(19) Europäisches Patentamt
European Patent Office
Office européen des brevets

(11) EP 4 393 915 A1

(12) # EUROPEAN PATENT APPLICATION
published in accordance with Art. 153(4) EPC

(43) Date of publication:
**03.07.2024 Bulletin 2024/27**

(21) Application number: **22860458.3**

(22) Date of filing: **22.08.2022**

(51) International Patent Classification (IPC):
***C07D 401/14*** (2006.01) ***C07D 419/14*** (2006.01)
***C07D 209/92*** (2006.01) ***A61K 31/4035*** (2006.01)
***A61P 35/00*** (2006.01)

(52) Cooperative Patent Classification (CPC):
**A61K 31/4035; A61P 35/00; C07D 209/92; C07D 401/14; C07D 419/14**

(86) International application number:
**PCT/CN2022/114058**

(87) International publication number:
**WO 2023/025112 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2021 CN 202110998156**
**06.04.2022 CN 202210358729**

(71) Applicant: **Hangzhou Glubio Pharmaceutical Co. Ltd.**
**Hangzhou, Zhejiang 310018 (CN)**

(72) Inventors:
- **FU, Liqiang**
  **Shanghai 201210 (CN)**
- **KONG, Linglong**
  **Shanghai 201210 (CN)**
- **ZHANG, Lei**
  **Shanghai 201210 (CN)**
- **LU, Gang**
  **San Diego, California 92121 (US)**
- **XIA, Yifeng**
  **San Diego, California 92121 (US)**
- **LU, Chin-Chun**
  **San Diego, California 92121 (US)**
- **SURKA, Christine**
  **San Diego, California 92121 (US)**

(74) Representative: **Cabinet Laurent & Charras**
**Le Contemporain**
**50 Chemin de la Bruyère**
**69574 Dardilly Cedex (FR)**

(54) **ISOINDOLINONE COMPOUND AND USE THEREOF**

(57) Provided are an isoindolinone compound represented by structural formula (I) or a pharmaceutically acceptable salt thereof, and a use thereof in the treatment of proliferative diseases. Further provided is a pharmaceutical composition comprising the compound or the salt thereof and a pharmaceutically acceptable carrier.

EP 4 393 915 A1

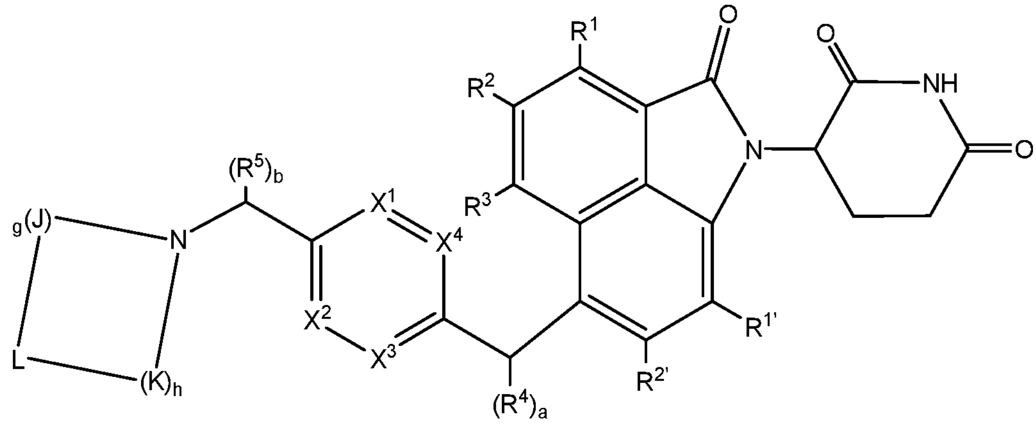

(I)

## Description

### FIELD OF THE INVENTION

The present application belongs to the field of medicine. The present application provides isoindolinone compounds represented by the structural formula(I) or a pharmaceutically acceptable salt thereof, and use thereof in the treatment of proliferative disorders. The present application also provides pharmaceutical compositions comprising a compound described herein or a salt thereof, and a pharmaceutically acceptable carrier.

### BACKGROUND

Cancer is a malignant disease that affects human health, and there is huge medical need for treating different types of cancers. Studies have shown that the occurrence of cancer is a complex event with the involvement of multiple factors and multiple steps.

Cereblon (CRBN) is a brain-related protein. CRBN has many functions. CRBN can interact with Cullins, regulators of ubiquitin ligase E3, to activate the activity of ubiquitin ligase E3 and promote the ligation between substrate protein and ubiquitin molecule to realize ubiquitination. The ubiquitinated protein will be recognized and degraded by proteasome. For example, CRBN can induce ubiquitination and degradation of transcription factors Ikaros (IKZF1) and Aiolos (IKZF3) containing zinc finger structures. Since these two transcription factors play a role in cancer cell proliferation, degrading both will produce toxic effects on cancer cells. CRBN is the direct target of Domides drugs, which can enhance the degradation of tumor-related factors by CRBN (Science, 2014, 343, 301-305). Domides drugs are also called CRBN molecular glue. However, there are many toxic and side effects of Domides drugs, which limit the application of Domides drugs as anti-cancer drugs. For example, thalidomide can cause fetal malformation.

SALL4 (Spaltlike transcription factor 4) is an embryonic Cys2-His2 (C2H2) zinc finger transcription factor involved in fetal limb development. CRBN molecular glue also degrades SALL4 factor, which may cause potential toxicity *in vivo* (Nature Chemical Biology, 2018, 14, 981-987). However, the ability of CRBN molecular glue to degrade other factors (such as SALL4) cannot be predicted in advance by its chemical structure. Therefore, careful analysis of its activity against SALL4 is required during the development of IKZF degrader.

In short, there is an urgent need to develop more effective and safer cancer treatment methods. IKZF degraders with low or no activity towards SALL4 appear attractive.

### BRIEF DESCRIPTION OF THE INVENTION

The present inventors surprisingly discovered that the compound or a pharmaceutically acceptable salt thereof of the present application can effectively degrade IKZF, but its ability to degrade SALL4 was significantly reduced, so that the compound or a pharmaceutically acceptable salt thereof of the present application has excellent anti-cancer activity and reduced toxic and side effects.

### BRIEF DESCRIPTION OF THE FIGURE(S)

Figure 1: the experimental result of IKZF 1 protein degradation kinetics.

### DETAILED DESCRIPTION OF THE INVENTION

The present application provides isoindolinone compounds represented by structural formula (I) or a pharmaceutically acceptable salt thereof.

The first embodiment of the present application is a compound having structural formula (I):

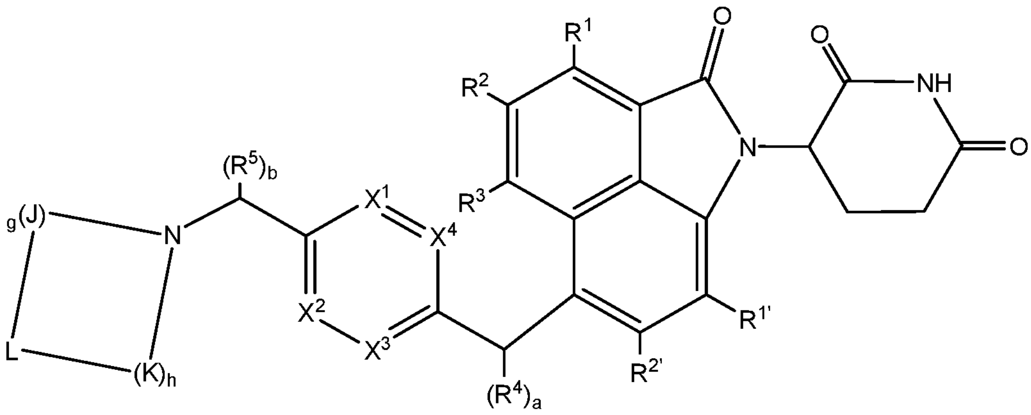

(I)

or a pharmaceutically acceptable salt thereof,
wherein:

$R^1$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{1'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^2$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{2'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^3$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or

$C_1$-$C_6$ alkyl;

$R^4$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^5$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

a is 0, 1 or 2;

b is 0, 1 or 2;

$X^1$, for each occurrence, is independently selected from $CR^{14}$ or N;

$X^2$, for each occurrence, is independently selected from $CR^{14'}$ or N;

$X^3$, for each occurrence, is independently selected from $CR^{14''}$ or N;

$X^4$, for each occurrence, is independently selected from $CR^{14'''}$ or N;

$R^{14}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14''}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14'''}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (monoand di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$

cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

L, for each occurrence, is independently selected from O, $NR^{21}$, $CR^{22}$, $CR^{23}R^{24}$, $NR^{25}$-$CR^{26}$, $CR^{27}$-$CR^{28}$,

$R^{21}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{22}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

when L is $CR^{23}R^{24}$, $R^{23}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{24}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or,

$R^{23}$ and $R^{24}$ are joined together with the connected carbon atom to form a monocyclic, bicyclic or tricyclic saturated or unsaturated ring system with 5-14 ring atoms, wherein 0, 1, 2, 3 or 4 of the ring atoms are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

when L is $NR^{25}$-$CR^{26}$, $R^{25}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{26}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or,

$R^{25}$ and $R^{26}$ are joined together with the connected nitrogen atom and carbon atom to form a monocyclic, bicyclic or tricyclic saturated or unsaturated ring system with 5-14 ring atoms, wherein 0, 1, 2, 3 or 4 of the remaining ring atoms except the nitrogen atom are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

when L is $CR^{27}$-$CR^{28}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{28}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or

$R^{27}$ and $R^{28}$ are joined together with the connected carbon atoms to form a monocyclic, bicyclic or tricyclic saturated or unsaturated ring system having 5-14 ring atoms, wherein 0, 1, 2, 3 or 4 of the ring atoms are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

J is $NR^{31}$, $CR^{32}R^{33}$;

g is 0, 1 or 2;

K is $NR^{34}$, $CR^{35}R^{36}$;

h is 0, 1 or 2;

$R^{31}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{32}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{33}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{34}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{35}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{36}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

In the second aspect of the first embodiment of the present invention, the compound of structural formula (I) is the compound of structural formula (II):

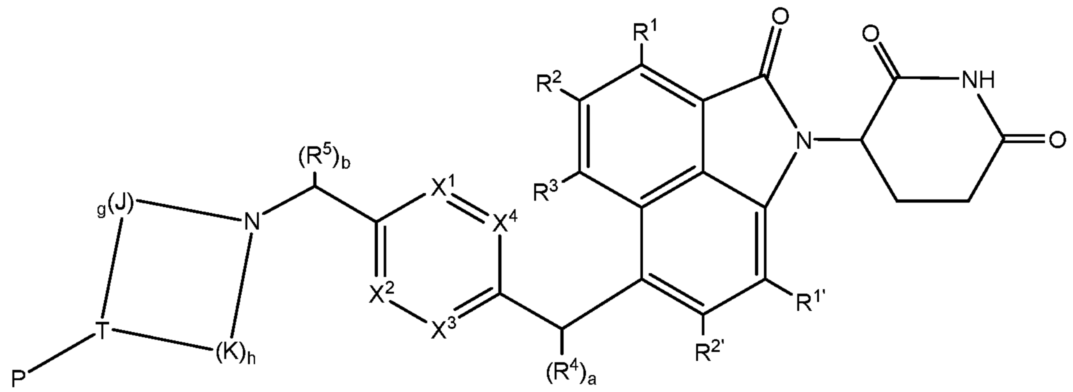

(II),

or a pharmaceutically acceptable salt thereof, wherein:

T is N or CH;

P, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (monoand di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

The remaining variables are as described and defined in the first embodiment and any aspect thereof.

In the third aspect of the first embodiment of the present invention, the compound of structural formula (I) is the compound of structural formula (III):

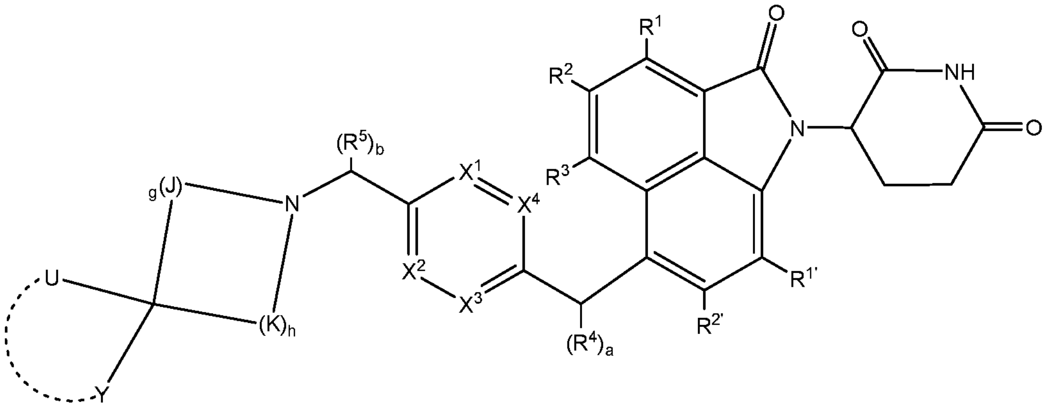

(III),

or a pharmaceutically acceptable salt thereof,
wherein the group

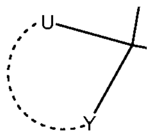

represents a monocyclic, bicyclic or tricyclic saturated or unsaturated ring system with 5-14 ring atoms, U is N, C or O, Y is N, C or O, the ring system further comprises additional 0, 1, 2, 3 or 4 heteroatoms selected from N, O, S, and the ring system is optionally substituted with 1, 2, 3 or 4 halogens, hydroxyl, nitro, cyano, amino, mercapto, -COOH, or $C_1$-$C_6$ alkyl;

The remaining variables are as described and defined in the first embodiment and any aspect thereof.

In the fourth aspect of the first embodiment of the present invention, the compound of structural formula (I) is the compound of structural formula (IV):

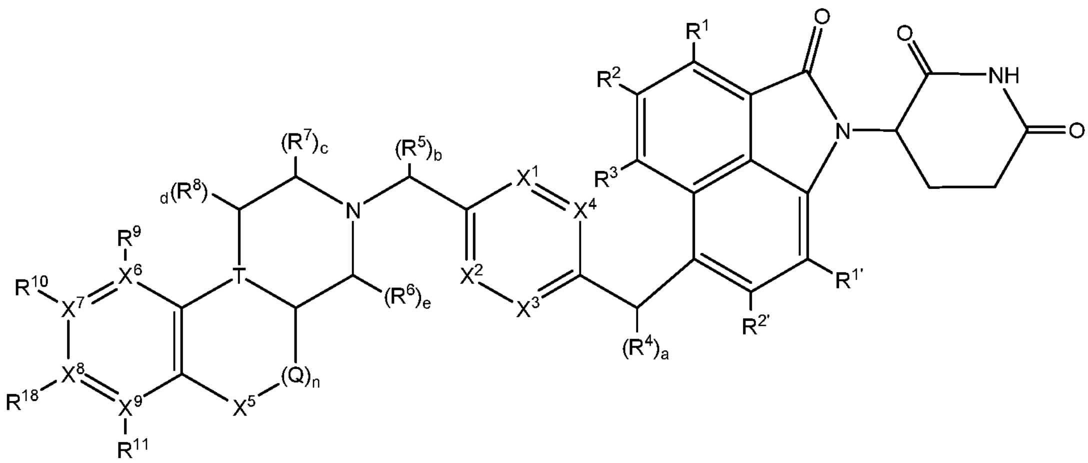

(IV),

or a pharmaceutically acceptable salt thereof,
wherein

$R^6$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or

$C_1$-$C_6$ alkyl;

$R^7$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^8$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^9$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{10}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{11}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

c is 0, 1 or 2;

d is 0, 1 or 2;

e is 0, 1 or 2;

Q is $CR^{12}R^{13}$, n is 0, 1 or 2;

$X^5$ is selected from O, $CR^{15}R^{16}$, or $NR^{17}$.

$R^{12}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or

$C_1$-$C_6$ alkyl;

$R^{13}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{15}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{16}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{17}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{18}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

T is N or CH;

$X^6$ isN orC;

$X^7$ is N or C;

$X^8$ is N or C;

$X^9$ is N or C;

The remaining variables are as described and defined in the first embodiment and any aspect thereof.

In the fifth aspect of the first embodiment of the present invention, the compound of structural formula (I) is the compound of structural formula (V):

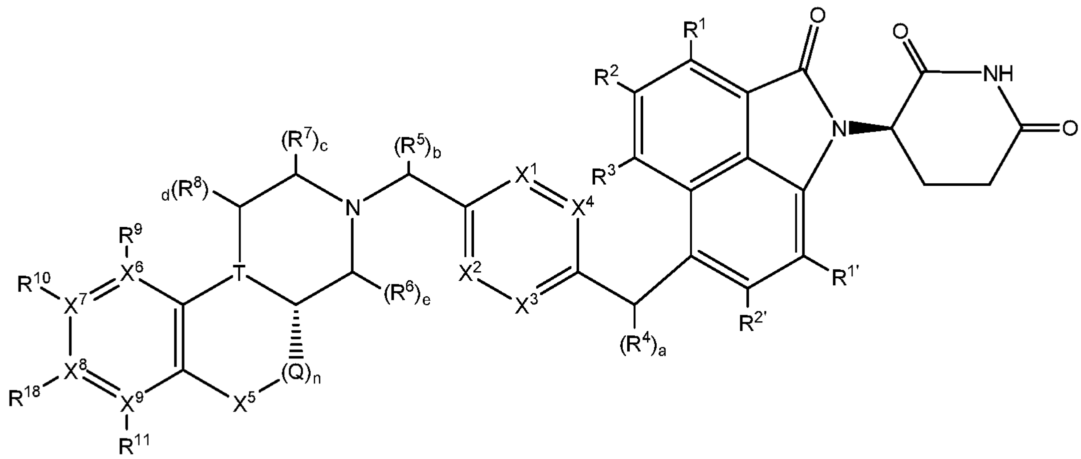

(V)

or a pharmaceutically acceptable salt thereof.

The remaining variables are as described and defined in the first embodiment and any aspect thereof.

In the sixth aspect of the first embodiment of the present invention, the compound of structural formula (I) is the compound of structural formula (VI):

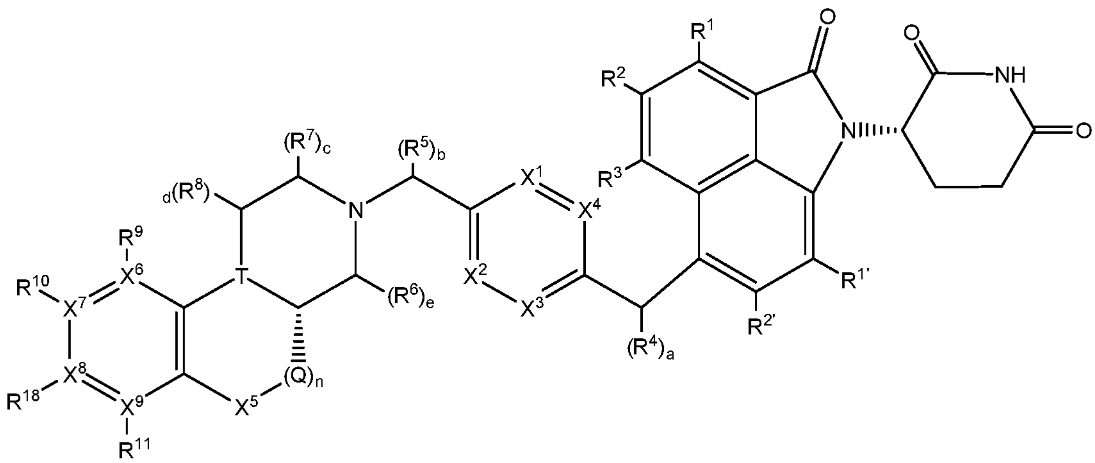

(VI)

or a pharmaceutically acceptable salt thereof.

The remaining variables are as described and defined in the first embodiment and any aspect thereof.

In the seventh aspect of the first embodiment of the present invention, the compound of structural formula (I) is the compound of structural formula (VII):

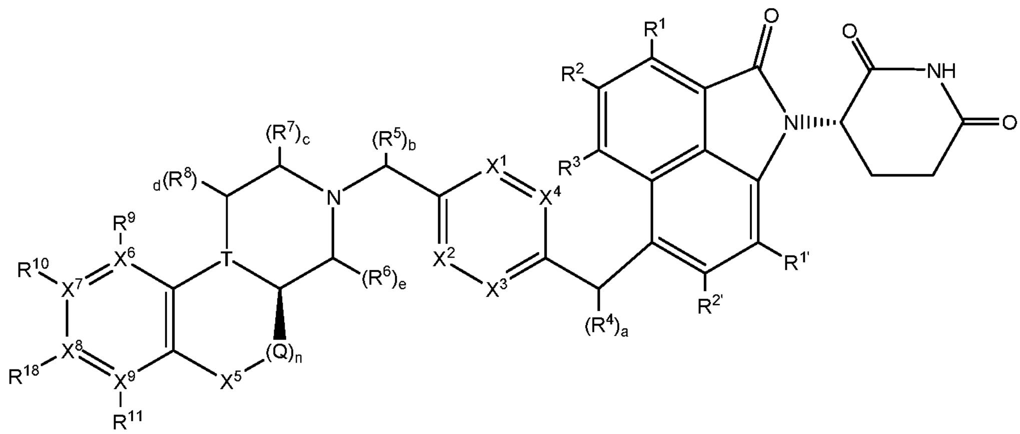

(VII)

or a pharmaceutically acceptable salt thereof.

The remaining variables are as described and defined in the first embodiment and any aspect thereof.

In the eighth aspect of the first embodiment of the present invention, the compound of structural formula (I) is the compound of structural formula (VIII):

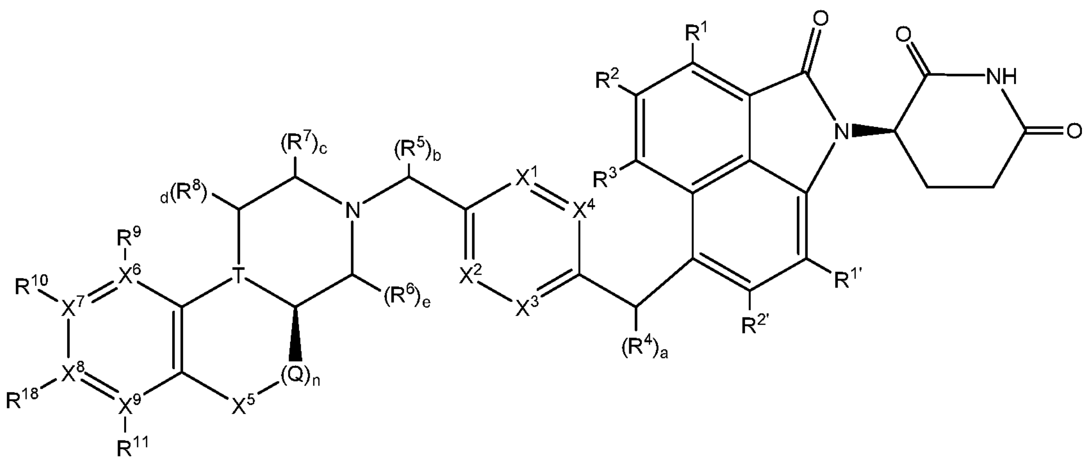

(VIII)

or a pharmaceutically acceptable salt thereof.

The remaining variables are as described and defined in the first embodiment and any aspect thereof.

In the ninth aspect of the first embodiment of the present invention, the compound of structural formula (I) is the compound of structural formula (IX):

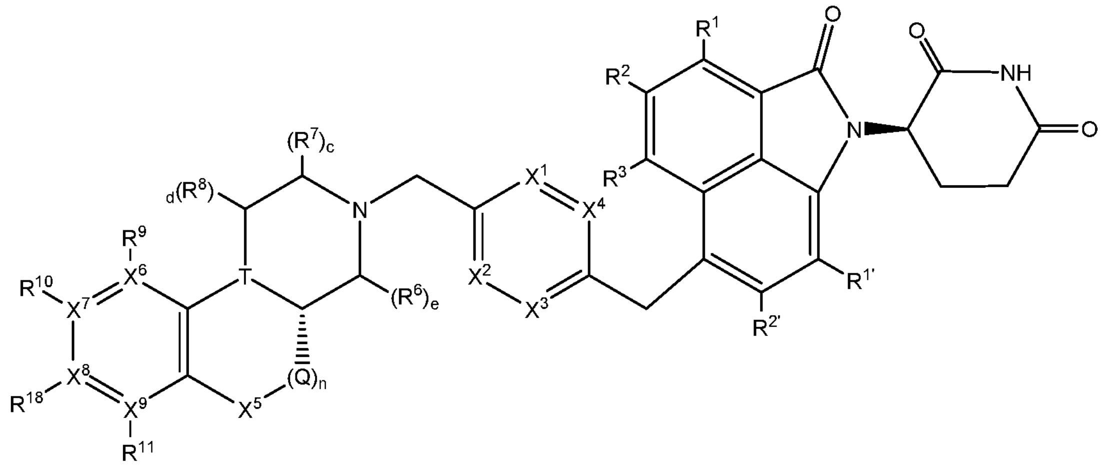

(IX)

or a pharmaceutically acceptable salt thereof.

The remaining variables are as described and defined in the first embodiment and any aspect thereof.

In the tenth aspect of the first embodiment of the present invention, the compound of structural formula (I) is the compound of structural formula (X):

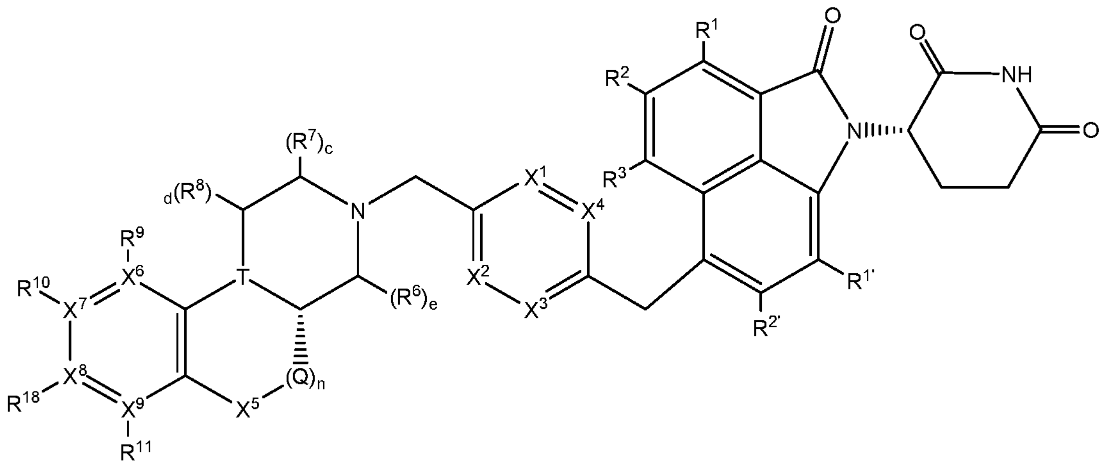

(X)

or a pharmaceutically acceptable salt thereof.

The remaining variables are as described and defined in the first embodiment and any aspect thereof.

In the eleventh aspect of the first embodiment of the present invention, the compound of structural formula (I) is the compound of structural formula (XI):

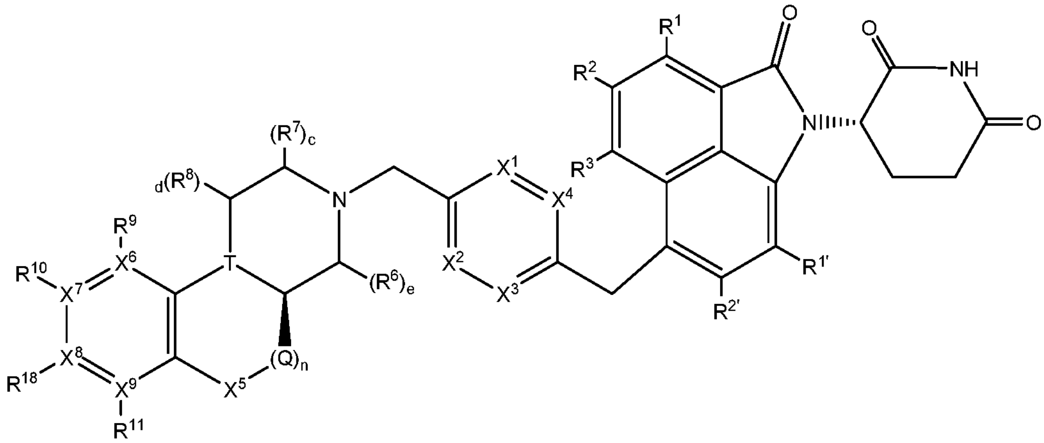

(XI)

or a pharmaceutically acceptable salt thereof.

The remaining variables are as described and defined in the first embodiment and any aspect thereof.

In the twelfth aspect of the first embodiment of the present invention, the compound of structural formula (I) is the compound of structural formula (XII):

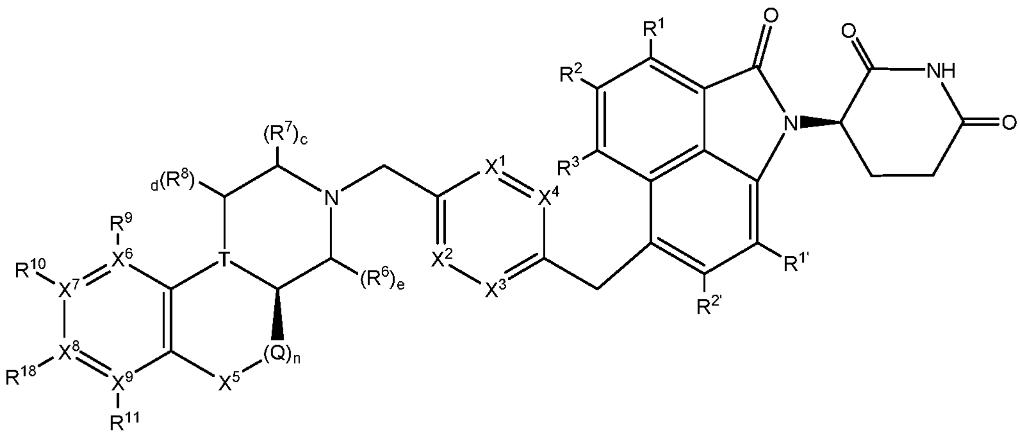

(XII)

or a pharmaceutically acceptable salt thereof.

The remaining variables are as described and defined in the first embodiment and any aspect thereof.

In the thirteenth aspect of the first embodiment of the present invention, the compound of structural formula (I) is the compound of structural formula (XIII):

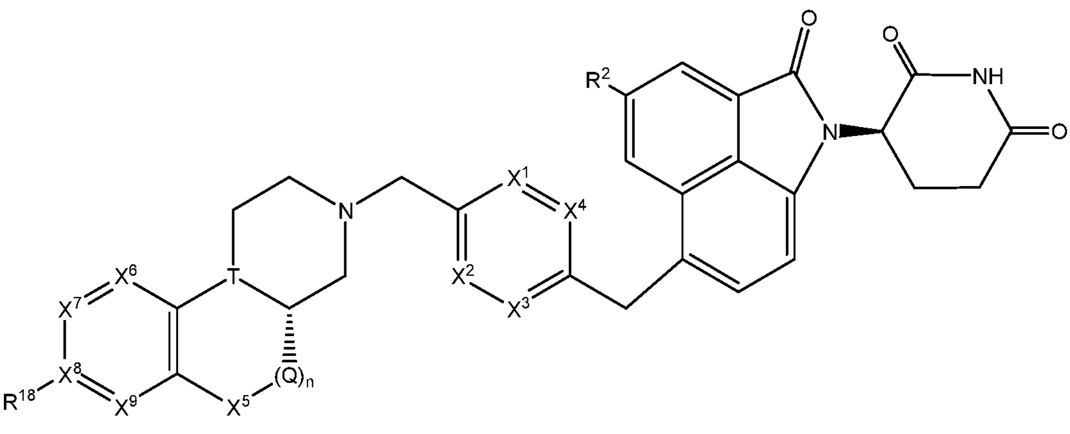

(XIII)

or a pharmaceutically acceptable salt thereof.

The remaining variables are as described and defined in the first embodiment and any aspect thereof.

In the fourteenth aspect of the first embodiment of the present invention, the compound of structural formula (I) is the compound of structural formula (XIV):

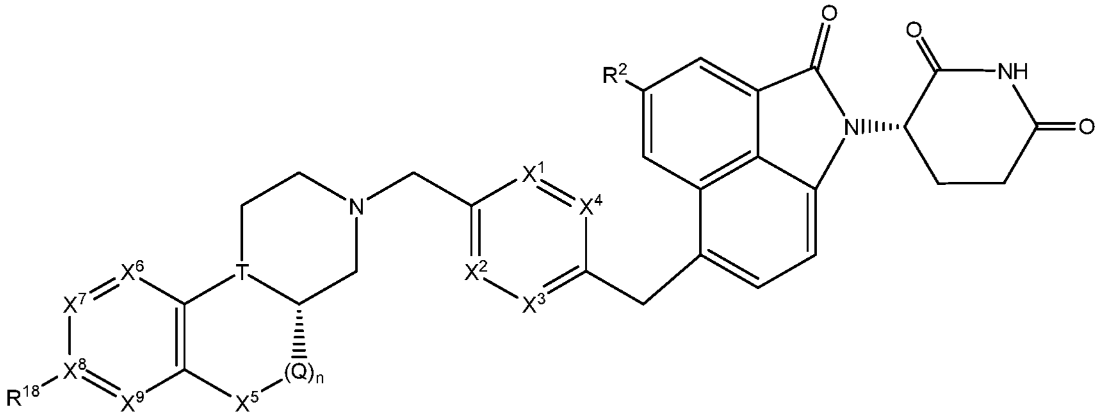

(XIV)

or a pharmaceutically acceptable salt thereof.

The remaining variables are as described and defined in the first embodiment and any aspect thereof.

In the fifteenth aspect of the first embodiment of the present invention, the compound of structural formula (I) is the compound of structural formula (XV):

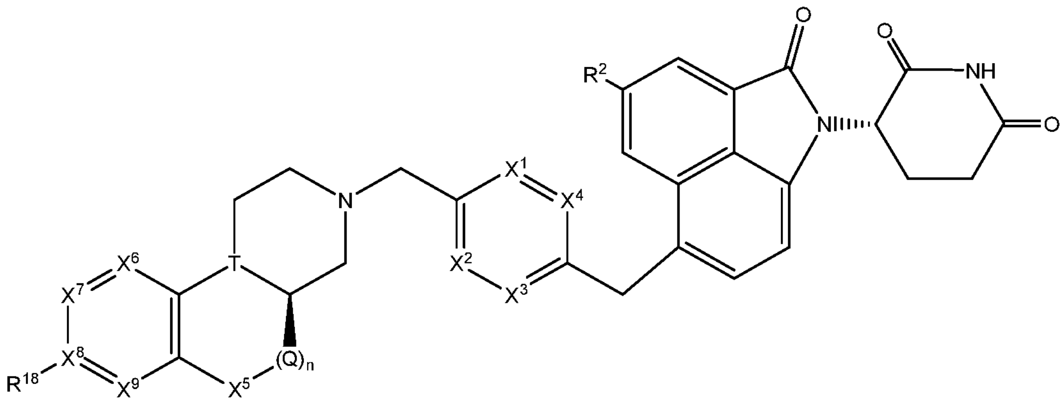

(XV)

or a pharmaceutically acceptable salt thereof.

The remaining variables are as described and defined in the first embodiment and any aspect thereof.

In the sixteenth aspect of the first embodiment of the present invention, the compound of structural formula (I) is the compound of structural formula (XVI):

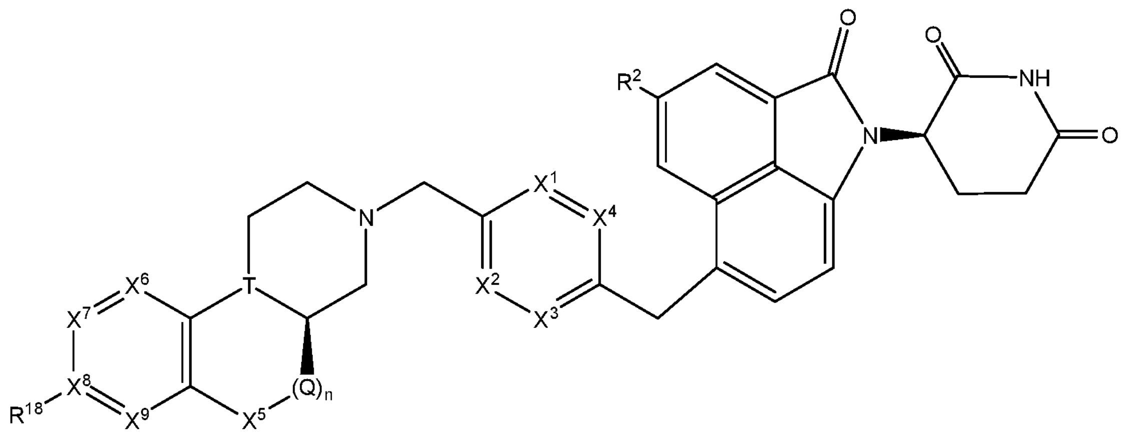

(XVI)

or a pharmaceutically acceptable salt thereof.

The remaining variables are as described and defined in the first embodiment and any aspect thereof.

In the seventeenth aspect of the first embodiment of the present invention, the compound of structural formula (I) is the compound of structural formula (XVII):

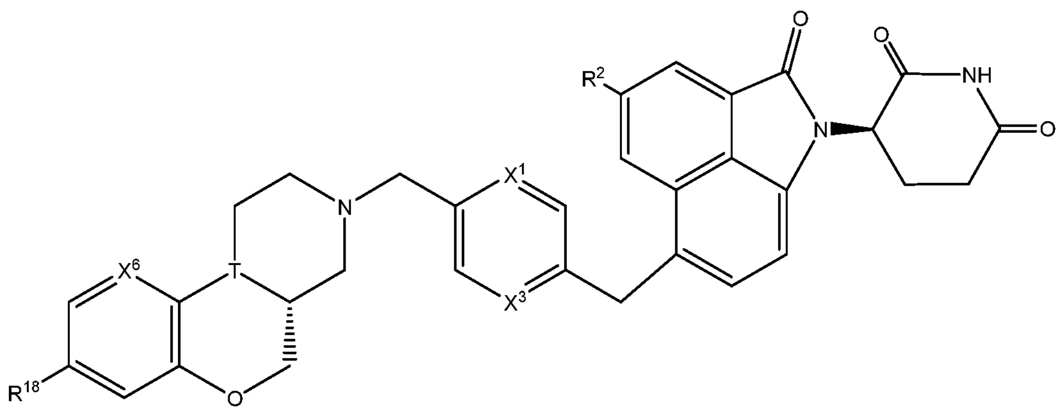

(XVII)

or a pharmaceutically acceptable salt thereof.

The remaining variables are as described and defined in the first embodiment and any aspect thereof.

In the eighteenth aspect of the first embodiment of the present invention, the compound of structural formula (I) is the compound of structural formula (XVIII):

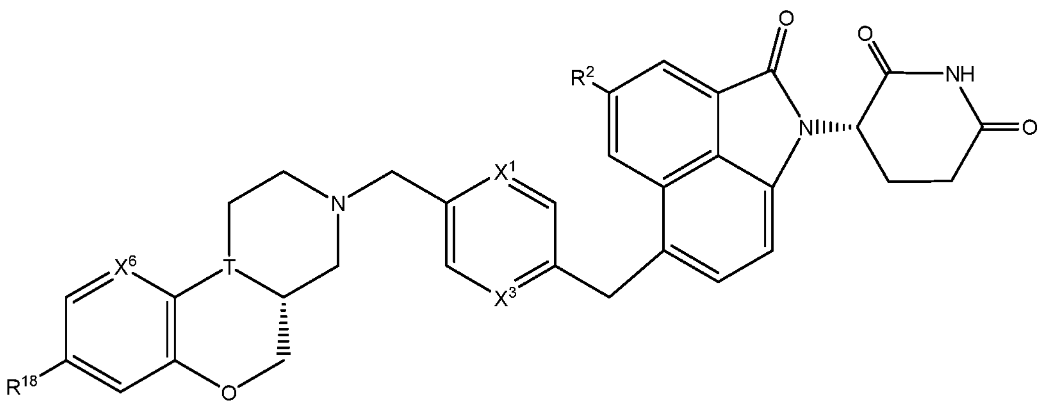

(XVIII)

or a pharmaceutically acceptable salt thereof.

The remaining variables are as described and defined in the first embodiment and any aspect thereof.

In the nineteenth aspect of the first embodiment of the present invention, the compound of structural formula (I) is the compound of structural formula (XIX):

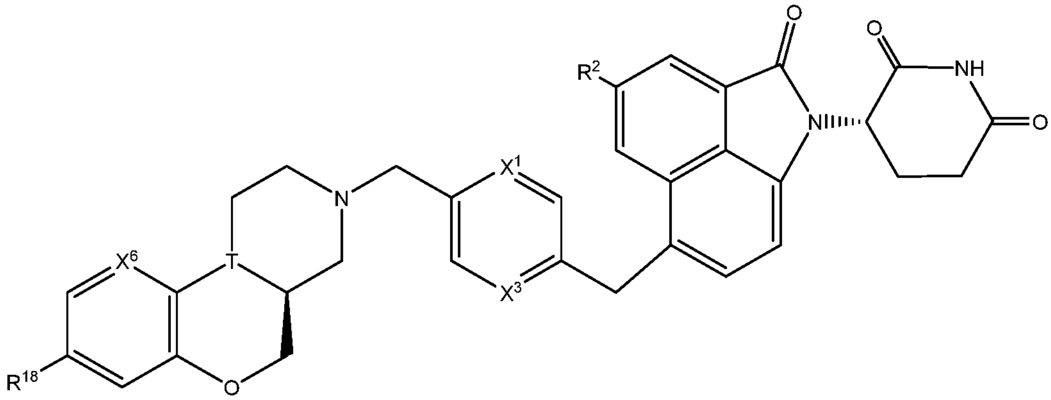

(XIX)

or a pharmaceutically acceptable salt thereof.

The remaining variables are as described and defined in the first embodiment and any aspect thereof.

In the 20th aspect of the first embodiment of the present invention, the compound of structural formula (I) is the compound of structural formula (XX):

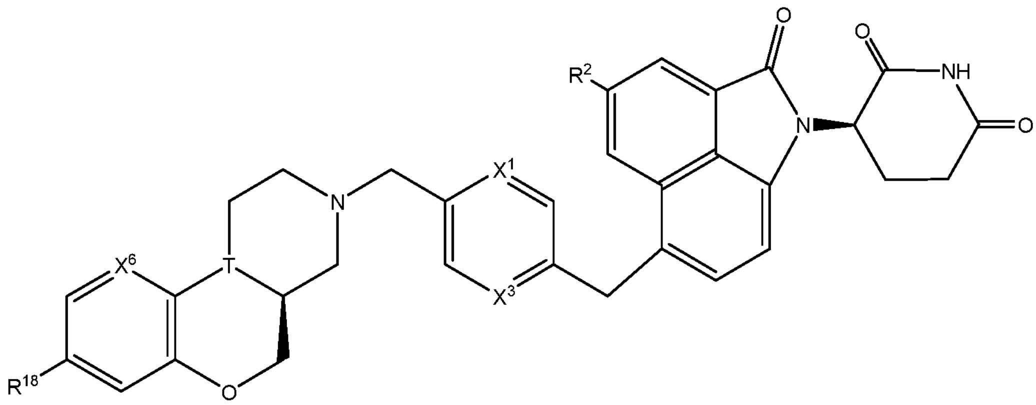

(XX)

or a pharmaceutically acceptable salt thereof.

The remaining variables are as described and defined in the first embodiment and any aspect thereof.

In the 21st aspect of the first embodiment of the present invention, the compound of structural formula (I) is the compound of structural formula (XXI):

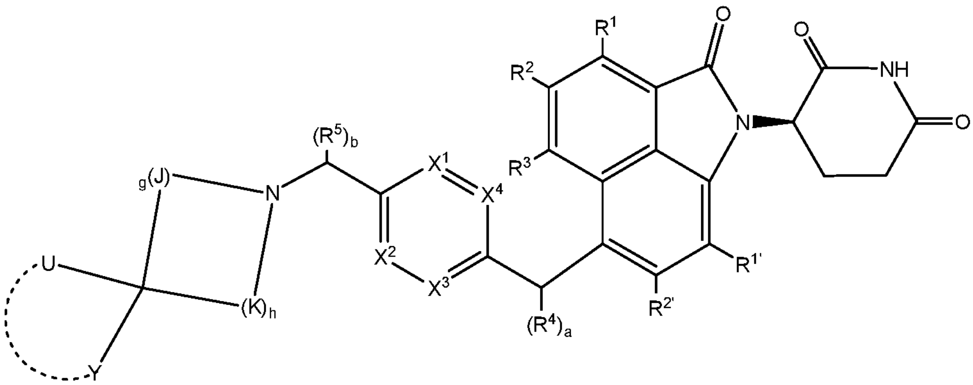

(XXI)

or a pharmaceutically acceptable salt thereof.

The remaining variables are as described and defined in the first embodiment and any aspect thereof.

In the 22nd aspect of the first embodiment of the present invention, the compound of structural formula (I) is the compound of structural formula (XXII):

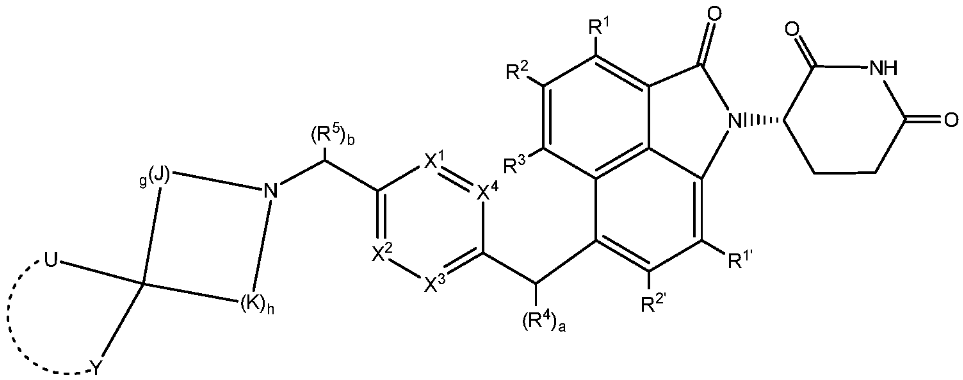

(XXII)

or a pharmaceutically acceptable salt thereof.

The remaining variables are as described and defined in the first embodiment and any aspect thereof.

In the 23rd aspect of the first embodiment of the present invention, the compound of structural formula (I) is the compound of structural formula (XXIII):

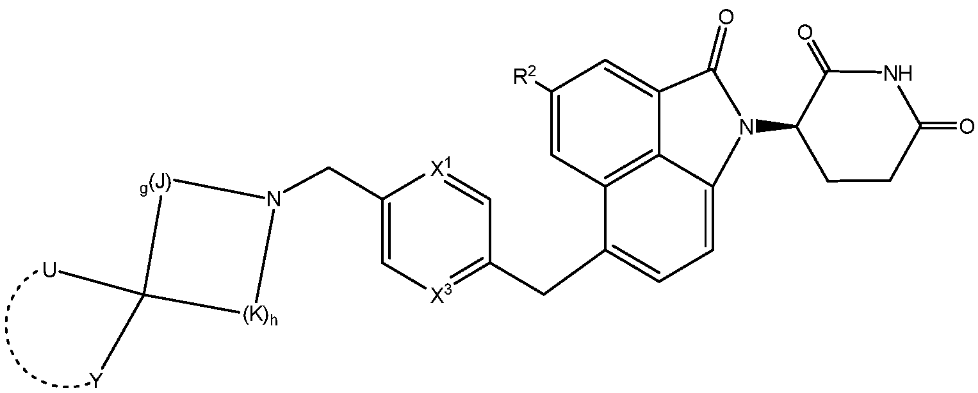

(XXIII)

or a pharmaceutically acceptable salt thereof.

The remaining variables are as described and defined in the first embodiment and any aspect thereof.

In the 24th aspect of the first embodiment of the present invention, the compound of structural formula (I) is

the compound of structural formula (XXIV):

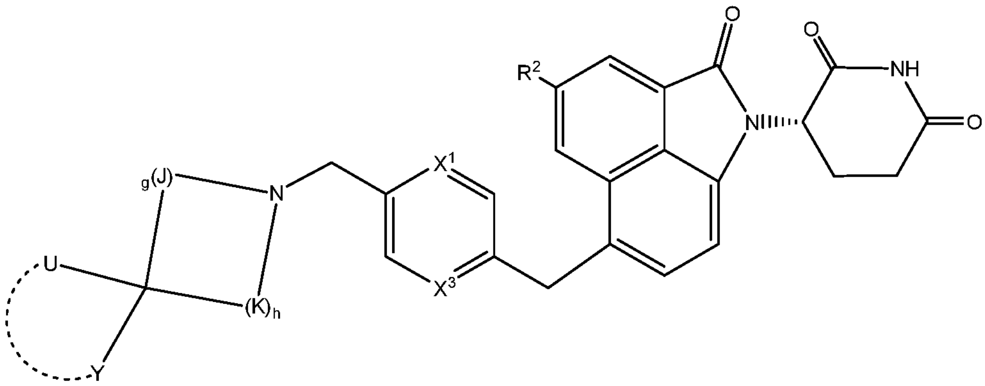

(XXIV)

or a pharmaceutically acceptable salt thereof.

The remaining variables are as described and defined in the first embodiment and any aspect thereof.

In the 25th aspect of the first embodiment of the present invention, the compound of structural formula (I) is the compound of structural formula (XXV):

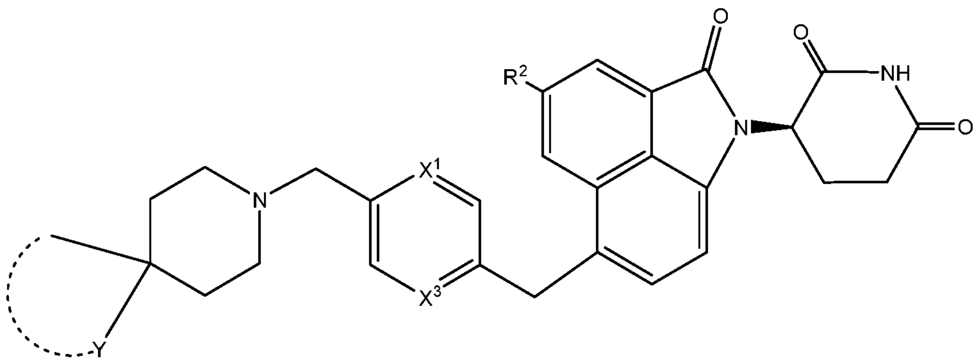

(XXV)

or a pharmaceutically acceptable salt thereof.

The remaining variables are as described and defined in the first embodiment and any aspect thereof.

In the 26th aspect of the first embodiment of the present invention, the compound of structural formula (I) is the compound of structural formula (XXVI):

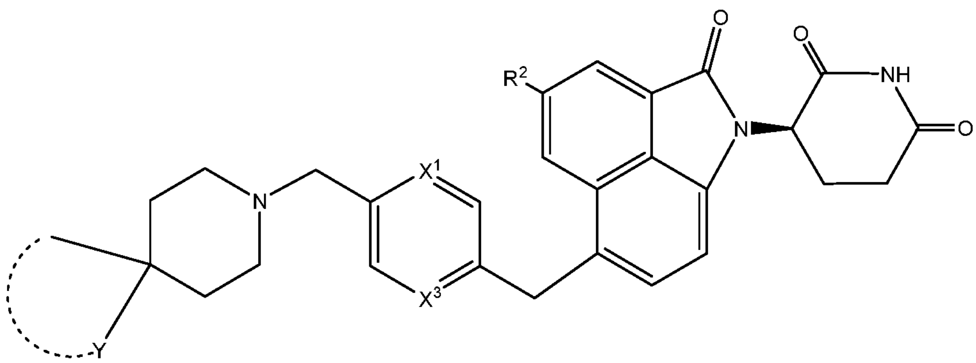

(XXV)

or a pharmaceutically acceptable salt thereof.

The remaining variables are as described and defined in the first embodiment and any aspect thereof.

In the 27th aspect of the first embodiment of the present invention, the compound of structural formula (I) is

the compound of structural formula (XXVII):

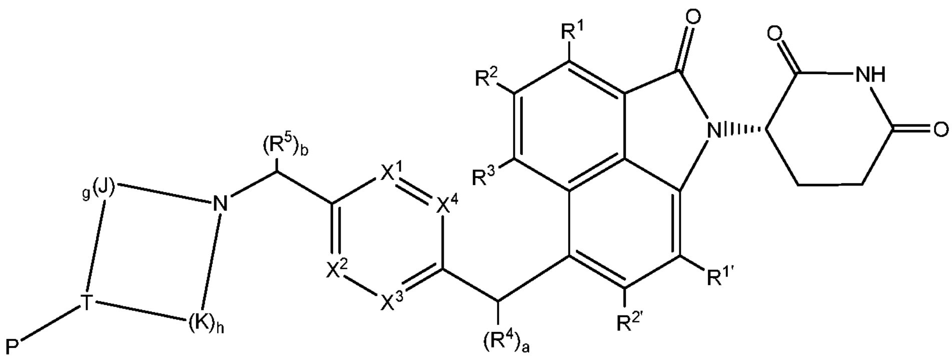

(XXVII)

or a pharmaceutically acceptable salt thereof.

The remaining variables are as described and defined in the first embodiment and any aspect thereof.

In the 28th aspect of the first embodiment of the present invention, the compound of structural formula (I) is the compound of structural formula (XXVIII):

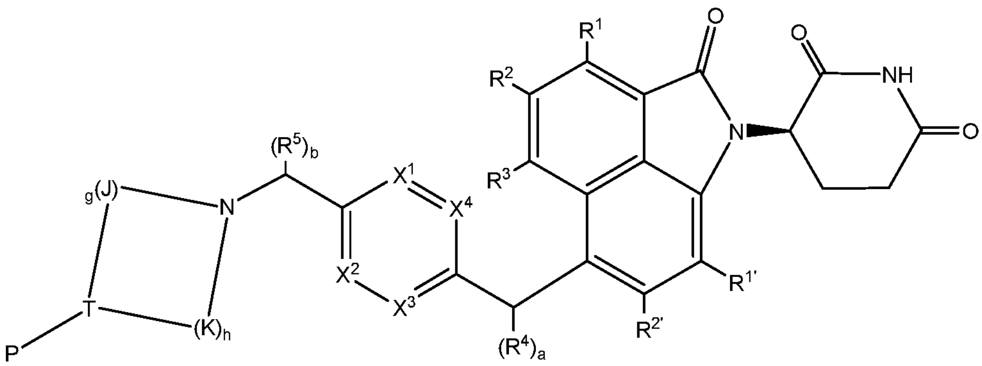

(XXVIII)

or a pharmaceutically acceptable salt thereof.

The remaining variables are as described and defined in the first embodiment and any aspect thereof.

In the 29th aspect of the first embodiment of the present invention, the compound of structural formula (I) is the compound of structural formula (XXIX):

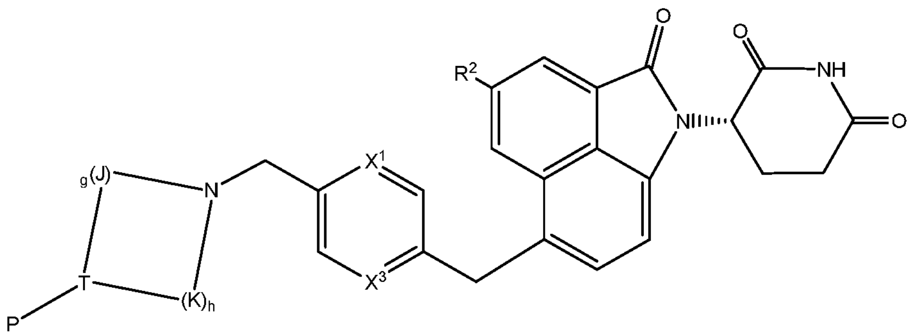

(XXIX)

or a pharmaceutically acceptable salt thereof.

The remaining variables are as described and defined in the first embodiment and any aspect thereof.

In the 30th aspect of the first embodiment of the present invention, the compound of structural formula (I) is the compound of structural formula (XXX):

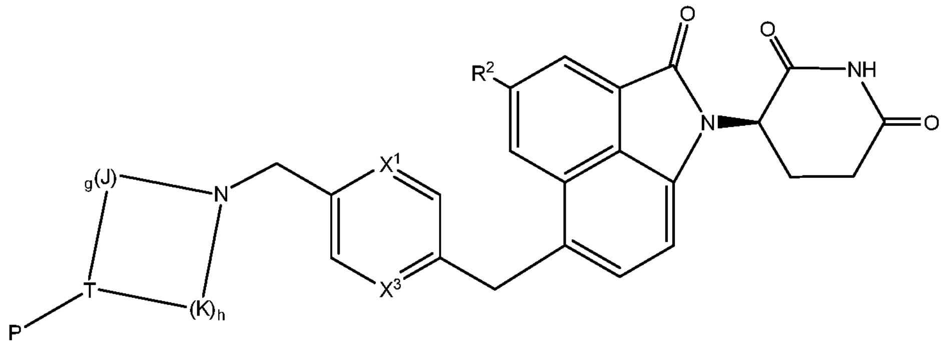

(XXX)

or a pharmaceutically acceptable salt thereof.

The remaining variables are as described and defined in the first embodiment and any aspect thereof.

In the 31st aspect of the first embodiment of the present invention, the compound of structural formula (I) is the compound of structural formula (XXXI):

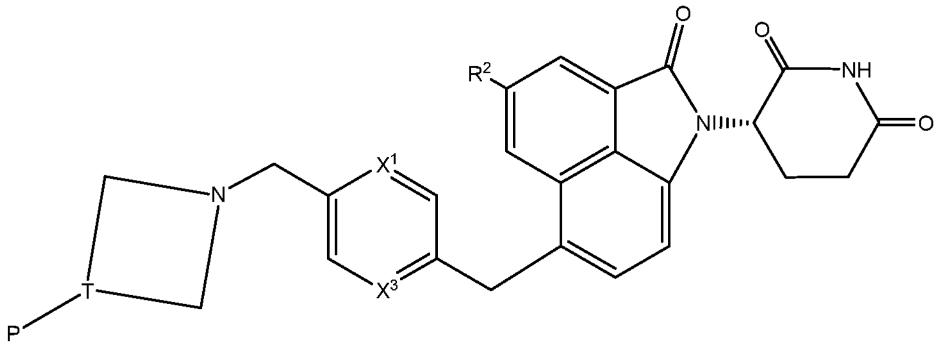

(XXXI)

or a pharmaceutically acceptable salt thereof.

The remaining variables are as described and defined in the first embodiment and any aspect thereof.

In the 32nd aspect of the first embodiment of the present invention, the compound of structural formula (I) is the compound of structural formula (XXXII):

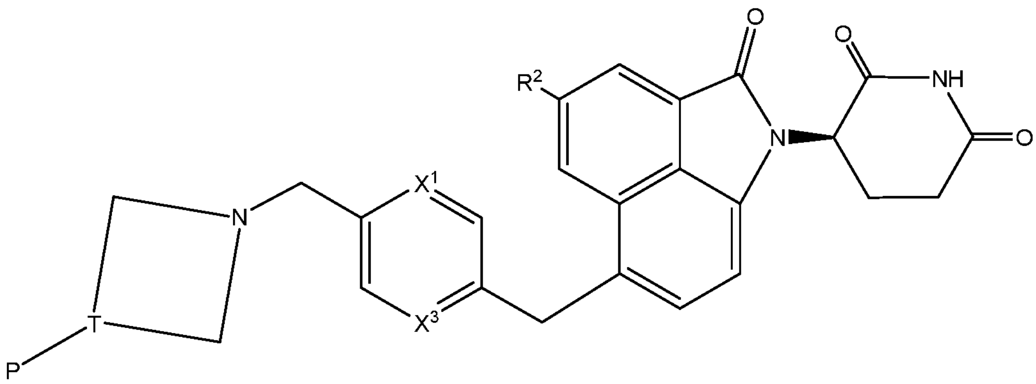

(XXXII)

or a pharmaceutically acceptable salt thereof.

The remaining variables are as described and defined in the first embodiment and any aspect thereof.

In the 33rd aspect of the first embodiment of the present invention, the compound of structural formula (I) is the compound of structural formula (XXXIII):

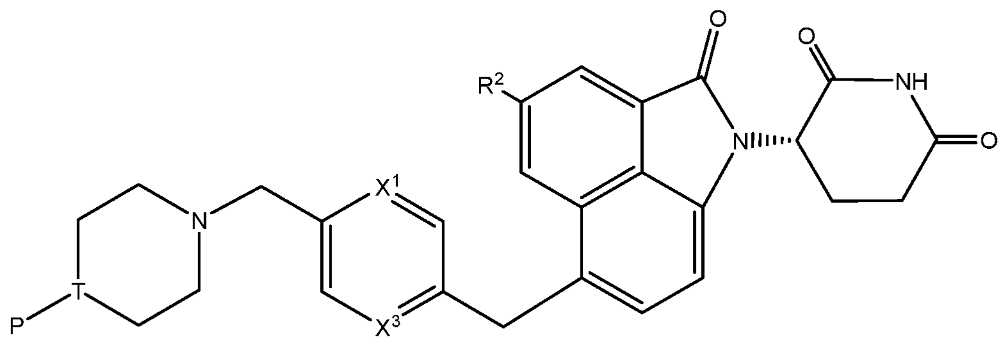

(XXXIII)

or a pharmaceutically acceptable salt thereof.

The remaining variables are as described and defined in the first embodiment and any aspect thereof.

In the 34th aspect of the first embodiment of the present invention, the compound of structural formula (I) is the compound of structural formula (XXXIV):

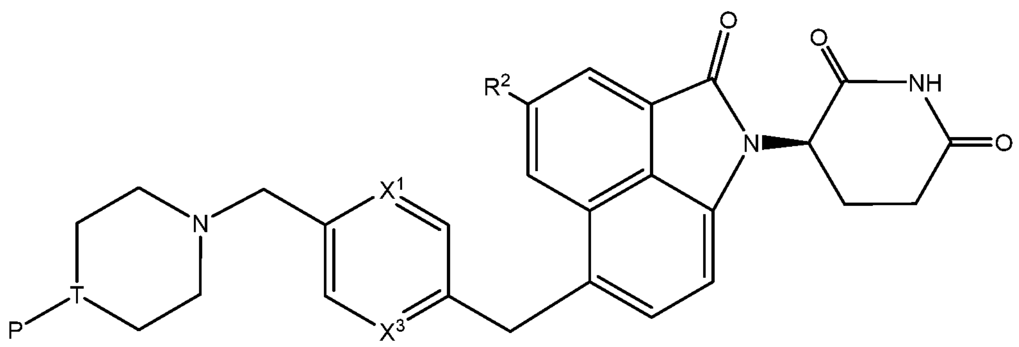

(XXXIV)

or a pharmaceutically acceptable salt thereof.

The remaining variables are as described and defined in the first embodiment and any aspect thereof.

In any implementation of the present application and any aspect thereof, each of $R^1$, $R^{1'}$, $R^2$, $R^{2'}$, $R^3$, $R^4$, $R^5$, $R^6$, $R^7$, $R^8$, $R^9$, $R^{10}$, $R^{11}$, $R^{12}$, $R^{13}$, $R^{14}$, $R^{14'}$ $R^{14''}$, $R^{14'''}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$ and P is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, any one of them, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

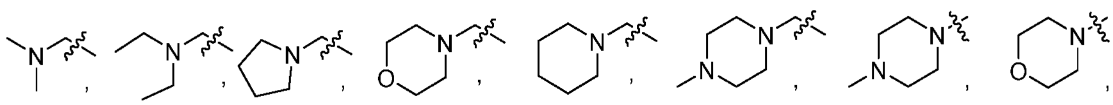
, , , , , , , ,

trifluoromethyl, monofluoromethyl, difluoromethyl, 2-fluoroethyl, pentafluoroethyl, fluorophenylacetonitrile; more preferably, any one of them, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

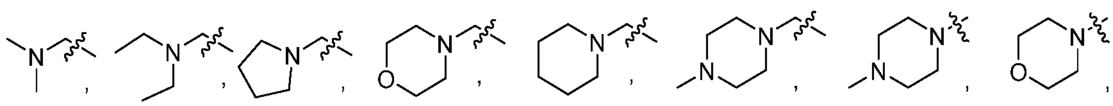
, , , , , , , ,

trifluoromethyl, monofluoromethyl, difluoromethyl, 2-fluoroethyl, pentafluoroethyl, fluorophenylacetonitrile; most preferably, any one of them, for each occurrence, is independently selected from hydrogen, fluorine, cyano, methyl, isopropyl, morpholinyl,

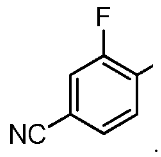.

In any implementation of the present application and any aspect thereof, $R^1$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^1$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

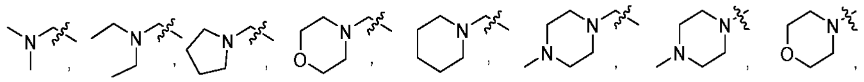

trifluoromethyl, monofluoromethyl, difluoromethyl, 2-fluoroethyl, pentafluoroethyl. More preferably, $R^1$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

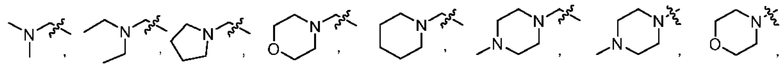

trifluoromethyl, monofluoromethyl, difluoromethyl, 2-fluoroethyl, pentafluoroethyl. Most preferably, $R^1$, for each occurrence, is independently selected from hydrogen, fluorine, cyano, methyl, isopropyl.

In any implementation of the present application and any aspect thereof, $R^{1'}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{1'}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

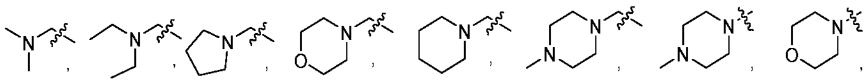

trifluoromethyl, monofluoromethyl, difluoromethyl, 2-fluoroethyl, pentafluoroethyl. More preferably, $R^{1'}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

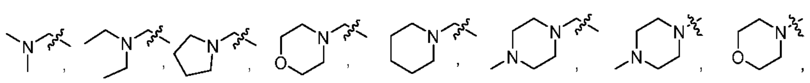

trifluoromethyl, monofluoromethyl, difluoromethyl, 2-fluoroethyl, pentafluoroethyl. Most preferably, $R^1$, for each occurrence, is independently selected from hydrogen, fluorine, cyano, methyl, isopropyl.

In any implementation of the present application and any aspect thereof, $R^2$, for each occurrence, is independently selected from absence, hydrogen, fluorine, chlorine, hydroxyl, nitro, cyano, amino, mercapto, -COOH, $C_1$-$C_6$ alkyl,

$C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^2$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

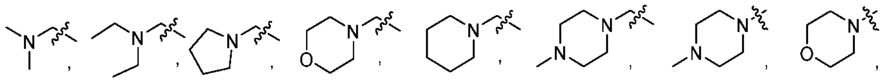
, , , , , , , ,

trifluoromethyl, monofluoromethyl, difluoromethyl, 2-fluoroethyl, pentafluoroethyl. More preferably, $R^2$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

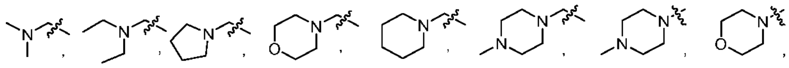
, , , , , , , ,

trifluoromethyl, monofluoromethyl, difluoromethyl, 2-fluoroethyl, pentafluoroethyl. Most preferably, $R^2$, for each occurrence, is independently selected from hydrogen, fluorine, chlorine, bromine, cyano, trifluoromethyl, methoxy, ethoxy, methyl, or isopropyl.

In any implementation of the present application and any aspect thereof, $R^2$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{2'}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

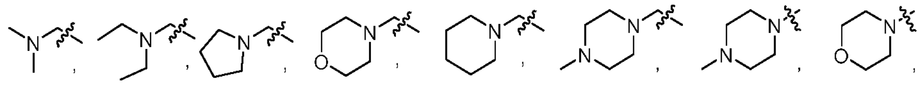
, , , , , , , ,

trifluoromethyl, monofluoromethyl, difluoromethyl, 2-fluoroethyl, pentafluoroethyl. More preferably, $R^{2'}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

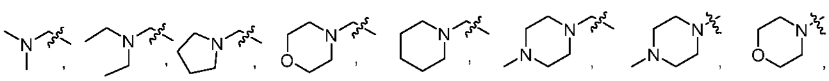
, , , , , , , ,

trifluoromethyl, monofluoromethyl, difluoromethyl, 2-fluoroethyl, pentafluoroethyl. Most preferably, $R^{2'}$, for each occurrence, is independently selected from hydrogen, fluorine, cyano, methyl, isopropyl.

In any implementation of the present application and any aspect thereof, $R^3$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^3$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

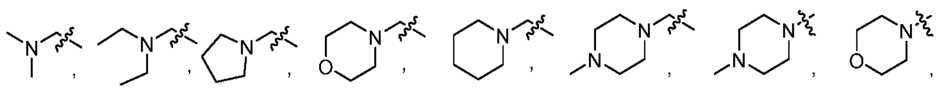
, , , , , , , ,

trifluoromethyl, monofluoromethyl, difluoromethyl, 2-fluoroethyl, pentafluoroethyl. More preferably, $R^3$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

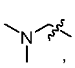, 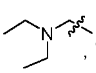, 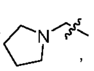, 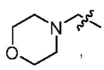, 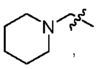, 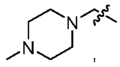, 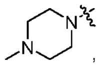, 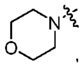,

trifluoromethyl, monofluoromethyl, difluoromethyl, 2-fluoroethyl, pentafluoroethyl. Most preferably, $R^3$, for each occurrence, is independently selected from hydrogen, fluorine, cyano, methyl, isopropyl.

In any implementation of the present application and any aspect thereof, $R^4$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^4$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

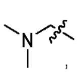, 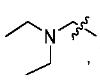, 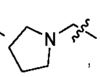, 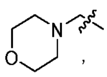, 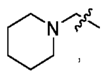, 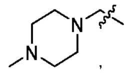, 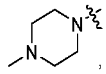, 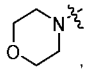,

trifluoromethyl, monofluoromethyl, difluoromethyl, 2-fluoroethyl, pentafluoroethyl. More preferably, $R^4$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

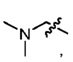, 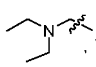, 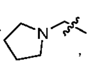, 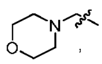, 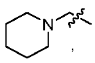, 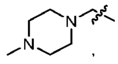, 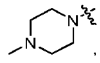, 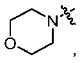,

trifluoromethyl, monofluoromethyl, difluoromethyl, 2-fluoroethyl, pentafluoroethyl. Most preferably, $R^4$, for each occurrence, is independently selected from hydrogen, fluorine, cyano, methyl, isopropyl.

In any implementation of the present application and any aspect thereof, $R^5$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^5$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

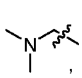, 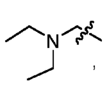, 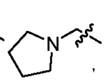, 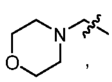, 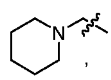, 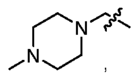, 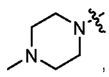, 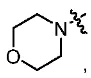,

trifluoromethyl, monofluoromethyl, difluoromethyl, 2-fluoroethyl, pentafluoroethyl. More preferably, $R^5$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

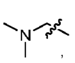, 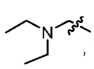, 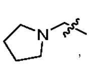, 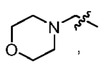, 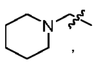, 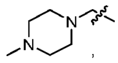, 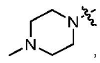, 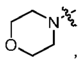,

trifluoromethyl, monofluoromethyl, difluoromethyl, 2-fluoroethyl, pentafluoroethyl. Most preferably, $R^5$, for each occurrence, is independently selected from hydrogen, fluorine, cyano, methyl, isopropyl.

In any implementation of the present application and any aspect thereof, $R^6$, for each occurrence, is independently selected from absence, hydrogen, fluorine, chlorine, hydroxyl, nitro, cyano, amino, mercapto, -COOH, $C_1$-$C_6$ alkyl,

$C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^6$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

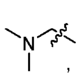, 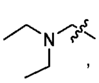, 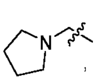, 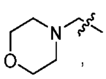, 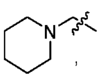, 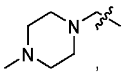, 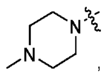, 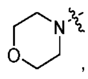,

trifluoromethyl, monofluoromethyl, difluoromethyl, 2-fluoroethyl, pentafluoroethyl. More preferably, $R^6$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

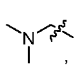, 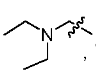, 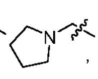, 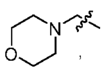, 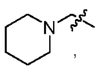, 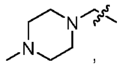, 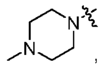, 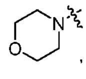,

trifluoromethyl, monofluoromethyl, difluoromethyl, 2-fluoroethyl, pentafluoroethyl. Most preferably, $R^6$, for each occurrence, is independently selected from hydrogen, fluorine, cyano, methyl, isopropyl.

In any implementation of the present application and any aspect thereof, $R^7$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^7$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

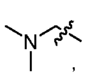, 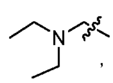, 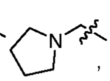, 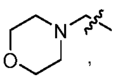, 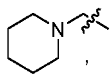, 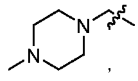, 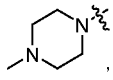, 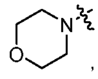,

trifluoromethyl, monofluoromethyl, difluoromethyl, 2-fluoroethyl, pentafluoroethyl. More preferably, $R^7$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

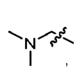, 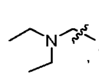, 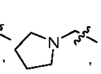, 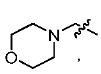, 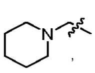, 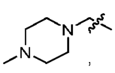, 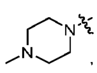, 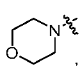,

trifluoromethyl, monofluoromethyl, difluoromethyl, 2-fluoroethyl, pentafluoroethyl. Most preferably, $R^7$, for each occurrence, is independently selected from hydrogen, fluorine, cyano, methyl, isopropyl.

In any implementation of the present application and any aspect thereof, $R^8$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^8$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

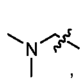, 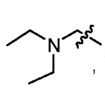, 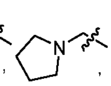, 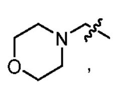, 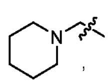, 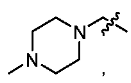, 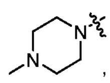, 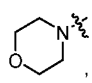,

trifluoromethyl, monofluoromethyl, difluoromethyl, 2-fluoroethyl, pentafluoroethyl. More preferably, $R^8$, for each occurrence-

rence, is independently selected from hydrogen, fluorine, chlorine, cyano, amino, mercapto, -COOH, methyl, ethyl, n-propyl, isopropyl, methoxy, ethoxy, n-propoxy, isopropoxy,

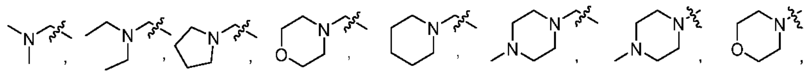 , , , , , , , ,

trifluoromethyl, monofluoromethyl, difluoromethyl, 2-fluoroethyl, pentafluoroethyl. Most preferably, $R^8$, for each occurrence, is independently selected from hydrogen, fluorine, cyano, methyl, isopropyl.

In any implementation of the present application and any aspect thereof, $R^9$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^9$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

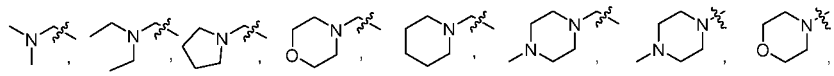 , , , , , , , ,

trifluoromethyl, monofluoromethyl, difluoromethyl, 2-fluoroethyl, pentafluoroethyl. More preferably, $R^9$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

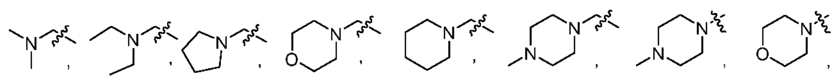 , , , , , , , ,

trifluoromethyl, monofluoromethyl, difluoromethyl, 2-fluoroethyl, pentafluoroethyl. Most preferably, $R^9$, for each occurrence, is independently selected from hydrogen, fluorine, cyano, methyl, isopropyl.

In any implementation of the present application and any aspect thereof, $R^{10}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{10}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

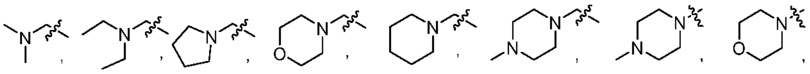 , , , , , , , ,

trifluoromethyl, monofluoromethyl, difluoromethyl, 2-fluoroethyl, pentafluoroethyl. More preferably, $R^{10}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

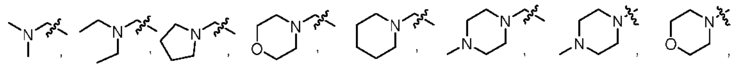 , , , , , , , ,

trifluoromethyl, monofluoromethyl, difluoromethyl, 2-fluoroethyl, pentafluoroethyl. Most preferably, $R^{10}$, for each occurrence, is independently selected from hydrogen, fluorine, cyano, methyl, isopropyl.

In any implementation of the present application and any aspect thereof, $R^{11}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$

alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{11}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

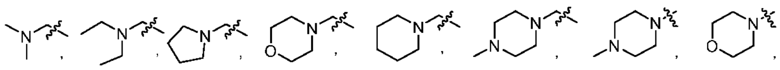,

trifluoromethyl, monofluoromethyl, difluoromethyl, 2-fluoroethyl, pentafluoroethyl. More preferably, $R^{11}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

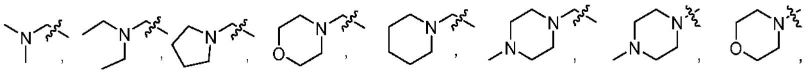,

trifluoromethyl, monofluoromethyl, difluoromethyl, 2-fluoroethyl, pentafluoroethyl. Most preferably, $R^{11}$, for each occurrence, is independently selected from hydrogen, fluorine, cyano, methyl, isopropyl.

In any implementation of the present application and any aspect thereof, $R^{12}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{12}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

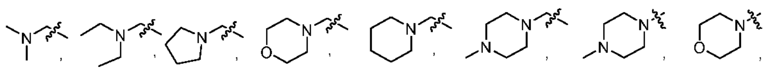,

trifluoromethyl, monofluoromethyl, difluoromethyl, 2-fluoroethyl, pentafluoroethyl. More preferably, $R^{12}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

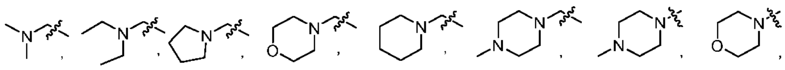,

trifluoromethyl, monofluoromethyl, difluoromethyl, 2-fluoroethyl, pentafluoroethyl. Most preferably, $R^{12}$, for each occurrence, is independently selected from hydrogen, fluorine, cyano, methyl, isopropyl.

In any implementation of the present application and any aspect thereof, $R^{13}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{13}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

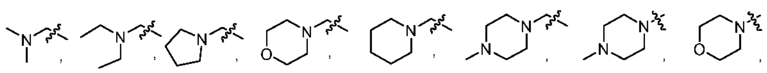,

trifluoromethyl, monofluoromethyl, difluoromethyl, 2-fluoroethyl, pentafluoroethyl. More preferably, $R^{13}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

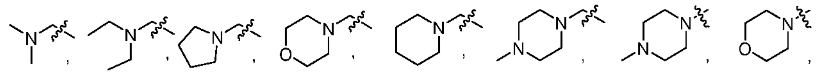

trifluoromethyl, monofluoromethyl, difluoromethyl, 2-fluoroethyl, pentafluoroethyl. Most preferably, $R^{13}$, for each occurrence, is independently selected from hydrogen, fluorine, cyano, methyl, isopropyl.

In any implementation of the present application and any aspect thereof, $R^{14}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

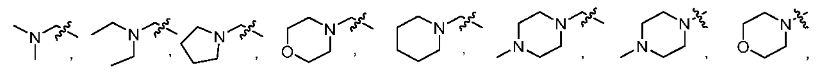

trifluoromethyl, monofluoromethyl, difluoromethyl, 2-fluoroethyl, pentafluoroethyl. More preferably, $R^{14}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

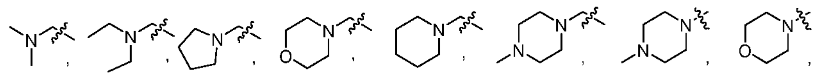

trifluoromethyl, monofluoromethyl, difluoromethyl, 2-fluoroethyl, pentafluoroethyl. Most preferably, $R^{14}$, for each occurrence, is independently selected from hydrogen, fluorine, cyano, methyl, isopropyl.

In any implementation of the present application and any aspect thereof, $R^{14}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

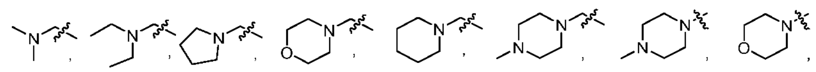

trifluoromethyl, monofluoromethyl, difluoromethyl, 2-fluoroethyl, pentafluoroethyl. More preferably, $R^{14}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

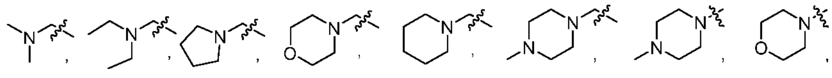

trifluoromethyl, monofluoromethyl, difluoromethyl, 2-fluoroethyl, pentafluoroethyl. Most preferably, $R^{14}$, for each occurrence, is independently selected from hydrogen, fluorine, cyano, methyl, isopropyl.

In any implementation of the present application and any aspect thereof, $R^{14}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein

the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

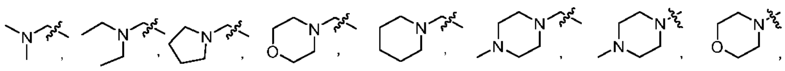

trifluoromethyl, monofluoromethyl, difluoromethyl, 2-fluoroethyl, pentafluoroethyl. More preferably, $R^{14''}$, , for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

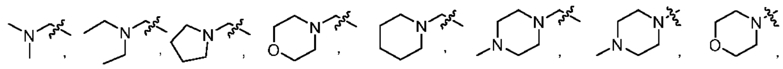

trifluoromethyl, monofluoromethyl, difluoromethyl, 2-fluoroethyl, pentafluoroethyl. Most preferably, $R^{14''}$, for each occurrence, is independently selected from hydrogen, fluorine, cyano, methyl, isopropyl.

In any implementation of the present application and any aspect thereof, $R^{14'''}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

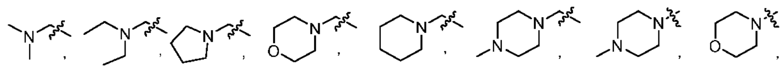

trifluoromethyl, monofluoromethyl, difluoromethyl, 2-fluoroethyl, pentafluoroethyl. More preferably, $R^{14'''}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

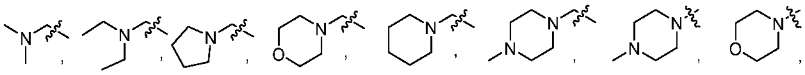

trifluoromethyl, monofluoromethyl, difluoromethyl, 2-fluoroethyl, pentafluoroethyl. Most preferably, $R^{14'''}$, for each occurrence, is independently selected from hydrogen, fluorine, cyano, methyl, isopropyl.

In any implementation of the present application and any aspect thereof, $R^{15}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$alkyl. Preferably, $R^{15}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

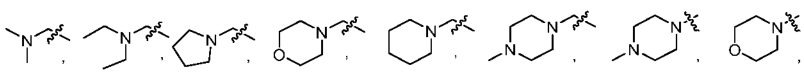

trifluoromethyl, monofluoromethyl, difluoromethyl, 2-fluoroethyl, pentafluoroethyl. More preferably, $R^{15}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

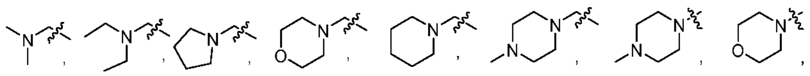

trifluoromethyl, monofluoromethyl, difluoromethyl, 2-fluoroethyl, pentafluoroethyl. Most preferably, $R^{15}$, for each occurrence, is independently selected from hydrogen, fluorine, cyano, methyl, isopropyl.

In any implementation of the present application and any aspect thereof, $R^{16}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{16}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

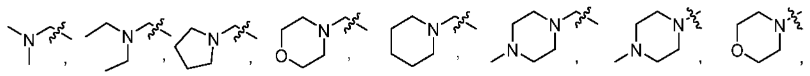

trifluoromethyl, monofluoromethyl, difluoromethyl, 2-fluoroethyl, pentafluoroethyl. More preferably, $R^{16}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

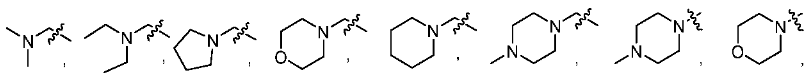

trifluoromethyl, monofluoromethyl, difluoromethyl, 2-fluoroethyl, pentafluoroethyl. Most preferably, $R^{16}$, for each occurrence, is independently selected from hydrogen, fluorine, cyano, methyl, isopropyl.

In any implementation of the present application and any aspect thereof, $R^{17}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{17}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

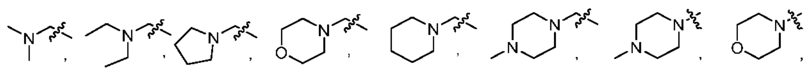

trifluoromethyl, monofluoromethyl, difluoromethyl, 2-fluoroethyl, pentafluoroethyl. More preferably, $R^{17}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

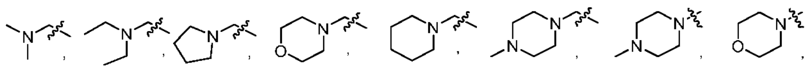

trifluoromethyl, monofluoromethyl, difluoromethyl, 2-fluoroethyl, pentafluoroethyl. Most preferably, $R^{17}$, for each occurrence, is independently selected from hydrogen, fluorine, cyano, methyl, isopropyl.

In any implementation of the present application and any aspect thereof, $R^{18}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{18}$, for each occurrence, is independently selected from

absence, hydrogen, fluorine, chlorine, cyano, amino, mercapto, -COOH, methyl, ethyl, n-propyl, isopropyl, methoxy, ethoxy, n-propoxy, isopropoxy,

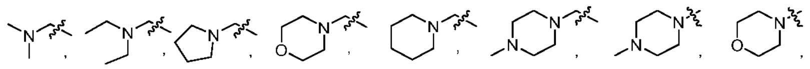
, , , , , , , ,

trifluoromethyl, monofluoromethyl, difluoromethyl, 2-fluoroethyl, pentafluoroethyl. More preferably, $R^{18}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

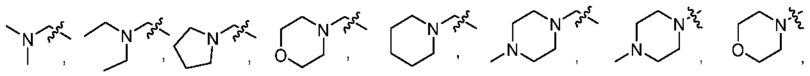
, , , , , , , ,

trifluoromethyl, monofluoromethyl, difluoromethyl, 2-fluoroethyl, pentafluoroethyl. Most preferably, $R^{18}$, for each occurrence, is independently selected from hydrogen, fluorine, chlorine, cyano, methyl, or isopropyl.

In any implementation of the present application and any aspect thereof, $R^{21}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{21}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

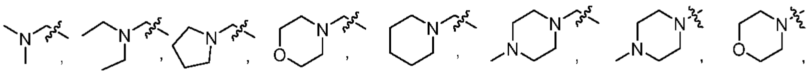
, , , , , , , ,

trifluoromethyl, monofluoromethyl, difluoromethyl, 2-fluoroethyl, pentafluoroethyl. More preferably, $R^{21}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

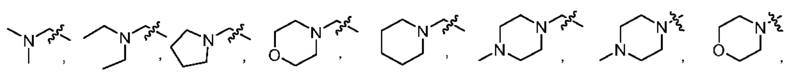
, , , , , , , ,

trifluoromethyl, monofluoromethyl, difluoromethyl, 2-fluoroethyl, pentafluoroethyl. Most preferably, $R^{21}$, for each occurrence, is independently selected from hydrogen, fluorine, cyano, methyl, isopropyl.

In any implementation of the present application and any aspect thereof, $R^{22}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{22}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

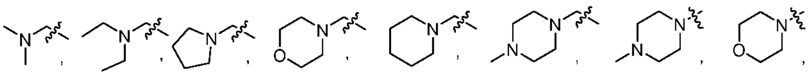
, , , , , , , ,

trifluoromethyl, monofluoromethyl, difluoromethyl, 2-fluoroethyl, pentafluoroethyl. More preferably, $R^{22}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

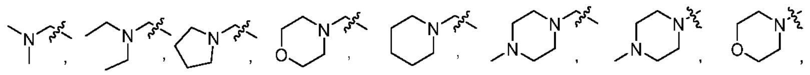
, , , , , , , ,

trifluoromethyl, monofluoromethyl, difluoromethyl, 2-fluoroethyl, pentafluoroethyl. Most preferably, $R^{22}$, for each occurrence, is independently selected from hydrogen, fluorine, cyano, methyl, isopropyl.

In any implementation of the present application and any aspect thereof, $R^{23}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{23}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

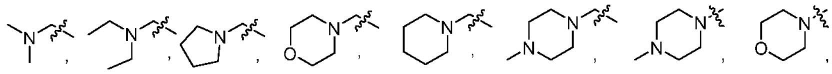
, , , , , , , ,

trifluoromethyl, monofluoromethyl, difluoromethyl, 2-fluoroethyl, pentafluoroethyl. More preferably, $R^{23}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

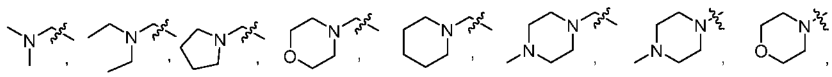
, , , , , , , ,

trifluoromethyl, monofluoromethyl, difluoromethyl, 2-fluoroethyl, pentafluoroethyl. Most preferably, $R^{23}$, for each occurrence, is independently selected from hydrogen, fluorine, cyano, methyl, isopropyl.

In any implementation of the present application and any aspect thereof, $R^{24}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{24}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

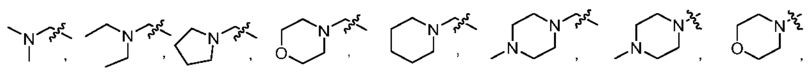
, , , , , , , ,

trifluoromethyl, monofluoromethyl, difluoromethyl, 2-fluoroethyl, pentafluoroethyl. More preferably, $R^{24}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

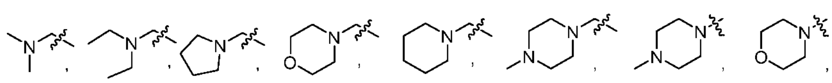
, , , , , , , ,

trifluoromethyl, monofluoromethyl, difluoromethyl, 2-fluoroethyl, pentafluoroethyl. Most preferably, $R^{24}$, for each occurrence, is independently selected from hydrogen, fluorine, cyano, methyl, isopropyl.

In any implementation of the present application and any aspect thereof, $R^{25}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{25}$, for each occurrence, is independently selected from

absence, hydrogen, fluorine, chlorine, cyano, amino, mercapto, -COOH, methyl, ethyl, n-propyl, isopropyl, methoxy, ethoxy, n-propoxy, isopropoxy,

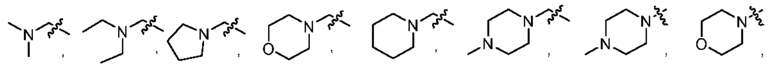

trifluoromethyl, monofluoromethyl, difluoromethyl, 2-fluoroethyl, pentafluoroethyl. More preferably, $R^{25}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

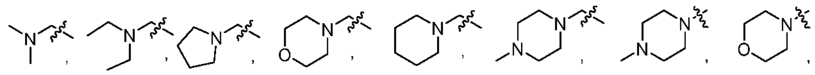

trifluoromethyl, monofluoromethyl, difluoromethyl, 2-fluoroethyl, pentafluoroethyl. Most preferably, $R^{25}$, for each occurrence, is independently selected from hydrogen, fluorine, cyano, methyl, isopropyl.

In any implementation of the present application and any aspect thereof, $R^{26}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$alkyl. Preferably, $R^{26}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

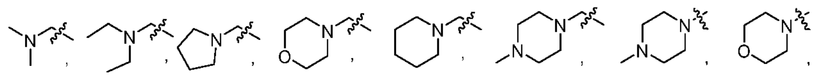

trifluoromethyl, monofluoromethyl, difluoromethyl, 2-fluoroethyl, pentafluoroethyl. More preferably, $R^{26}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

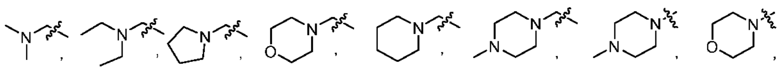

trifluoromethyl, monofluoromethyl, difluoromethyl, 2-fluoroethyl, pentafluoroethyl. Most preferably, $R^{26}$, for each occurrence, is independently selected from hydrogen, fluorine, cyano, methyl, isopropyl.

In any implementation of the present application and any aspect thereof, $R^{27}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{27}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

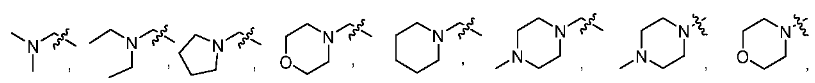

trifluoromethyl, monofluoromethyl, difluoromethyl, 2-fluoroethyl, pentafluoroethyl. More preferably, $R^{27}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

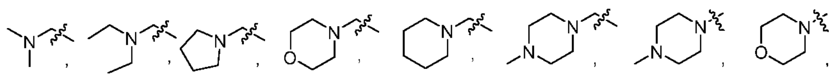
, , , , , , , ,

trifluoromethyl, monofluoromethyl, difluoromethyl, 2-fluoroethyl, pentafluoroethyl. Most preferably, $R^{27}$, for each occurrence, is independently selected from hydrogen, fluorine, cyano, methyl, isopropyl.

In any implementation of the present application and any aspect thereof, $R^{28}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{28}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

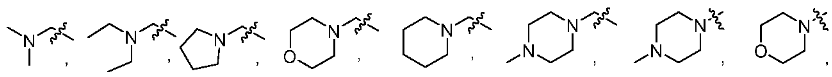
, , , , , , , ,

trifluoromethyl, monofluoromethyl, difluoromethyl, 2-fluoroethyl, pentafluoroethyl. More preferably, $R^{28}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

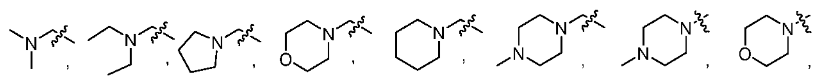
, , , , , , , ,

trifluoromethyl, monofluoromethyl, difluoromethyl, 2-fluoroethyl, pentafluoroethyl. Most preferably, $R^{28}$, for each occurrence, is independently selected from hydrogen, fluorine, cyano, methyl, isopropyl.

In any implementation of the present application and any aspect thereof, $R^{31}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{31}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

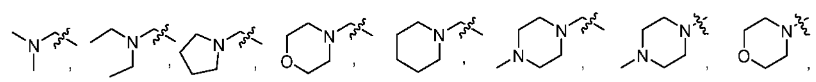
, , , , , , , ,

trifluoromethyl, monofluoromethyl, difluoromethyl, 2-fluoroethyl, pentafluoroethyl. More preferably, $R^{31}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

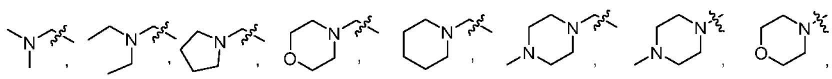
, , , , , , , ,

trifluoromethyl, monofluoromethyl, difluoromethyl, 2-fluoroethyl, pentafluoroethyl. Most preferably, $R^{31}$, for each occurrence, is independently selected from hydrogen, fluorine, cyano, methyl, isopropyl.

In any implementation of the present application and any aspect thereof, $R^{32}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{32}$, for each occurrence, is independently selected from

absence, hydrogen, fluorine, chlorine, cyano, amino, mercapto, -COOH, methyl, ethyl, n-propyl, isopropyl, methoxy, ethoxy, n-propoxy, isopropoxy,

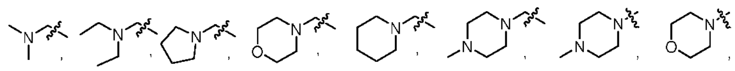 , , , , , , , ,

trifluoromethyl, monofluoromethyl, difluoromethyl, 2-fluoroethyl, pentafluoroethyl. More preferably, $R^{32}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

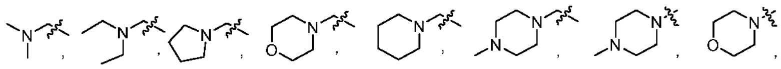 , , , , , , , ,

trifluoromethyl, monofluoromethyl, difluoromethyl, 2-fluoroethyl, pentafluoroethyl. Most preferably, $R^{32}$, for each occurrence, is independently selected from hydrogen, fluorine, cyano, methyl, isopropyl.

In any implementation of the present application and any aspect thereof, $R^{33}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{33}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

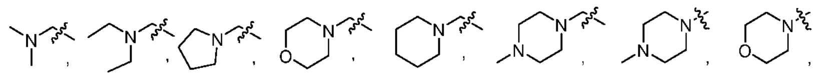 , , , , , , , ,

trifluoromethyl, monofluoromethyl, difluoromethyl, 2-fluoroethyl, pentafluoroethyl. More preferably, $R^{33}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

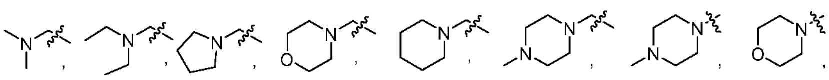 , , , , , , , ,

trifluoromethyl, monofluoromethyl, difluoromethyl, 2-fluoroethyl, pentafluoroethyl. Most preferably, $R^{33}$, for each occurrence, is independently selected from hydrogen, fluorine, cyano, methyl, isopropyl.

In any implementation of the present application and any aspect thereof, $R^{34}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{34}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

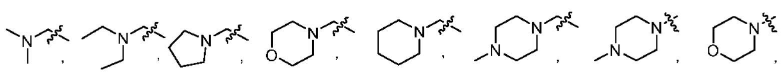 , , , , , , , ,

trifluoromethyl, monofluoromethyl, difluoromethyl, 2-fluoroethyl, pentafluoroethyl. More preferably, $R^{34}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

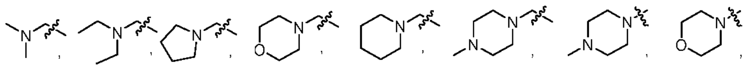

trifluoromethyl, monofluoromethyl, difluoromethyl, 2-fluoroethyl, pentafluoroethyl. Most preferably, $R^{34}$, for each occurrence, is independently selected from hydrogen, fluorine, cyano, methyl, isopropyl.

In any implementation of the present application and any aspect thereof, $R^{35}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$alkyl. Preferably, $R^{35}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

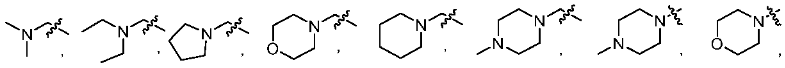

trifluoromethyl, monofluoromethyl, difluoromethyl, 2-fluoroethyl, pentafluoroethyl. More preferably, $R^{35}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

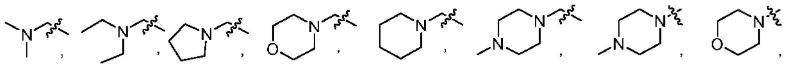

trifluoromethyl, monofluoromethyl, difluoromethyl, 2-fluoroethyl, pentafluoroethyl. Most preferably, $R^{35}$, for each occurrence, is independently selected from hydrogen, fluorine, cyano, methyl, isopropyl.

In any implementation of the present application and any aspect thereof, $R^{36}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, $R^{36}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

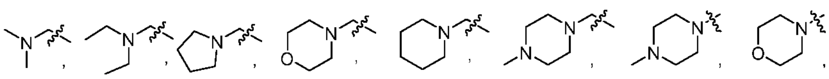

trifluoromethyl, monofluoromethyl, difluoromethyl, 2-fluoroethyl, pentafluoroethyl. More preferably, $R^{36}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

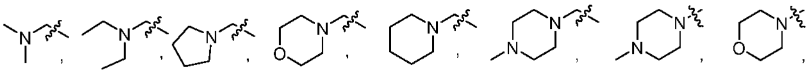

trifluoromethyl, monofluoromethyl, difluoromethyl, 2-fluoroethyl, pentafluoroethyl. Most preferably, $R^{36}$, for each occurrence, is independently selected from hydrogen, fluorine, cyano, methyl, isopropyl.

In any implementation of the present application and any aspect thereof, a is 0. In any implementation of the present application and any aspect thereof, a is 1. In any implementation of the present application and any aspect thereof, a is 2.

In any implementation of the present application and any aspect thereof, b is 0. In any implementation of the present application and any aspect thereof, b is 1. In any implementation of the present application and any aspect thereof, b is 2.

In any implementation of the present application and any aspect thereof, c is 0. In any implementation of the present application and any aspect thereof, c is 1. In any implementation of the present application and any aspect thereof, c is 2.

In any implementation of the present application and any aspect thereof, d is 0. In any implementation of the present application and any aspect thereof, d is 1. In any implementation of the present application and any aspect thereof, d is 2.

In any implementation of the present application and any aspect thereof, e is 0. In any implementation of the present application and any aspect thereof, e is 1. In any implementation of the present application and any aspect thereof, e is 2.

In any implementation of the present application and any aspect thereof, n is 0. In any implementation of the present application and any aspect thereof, n is 1. In any implementation of the present application and any aspect thereof, n is 2.

In any implementation of the present application and any aspect thereof, g is 0. In any implementation of the present application and any aspect thereof, g is 1. In any implementation of the present application and any aspect thereof, g is 2.

In any implementation of the present application and any aspect thereof, h is 0. In any implementation of the present application and any aspect thereof, h is 1. In any implementation of the present application and any aspect thereof, h is 2.

In any implementation of the present application and any aspect thereof, $X^1$ is N. In any implementation of the present application and any aspect thereof, $X^1$ is $CR^{14}$.

In any implementation of the present application and any aspect thereof, $X^2$ is N. In any implementation of the present application and any aspect thereof, $X^2$ is $CR^{14'}$.

In any implementation of the present application and any aspect thereof, $X^3$ is N. In any implementation of the present application and any aspect thereof, $X^3$ is $CR^{14''}$.

In any implementation of the present application and any aspect thereof, $X^4$ is N. In any implementation of the present application and any aspect thereof, $X^4$ is $CR^{14'''}$.

In any implementation of the present application and any aspect thereof, $X^5$ is O. In any implementation of the present application and any aspect thereof, $X^5$ is $CR^{15}R^{16}$. In any implementation of the present application and any aspect thereof, $X^5$ is $NR^{17}$.

In any implementation of the present application and any aspect thereof, $X^6$ is N. In any implementation of the present application and any aspect thereof, $X^6$ is C.

In any implementation of the present application and any aspect thereof, $X^7$ is N. In any implementation of the present application and any aspect thereof, $X^7$ is C.

In any implementation of the present application and any aspect thereof, $X^8$ is N. In any implementation of the present application and any aspect thereof, $X^6$ is C.

In any implementation of the present application and any aspect thereof, $X^9$ is N. In any implementation of the present application and any aspect thereof, $X^9$ is C.

In any implementation of the present application and any aspect thereof, T is N. In any implementation of the present application and any aspect thereof, T is CH.

In any implementation of the present application and any aspect thereof, U is N. In any implementation of the present application and any aspect thereof, U is C. In any implementation of the present application and any aspect thereof, U is O.

In any implementation of the present application and any aspect thereof, Y is N. In any implementation of the present application and any aspect thereof, Y is C. In any implementation of the present application and any aspect thereof, Y is O.

In any implementation of the present application and any aspect thereof, J is $NR^{31}$. In any implementation of the present application and any aspect thereof, J is $CR^{32}R^{33}$.

In any implementation of the present application and any aspect thereof, K is $NR^{34}$. In any implementation of the present application and any aspect thereof, K is $CR^{35}R^{36}$.

In any implementation of the present application and any aspect thereof, L is O. In any implementation of the present application and any aspect thereof, L is $NR^{21}$. In any implementation of the present application and any aspect thereof, L is $CR^{22}$. In any implementation of the present application and any aspect thereof, L is $CR^{23}R^{24}$. In any implementation of the present application and any aspect thereof, L is $NR^{25}$-$CR^{26}$. In any implementation of the present application and any aspect thereof, L is $CR^{27}$-$CR^{28}$.

In any implementation of the present application and any aspect thereof,

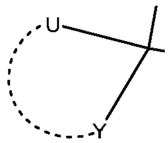

is 5-membered monocyclic ring. In any implementation of the present application and any aspect thereof,

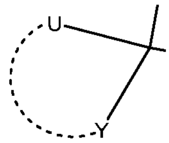

is 6-membered monocyclic ring. In any implementation of the present application and any aspect thereof,

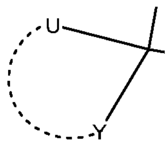

is 7-membered monocyclic ring. In any implementation of the present application and any aspect thereof,

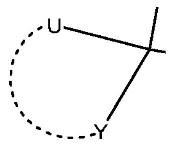

is substituted with 1 substituent. In any implementation of the present application and any aspect thereof,

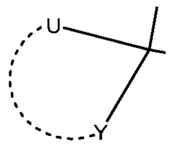

is substituted with 2 substituents. In any implementation of the present application and any aspect thereof,

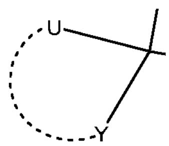

is

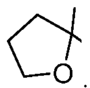.

In any implementation of the present application and any aspect thereof,

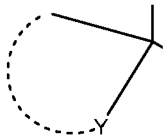

is 5-membered monocyclic ring. In any implementation of the present application and any aspect thereof,

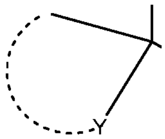

is 6-membered monocyclic ring. In any implementation of the present application and any aspect thereof,

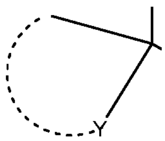

is 7-membered monocyclic ring. In any implementation of the present application and any aspect thereof,

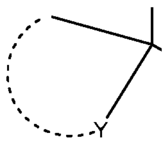

is substituted with 1 substituent. In any implementation of the present application and any aspect thereof,

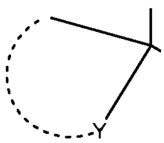

is substituted with 2 substituents. In any implementation of the present application and any aspect thereof,

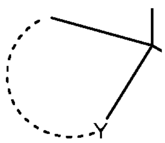

is

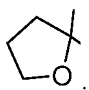.

In any implementation of the present application and any aspect thereof, P, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl. Preferably, P, for each occurrence, is independently selected from absence,

hydrogen, fluorine, chlorine, cyano, amino, mercapto, -COOH, methyl, ethyl, n-propyl, isopropyl, methoxy, ethoxy, n-propoxy, isopropoxy,

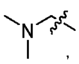, 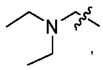, 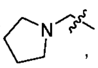, 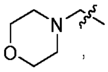, 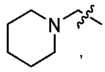, 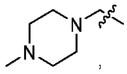, 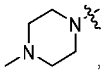, 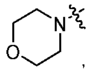,

trifluoromethyl, monofluoromethyl, difluoromethyl, 2-fluoroethyl, pentafluoroethyl, fluorophenylacetonitrile. More preferably, P, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

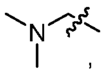, 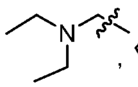, 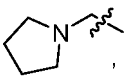, 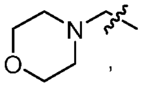, 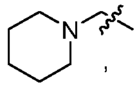, 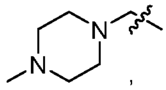, 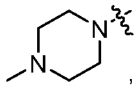, 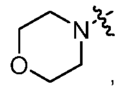,

trifluoromethyl, monofluoromethyl, difluoromethyl, 2-fluoroethyl, pentafluoroethyl, fluorophenylacetonitrile. Most preferably, P, for each occurrence, is independently selected from hydrogen, fluorine, cyano, methyl, isopropyl, tert-butyl, trifluoromethyl, morpholinyl,

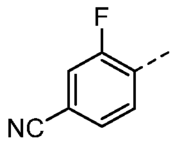 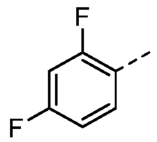 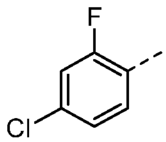 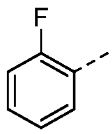.

In one aspect, the compounds of the present invention are compounds having the following structural formula (I):

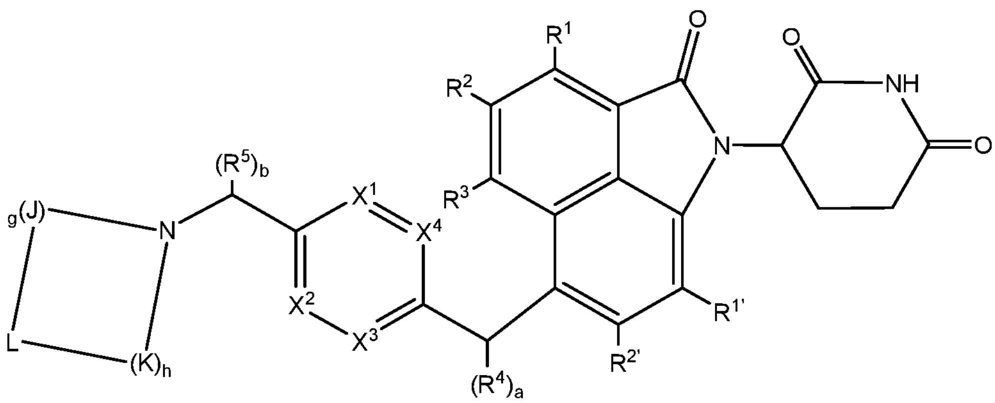

(I)

or a pharmaceutically acceptable salt thereof.
wherein:

$R^1$, $R^3$, $R^1$, $R^{2'}$, $R^4$ and $R^5$ are all hydrogen;

Both a and b are both 2;

Both g and h are both 2;

Both J and K are $CH_2$;

L is O;

$X^2$ and $X^4$ are both CH;

$X^1$ is N, CH, CF, C-$CH_3$;

$X^3$ is N, CH, CF, C-$CH_3$; and

$R^2$ is hydrogen, fluorine, cyano, trifluoromethyl, methoxy, ethoxy or methyl.

In one aspect, the compound of the present invention is the compound of structural formula (XIII):

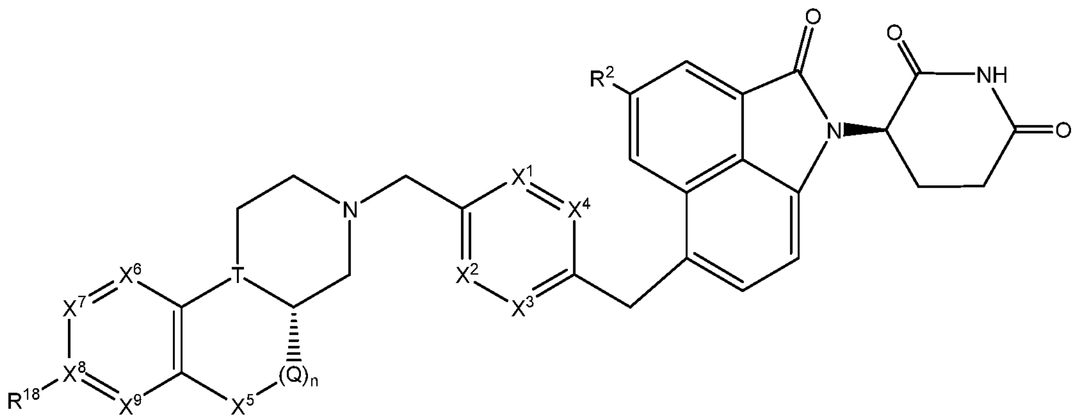

(XIII)

or a pharmaceutically acceptable salt thereof,
wherein:

TisN;
$R^2$ is hydrogen, fluorine, cyano, trifluoromethyl, methoxy, ethoxy or methyl;
$X^8$ is C, and $R^{18}$ is hydrogen, fluorine, chlorine, cyano, or methyl;
$-(Q)_n-$ is $-(CH_2)-$ or$-(CH_2)_2-$;
$X^5$ is O;
$X^1$ is N, CH, CF, C-$CH_3$;
$X^2$ is N, CH, CF, C-$CH_3$;
$X^3$ is N, CH, CF, C-$CH_3$;
$X^4$ is N, CH, CF, C-$CH_3$;
$X^6$ is N, CH, CF, C-$CH_3$;
$X^7$ is N, CH, CF, C-$CH_3$;
$X^9$ is N, CH, CF, C-$CH_3$.

In one aspect, the compound of the present invention is the compound of structural formula (XIV):

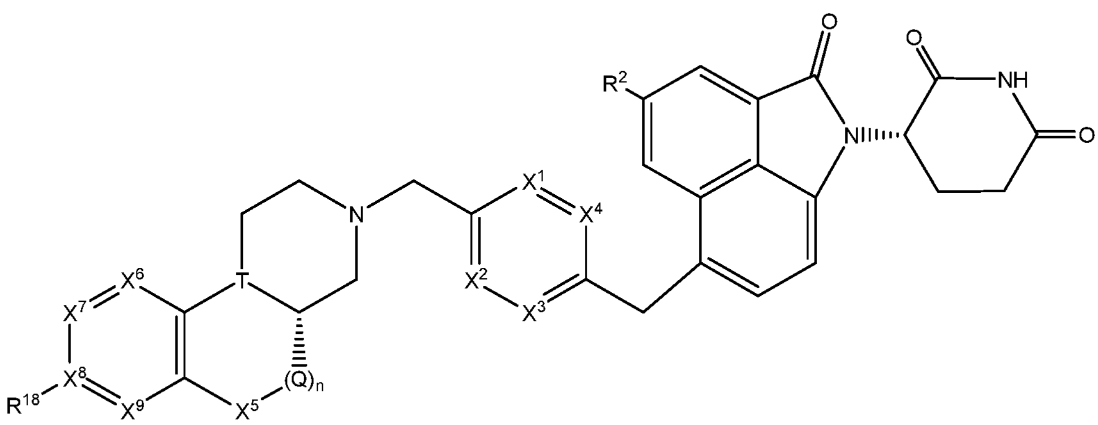

(XIV)

or a pharmaceutically acceptable salt thereof,
wherein:

TisN;
$R^2$ is hydrogen, fluorine, cyano, trifluoromethyl, methoxy, ethoxy or methyl;
$X^8$ is C, and $R^{18}$ is hydrogen, fluorine, chlorine, cyano, or methyl;
$-(Q)_n-$ is $-(CH_2)-$ or$-(CH_2)_2-$;
$X^5$ is O;
$X^1$ is N, CH, CF, $C-CH_3$;
$X^2$ is N, CH, CF, $C-CH_3$;
$X^3$ is N, CH, CF, $C-CH_3$;
$X^4$ is N, CH, CF, $C-CH_3$;
$X^6$ is N, CH, CF, $C-CH_3$;
$X^7$ is N, CH, CF, $C-CH_3$;
$X^9$ is N, CH, CF, $C-CH_3$.

In one aspect, the compound of the present invention is the compound of structural formula (XV):

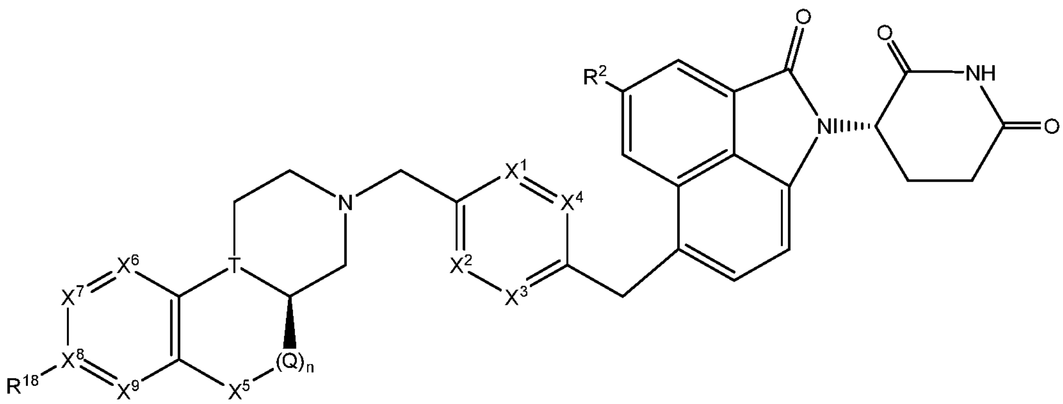

(XV)

or a pharmaceutically acceptable salt thereof,
wherein:

TisN;
$R^2$ is hydrogen, fluorine, cyano, trifluoromethyl, methoxy, ethoxy or methyl;
$X^8$ is C, and $R^{18}$ is hydrogen, fluorine, chlorine, cyano, or methyl;
$-(Q)_n-$ is $-(CH_2)-$ or$-(CH_2)_2-$;
$X^5$ is O;
$X^1$ is N, CH, CF;
$X^2$ is N, CH, CF, $C-CH_3$;
$X^3$ is N, CH, CF, $C-CH_3$;
$X^4$ is N, CH, CF, $C-CH_3$;
$X^6$ is N, CH, CF, $C-CH_3$;
$X^7$ is N, CH, CF, $C-CH_3$;
$X^9$ is N, CH, CF, $C-CH_3$.

In one aspect, the compound of the present invention is the compound of structural formula (XVI):

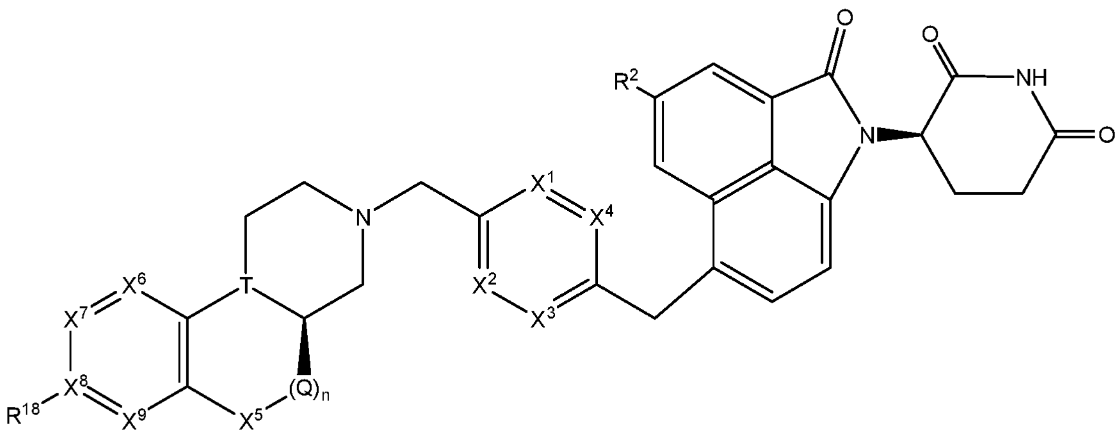

(XVI)

or a pharmaceutically acceptable salt thereof,
wherein:

- T is N;
- $R^2$ is hydrogen, fluorine, cyano, trifluoromethyl, methoxy, ethoxy or methyl;
- $X^8$ is C, and $R^{18}$ is hydrogen, fluorine, chlorine, cyano, or methyl;
- $-(Q)_n-$ is $-(CH_2)-$ or $-(CH_2)_2-$;
- $X^5$ is O;
- $X^1$ is N, CH, CF, C-$CH_3$;
- $X^2$ is N, CH, CF, C-$CH_3$;
- $X^3$ is N, CH, CF, C-$CH_3$;
- $X^4$ is N, CH, CF, C-$CH_3$;
- $X^6$ is N, CH, CF, C-$CH_3$;
- $X^7$ is N, CH, CF, C-$CH_3$;
- $X^9$ is N, CH, CF, C-$CH_3$.

In one aspect, the compound of the present invention is the compound of structural formula (XVII):

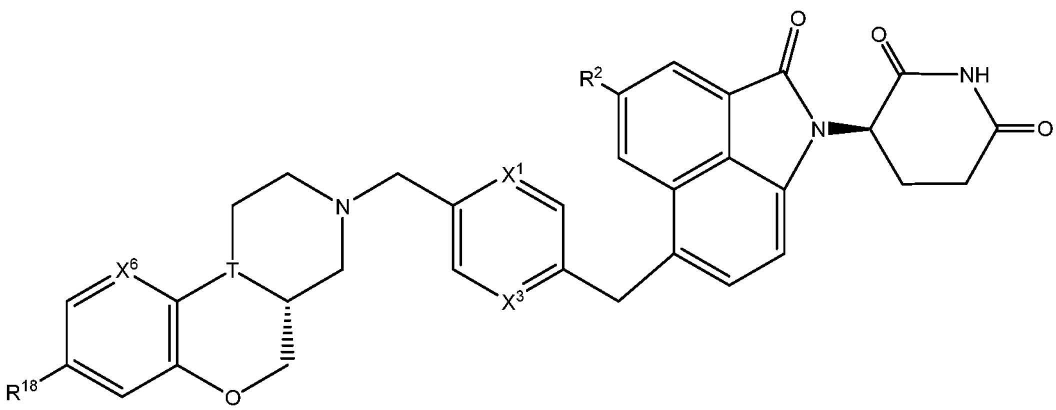

(XVII)

or a pharmaceutically acceptable salt thereof,
wherein:

- T is N;
- $R^2$ is hydrogen, fluorine, cyano, trifluoromethyl, methoxy, ethoxy or methyl;
- $R^{18}$ is hydrogen, fluorine, chlorine, cyano, or methyl;

$X^1$ is N, CH, CF, C-$CH_3$;
$X^3$ is N, CH, CF, C-$CH_3$;
$X^6$ is N, CH, CF, C-$CH_3$.

In one aspect, the compound of the present invention is the compound of structural formula (XVIII):

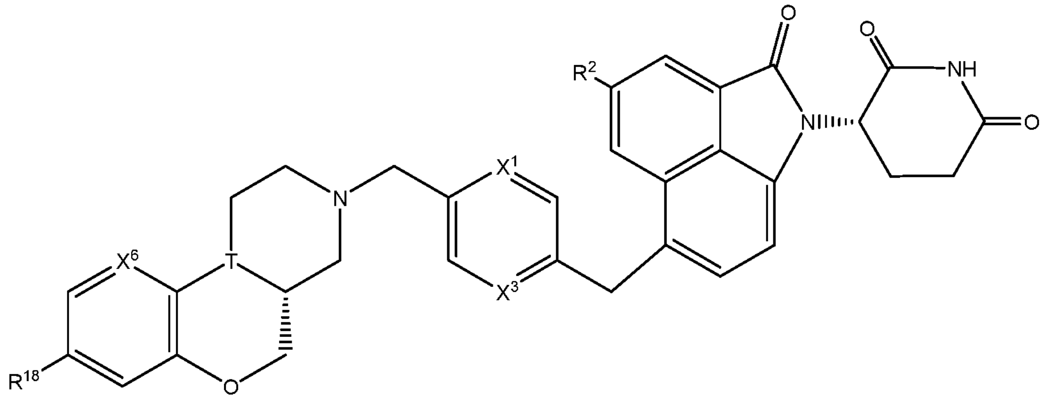

(XVIII)

or a pharmaceutically acceptable salt thereof,
wherein:

TisN;
$R^2$ is hydrogen, fluorine, cyano, trifluoromethyl, methoxy, ethoxy or methyl;
$R^{18}$ is hydrogen, fluorine, chlorine, cyano, or methyl;
$X^1$ is N, CH, CF, C-$CH_3$;
$X^3$ is N, CH, CF, C-$CH_3$;
$X^6$ is N, CH, CF, C-$CH_3$.

In one aspect, the compound of the present invention is the compound of structural formula (XIX) :

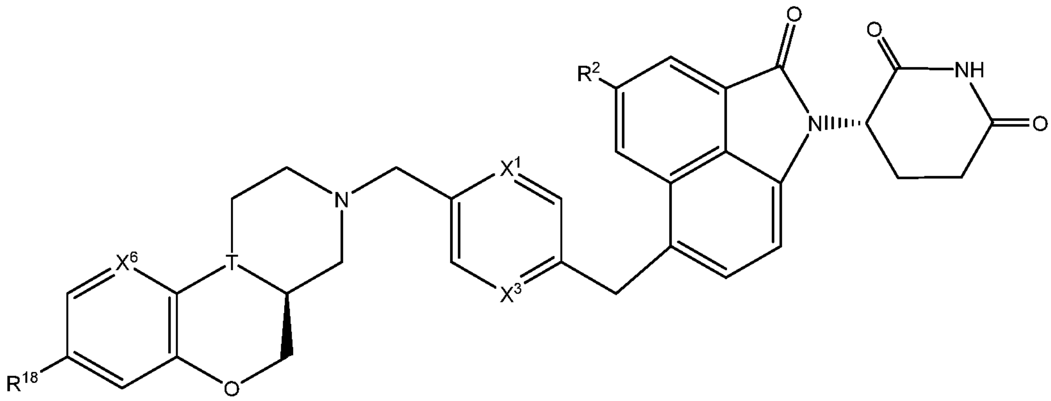

(XIX)

or a pharmaceutically acceptable salt thereof,
wherein:

TisN;
$R^2$ is hydrogen, fluorine, cyano, trifluoromethyl, methoxy, ethoxy or methyl;
$R^{18}$ is hydrogen, fluorine, chlorine, cyano, or methyl;
$X^1$ is N, CH, CF, C-$CH_3$;
$X^3$ is N, CH, CF, C-$CH_3$;
$X^6$ is N, CH, CF, C-$CH_3$.

In one aspect, the compound of the present invention is the compound of structural formula (XX):

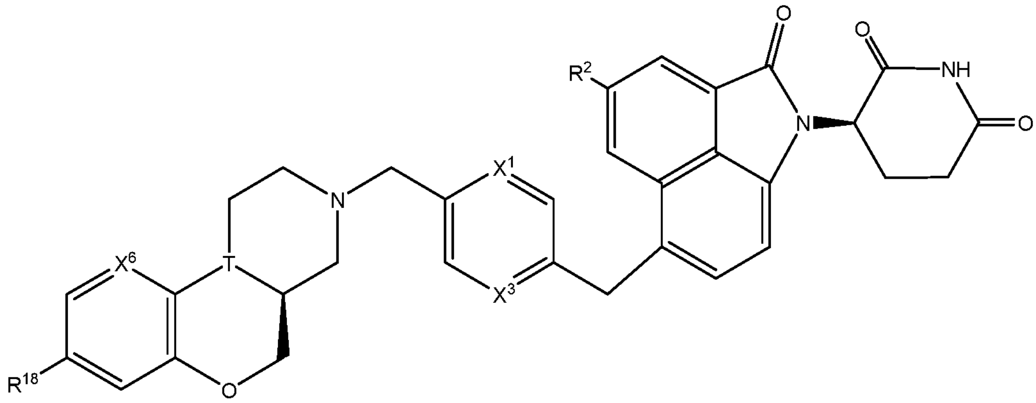

(XX)

or a pharmaceutically acceptable salt thereof,
wherein:

TisN;
$R^2$ is hydrogen, fluorine, cyano, trifluoromethyl, methoxy, ethoxy or methyl;
$R^{18}$ is hydrogen, fluorine, chlorine, cyano, or methyl;
$X^1$ is N, CH, CF, C-$CH_3$;
$X^3$ is N, CH, CF, C-$CH_3$;
$X^6$ is N, CH, CF, C-$CH_3$.

In one aspect, the compound of the present invention is the compound of structural formula (XXIII):

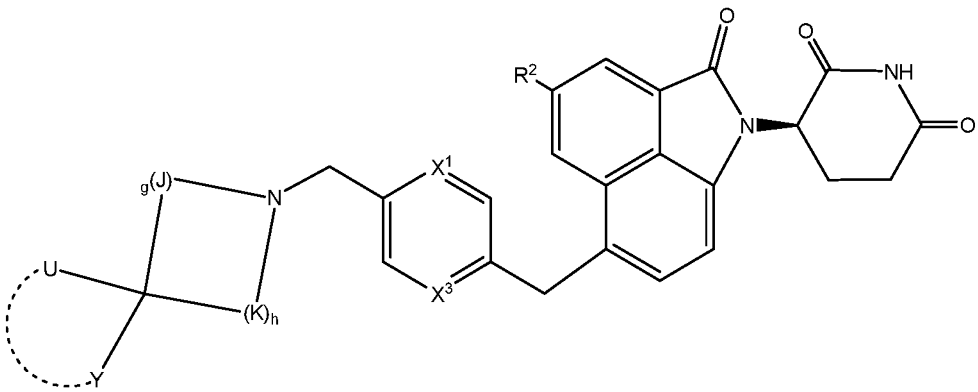

(XXIII)

or a pharmaceutically acceptable salt thereof,
wherein:

$R^2$ is hydrogen, fluorine, chlorine, bromine, cyano, trifluoromethyl, methoxy, ethoxy or methyl;

$X^1$ is N, CH, CF, C-$CH_3$;

$X^3$ is N, CH, CF, C-$CH_3$;

$-(J)_g$ -is $-(CH_2)-$ or $-(CH_2)_2-$;

$-(K)_h$ -is $-(CH_2)-$ or $-(CH_2)_2-$;

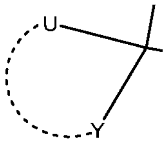

is a 5, 6 or 7 membered monocyclic ring;

U is C, N or O;

Y is C, N or O. In this aspect, preferably,

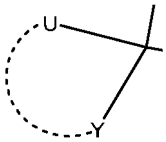

is

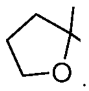

In one aspect, the compound of the present invention is the compound of structural formula (XXIV):

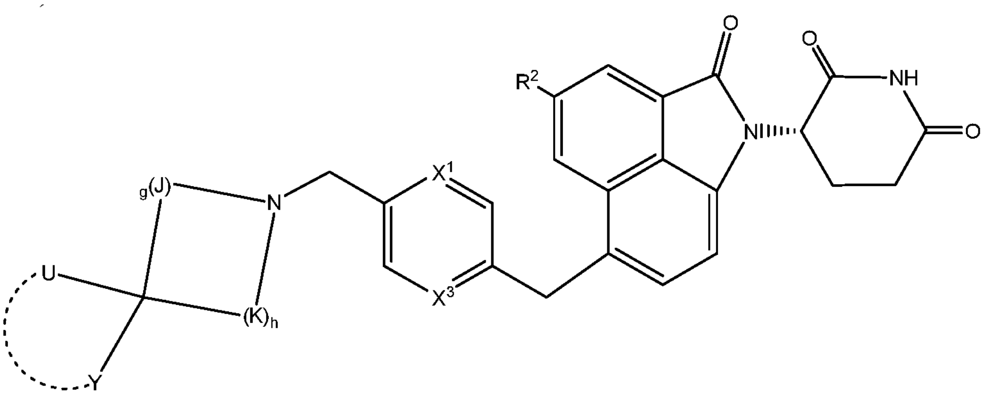

(XXIV)

or a pharmaceutically acceptable salt thereof,
wherein:

$R^2$ is hydrogen, fluorine, chlorine, bromine, cyano, trifluoromethyl, methoxy, ethoxy or methyl;

$X^1$ is N, CH, CF, C-$CH_3$;

$X^3$ is N, CH, CF, C-$CH_3$;

-$(J)_g$ -is -$(CH_2)$- or -$(CH_2)_2$-;

-$(K)_h$ -is -$(CH_2)$- or -$(CH_2)_2$-;

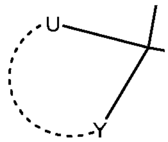

is a 5, 6 or 7 membered monocyclic ring;

U is C, N or O;

Y is C, N or O. In this aspect, preferably,

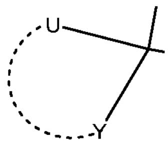

is

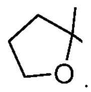

In one aspect, the compound of the present invention is the compound of structural formula (XXV):

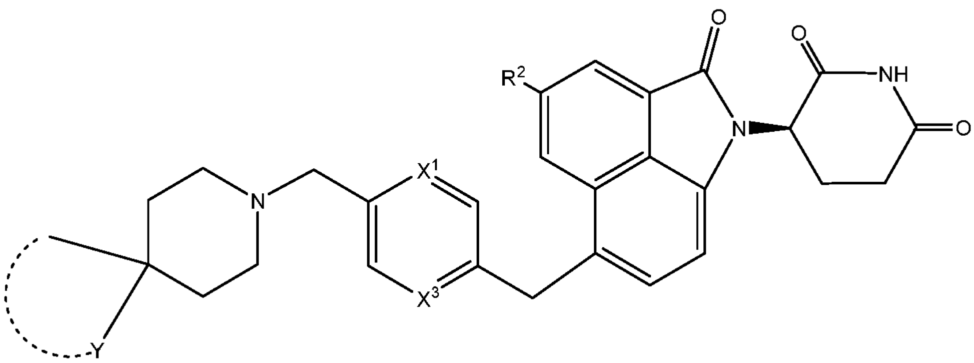

(XXV)

or a pharmaceutically acceptable salt thereof,

wherein:

$R^2$ is hydrogen, fluorine, chlorine, bromine, cyano, trifluoromethyl, methoxy, ethoxy or methyl;

$X^1$ is N, CH, CF, C-$CH_3$;

$X^3$ is N, CH, CF, C-$CH_3$;

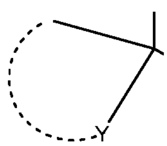

is a 5, 6 or 7 membered monocyclic ring;

Y is C, N or O. In this aspect, preferably,

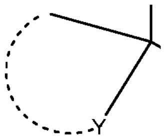

is

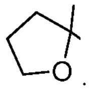

In one aspect, the compound of the present invention is the compound of structural formula (XXVI):

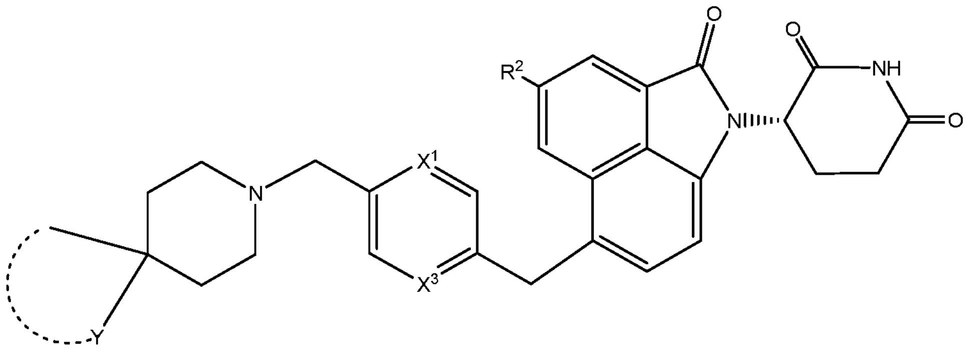

(XXVI)

or a pharmaceutically acceptable salt thereof,
wherein:

$R^2$ is hydrogen, fluorine, chlorine, bromine, cyano, trifluoromethyl, methoxy, ethoxy or methyl;

$X^1$ is N, CH, CF, C-$CH_3$;

$X^3$ is N, CH, CF, C-$CH_3$;

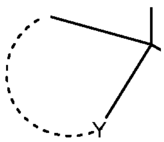

is a 5, 6 or 7 membered monocyclic ring;

Y is C, N or O. In this aspect, preferably,

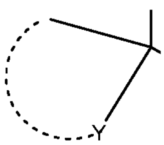

is

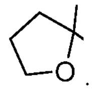

In one aspect, the compound of the present invention is the compound of structural formula (XXIX):

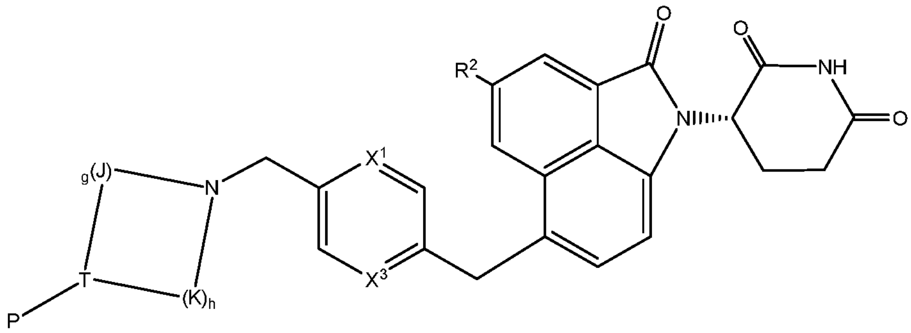

(XXIX)

or a pharmaceutically acceptable salt thereof,
wherein:

T is CorN;
-$(J)_g$ -is -$(CH_2)$- or -$(CH_2)_2$-;
-$(K)_h$ -is -$(CH_2)$- or -$(CH_2)_2$-;
$R^2$ is hydrogen, fluorine, chlorine, bromine, cyano, trifluoromethyl, methoxy, ethoxy or methyl;
$X^1$ is N, CH, CF, C-$CH_3$;
$X^3$ is N, CH, CF, C-$CH_3$;
P is hydrogen, fluorine, cyano, methyl, isopropyl, tert-butyl, trifluoromethyl, morpholinyl,

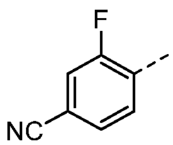

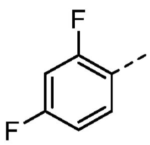

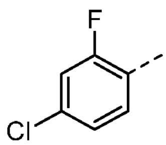

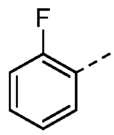

.

In one aspect, the compound of the present invention is the compound of structural formula (XXX):

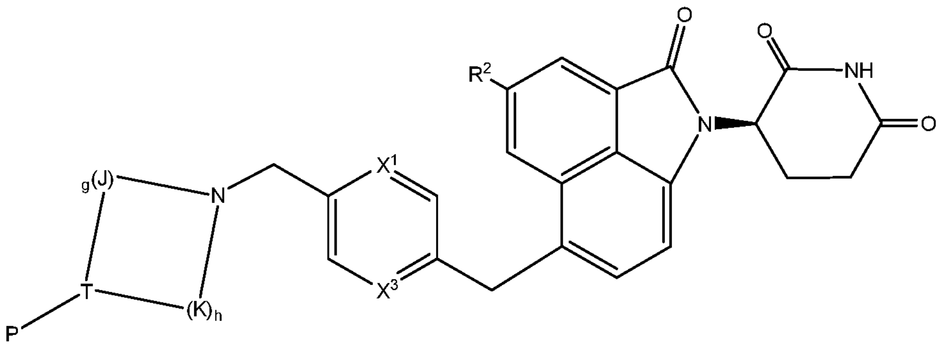

(XXX)

or a pharmaceutically acceptable salt thereof,
wherein:

TisCorN;
-$(J)_g$ -is -$(CH_2)$- or -$(CH_2)_2$-;
-$(K)_h$ -is -$(CH_2)$- or -$(CH_2)_2$-;
$R^2$ is hydrogen, fluorine, chlorine, bromine, cyano, trifluoromethyl, methoxy, ethoxy or methyl;

$X^1$ is N, CH, CF, C-$CH_3$;
$X^3$ is N, CH, CF, C-$CH_3$;
P is hydrogen, fluorine, cyano, methyl, isopropyl, tert-butyl, trifluoromethyl, morpholinyl,

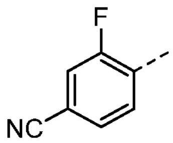 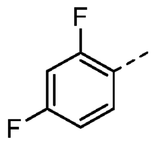 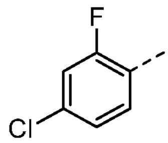 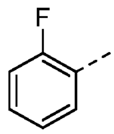 .

In one aspect, the compound of the present invention is the compound of structural formula (XXXI):

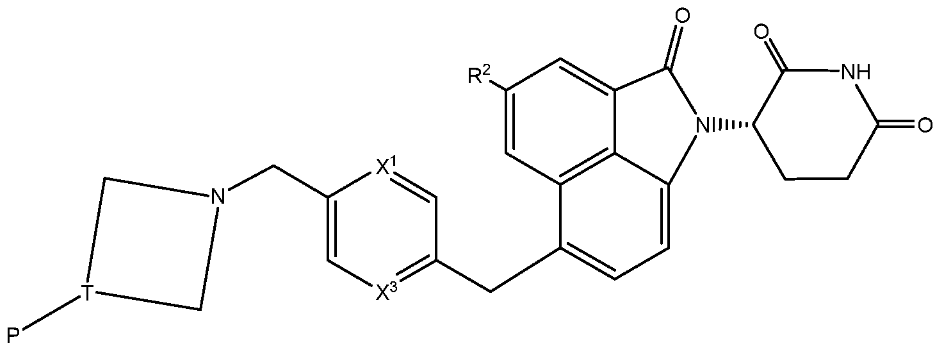

(XXXI)

or a pharmaceutically acceptable salt thereof,
wherein:

TisCorN;
$R^2$ is hydrogen, fluorine, chlorine, bromine, cyano, trifluoromethyl, methoxy, ethoxy or methyl;
$X^1$ is N, CH, CF, C-$CH_3$;
$X^3$ is N, CH, CF, C-$CH_3$;
P is hydrogen, fluorine, cyano, methyl, isopropyl, tert-butyl, trifluoromethyl, morpholinyl,

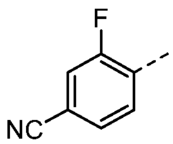 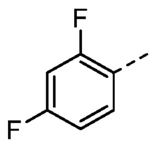 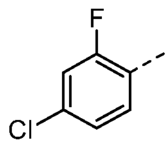 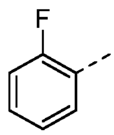 .

In one aspect, the compound of the present invention is the compound of structural formula (XXXII):

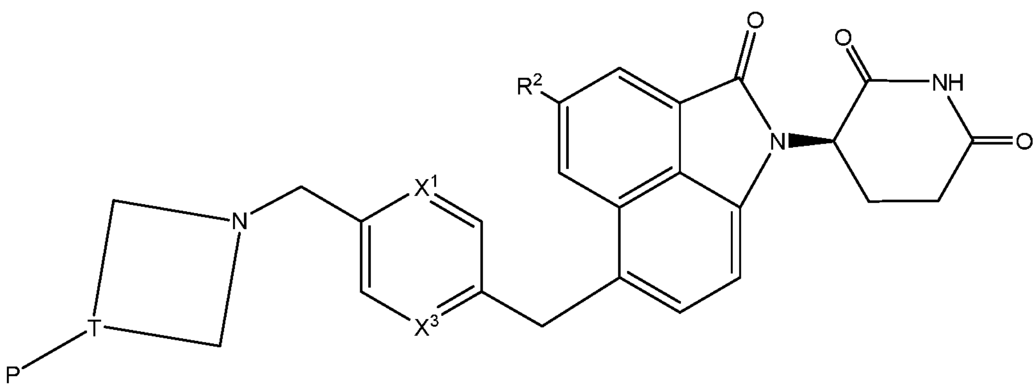

(XXXII)

or a pharmaceutically acceptable salt thereof,
wherein:

T is C or N;

$R^2$ is hydrogen, fluorine, chlorine, bromine, cyano, trifluoromethyl, methoxy, ethoxy or methyl;

$X^1$ is N, CH, CF, C-$CH_3$;

$X^3$ is N, CH, CF, C-$CH_3$;

P is hydrogen, fluorine, cyano, methyl, isopropyl, tert-butyl, trifluoromethyl, morpholinyl,

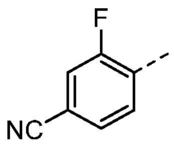 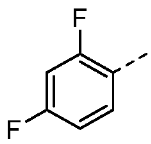 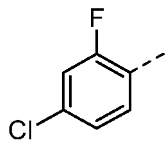 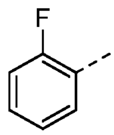.

In one aspect, the compound of the present invention is the compound of structural formula (XXXIII):

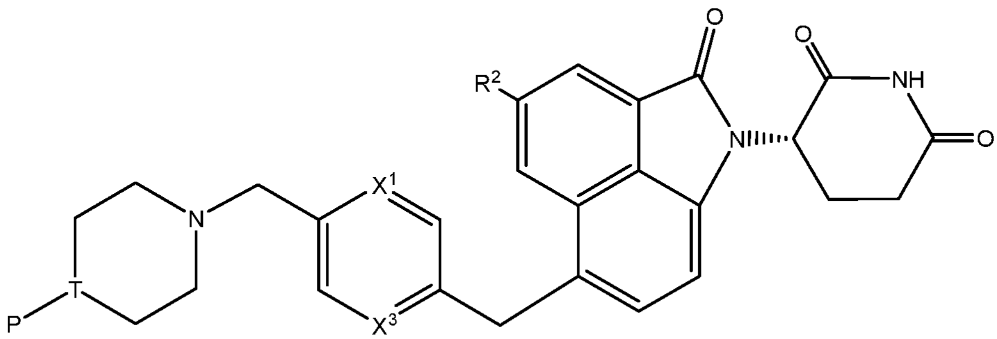

(XXXIII)

or a pharmaceutically acceptable salt thereof,
wherein:

T is C or N;
$R^2$ is hydrogen, fluorine, chlorine, bromine, cyano, trifluoromethyl, methoxy, ethoxy or methyl;
$X^1$ is N, CH, CF, C-$CH_3$;
$X^3$ is N, CH, CF, C-$CH_3$;
P is hydrogen, fluorine, cyano, methyl, isopropyl, tert-butyl, trifluoromethyl, morpholinyl,

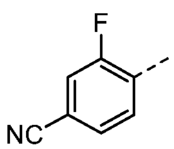 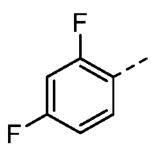 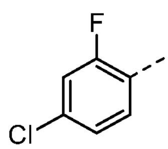 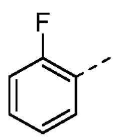.

In one aspect, the compound of the present invention is the compound of structural formula (XXXIV):

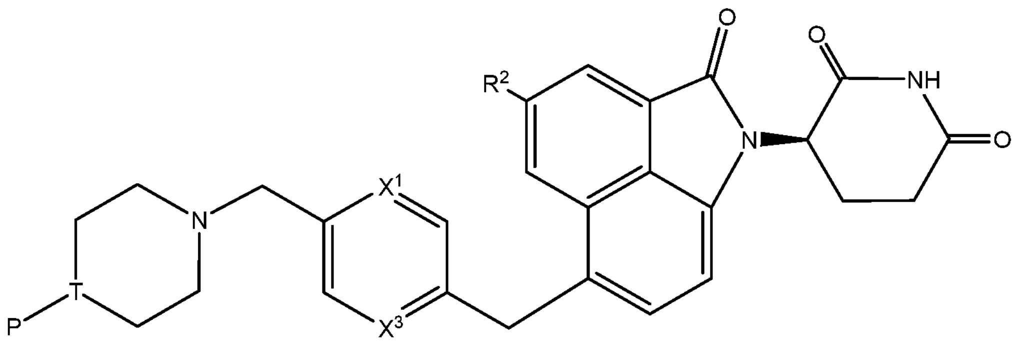

(XXXIV)

or a pharmaceutically acceptable salt thereof, wherein:

T is CorN;

$R^2$ is hydrogen, fluorine, chlorine, bromine, cyano, trifluoromethyl, methoxy, ethoxy or methyl;

$X^1$ is N, CH, CF, C-$CH_3$;

$X^3$ is N, CH, CF, C-$CH_3$;

P is hydrogen, fluorine, cyano, methyl, isopropyl, tert-butyl, trifluoromethyl, morpholinyl,

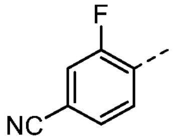

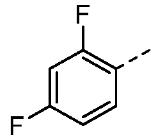

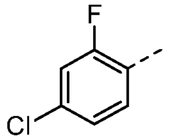

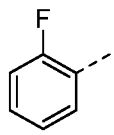.

The term "a pharmaceutically acceptable salt" refers to pharmaceutically acceptable organic or inorganic salts of the compounds of the present invention. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, gluconate, glucuronate, saccharate, formate, benzoate, glutamate, mesylate, ethanesulfonate, benzenesulfonate, p-toluenesulfonate, pamoate (i.e., 1,1'-methylene-bis-(2-hydroxy-3-naphthoic acid) salt), alkali metal (e.g., sodium and potassium) salts, alkaline earth metal (e.g., magnesium) salts, and ammonium salt. Pharmaceutically-acceptable salts may involve the inclusion of another molecule, such as acetate ions, succinate ions, or other counterions. The counterion may be any organic or inorganic moiety that stabilizes the charge on the parent compound. In addition, a pharmaceutically acceptable salt may have more than one charged atom in its structure. Examples where multiple charged atoms are part of a pharmaceutically acceptable salt may have multiple counterions. Thus, a pharmaceutically acceptable salt may have one or more charged atoms and/or one or more counterions.

If the compound of the present invention is a base, the desired pharmaceutically acceptable salt can be prepared by any suitable method available in the art, for example,by treating the free base with an inorganic acid (such as hydrochloric acid, hydrobromic acid, sulfuric acid, nitric acid, methanesulfonic acid, phosphoric acid) or the organic acid (such as acetic acid, maleic acid, succinic acid, mandelic acid, fumaric acid, malonic acid, pyruvic acid, oxalic acid, glycolic acid, salicylic acid, pyranosidic acid (such as glucuronic acid or galacturonic acid), $\alpha$-hydroxy acid (such as citric acid or tartaric acid), amino acid (such as aspartic acid or glutamic acid), aromatic acid (such as benzoic acid or cinnamic acid), sulfonic acid (such as p-toluenesulfonic acid or ethanesulfonic acid), and the like.)

If the compound of the present invention is an acid, the desired pharmaceutically acceptable salt can be prepared by any suitable method, for example, by treating the free acid with an inorganic or organic base, such as an amine (primary, secondary or tertiary amine), alkali metal hydroxide or alkaline earth metal hydroxide. Illustrative examples of suitable salts include, but are not limited to, organic salts derived from amino acids (such as glycine and arginine), ammonia, primary, secondary and tertiary amines and cyclic amines (such as piperidine, morpholine and piperazine), and inorganic salts derived from sodium, calcium, potassium, magnesium, manganese, iron, copper, zinc, aluminum and lithium.

The term "pharmaceutically acceptable" indicates that the substance or composition must be chemically and/or toxicologically compatible with other ingredients of the formulation and/or mammals treated therewith.

The compounds of the present application may exist in various stereoisomeric forms. The term "stereoisomer" refers to compounds having the same chemical composition and connectivity, but whose atoms have different orientations in space that cannot be interconverted by rotation about a single bond. "Stereoisomer" may include "diastereomer" and "enantiomer". "Diastereomer" refers to a stereoisomer having two or more chiral centers and whose molecules are not mirror images of each other. "Enantiomer" refers to two stereoisomers of a compound, which are non-overlapping mirror images of each other. "R" and "S" represent substituent configurations surrounding one or more chiral atoms. The compounds of the present application can be prepared as individual isomers by chiral synthesis or resolution from mixtures of isomers. The disclosed compounds may have one or more stereocenters, and each stereocenter may independently exist in an R or S configuration. When the absolute stereochemistry of the stereocenter is not determined, the stereochemistry configuration can be designated (*) at the designated center. In one embodiment, the compounds described herein are present in optically active or racemic form. It should be understood that the compounds described herein include racemic, optically active, regioisomeric and stereoisomeric forms or combinations thereof having the therapeutically useful properties described herein.

In one embodiment, the compounds described herein contain one or more chiral centers. These compounds can be prepared by any means, including stereoselective synthesis, enantioselective synthesis, or separation of mixtures of enantiomers or diastereomers. Resolution of compounds and their isomers can be achieved by any means, including but not limited to chemical processes, enzymatic processes, fractional crystallization, distillation and chromatography.

Definition

The term "substituted" means that any one or more hydrogen on the designated atom or group is replaced by a moiety selected from the designated group, provided that the designated atom's normal valence is not exceeded.

"Alkyl" is a branched or straight chain saturated aliphatic hydrocarbon group. In one embodiment, the alkyl group contains 1 to about 12 carbon atoms, more typically 1 to about 6 carbon atoms or 1 to about 4 carbon atoms. In one embodiment, the alkyl group contains 1 to about 8 carbon atoms. In certain embodiments, the alkyl group is $C_1$-$C_2$, $C_1$-$C_3$, or $C_1$-$C_6$. The specified ranges as used herein indicate an alkyl group having each member of the range described as an independent species. For example, the term $C_1$-$C_6$ alkyl as used herein indicates a straight or branched alkyl group having from 1, 2, 3, 4, 5, or 6 carbon atoms and is intended to mean that each of these is described as an independent species. For example, the term $C_1$-$C_4$ alkyl as used herein indicates a straight or branched alkyl group having from 1, 2, 3, or 4 carbon atoms and is intended to mean that each of these is described as an independent species. When $C_0$-$C_n$ alkyl is used herein in conjunction with another group, for example, ($C_3$-$C_7$ cycloalkyl) $C_0$-$C_4$ alkyl, or -$C_0$-$C_4$ alkyl ($C_3$-$C_7$ cycloalkyl), the indicated group, in this case cycloalkyl, is either directly bound by a single covalent bond ($C_0$ alkyl), or attached by an alkyl chain in this case 1, 2, 3, or 4 carbon atoms. Alkyls can also be attached via other groups such as heteroatoms as in -O-$C_0$-$C_4$ alkyl ($C_3$-$C_7$ cycloalkyl). Examples of alkyl include, but are not limited to, methyl, ethyl, n-propyl, isopropyl, n-butyl, isobutyl, sec-butyl, t-butyl, n-pentyl, isopentyl, tertpentyl, neopentyl, n-hexyl, 2-methylpentyl, 3-methylpentyl, 2,2-dimethylbutyl and 2,3-dimethylbutyl. In one embodiment, the alkyl group is optionally substituted as described above.

"Alkenyl" is a branched or straight chain aliphatic hydrocarbon group having one or more carbon-carbon double bonds that may occur at a stable point along the chain. Non-limiting examples are $C_2$-$C_8$ alkenyl, $C_2$-$C_6$ alkenyl and $C_2$-$C_4$ alkenyl. The specified ranges as used herein indicate an alkenyl group having each member of the range described as an independent species, as described herein for the alkyl moiety. Examples of alkenyl include, but are not limited to, ethenyl, propenyl. In one embodiment, the alkenyl group is optionally substituted as described above.

"Alkynyl" is a branched or straight chain aliphatic hydrocarbon group having one or more carbon-carbon triple bonds that may occur at any stable point along the chain, for example, $C_2$-$C_8$ alkynyl or $C_2$-$C_6$ alkynyl. The specified ranges as used herein indicate an alkynyl group having each member of the range described as an independent species, as described herein for the alkyl moiety. Examples of alkynyl include, but are not limited to, ethynyl, propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl. In one embodiment, the alkynyl group is optionally substituted as described above.

"Alkoxy" is an alkyl group as defined above covalently bound through an oxygen bridge (-O-). Examples of alkoxy include, but are not limited to, methoxy, ethoxy, n-propoxy, i-propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3-methylpentoxy. Similarly an "alkylthio" or a "thioalkyl" group is an alkyl group as defined above with the indicated number of carbon atoms covalently bound through a sulfur bridge (-S-). In one embodiment, the alkoxy group is optionally substituted as described above.

"Alkenyloxy" is an alkenyl group as defined above covalently bound to the group it substitutes by an oxygen bridge (-O-).

"Alkanoyl" is an alkyl group as defined above covalently bound through a carbonyl (C=O) bridge. The carbonyl

carbon is included in the number of carbons, that is $C_2$ alkanoyl is a $CH_3(C=O)$- group. In one embodiment, the alkanoyl group is optionally substituted as described above.

"Alkylester" is an alkyl group as defined covalently bound through an ester linkage. The ester linkage may be in either orientation, e.g., a group of the formula -O(C=O)alkyl or a group of the formula -(C=O)Oalkyl.

"Carbocyclyl", "carbocyclic ring", or "cycloalkyl" is a saturated or partially unsaturated (i.e., not aromatic) group containing all carbon ring atoms. A carbocyclic group typically contains 1 ring of 3 to 7 carbon atoms or 2 fused rings each containing 3 to 7 carbon atoms. Cycloalkyl substituents may be pendant from a substituted nitrogen or carbon atom, or a substituted carbon atom that may have two substituents can have a cycloalkyl group, which is attached as a spiro group. Examples of carbocyclic rings include cyclohexenyl, cyclohexyl, cyclopentenyl, cyclopentyl, cyclobutenyl, cyclobutyl and cyclopropyl rings. In one embodiment, the carbocyclic ring is optionally substituted as described above. In one embodiment, the cycloalkyl is a partially unsaturated (i.e., not aromatic) group containing all carbon ring atoms.

"Carbocycle-oxy group" is a monocyclic carbocyclic or mono- or bi-cyclic carbocyclic group as described above attached to the group it substitutes by an oxygen-O-linker.

"Haloalkyl" indicates both branched and straight-chain alkyl groups substituted with 1 or more halogen atoms, up to the maximum allowable number of halogen atoms. Examples of haloalkyl include, but are not limited to, trifluoromethyl, monofluoromethyl, difluoromethyl, 2-fluoroethyl, and penta-fluoroethyl.

"Haloalkoxy" indicates a haloalkyl group as defined herein attached through an oxygen bridge (oxygen of an alcohol radical).

"Hydroxyalkyl" is an alkyl group as previously described substituted with at least one hydroxy substituent.

"Aminoalkyl" is an alkyl group as previously described substituted with at least one amino substituent.

"Halo" indicates independently any of fluoro, chloro, bromo, and iodo.

"Aryl" indicates aromatic groups containing only carbon in the aromatic ring or rings. In one embodiment, the aryl groups contain 1 to 3 separate or fused rings and is 6 to about 18 ring atoms (such as 6, 7, 8, 9, 10, 11, 12, 13, 14, 15, 16, 17, or 18 ring atoms), without heteroatoms as ring members. When indicated, such aryl groups may be further substituted with carbon or non-carbon atoms or groups. Such substitution may include fusion to a 5 to 7-membered saturated cyclic group that optionally contains 1 or 2 heteroatoms independently chosen from N, O, and S, to form, for example, a 3,4-methylenedioxyphenyl group. Aryl groups include, for example, phenyl, naphthyl, including 1-naphthyl and 2-naphthyl. In one embodiment, aryl groups are pendant. An example of a pendant ring is a phenyl group substituted with a phenyl group. In one embodiment, the aryl group is optionally substituted as described above. Aryl groups include bicyclic groups comprising an aromatic ring fused to a saturated, partially unsaturated ring or an aromatic carbocyclic or heterocyclic ring. Typical aryl groups include, but are not limited to, those derived from benzene (phenyl), substituted benzene, naphthalene, anthracene, indenyl, indanyl, 1,2-dihydronaphthalene, 1,2,3,4-tetrahydro groups and the like. Preferably, the aryl group is a phenyl group.

The term "heterocyclyl" as used herein refers to a saturated or a partially unsaturated (i.e., having one or more double and/or triple bonds within the ring without aromaticity) carbocyclic radicals of 3 to 18 (such as 3, 4, 5, 6, 7, 8, 9, 10, 11, 12, 13, 14, 15, 16, 17, or 18) ring atoms wherein at least one ring atom is a heteroatom selected from nitrogen, oxygen, phosphorus and sulfur, the remaining ring atoms being C, where one or more ring atoms is optionally substituted independently with one or more substituents described above. A heterocycle may be a monocycle having 3 to 7 ring members (2 to 6 carbon atoms and 1 to 4 heteroatoms selected from N, O, P and S) or a bicycle having 6 to 10 ring members (4 to 9 carbon atoms and 1 to 6 heteroatoms selected from N, O, P and S), for example: a bicyclo [4,5], [5,5], [5,6], or [6,6] system. In one embodiment, the only heteroatom is nitrogen. In one embodiment, the only heteroatom is oxygen. In one embodiment, the only heteroatom is sulfur. Heterocycles are described in Paquette, Leo A.; "Principles of Modern Heterocyclic Chemistry" (W. A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Chapters 13, 14, 16, 19, and 28; and J. Am. Chem. Soc. (1960) 82:5566. Examples of heterocyclic rings include, but are not limited to, pyrrolidinyl, dihydrofuranyl, tetrahydrothienyl, tetrahydropyranyl, dihydropyranyl, tetrahydrothiopyranyl, piperidino, piperidonyl, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, dihydroisoquinolinyl, tetrahydroisoquinolinyl, pyrazolidinylimidazolinyl, imidazolidinyl, 2-oxa-5-azabicyclo[2.2.2]octane, 3-oxa-8-azabicyclo[3.2.1]octane, 8-oxa-3-azabicyclo[3.2.1]octane, 6-oxa-3-azabicyclo[3.1.1]heptane, 2-oxa-5-azabicyclo[2.2.1]heptane, 3-azabicyco[3.1.0]hexanyl, 3-azabicyclo[4.1.0]heptanyl, azabicyclo[2.2.2]hexanyl, 3H-indolyl, quinolizinyl, N-pyridyl ureas, and pyrrolopyrimidine. Spiro moieties are also included within the scope of this definition. Examples of a heterocyclic group wherein 1 or 2 ring carbon atoms are substituted with oxo (=O) moieties are pyrimidinonyl and 1,1-dioxo-thiomorpholinyl. The heterocycle groups herein are optionally substituted independently with one or more substituents described herein. "Heterocyclyl" includes "heterocycloalkyl". "Heterocycloalkyl" is a saturated ring group. It may have, for example, 1, 2, 3 or 4 heteroatoms independently selected from N, S and O, the remaining ring atoms being carbon. In a typical embodiment, nitrogen is a heteroatom. Monocyclic heterocycloalkyl groups typically have 3 to about 8 ring atoms or 4 to 6 ring atoms. Examples of heterocycloalkyl groups include morpholino, piperazinyl, piperidino, and pyrrolinyl. "Heterocyclyl" may contain 1, 2, 3, 4, or 5 heteroatoms selected from N, O, and S.

"Heterocyclicoxy group" is a monocyclic heterocyclic ring or a bicyclic heterocyclic group as described previously linked to the group it substitutes via an oxygen-O-linker.

"Heteroaryl" can be a stable monocyclic aromatic ring which contains from 1 to 3, or in some embodiments from 1 to 2, heteroatoms chosen from N, O, and S, with remaining ring atoms being carbon, or a stable bicyclic or tricyclic system containing at least one 5- to 10-membered (5, 6, 7, 8, 9, 10 membered) aromatic rings which contains from 1 to 3, or in some embodiments from 1 to 2, heteroatoms chosen from N, O, and S, with remaining ring atoms being carbon. In one embodiment, the only heteroatom is nitrogen. In one embodiment, the only heteroatom is oxygen. In one embodiment, the only heteroatom is sulfur. Monocyclic heteroaryl groups typically have from 5 to 8 ring atoms (5, 6, 7, 8 ring atoms). In some embodiments, bicyclic heteroaryl groups are 9- to 10-membered heteroaryl groups, that is, groups containing 9 or 10 ring atoms wherein one 5- to 7-membered aromatic ring is fused to a second aromatic or nonaromatic ring. When the total number of S and O atoms in the heteroaryl group exceeds 1, these heteroatoms are not adjacent to one another. In one embodiment, the total number of S and O atoms in the heteroaryl group is not more than 2. In another embodiment, the total number of S and O atoms in the aromatic heterocycle is not more than 1. Examples of heteroaryl groups include, but are not limited to, pyridinyl (including, for example, 2-hydroxypyridinyl), imidazolyl, imidazopyridinyl, pyrimidinyl (including, for example, 4-hydroxypyrimidinyl), pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, tetrahydrofuranyl, and furopyridinyl. Heteroaryl groups are optionally substituted independently with one or more substituents described herein. "Heteroaryloxy" is a heteroaryl group as described bound to the group it substituted via an oxygen-O-linker. "Heteroaryl" may contain 1, 2, 3, 4, or 5 heteroatoms selected from N, O, and S.

The present application also covers bicyclic, tricyclic, tetracyclic, pentacyclic and other polycyclic ring systems formed by one or more monocyclic rings selected from "cycloalkyl", "aryl" and "heteroaryl" in a valence-allowed manner.

The term cyano refers to -CN.

The term "monocyclic, bicyclic or tricyclic saturated or unsaturated ring system having 5-14 ring atoms" means that the ring system has 5-14 ring atoms (eg 5, 6, 7, 8, 9, 10, 11, 12, 13, or 14 ring atoms); the ring system contains one, two, or three rings; each ring in the ring system can independently be saturated, unsaturated, or even aromatic ring. Preferably, each of the foregoing saturated, unsaturated, and aromatic rings may contain ring heteroatoms.

Applicants have found that the compound or a pharmaceutically acceptable salt thereof of the present application can effectively degrade IKZF. Accordingly, the compounds or salts thereof described herein are useful in the treatment of proliferative disorders. Furthermore, Applicants have also discovered that the compounds or salts thereof described herein do not simultaneously inhibit/degrade SALL4 when degrading IKZF. In other words, the compounds or salts thereof described herein can selectively degrade IKZF with reduced or no effect on other proteins/kinases, thereby exhibiting reduced or no toxic side effects compared to previous IKZF degraders/inhibitors.

The compounds or a pharmaceutically acceptable salt thereof described herein are useful in the treatment of proliferative disorders. The present invention also provides the use of the compound or a pharmaceutically acceptable salt thereof described herein in the manufacture of a medicament for the treatment of proliferative disorders. The present invention further provides the compounds or a pharmaceutically acceptable salt thereof for use in the treatment of proliferative disorders. The application further provides a method of treating proliferative disorders, comprising administering to a subject in need thereof a therapeutically effective amount of the compound or a pharmaceutically acceptable salt thereof.

The term "proliferative disorder" or "cell proliferative disorder" refers to disorders that are associated with some degree of abnormal cell proliferation, whether malignant or benign. In some embodiments, the proliferative disorder is a cancer. In some aspects, the cancer is a solid tumor. In some aspects, the cancer is a hematologic malignancy. The terms "proliferative disorder", "cell proliferative disorder", "cancer", "cancerous", and "tumor" are not mutually exclusive as referred to herein.

The term "cancer" comprises cancerous cells, and/or benign or pre-cancerous cells. Examples of cancer include, but are not limited to, breast cancer, colon cancer, brain cancer, prostate cancer, kidney cancer, pancreatic cancer, ovarian cancer, head and neck cancer, melanoma, colorectal cancer, gastric cancer, squamous cancer, small-cell lung cancer, non small-cell lung cancer, testicular cancer, Merkel cell carcinoma, glioblastoma, neuroblastoma, cancers of lymphatic organs and hematological malignancy including Leukemia (Acute lymphoblastic leukemia (ALL), Acute myelogenous leukemia (AML), Chronic lymphocytic leukemia (CLL), Chronic myelogenous leukemia (CML), Acute monocytic leukemia (AMOL), Hairy cell leukemia (HCL), T-cell prolymphocytic leukemia (T-PLL), Large granular lymphocytic leukemia, Adult T-cell leukemia), Lymphoma (small lymphocytic lymphoma (SLL), Hodgkin's lymphomas (Nodular sclerosis,

Mixed cellularity, Lymphocyte-rich, Lymphocyte depleted or not depleted, and Nodular lymphocyte-predominant Hodgkin lymphoma), Non-Hodgkin's lymphomas (all subtypes), Chronic lymphocytic leukemia/Small lymphocytic lymphoma, B-cell prolymphocytic leukemia, Lymphoplasmacytic lymphoma (such as Waldenström macroglobulinemia), Splenic marginal zone lymphoma, Plasma cell neoplasms (Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma, Burkitt lymphoma/leukemia, T cell prolymphocytic leukemia, T cell large granular lymphocytic leukemia, Aggressive NK cell leukemia, Adult T cell leukemia/lymphoma, Extranodal NK/T cell lymphoma (nasal type), Enteropathy-type T cell lymphoma, Hepatosplenic T cell lymphoma, Blastic NK cell lymphoma, Mycosis fungoides / Sezary syndrome, Primary cutaneous CD30-positive T cell lymphoproliferative disorders, Primary cutaneous anaplastic large cell lymphoma, Lymphomatoid papulosis, Angioimmunoblastic T cell lymphoma, Peripheral T cell lymphoma (unspecified), Anaplastic large cell lymphoma), multiple myeloma (plasma cell myeloma or Kahler's disease). The term "cancer" also includes leukemia, carcinoma and sarcoma. Exemplary cancers include brain cancer, breast cancer, cervical cancer, colon cancer, head and neck cancer, liver cancer, kidney cancer, lung cancer, non-small cell lung cancer, melanoma, mesothelioma, ovarian cancer, sarcoma, stomach cancer, uterine cancer and Medulloblastoma. Additional examples include Hodgkin's disease, non-Hodgkin's lymphoma, multiple myeloma, neuroblastoma, ovarian cancer, rhabdomyosarcoma, essential thrombocythemia, primary macroglobulinemia, primary brain tumor, cancer, malignant pancreatic insulinoma, malignant carcinoid, bladder cancer, premalignant skin lesions, testicular cancer, lymphoma, thyroid cancer, neuroblastoma, esophageal cancer, genitourinary tract cancer, malignant hypercalcemia , endometrial cancer, adrenocortical carcinoma, tumors of the endocrine and exocrine pancreas, and prostate cancer. The term "cancer"also includes leukemia, multiple myeloma, lymphoma, liver cancer, stomach cancer, breast cancer, cholangiocarcinoma, pancreatic cancer, lung cancer, colorectal cancer, osteosarcoma, melanoma, human cervical cancer, glioma, nasopharyngeal cancer, laryngeal cancer, esophageal cancer, middle ear tumor, prostate cancer, etc.

The present invention further provides a pharmaceutical composition comprising the compound or a pharmaceutically acceptable salt thereof described herein, and a pharmaceutically acceptable carrier. The pharmaceutical composition described herein may be a tablet, a capsule, a granule, a syrup, a suspension, a solution, a dispersion, a slow release preparation for oral or non-oral administration, an intravenous injection preparation, a subcutaneous injection preparation, an inhalation preparation, a transdermal preparation, a rectal or vaginal suppository.

The pharmaceutically acceptable carrier described herein refers to a pharmaceutically acceptable carrier well known to those skilled in the art, and the pharmaceutically acceptable carrier of the present invention includes, but is not limited to, a filler, a wetting agent, a binder, a disintegrating agent, a lubricant, a binder, a glidant, a flavoring agent, a surfactant, a preservative, and the like. Fillers include, but are not limited to, lactose, microcrystalline cellulose, starch, powdered sugar, dextrin, mannitol, calcium sulfate, and the like. Wetting agents and binders include, but are not limited to, sodium carboxymethylcellulose, hydroxypropylcellulose, hydroxypropyl methyl cellulose, gelatin, sucrose, polyvinylpyrrolidone, and the like. Disintegrating agents include, but are not limited to, sodium carboxymethyl starch, crosslinked polyvinylpyrrolidone, croscarmellose sodium, low substituted hydroxypropylcellulose, and the like. Lubricants include, but are not limited to, magnesium stearate, aerosil, talc, hydrogenated vegetable oil, polyethylene glycol, magnesium lauryl sulfate, and the like. Binders include, but are not limited to, gum arabic, alginic acid, calcium carboxymethylcellulose, sodium carboxymethylcellulose, glucose, dextrin, dextrose, ethylcellulose, gelatin, liquid glucose, guar gum, hydroxyethyl cellulose, hydroxypropyl cellulose, hydroxypropyl methyl cellulose, magnesium aluminum silicate, maltodextrin, methyl cellulose, polymethacrylate, polyvinylpyrrolidone, pregelatinized starch, sodium alginate, sorbitol, starch, syrup and tragacanth. Glidants include, but are not limited to, colloidal silica, powdered cellulose, magnesium trisilicate, silica, and talc. Flavoring agents include, but are not limited to, aspartame, stevioside, fructose, glucose, syrup, honey, xylitol, mannitol, lactose, sorbitol, maltitol, glycyrrhizin. Surfactants include, but are not limited to, Tween-80, poloxamer. Preservatives include, but are not limited to, paraben, sodium benzoate, potassium sorbate, and the like.

Methods of preparing various pharmaceutical compositions containing various amounts of active ingredients are known, or will be apparent to those skilled in the art in light of this disclosure, as described in REMINGTON'S PHARMACEUTICAL SCIENCES, Martin, E.W., ed., Mack Publishing Company, 19th ed. (1995). Methods of preparing the pharmaceutical compositions include incorporation of suitable pharmaceutical excipients, carriers, diluents and the like. The pharmaceutical compositions described herein are made in a known manner, including conventional methods of mixing, dissolving or lyophilizing.

In the pharmaceutical compositions described herein, the amount of active ingredient may vary from about 0.01% to about 99% by weight of a given unit dosage form. In such therapeutically useful pharmaceutical composition formulations, the active ingredient is in an amount such that an effective dosage level can be obtained.

The tablet, capsule and the like described herein may comprise: a binder such as tragacanth, gum arabic, corn starch or gelatin; an excipient such as dicalcium phosphate; a disintegrating agent such as corn starch, potato starch, alginic acid, etc.; a lubricant such as magnesium stearate; and a sweetener such as sucrose, fructose, lactose or

aspartame; or a flavoring agent such as mint, wintergreen or cherry flavor. When the unit dosage form is a capsule, it may contain, in addition to materials of the above types, a liquid carrier such as vegetable oil or polyethylene glycol. Various other materials may be present, as a coating, or otherwise alter the physical form of the solid unit dosage form. For example, tablets or capsules may be coated with gelatin, wax, shellac or sugar, etc. The syrup may contain an active ingredient, sucrose or fructose as a sweetener, methylparaben or propylparaben as a preservative, a dye and a flavoring agent such as cherry or orange flavor. Of course, any material used to prepare any unit dosage form should be pharmaceutically acceptable and non-toxic in the amounts employed. In addition, the active ingredient can be incorporated into a slow release formulations and a slow release device.

The active ingredient can also be administered intravenously or intraperitoneally by infusion or injection. An aqueous solution of the active ingredient or a salt thereof can be prepared, optionally with a non-toxic surfactant. Dispersions in glycerol, liquid polyethylene glycol, triacetin and mixtures thereof, and oils can also be prepared. Under ordinary conditions of storage and use, these formulations contain a preservative to prevent the growth of microorganisms.

Pharmaceutical composition dosage forms suitable for injection or infusion may comprise sterile aqueous solution or dispersion or sterile powder comprising an active ingredient suitable for the ready-to-use preparation of a sterile, injectable or infusible solution or dispersion (optionally encapsulated in a liposome). In all cases, the final dosage forms must be sterile, liquid, and stable under the conditions of manufacture and storage. The liquid carrier may be a solvent or liquid dispersion medium including, for example, water, ethanol, polyols (for example, glycerol, propylene glycol, liquid polyethylene glycol, and the like), vegetable oils, non-toxic glycerides, and suitable mixtures thereof. Proper fluidity can be maintained, for example, by liposome formation, by maintaining the desired particle size in the case of dispersion, or by the use of a surfactant. The prevention of microorganisms can be achieved by using various antibacterial and antifungal agents such as parabens, chlorobutanol, phenol, sorbic acid, thimerosal, and the like. In many cases, it will be preferred to include isotonic agents, such as sugars, buffers or sodium chloride. Prolonged absorption of the injectable composition can be brought about by the use of a composition that comprises an absorption delaying agent (for example, aluminum monostearate and gelatin).

Sterile injectable solutions are prepared by combining the active ingredient in a desired amount in a suitable solvent with the various other ingredients listed above, followed by filter sterilization. In the case of sterile powders for the preparation of sterile injectable solutions, the preferred preparation methods are vacuum drying and lyophilization techniques which result in powders of the active ingredient plus any additionally desired ingredients present in the sterile filtration solution.

Useful solid carriers include comminuted solids (e.g., talc, clay, microcrystalline cellulose, silica, alumina, etc.). Useful liquid carriers include water, ethanol or ethylene glycol or a water-ethanol/ethylene glycol mixture, and the pharmaceutical compositions of the present invention may be dissolved or dispersed in the liquid carriers in an effective amount, optionally with the aid of a non-toxic surfactant. Adjuvants (such as fragrances) and additional antimicrobial agents can be added to optimize the properties for a given use.

Thickeners (such as synthetic polymers, fatty acids, fatty acid salts and esters, fatty alcohols, modified celluloses or modified inorganic materials) can also be used with liquid carriers to form coatable pastes, gels, ointments, soaps, etc. that are used directly on the user’s skin.

The therapeutically effective amount of the active ingredient will depend not only on the particular salt selected, but also on the administration route, the property of the disease to be treated, and the age and conditions of the patient, and will ultimately depend on the decision of the attending physician or clinician.

The above formulations may be presented in unit dosage form, which is a physically discrete unit containing a unit dose suitable for administration to humans and other mammalian bodies. The unit dosage form may be a capsule or a tablet. The amount of active ingredient in the unit dose may vary or be adjusted between about 0.01 to about 1000 mg or more, depending on the particular treatment involved.

The term "treatment" as used herein generally refers to the acquisition of the desired pharmacological and/or physiological effect. The effect may be prophylactic according to the prevention of the disease or its symptoms in whole or in part; and/or may be therapeutic according to the partial or complete stabilization or cure of the disease and/or the side effect due to the disease. As used herein, "treatment" encompasses any treatment for the disease of a patient, including: (a) prevention of the disease or condition in the patient that may be predisposed to the disease or condition but has not yet been diagnosed; (b) inhibition of the symptoms of the disease, i.e., preventing its development; or (c) remission of the symptoms of the disease, i.e., causing regression of the disease or symptoms in whole or in part. The compound or a pharmaceutically acceptable salt thereof described herein may also be administered in combination with one or more additional therapeutic agents for the treatment of cancers. Such additional therapeutic agents include, but are not limited to, anthracyclines, cyclophosphamide, 5-fluorouracil, cisplatin, and the like.

Unless otherwise stated, percentages, ratios, proportions or parts used in this application are by weight or volume. The amount used in this application is a weight or volume amount. Those skilled in the art can easily determine.

In yet another aspect of the present application, the present application further includes the compound or a pharmaceutically acceptable salt thereof obtained by combining any group defined at any variable group of the present

application.

The present application also includes the following exemplary compounds.

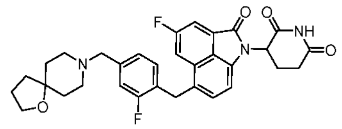
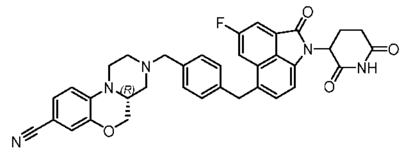
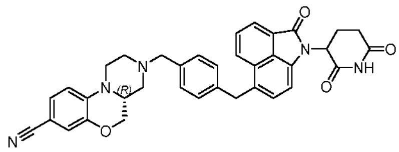
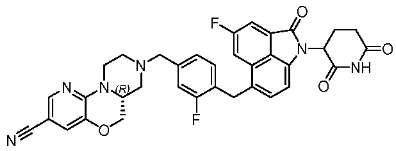
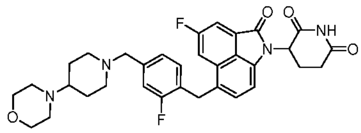
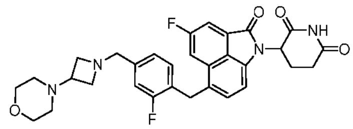
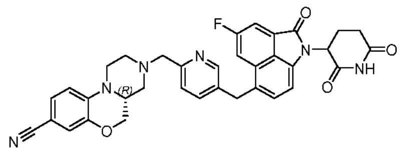
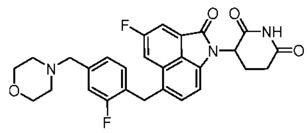
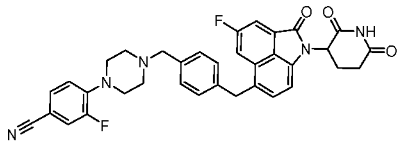
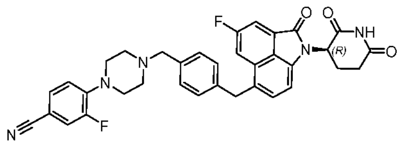
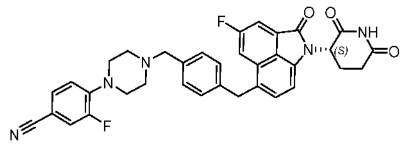
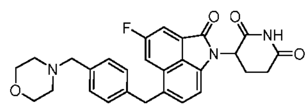
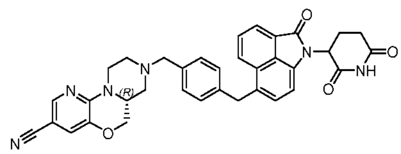
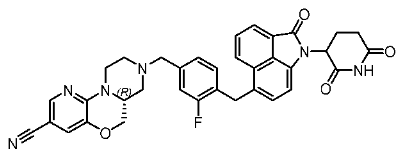
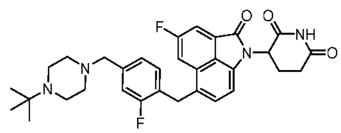
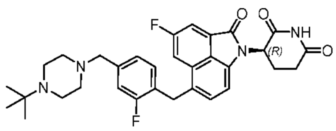
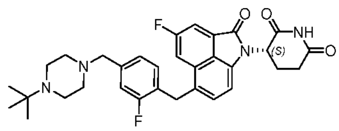
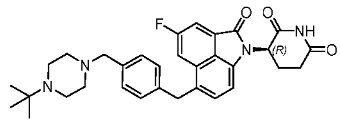
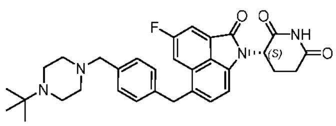
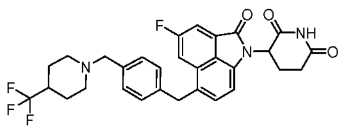
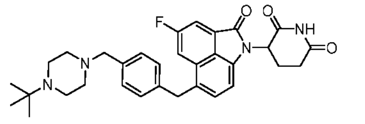
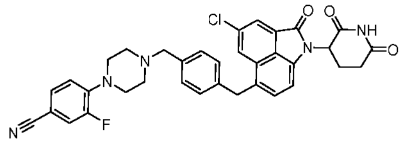
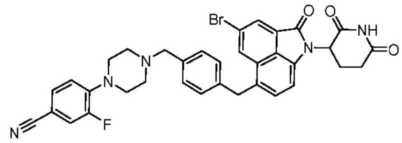
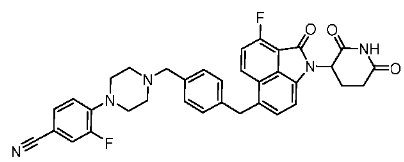
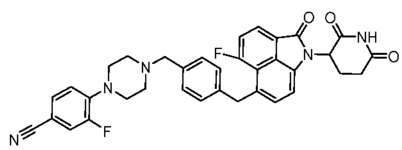

Hereinafter, the present application will illustrate the beneficial effects of the present application by Examples.

Those skilled in the art will recognize that these Examples are illustrative and not limiting. These Examples do not limit the scope of the present application in any way. The experimental methods described in the following Examples are conventional unless otherwise specified. Unless otherwise specified, reagents and materials are commercially available.

## Examples.

The compounds of the present application can be synthesized according to the following exemplary general schemes or the like. Under the premise of the existence of valence, various intermediates can have various substituents described in this application.

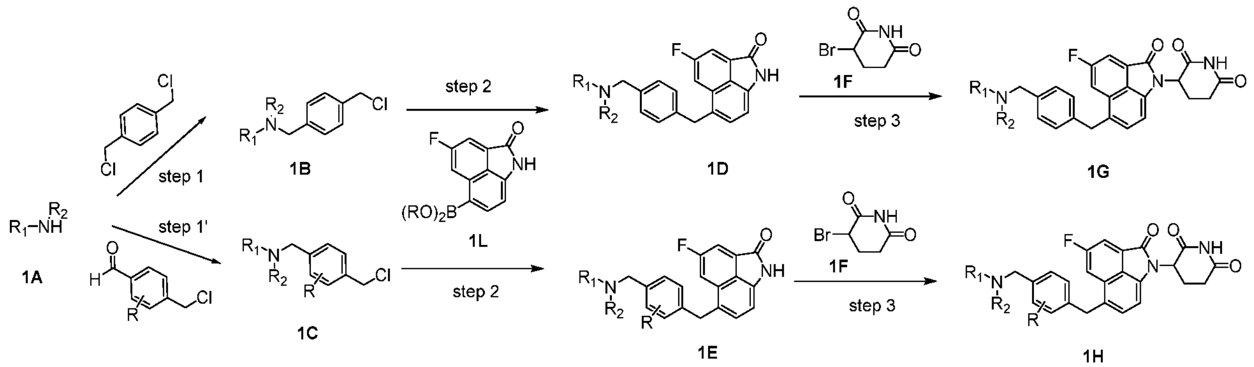

1A 1B 1C 1L 1D 1E 1F 1G 1H

Compound **1A** and chlorotoluene react to produce compound **1B** in the presence of a base, or compound **1A** and aromatic aldehyde react to produce compound **1C** through Borch reduction. Compound **1B** (or **1C)** and compound **1L** react to produce compound **1D** (or **1E)** through coupling reaction in the presence of a catalyst, then compound **1D** (or **1E)** and compound **1F** react to produce compound **1G** (or **1H)** in the presence of a base.

Compound **1A** can also be a commercially available spiro ring, bicyclic ring, can also be a spiro ring, bicyclic ring synthesized according to known methods, and can also be a ring system synthesized according to the following exemplary general synthetic methods or similar methods.

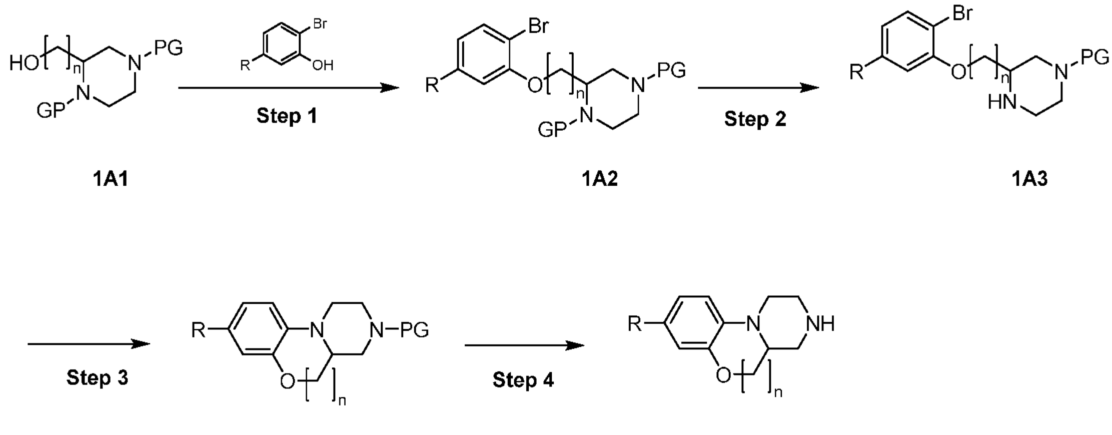

1A1 1A2 1A3 1A4 1A

Compound **1A2** can be made from a compound **1A1** and a compound of substituted phenol, in a Mitsunobu reaction. Compound **1A4** can be made from a compound **1A2,** in a deprotection, and a coupling reaction. Compound **1A** can be made from a compound **1A4,** in a deprotection reaction.

Synthesis of key intermediate **B1**

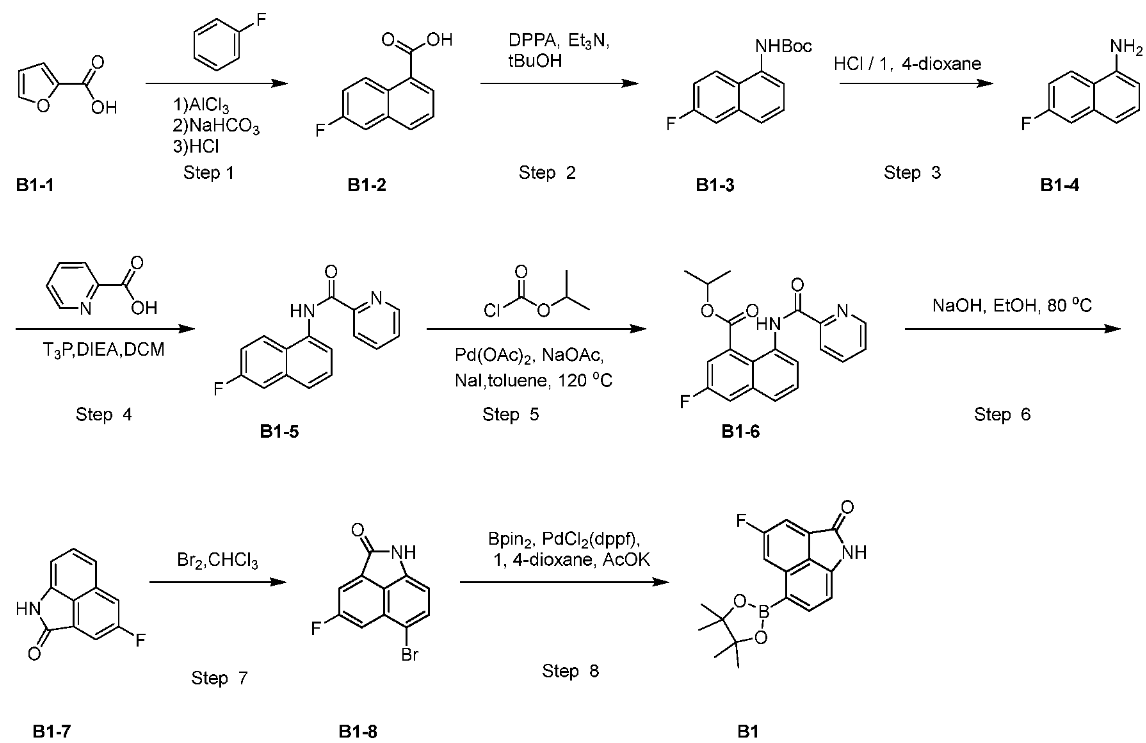


B1-1 B1-2 B1-3 B1-4 B1-5 B1-6 B1-7 B1-8 B1

**Step 1.** Synthesis of Intermediate **B1-2**

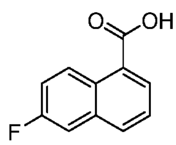

**B1-2**

To a solution of intermediate **B1-1** (10 g, 89.2 mmol) in fluorobenzene (51.0 g, 531 mmol, 50 mL) was added $AlCl_3$ (22 g, 165 mmol) slowly at 0°C. The reaction mixture was stirred at 0°C for 1 hour, then slowly heated to 75°C, stirred for 18 hours. The reaction mixture was cooled to room temperature, poured into 2N HCl (500 mL), extracted with EtOAc (300 mL x 3). The organic phase was washed with $H_2O$ (300 mL), extracted with sat. $NaHCO_3$ (300 mL x 3). The water phase was adjusted pH to 2-3, extracted with EtOAc (300 mL x 2). The organic phase was dried over $Na_2SO_4$, filtered, and concentrated to give a crude. Intermediate **B1-2** (7.3 g, 38.39 mmol, 43.0% yield) was obtained as a yellow solid. The crude product was used for the next step without further purification. $^1$H NMR (400 MHz, $CDCl_3$) $\delta$ 9.04 - 8.62 (m, 1H), 8.32 - 8.11 (m, 1H), 8.04 - 7.78 (m, 1H), 7.54 - 7.40 (m, 2H), 7.36 - 7.29 (m, 1H).

**Step 2.** Synthesis of Intermediate **B1-3**

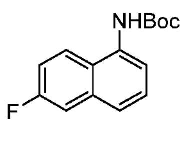

**B1-3**

To a solution of intermediate **B1-2** (1 g, 5.26 mmol) and TEA (1.65 g, 16.30 mmol, 2.27 mL) in t-BuOH (10 mL) was added DPPA (1.55 g, 5.62 mmol, 1.22 mL) at 20 °C. The reaction mixture was stirred at 100 °C for 17 hours under $N_2$ atmospheres. The reaction mixture was concentrated in vacuo to give a crude. The residue was purified by column chromatography (Petroleum ether/Ethyl acetate gradient = 1/0 to 10/1). Intermediate **B1-3** (1.10 g, 80.1% yield) was obtained as a white solid.
LCMS (ESI$^+$): m/z 206.99 [M+H-56]$^+$.

**Step 3.** Synthesis of Intermediate **B1-4**

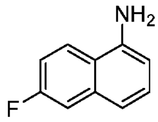

**B1-4**

To a solution of intermediate **B1-3** (1.10 g, 4.21 mmol) in DCM (10 mL) was added HCl/dioxane (10 mL, 4M). The reaction mixture was stirred 20 °C for 2 hours. The reaction mixture was concentrated to dryness under reduced pressure to give intermediate **B1-4** (770 mg, crude) as a white solid. The crude product was used for the next step without further purification.
LCMS (ESI$^+$): m/z 161.96 [M+H]$^+$.

**Step 4.** Synthesis of Intermediate **B1-5**

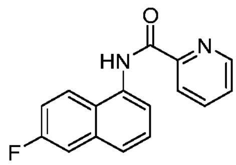

**B1-5**

To a mixture of pyridine-2-carboxylic acid (500 mg, 4.06 mmol), hydrochloride salt of intermediate **B1-4** (770 mg, 3.90 mmol) and DIEA (1.51 g, 11.7 mmol, 2.04 mL) in DCM (5 mL) was added $T_3P$ (3.72 g, 5.84 mmol, 3.48 mL). The reaction mixture was stirred at 20 °C for 18 hours. The reaction mixture was concentrated to dryness under reduced pressure to give a residue. The residue was purified by column chromatography (Petroleum ether/Ethyl acetate=1/1 to 5/1). Intermediate **B1-5** (860 mg, 69.6% yield) was obtained as a white solid.
LCMS (ESI$^+$): m/z 267.1 [M+H]$^+$.

**Step 5.** Synthesis of Intermediate **B1-6**

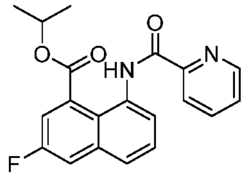

**B1-6**

A mixture of intermediate **B1-5** (840 mg, 3.15 mmol), isopropyl carbonochloridate (1.16 g, 9.46 mmol, 1.31 mL), $Pd(OAc)_2$ (70 mg, 311.79 μmol), NaI (480 mg, 3.20 mmol) and NaOAc (520 mg, 6.34 mmol) in toluene (10 mL)

was displaced with $N_2$ for 3 times, and then the mixture was stirred at 120 °C for 24 hours under $N_2$ atmosphere. The reaction mixture was filtered and concentrated to dryness under reduced pressure to give a residue. The residue was purified by column chromatography (Petroleum ether/Ethyl acetate gradient = 1/0 to 5/2) to give intermediate **B1-6** (660 mg, 44.55% yield) as a yellow solid. LCMS (ESI+): m/z 353.0 $[M+H]^+$.

**Step 6.** Synthesis of Intermediate **B1-7**

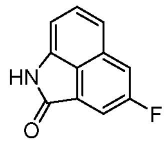

**B1-7**

To a solution of intermediate **B1-6** (660 mg, 1.87 mmol) in EtOH (15 mL) was added NaOH (224.75 mg, 5.62 mmol). The reaction mixture was stirred at 80 °C for 18 hours. The reaction mixture was concentrated to dryness under reduced pressure to give a crude, then added $H_2O$ (5 mL), adjust pH to 2 by 1N HCl, extracted with EtOAc (15 mL x 3). The organic phase was washed with sat. $NaHCO_3$ (15 mL x 2), dried over $Na_2SO_4$, filtered, and concentrated to dryness under reduced pressure to give a crude. The residue was purified by column chromatography (Petroleum ether/Ethyl acetate = 1/0 to 3/1) to give intermediate **B1-7** (110 mg, 27.30% yield) as a yellow solid.

**Step 7.** Synthesis of Intermediate **B1-8**

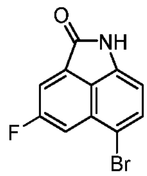

**B1-8**

To a solution intermediate **B1-7** (110 mg, 588 μmol) in $CHCl_3$ (5 mL) was added $Br_2$ (93.9 mg, 588 μmol, 30.30 μL) in $CHCl_3$ (3 mL) dropwise for 3 min. The reaction mixture was stirred at 20 °C for 20 hours. The reaction mixture was concentrated to dryness under reduced pressure to give intermediate **B1-8** (156 mg, crude) as a yellow solid, which was used for the next step without further purification.

**Step 8.** Synthesis of Intermediate **B1-9**

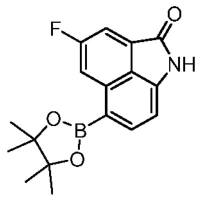

**B1**

A mixture of intermediate **B1-8** (150 mg, 564 μmol), 4,4,5,5-tetramethyl-2-(4,4,5,5-tetramethyl-1,3,2-dioxaborolan-2-yl)-1,3,2-dioxaborolane (150 mg, 591 μmol), KOAc (166 mg, 1.69 mmol), $Pd(dppf)Cl_2$ (41.25 mg, 56.4 μmol) in dioxane (5 mL) was stirred at 100°C for 16 hours under $N_2$ atmospheres. The reaction mixture was concentrated to dryness under reduced pressure to give a crude. The residue was purified by column chromatography (Petroleum ether/Ethyl acetate=8/1 to 5/1) to give intermediate **B1-9** (134 mg, 56.2% yield) as a yellow solid. The crude product was used for the next step without further purification.
LCMS (ESI+): m/z 314.0 $[M+H]^+$.

Synthesis of Intermediate **B2**

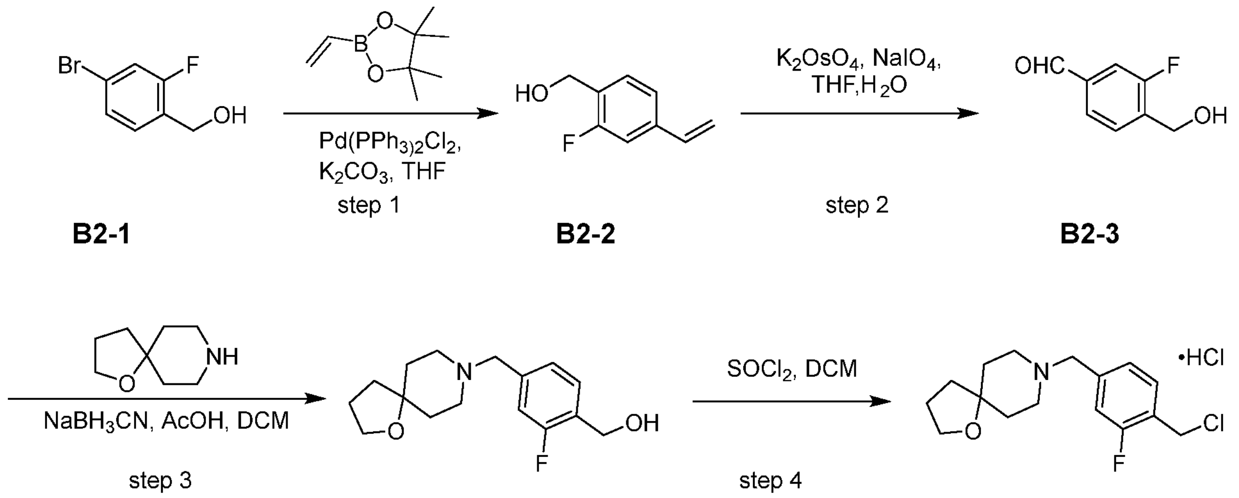


**B2-1** **B2-2** **B2-3**

**B2-4** **B2**

**Step 1.** Synthesis of Intermediate **B2-2**

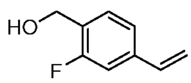

**B2-2**

To a solution of intermediate **B2-1** (5.00 g, 24.4 mmol), 4,4,5,5-tetramethyl-2-vinyl-1,3,2-dioxaborolane (5.63 g, 36.6 mmol) and $K_2CO_3$ (10.3 g, 74.4 mmol) in dioxane (50 mL) and $H_2O$ (5 mL) was added $Pd(dppf)Cl_2$ (1.07 g, 1.46 mmol). The reaction mixture was degassed and purged with $N_2$ for 3 times, and then stirred at 90 °C for 16 h. The reaction mixture was cooled to room temperature and quenched with $H_2O$ (30 mL). The mixture was then extracted with EA (50 mL * 3). The combined organic layers were washed with brine (10 mL*2), dried over anhydrous $Na_2SO_4$, filtered and concentrated under reduced pressure to give a residue. The residue was purified by column chromatography (Petroleum ether/Ethyl acetate gradient = 1/0 to 3/1) to give intermediate **B2-2** (2.86 g, 73% yield) as yellow oil.

**Step 2.** Synthesis of Intermediate **B2-3**

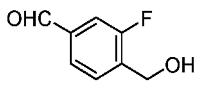

B2-3

Intermediate **B2-2** (2.86 g, 18.8 mmol) was dissolved in THF (30 mL) and $H_2O$ (8 mL). The mixture was cooled to 0 °C. $K_2O_SO_4 \cdot H_2O$ (360 mg, 0.977 mmol) and $NaIO_4$ (16.1 g, 75.2 mmol) was added to the above mixture at 0 °C. The mixture was stirred at 0 °C for 1 h. The mixture was quenched with $H_2O$ (10 mL) and then extracted with EA (10 mL * 3). The combined organic layers were washed with brine (10 mL), dried over anhydrous $Na_2SO_4$, filtered and concentrated under reduced pressure to give a residue. The residue was purified by column chromatography (Petroleum ether/Ethyl acetate gradient = 1/0 to 3/1) to give intermediate **B2-3** (1.47 g, 51% yield) as light red oil.

**Step 3.** Synthesis of Intermediate **B2-4**

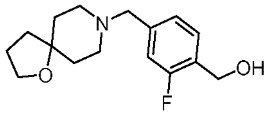

B2-4

Intermediate **B2-3** (210 mg, 1.36 mmol), 1-oxa-8-azaspiro[4.5]decane (242 mg, 1.36 mmol) and AcOH (8.18 mg, 0.136 mmol) were dissolved in DCM (10 mL). The mixture was stirred at 25 °C for 0.5 h. $NaBH_3CN$ (128 mg, 2.04 mmol) was added to the mixture. The mixture was stirred at 25 °C for 12 h. The reaction mixture was quenched with saturated $NaHCO_3$ (10 mL) and extracted with DCM (20 mL*3). The combined organic layers were dried over anhydrous $Na_2SO_4$, filtered and concentrated under reduced pressure to give a residue. The residue was purified by column chromatography (dichloromethane: methanol = 1/0 to 20/1) to give intermediate **B2-4** (160 mg, 39.9% yield) as colorless oil.
LCMS ($ESI^+$): m/z 279.6 $[M+H]^+$.

**Step 4.** Synthesis of Intermediate **B2**

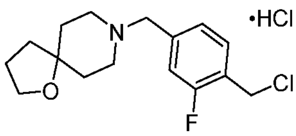

**B2**

To a solution of intermediate **B2-4** (160 mg, 573 μmol) in DCM (5 mL) was added $SOCl_2$ (681 mg, 5.73 mmol) dropwise. The mixture was stirred at 25 °C for 12 h. The mixture was concentrated to dryness under reduced pressure to give intermediate **B2** (190 mg, crude) as a white solid.
LCMS ($ESI^+$): m/z 298.11 $[M+H]^+$.

Intermediates **B3, B4,** and **B5** were prepared according to the representative procedure for intermediate **B2.**

Example 2: Intermediate **B3**

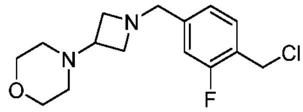

**B3**

LCMS (ESI+): m/z 298.8 [M+H]+.

Example 3: Intermediate **B4**

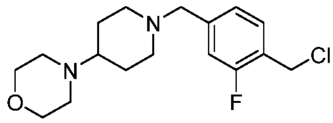

**B4**

LCMS (ESI+): m/z 327.09 [M+H]+.

Example 4: Intermediate **B5**

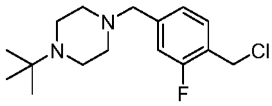

**B5**

LCMS (ESI+): m/z 299.7 [M+H]+.

Synthesis of **B6**

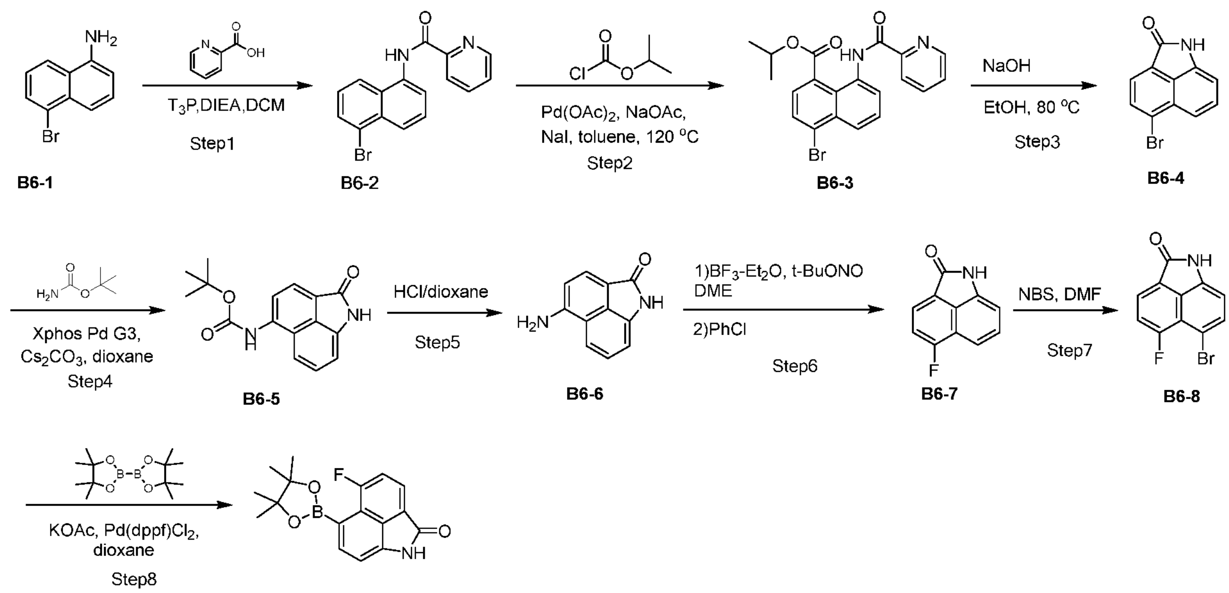


B6-1 B6-2 B6-3 B6-4 B6-5 B6-6 B6-7 B6-8 B6

**Step1:** Synthesis of **B6-2**

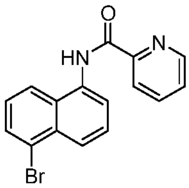

**B6-2**

$T_3P$ (343 g, 540 mmol, 50% in EtOAc) was added dropwise to a mixture of intermediate **B6-1** (80.0 g, 360 mmol), pyridine-2-carboxylic acid (46.6 g, 378 mmol), TEA (109 g, 1.08 mol) and DCM (500 mL). The reaction mixture was stirred at 20 °C for 16 hrs. The reaction mixture was quenched by addition $H_2O$ (500 mL), and then extracted with DCM (300 mL x 2). The combined organic layers were washed with aq. NaCl (200 mL), dried over anhydrous $Na_2SO_4$, filtered and concentrated under reduced pressure to give a residue. The residue was purified by column chromatography (Petroleum ether/Ethyl acetate gradient = 1/0 to 5/1). Intermediate **B6-2** (100 g, 84.0% yield) was obtained as a brown solid.

$^1$H NMR (400 MHz, DMSO-$d_6$) $\delta$ 11.00 (s, 1H), 8.91 - 8.75 (m, 1H), 8.24 - 8.17 (m, 1H), 8.15 - 8.07 (m, 2H), 8.03 (d, *J* = 8.4 Hz, 1H), 7.99 - 7.92 (m, 2H), 7.81 - 7.68 (m, 2H), 7.51 (dd, *J*= 7.6, 8.4 Hz, 1H).

**Step 2:** Synthesis of **B6-3**

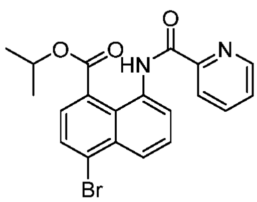

**B6-3**

A mixture of intermediate **B6-2** (10.0 g, 30.6 mmol), isopropyl carbonochloridate (11.2 g, 91.7 mmol), NaOAc (6.01 g, 73.3 mmol) , NaI (4.58 g, 30.6 mmol) and $Pd(OAc)_2$ (1.37 g, 6.11 mmol) in Toluene (300 mL) was degassed

and purged with $N_2$ for 3 times, and then the mixture was stirred at 135 °C for 3 hrs under $N_2$ atmosphere. The reaction mixture was cooled to room temperature, DCM (300 mL) was added to the reaction system. The reaction mixture was adjusted pH > 7 with TEA, and the resulting mixture was filtered and concentrated under vacuum. The residue was purified by column chromatography ($SiO_2$, (Petroleum ether/DCM = 1:1)/Ethyl acetate=1/0 to 1/1). Intermediate **B6-3** (7.10 g, 56.21% yield) was obtained as a pale yellow solid.

**Step 3:** Synthesis of **B6-4**

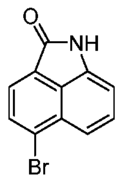

**B6-4**

NaOH (1.03 g, 25.8 mmol) was added to a mixture consisting of intermediate **B6-3** (7.10 g, 17.2 mmol) and EtOH (80 mL). The reaction mixture was stirred at 80 °C for 16 hrs. The reaction mixture was adjusted to pH 6 with 1M HCl and then filtered to give a cake. The cake was used for next step without further purification. Intermediate **B6-4** (3.60 g, 84.5% yield) was obtained as a yellow solid.
$^1$H NMR (400 MHz, DMSO-$d_6$) $\delta$ 10.88 (br. s., 1H), 8.07 (d, *J*= 7.2 Hz, 1H), 7.89 (d, *J*= 7.2 Hz, 1H), 7.67 - 7.59 (m, 1H), 7.57 - 7.51 (m, 1H), 7.05 (d, *J*= 6.8 Hz, 1H).

**Step 4:** Synthesis of **B6-5**

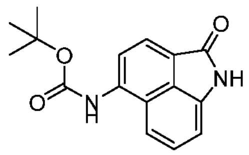

**B6-5**

Intermediate **B6-4** (2.00 g, 8.06 mmol), tert-butyl carbamate (3.78 g, 32.3 mmol), $Cs_2CO_3$ (7.88 g, 24.2 mmol) and XPhos Pd $G_3$ (1.36 g, 1.61 mmol) in dioxane (20 mL) was de-gassed for 3 times and then heated to 110 °C for 6 hours under $N_2$. The reaction mixture was filtered and concentrated under reduced pressure to give a residue. The residue was purified by column chromatography (ISCO®; 40 g SepaFlash® Silica Flash Column, Petroleum ether/Ethyl acetate gradient = 1/0 to 1/2). Intermediate **B6-5** (1.50 g, 65.4% yield) was obtained as a yellow solid.
LCMS (ESI$^+$): m/z 285.3 [M+H]$^+$.

**StepS:** Synthesis of **B6-6**

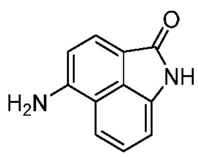

**B6-6**

To a mixture of intermediate **B6-5** (1.47 g, 5.17 mmol) in DCM (4 mL) was added HCl/dioxane (4 M, 10 mL). The reaction mixture was stirred at 25 °C for 10 min. The reaction mixture was concentrated under reduced pressure

to give a residue. The residue was diluted with DCM (20 mL) and sat. $NaHCO_3$ (20 mL), extracted with DCM (20 mL x 2), The combined organic layer was dried over $Na_2SO_4$, filtered and concentrated under reduce pressure to give a residue. Intermediate **B6-6** (950 mg, 99.8% yield) was obtained as a light yellow solid.

LCMS (ESI$^+$): m/z 185.1 $[M+H]^+$.
$^1$H NMR (400 MHz, DMSO-$d_6$) $\delta$ 10.18 (br. s., 1H), 7.70 (d, *J* = 8.4 Hz, 1H), 7.64 (d, *J* = 7.6 Hz, 1H), 7.28 - 7.21 (m, 1H), 7.04 - 6.93 (m, 2H), 6.83 (d, *J*= 6.8 Hz, 1H), 6.70 (d, *J* = 8.0 Hz, 1H)

**Step6:** Synthesis of **B6-7**

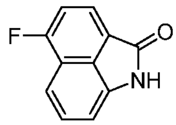

**B6-7**

To a solution of $BF_3$•$Et_2O$ (1.41 g, 4.78 mmol, 1.23 mL, 48%) in DME (20 mL) was added intermediate B6-6 (550 mg, 2.99 mmol) in DME (12 mL) at -5 °C dropwise over 10 min. The reaction mixture was stirred at 0 °C for 1 hr. Then t-BuONO (370 mg, 3.58 mmol) in DME (8 mL) was added into above the mixture at 0 °C. The reaction mixture was stirred at 25 °C for 2 hrs. The reaction mixture was concentrated under reduced pressure, PhCl (10 mL) was added to the residue and heated to 140 °C for 50 min. PhCl was removed under reduced pressure to give the residue. The residue was purified by column chromatography (Petroleum ether/Ethyl acetate gradient = 1/0 to 1/2). Intermediate **B6-7** (200 mg, 35.8% yield) was obtained as a yellow solid.

LCMS (ESI$^+$): m/z 188.1 $[M+H]^+$.
$^1$H NMR (500 MHz, DMSO-$d_6$) $\delta$ 10.97 - 10.76 (m, 1H), 8.05 - 8.01 (m, 1H), 7.64 - 7.54 (m, 3H), 7.06 (d, *J*= 6.5 Hz, 1H).

**Step7:** Synthesis of **B6-8**

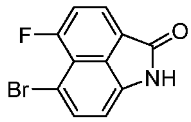

**B6-8**

To a mixture of intermediate **B6-7** (200 mg, 1.07 mmol) in DMF (2 mL) was added NBS (190.18 mg, 1.07 mmol). The reaction mixture was stirred at 25 °C for 2 hrs. The reaction mixture was poured into sat. $NaHCO_3$, filtered and the filter cake was dried under reduce pressure. The residue was purified by column chromatography (Petroleum ether/Ethyl acetate gradient = 1/0 to 1/2). Intermediate **B6-8** (135 mg, 39.4% yield) was obtained as a yellow solid.

LCMS (ESI$^+$): m/z 268.0 $[M+H]^+$.
$^1$H NMR (400 MHz, DMSO-$d_6$) $\delta$ 11.26 - 10.62 (m, 1H), 8.13 - 8.07 (m, 1H), 7.82 - 7.76 (m, 1H), 7.70 - 7.61 (m, 1H), 6.99 - 6.92 (m, 1H).

Step8 : Synthesis of **B6**

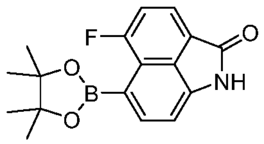

**B6**

Intermediate **B6-8** (135 mg, 507 μmol), 4,4,5,5-tetramethyl-2-(4,4,5,5-tetramethyl-1,3,2-dioxaborolan-2-yl)-1,3,2-dioxaborolane (193 mg, 761 μmol), KOAc (149 mg, 1.52 mmol,) and Pd(dppf)$Cl_2$ (37.1 mg, 50.7 μmol) in dioxane (5 mL), the mixture was degassed for 3 times and then heated to 90 °C for 4 hours under $N_2$. The reaction mixture was concentrated under reduced pressure to give a residue. The residue was purified by column chromatography (Petroleum ether/Ethyl acetate gradient= 1/0 to 1/2). Intermediate **B6** (230 mg, crude) was obtained as a yellow solid.
LCMS (ESI+): m/z 314.1 [M+H]+.

Synthesis of **B8**

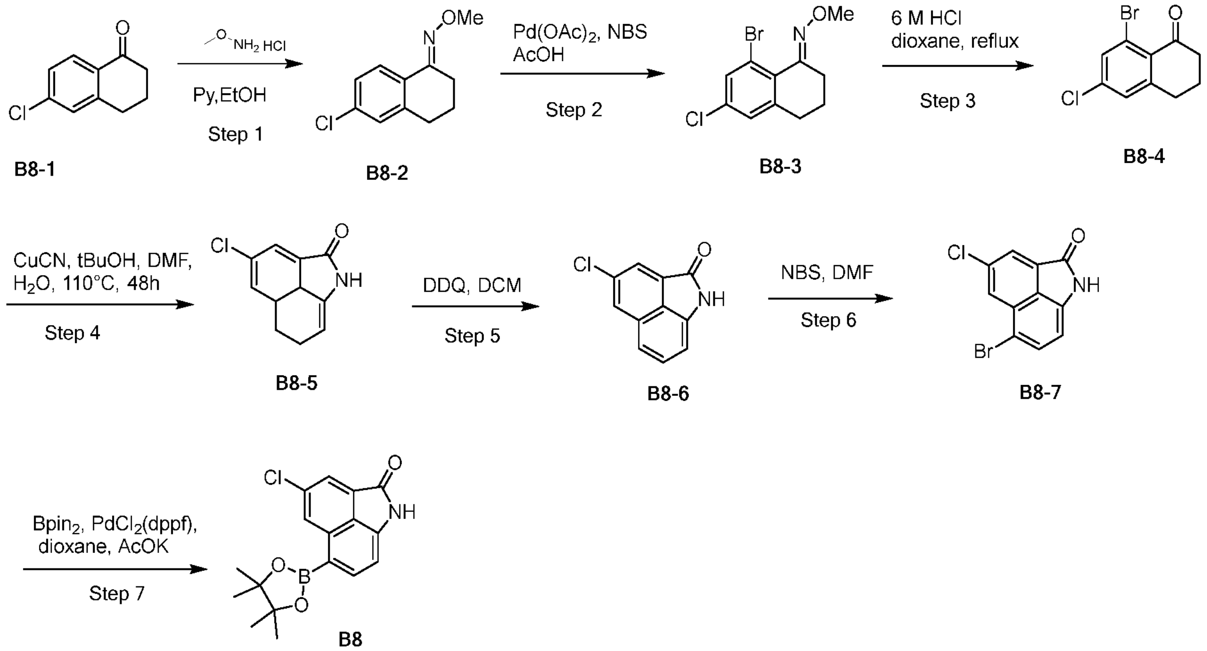


**Step1:** Synthesis of **B8-2**

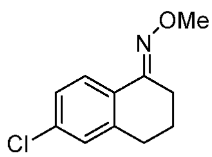

**B8-2**

A solution of intermediate **B8-1** (5.00 g, 27.7 mmol), O-methyl hydroxylamine; hydrochloride (3.01 g, 36.0 mmol) and Pyridine (3.28 g, 41.5 mmol) in MeOH (40 mL) was degassed and purged with $N_2$ for 3 times, and then the mixture was stirred at 20°C for 16 hours under $N_2$ atmosphere. The reaction mixture was concentrated under reduced pressure to give a crude. The crude was purified by column chromatography ($SiO_2$, Petroleum ether/Ethyl acetate=1/0). Intermediate **B8-2** (5.48 g, 94.4% yield) was obtained as a yellow oil.

$^1H$ NMR (400MHz, $CDCl_3$) $\delta$7.92 (d, $J$ = 8.4 Hz, 1H), 7.19 - 7.09 (m, 2H), 3.99 (s, 3H), 2.78 - 2.65 (m, 4H), 1.90 - 1.78 (m, 2H).

**Step2:** Synthesis of **B8-3**

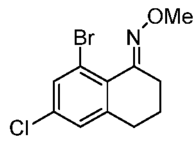

B8-3

To a solution of intermediate **B8-2** (5.48 g, 26.1 mmol), NBS (5.44 g, 30.6 mmol) in AcOH (30 mL) was added $Pd(OAc)_2$ (58.7 mg, 261 μmol), and the reaction mixture was degassed and purged with $N_2$ for 3 times, and then the mixture was stirred at 80 °C for 0.5 hour under $N_2$ atmosphere. The reaction mixture was concentrated under reduced pressure to give a crude. The crude was purified by column chromatography ($SiO_2$, Petroleum ether/Ethyl acetate=1/0). Intermediate **B8-3** (7.48 g, 99.2% yield) was obtained as a yellow oil.

**Step3:** Synthesis of **B8-4**

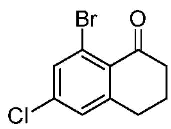

B8-4

To a solution of intermediate **B8-3** (7.48 g, 25.9 mmol) in dioxane (50 mL) was added HCl (6 M, 70 mL). The mixture was stirred at 100°C for 1 hour. The reaction mixture was added 10% NaOH to adjust pH = 7. The reaction mixture was partitioned between EA 100 mL and $H_2O$ 60 mL. The organic phase was separated, washed with brine 80 mL (40 mL*2), dried over $Na_2SO_4$, filtered and concentrated under reduced pressure to give a residue. The residue was purified by column chromatography ($SiO_2$, Petroleum ether/Ethyl acetate=1/0 to 10/1). Intermediate **B8-4** (3.4 g, 13.10 mmol, 50.5% yield) was obtained as a yellow oil.
$^1H$ NMR (500MHz, DMSO-$d_6$) $\delta$ 7.70 (d, $J$ = 2.0 Hz, 1H), 7.55 - 7.51 (m, 1H), 2.97 (t, $J$ = 6.0 Hz, 2H), 2.63 (t, $J$ = 7.0 Hz, 2H), 2.02 - 1.96 (m, 2H).

**Step 4:** Synthesis of **B8-5**

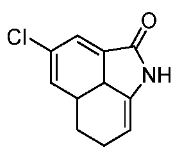

B8-5

To a solution of intermediate **B8-4** (500 mg, 1.93 mmol) in DMF (2 mL), $H_2O$ (1 mL), t-BuOH (2 mL) was added CuCN (173 mg, 1.93 mmol). The mixture was stirred at 110°C for 48 hours. The reaction mixture was quenched by addition $H_2O$ 20 mL at 20°C, and then extracted with DCM 60 mL (20 mL*3). The combined organic layers were washed with brine 10 mL, dried over $Na_2SO_4$, filtered and concentrated under reduced pressure to give a residue. The residue

was purified by column chromatography ($SiO_2$, Petroleum ether/Ethyl acetate=1/0 to 1/1). Intermediate **B8-5** (172 mg, 43.4% yield) was obtained as a yellow solid.
$^1H$ NMR (500MHz, DMSO-$d_6$) $\delta$ 10.50 (s, 1H), 7.53 - 7.42 (m, 2H), 5.45 (t, *J* = 4.4 Hz, 1H), 2.95 (t, *J* = 8.0 Hz, 2H), 2.64 (t, *J* = 8.0 Hz, 2H).

**Step 5:** Synthesis of **B8-6**

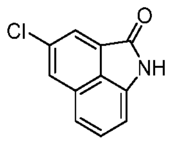

B8-6

To a solution of intermediate **B8-5** (170 mg, 827 μmol) in DCM (4 mL) was added DDQ (188 mg, 827 μmol). The mixture was stirred at 20 °C for 16 hours. The reaction mixture was concentrated under reduced pressure to give a residue. The residue was purified by column chromatography ($SiO_2$, Petroleum ether/Ethyl acetate = 1/0 to 3/1). Intermediate **B8-6** (360 mg, crude) was obtained as a yellow solid.

LCMS (ESI$^+$): m/z 204.1 $[M+H]^+$.
$^1H$ NMR (400MHz, DMSO-$d_6$) $\delta$ 10.92 (s, 1H), 8.30 (d, *J* = 1.2 Hz, 1H), 8.01 (d, *J* = 1.2 Hz, 1H), 7.59 - 7.50 (m, 2H), 7.00 (dd, *J*= 1.2, 6.4 Hz, 1H).

**Step 6:** Synthesis of **B8-7**

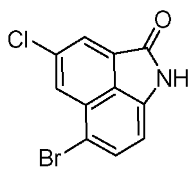

B8-7

To a solution of intermediate **B8-6** (300 mg, 1.47 mmol) in DMF (7 mL) was added NBS (262 mg, 1.47 mmol) at 0 °C. The mixture was stirred at 0 °C for 2 hours. The reaction mixture was partitioned between EA 60 mL and $H_2O$ 40 mL. The organic phase was separated, washed with brine (20 mL * **2),** dried over $Na_2SO_4$, filtered and concentrated under reduced pressure to give a residue. The residue was purified by column chromatography ($SiO_2$, Petroleum ether/Ethyl acetate=1/0 to 3/1). Intermediate **B8-7** (300 mg, 50.5% yield) was obtained as a yellow solid.
$^1H$ NMR (400MHz, DMSO-$d_6$) $\delta$ 11.05 (s, 1H), 8.14 (s, 1H), 8.11 (s, 1H), 7.81 (d, *J* = 7.6 Hz, 1H), 6.93 (d, *J* = 8.0 Hz, 1H).

**Step7:** Synthesis of **B8**

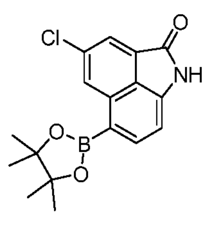

B8

To a solution of intermediate **B8-7** (240 mg, 850 μmol), 4,4,5,5-tetramethyl-2-(4,4,5,5-tetramethyl-1,3,2-dioxaborolan-2-yl)-1,3,2-dioxaborolane (324 mg, 1.27 mmol), Pd(dppf)$Cl_2$ (62.2 mg, 85.0 μmol), KOAc (250 mg, 2.55 mmol) in dioxane (4 mL) was degassed and purged with $N_2$ for 3 times, and then the mixture was stirred at 90°C for 2 hr under $N_2$ atmosphere. The reaction mixture was partitioned between EA 40 mL and $H_2O$ 40 mL. The organic phase was separated, washed with brine (30 mL * 2), dried over $Na_2SO_4$, filtered and concentrated under reduced pressure to give a residue. The residue was purified by column chromatography ($SiO_2$, Petroleum ether/Ethyl acetate=1/0 to 3/1). Intermediate **B8** (198 mg, 57.4% yield) was obtained as a yellow solid.
LCMS (ESI$^+$): m/z 330.1 [M+H]$^+$.

Synthesis of **B9**

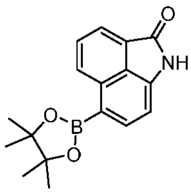

**B9**

**B9** was synthesized following the method of step 7 of **B8.**
LCMS (ESI$^+$): m/z 296.3 [M+H]$^+$.

Synthesis of **B10**

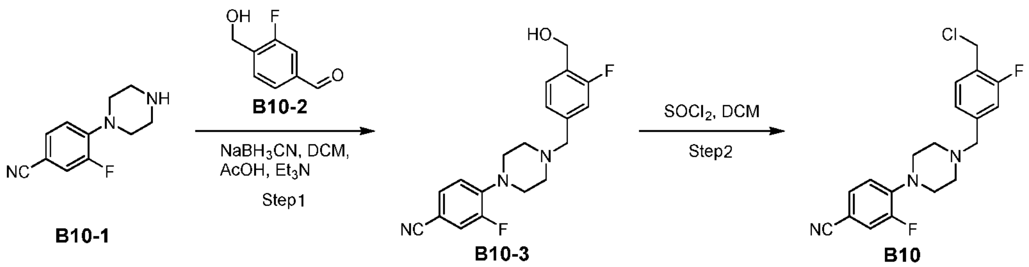


**Step1:** Synthesis of **B10-3**

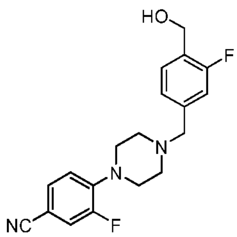

**B10-3**

To a solution of intermediate **B10-2** (2 g, 9.75 mmol) and intermediate **B10-1** (1.50 g, 9.75 mmol) in DCM (20 mL) was added TEA (4.93 g, 48.7 mmol, 6.78 mL) and AcOH (2.93 g, 48.7 mmol, 2.79 mL). The mixture was stirred at 20 °C for 0.5 hr. Then $NaBH_3CN$ (1.84 g, 29.2 mmol) was added and the reaction mixture was stirred at 20 °C for 16 hrs. The reaction mixture was diluted with $H_2O$ (30 mL) and extracted with DCM (30 mL * 3). The combined organic layers were washed with aqueous NaCl (150 mL), dried over $Na_2SO_4$, filtered and concentrated under reduced pressure

to give a residue. The residue was purified by column chromatography ($SiO_2$, Petroleum ether/Ethyl acetate=1/0 to 1/1) to obtained compound 3-fluoro-4-[4-[[3-fluoro-4-(hydroxymethyl)phenyl]methyl]piperazin-1-yl]benzonitrile **B10-3** (2.9 g, 86.7% yield) was obtained as a white solid.
LCMS ($ESI^+$): m/z 343.9 $[M+H]^+$.

**Step 2:** Synthesis of **B10**

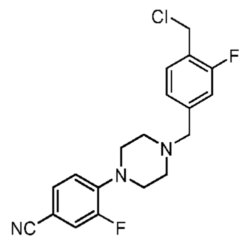

**B10**

To a solution of intermediate **B10-3** (750 mg, 2.18 mmol) in DCM (3 mL) was added $SOCl_2$ (6.8 mL) The mixture was stirred at 25 °C for 16 hr. The reaction mixture was concentrated under reduced pressure to remove $SOCl_2$. The residue was diluted with EA 50 mL then added sat. $NaHCO_3$ (20 mL). The organic phase was separated, dried over $Na_2SO_4$, filtered and concentrated under reduced pressure to give a residue. The residue was purified by column chromatography ($SiO_2$, Petroleum ether/Ethyl acetate = 5/1 to 1/1). Intermediate **B10** (445 mg, 52.4% yield) was obtained as a yellow oil.
LCMS ($ESI^+$): m/z 363.2 $[M+H]^+$.

Synthesis of **B11**

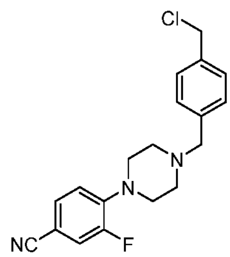

**B11**

**B11** was synthesized following the method of **B10.**
LCMS ($ESI^+$): m/z 343.8 $[M+H]^+$.

**Example 1.** Compound **1**

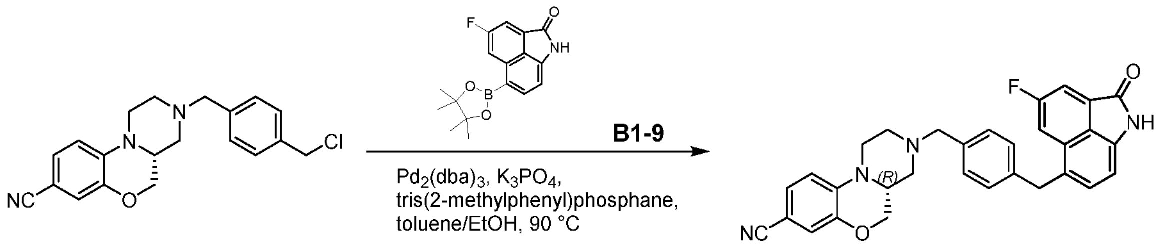


**1-1** **1-2**

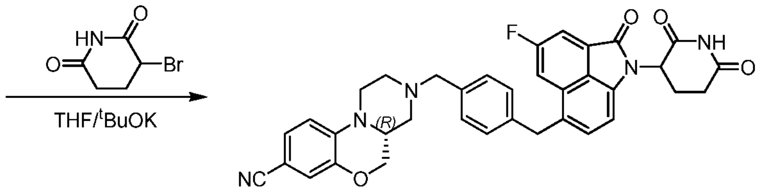


**1**

Step 1. Synthesis of Intermediate 1-2

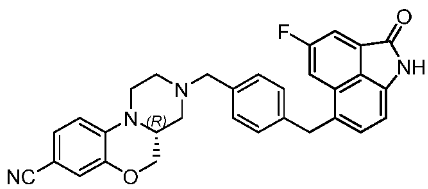

**1-2**

A black suspension of intermediate 1-1 (110 mg, 311 μmol) , intermediate B1-9 (195 mg, 622 μmol), $K_3PO_4$ (198 mg, 933 μmol), $Pd_2(dba)_3$ (42.7 mg, 46.6 μmol) and tris(2-methylphenyl)phosphane (18.9 mg, 62.2 μmol) in toluene (3 mL) and EtOH (0.5 mL) was degassed with $N_2$ for 1 min and stirred at 80°C for 16 hours. The reaction mixture was concentrated to dryness under reduced pressure to give a residue. The residue was purified by prep-TLC (PE:EA=1: 1) to give intermediate 2-2 (105 mg, 62.8% yield) as a yellow solid.
LCMS (ESI$^+$): m/z 505.0 $[M+H]^+$.

**Step 2.** Synthesis of compound **1**

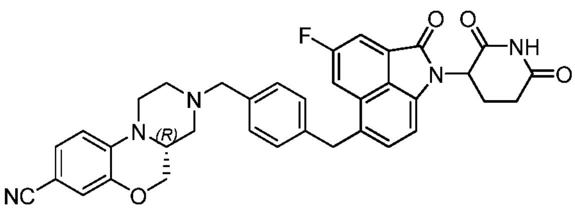

**1**

To a grey suspension of NaH (83.2 mg, 2.08 mmol) in THF (1 mL) was added a yellow solution of intermediate **2-2** (105 mg, 208.10 μmol) in THF (2 mL) at 0 °C. Then a colorless solution of 3-bromopiperidine-2,6-dione (199.79 mg, 1.04 mmol, 5.0 *eq*) in THF (1 mL) was added at 0°C. Then the yellow suspension was stirred at 60 °C for 1 hour. The

reaction mixture was diluted with EA (50 mL) and washed with ice water (50 mL). The organic layer was washed with saturated NaCl (30 mL), dried over $Na_2SO_4$, filtered, and concentrated to dryness under reduced pressure to give a crude residue. The residue was purified by prep-TLC (PE: EA = 1:1) to give **Compound 1** (32.0 mg, 23.8% yield) as a yellow solid.

LCMS (ESI$^+$): m/z 616.2 [M+H] $^+$.
$^1$H NMR (400 MHz, DMSO-$d_6$) $\delta$ 11.15 (s, 1H), 8.16 (d, *J* = 11.2 Hz, 1H), 8.03 (d, *J* = 7.6 Hz, 1H), 7.45 (d, *J*= 7.2 Hz, 1H), 7.30 - 7.16 (m, 5H), 7.13 - 7.01 (m, 2H), 6.94 (d, *J* = 8.8 Hz, 1H), 5.44 (dd, *J* = 5.2, 12.8 Hz, 1H), 4.35 (s, 2H), 4.25 (dd, *J* = 2.8, 10.8 Hz, 1H), 3.95 - 3.77 (m, 2H), 3.55 - 3.41 (m, 2H), 3.21 - 3.14 (m, 1H), 3.00 - 2.82 (m, 2H), 2.81 - 2.72 (m, 2H), 2.64 - 2.58 (m, 1H), 2.43 - 2.42 (m, 1H), 2.16 - 2.01 (m, 2H), 1.79 - 1.60 (m, 1H).

Examples **2** to **19,** were prepared according to the representative procedure of compound **1.**

**Example 2.** Compound **2**

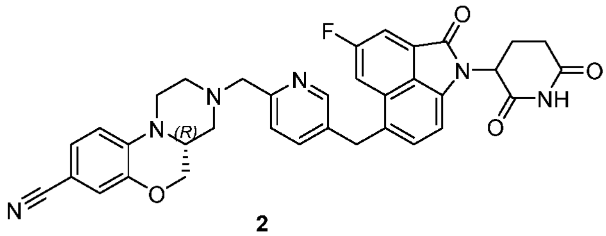

**2**

LCMS (ESI$^+$): m/z 617.3 [M+H] $^+$.
$^1$H NMR (400 MHz, DMSO-$d_6$) $\delta$ 11.15 (s, 1H), 8.57 (d, *J* = 1.6 Hz, 1H), 8.25 (dd, *J* = 1.6, 11.2 Hz, 1H), 8.05 (dd, *J*= 1.6, 7.6 Hz, 1H), 7.65 (dd, *J*= 2.0, 8.0 Hz, 1H), 7.48 (d, *J* = 7.6 Hz, 1H), 7.36 (d, *J* = 8.0 Hz, 1H), 7.21 (dd, *J* = 2.0, 8.4 Hz, 1H), 7.11 (d, *J* = 7.2 Hz, 1H), 7.07 (d, *J* = 2.0 Hz, 1H), 6.94 (d, *J* = 8.8 Hz, 1H), 5.44 (dd, *J* = 5.2, 12.8 Hz, 1H), 4.38 (s, 2H), 4.29 - 4.18 (m, 1H), 3.93 - 3.77 (m, 2H), 3.66 - 3.51 (m, 2H), 3.24 - 3.17 (m, 1H), 2.90 (d, *J* = 9.6 Hz, 2H), 2.83 - 2.69 (m, 2H), 2.53 - 2.47 (m, 1H), 2.45 - 2.42 (m, 1H), 2.23 - 2.14 (m, 1H), 2.13 - 2.04 (m, 1H), 1.81 (t, *J* = 10.8 Hz, 1H).

**Example 3.** Compound **3**

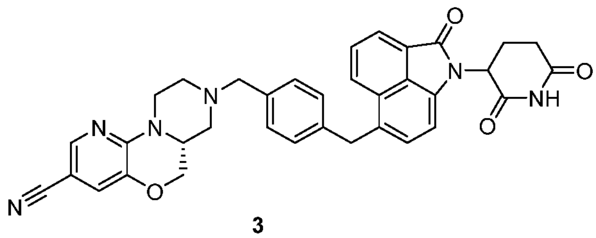

**3**

LCMS (ESI$^+$): m/z 599.3[M+H] $^+$.
$^1$H NMR (400 MHz, DMSO-$d_6$) $\delta$ 11.15 (s, 1H), 8.46 - 8.27 (m, 1H), 8.19 - 8.04 (m, 2H), 7.89 - 7.73 (m, 1H), 7.43 (d, *J* = 7.2 Hz, 1H), 7.34 (d, *J* = 2.0 Hz, 1H), 7.29 - 7.17 (m, 4H), 7.12 (d, *J*= 7.2 Hz, 1H), 5.45 (dd, *J*= 5.2, 12.8 Hz, 1H), 4.52 - 4.34 (m, 3H), 4.33 - 4.25 (m, 1H), 3.94 - 3.81 (m, 1H), 3.58 - 3.49 (m, 1H), 3.45 (d, *J* = 7.2 Hz, 1H), 3.34 - 3.33 (m, 1H), 3.01 - 2.82 (m, 4H), 2.81 - 2.70 (m, 1H), 2.44 - 2.36 (m, 1H), 2.15 - 1.97 (m, 2H), 1.74 (t, *J*= 10.8 Hz, 1H).

**Example 4.** Compound **4**

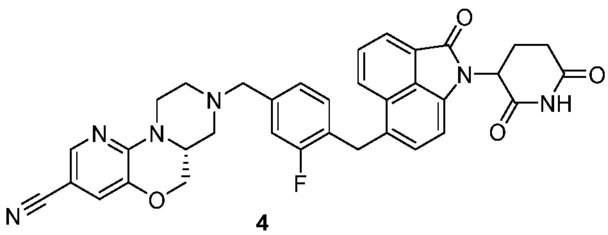

**4**

LCMS (ESI+): m/z 617.4 [M+H] +.
$^1$H NMR (400 MHz, DMSO-$d_6$) $\delta$ 11.14 (s, 1H), 8.37 (d, $J$ = 8.4 Hz, 1H), 8.19 - 7.98 (m, 2H), 7.91 - 7.76 (m, 1H), 7.40 - 7.31 (m, 2H), 7.25 - 6.99 (m, 4H), 5.56 - 5.40 (m, 1H), 4.52 - 4.36 (m, 3H), 4.30 (s, 1H), 3.87 (dd, $J$ = 8.4, 11.2 Hz, 1H), 3.57 - 3.49 (m, 2H), 3.03 - 2.83 (m, 4H), 2.82 - 2.71 (m, 1H), 2.64 - 2.56 (m, 1H), 2.47 - 2.43 (m, 1H), 2.14 - 1.99 (m, 2H), 1.77 (t, $J$= 10.8 Hz, 1H).

**Example 5.** Compound **5**

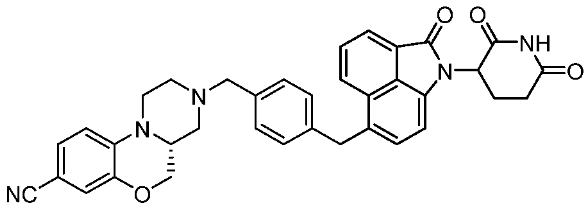

**5**

LCMS (ESI+): m/z 598.3 [M+1]+.
$^1$H NMR (400MHz, DMSO-$d_6$) $\delta$ 11.15 (br s, 1H), 8.37 - 8.31 (m, 1H), 8.13 - 8.04 (m, 1H), 7.85 - 7.78 (m, 1H), 7.49 - 7.40 (m, 1H), 7.29 - 7.25 (m, 2H),7.24 - 7.20 (m, 3H), 7.14 - 7.10 (m, 1H), 7.09 - 7.03 (m, 1H), 6.96 - 6.91 (m, 1H), 5.49 - 5.39 (m, 1H), 4.39 (s, 2H), 4.27 - 4.22 (m, 1H), 3.88 - 3.80 (m, 2H), 3.45 - 3.42 (m, 2H), 3.22 - 3.17 (m, 1H), 2.98 - 2.79 (m, 4H), 2.70 - 2.61 (m, 2H), 2.15 - 2.03 (m, 2H), 1.76 - 1.66 (m, 1H).

**Example 6.** Compound **6**

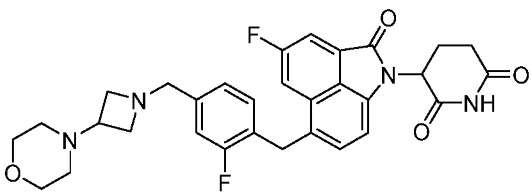

**6**

LCMS (ESI+): m/z 561.3[M+H]+.
$^1$H NMR (400 MHz, DMSO-$d_6$) $\delta$ 11.15 (br s, 1H), 8.24 - 8.13 (m, 1H), 8.10 - 8.01 (m, 1H), 7.38 - 7.29 (m, 1H), 7.21 (t, $J$ = 8.0 Hz, 1H), 7.10 - 6.99 (m, 3H), 5.50 - 5.39 (m, 1H), 4.35 (s, 2H), 3.56 - 3.50 (m, 6H), 3.33 - 3.27 (m, 5H), 2.99 - 2.89 (m, 1H), 2.75 - 2.62 (m, 2H), 2.25 - 2.15 (m, 4H), 2.13 - 2.05 (m, 1H).

**Example 7.** Compound **7**

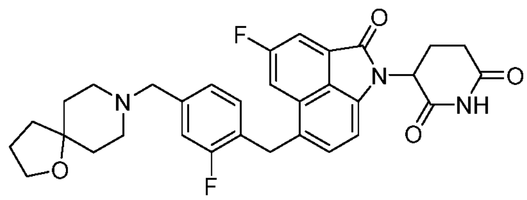

**7**

LCMS (ESI$^+$): m/z 560.3[M+H] $^+$.
$^1$H NMR (400 MHz, DMSO-$d_6$) $\delta$ 11.14 (s, 1H), 8.18 (dd, *J*= 1.6, 11.2 Hz, 1H), 8.05 (dd, *J*= 1.6, 7.6 Hz, 1H), 7.33 (d, *J* = 7.2 Hz, 1H), 7.21 (t, *J* = 7.6 Hz, 1H), 7.14 - 7.00 (m, 3H), 5.44 (dd, *J* = 5.2, 12.8 Hz, 1H), 4.35 (s, 2H), 3.66 (t, *J* = 6.8 Hz, 2H), 3.41 (s, 2H), 3.00 - 2.88 (m, 1H), 2.79 - 2.60 (m, 2H), 2.43 - 2.26 (m, 4H), 2.14 - 2.05 (m, 1H), 1.86 - 1.76 (m, 2H), 1.64 - 1.56 (m, 2H), 1.54 - 1.47 (m, 4H).

**Example 8.** Compound **8**

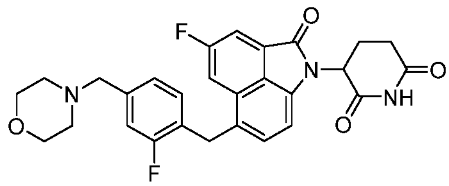

**8**

LCMS (ESI$^+$): m/z 506.3[M+H] $^+$.
$^1$H NMR (400 MHz, DMSO-$d_6$) $\delta$ 11.15 (s, 1H), 8.24 - 8.15 (m, 1H), 8.10 - 7.98 (m, 1H), 7.42 - 7.31 (m, 1H), 7.28 - 7.19 (m, 1H), 7.17 - 6.95 (m, 3H), 5.53 - 5.35 (m, 1H), 4.36 (s, 2H), 3.66 - 3.49 (m, 4H), 3.43 (s, 2H), 3.01 - 2.88 (m, 1H), 2.80 - 2.59 (m, 2H), 2.38 - 2.21 (m, 4H), 2.15 - 2.04 (m, 1H).

**Example 9.** Compound **9**

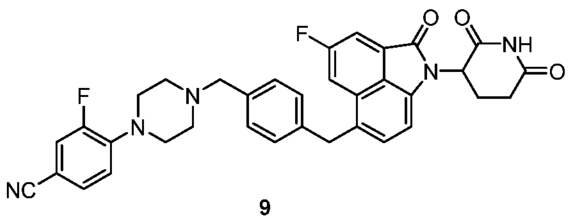

**9**

LCMS (ESI$^+$): m/z 606.1 [M+H] $^+$.
$^1$H NMR (400 MHz, DMSO-$d_6$) $\delta$ 11.16 (s, 1H), 8.22 - 8.12 (m, 1H), 8.08 - 7.99 (m, 1H), 7.73 - 7.64 (m, 1H), 7.60 - 7.51 (m, 1H), 7.45 (d, *J* = 7.2 Hz, 1H), 7.32 - 7.19 (m, 4H), 7.14 - 7.04 (m, 2H), 5.50 - 5.39 (m, 1H), 4.35 (s, 2H), 3.46 (s, 2H), 3.14 (br s, 4H), 3.01 - 2.88 (m, 1H), 2.82 - 2.71 (m, 1H), 2.68 (br s, 1H), 2.47 (br s, 4H), 2.16 - 2.05 (m, 1H). **Chiral SFC separation condition:** DAICEL CHIRALPAK IA(250mm*30mm, 10μm); Mobile phase: A: Supercritical $CO_2$, B: ethanol (neutral), A:B =15:85 at 80mL/min; Column Temp: 38°C; Nozzle Pressure: 100 Bar; Nozzle Temp: 60°C; Evaporator Temp: 20°C; Trimmer Temp: 25°C; Wavelength: 220nm).

The first eluted peak during SFC separation was assigned as Compound **9-B** and second eluted peak was assigned as Compound **9-A.**

Compound **9-A-**Peak-1-SFC RT: 7.689 min
Compound **9-B**-Peak-2-SFC RT: 5.503min

Compound **9-A**

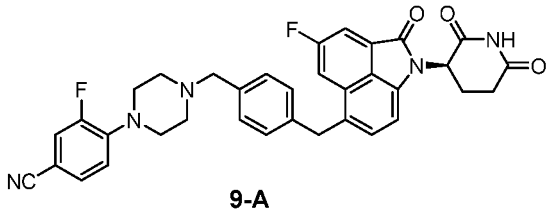

**9-A**

LCMS (ESI+): m/z 606.4 $[M+H]^+$.

$^1H$ NMR (400 MHz, DMSO-$d_6$) $\delta$ 11.14 (s, 1H), 8.19 - 8.12 (m, 1H), 8.03 (d, *J* = 7.6 Hz, 1H), 7.67 (dd, *J*= 1.6, 13.2 Hz, 1H), 7.60 - 7.53 (m, 1H), 7.44 (d, *J* = 7.2 Hz, 1H), 7.30 - 7.18 (m, 4H), 7.15 - 7.03 (m, 2H), 5.44 (dd, *J* = 5.2, 12.8 Hz, 1H), 4.35 (s, 2H), 3.45 (s, 2H), 3.14 (br s, 4H), 3.03 - 2.88 (m, 1H), 2.81 - 2.71 (m, 1H), 2.66 - 2.56 (m, 1H), 2.47 (br s, 4H), 2.14 - 2.04 (m, 1H).

Compound **9-B**

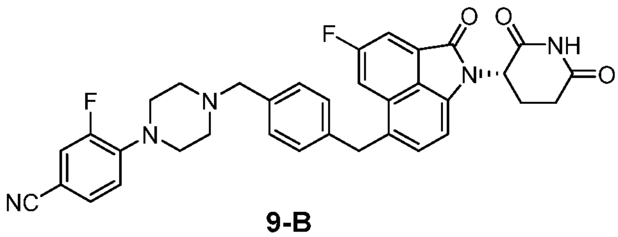

**9-B**

LCMS (ESI+): m/z 606.4 $[M+H]^+$.

$^1H$ NMR (400 MHz, DMSO-$d_6$) $\delta$11.14 (s, 1H), 8.22 - 8.19 (s, 0.07 H), 8.15 (dd, *J*= 2.0, 11.2 Hz, 1H), 8.02 (dd, *J*= 2.0, 7.6 Hz, 1H), 7.67 (dd, *J*= 2.0, 13.2 Hz, 1H), 7.55 (dd, *J* = 1.6, 8.4 Hz, 1H), 7.44 (d, *J* = 7.6 Hz, 1H), 7.31 - 7.19 (m, 4H), 7.14 - 6.96 (m, 2H), 5.44 (dd, *J* = 5.2, 12.8 Hz, 1H), 4.35 (s, 2H), 3.45 (s, 2H), 3.14 (br s, 4H), 2.99 - 2.86 (m, 1H), 2.81 - 2.70 (m, 1H), 2.62 (br s, 1H), 2.48 - 2.42 (m, 4H), 2.16 - 2.01 (m, 1H).

**Example 10.** Compound **10**

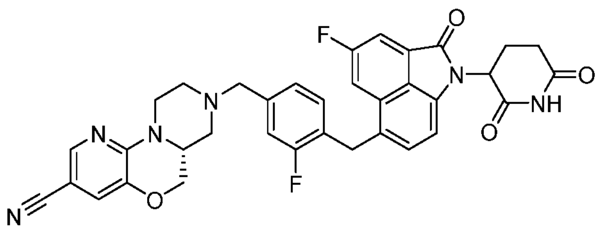

**10**

LCMS ($ESI^+$): m/z 635.3 $[M+H]^+$.

$^1H$ NMR (400 MHz, DMSO-$d_6$) $\delta$ 11.15 (br s, 1H), 8.26 - 8.00 (m, 3H), 7.44 - 7.32 (m, 2H), 7.27 (t, *J* = 8.0 Hz, 1H), 7.15 (d, *J* = 11.2 Hz, 1H), 7.08 (d, *J* = 7.6 Hz, 2H), 5.52 - 5.37 (m, 1H), 4.45 (d, *J*= 13.2 Hz, 1H), 4.39 - 4.22 (m, 3H), 3.94 - 3.82 (m, 1H), 3.58 - 3.52 (m, 2H), 2.98 - 2.86 (m, 4H), 2.78 - 2.70 (m, 1H), 2.64 - 2.59 (m, 1H), 2.43 - 2.40 (m, 1H), 2.17 - 2.02 (m, 2H), 1.85 - 1.71 (m, 1H).

**Example 11.** Compound **11**

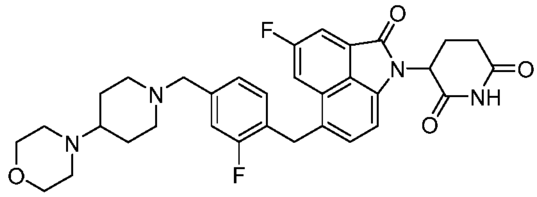

**11**

LCMS (ESI+): m/z 589.4 [M+H] +.

$^1$H NMR (400 MHz, DMSO-$d_6$) $\delta$ 11.15 (s, 1H), 8.17 (dd, *J*= 1.6, 11.2 Hz, 1H), 8.04 (dd, *J*= 1.6, 7.6 Hz,1H), 7.32 (d, *J*= 7.6 Hz, 1H), 7.22 (t, *J* = 8.0 Hz, 1H), 7.12 -7.05 (m, 2H), 7.03 (d, *J*= 8.0 Hz, 1H), 5.48- 5.37 (m, 1H), 4.34 (s, 2H), 3.56 - 3.52 (m, 4H), 3.36 - 3.35 (m, 1H), 2.81 - 2.62 (m, 4H), 2.42 - 2.37 (m, 4H), 2.13 - 2.02 (m, 2H), 1.93 - 1.82 (m, 2H), 1.73 - 1.65 (m, 2H), 1.40 - 1.29 (m, 2H), 0.81 - 0.68 (m, 2H).

**Example 12.** Compound **12**

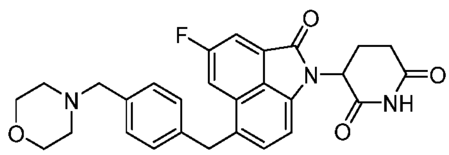

**12**

LCMS (ESI+): m/z 488.2 [M+H] +.

$^1$H NMR (400 MHz, DMSO-$d_6$) $\delta$ 11.16 (s, 1H), 8.15 (dd, *J*= 1.6, 11.2 Hz, 1H), 8.03 (dd, *J* = 1.6, 7.6 Hz, 1H), 7.44 (d, *J* = 7.6 Hz, 1H), 7.25 (d, *J* = 8.0 Hz, 2H), 7.21 (d, *J* = 8.0 Hz, 2H), 7.10 (d, *J* = 7.6 Hz,1H), 5.44 (dd, *J* = 5.2, 12.8 Hz, 1H), 4.34 (s, 2H), 3.57 - 3.49 (m, 4H), 3.36 - 3.27 (m, 2H), 3.00 -2.89 (m, 1H), 2.81 - 2.65 (m, 2H), 2.34 - 2.25 (m, 4H), 2.15 - 2.06 (m, 1H).

**Example 13.** Compound **13**

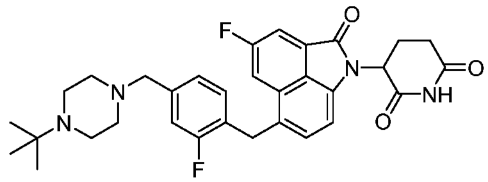

**13**

LCMS (ESI+): m/z 561.4 [M+H]+.

$^1$H NMR (400 MHz, DMSO-$d_6$) $\delta$ 11.15 (s, 1H), 8.31 (s, 0.952, FA), 8.18 (d, *J* = 11.20 Hz, 1H), 8.05 (d, *J* = 7.60 Hz, 1H), 7.32 (d, *J* = 7.60 Hz, 1H), 7.22 (t, *J* = 8.0 Hz, 1H), 7.06 - 7.11 (m, 2H), 7.03 (d, *J* = 8.0 Hz, 1H), 5.44 (d, *J* = 1.20 Hz, 1H), 4.35 (s, 2H), 3.39 (s, 2H), 2.84 - 3.04 (m, 2H), 2.69 -2.80 (m, 1H), 2.59 - 2.68 (m, 2H), 2.56 (br s, 3H), 2.22-2.40 (m, 3H), 2.05- 2.14 (m, 1H), 0.97 (s, 9H).

**Example 14.** Compound **14**

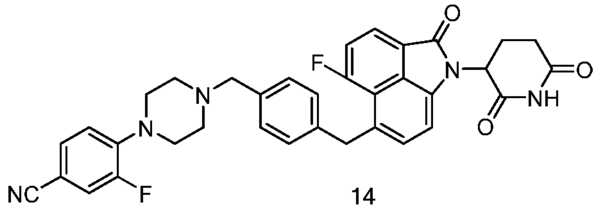

**14**

LCMS (ESI+): m/z 606.1 [M+H]+.

$^1$H NMR (400 MHz, DMSO-$d_6$) $\delta$ 11.15 (br. s., 1H), 8.15 - 8.09 (m, 1H), 7.72 - 7.65 (m, 1H), 7.61 - 7.52 (m, 2H), 7.48 - 7.42 (m, 1H), 7.25 - 7.05 (m, 6H), 5.51 - 5.42 (m, 1H), 4.41 (s, 2H), 3.47 - 3.43 (m, 2H), 3.19 - 3.09 (m, 4H), 3.00 - 2.88 (m, 1H), 2.82 - 2.72 (m, 1H), 2.69 - 2.62 (m, 1H), 2.49 - 2.44 (m, 4H), 2.14 - 2.05 (m, 1H).

**Example 15.** Compound **15**

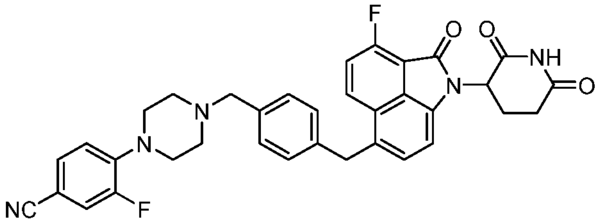

**15**

LCMS (ESI+): m/z 606.1 [M+H]+.

$^1$H NMR (400MHz, DMSO-$d_6$) $\delta$ 11.15 (br s, 1H), 8.44 (d, *J* = 8.4 Hz, 1H), 7.72 - 7.59 (m, 2H), 7.54 (d, *J* = 7.6 Hz, 1H), 7.41 (d, *J*= 6.8 Hz, 1H), 7.23 (br s, 5H), 7.12 - 7.03 (m, 1H), 5.45 (d, *J*= 9.2 Hz, 1H), 4.39 (br s, 2H), 3.45 (br s, 2H), 3.13 (br s, 4H), 2.92 (d, *J* = 12.0 Hz, 1H), 2.78 - 2.61 (m, 2H), 2.46 (br s, 4H), 2.10 (br s, 1H).

**Example 16.** Compound **17**

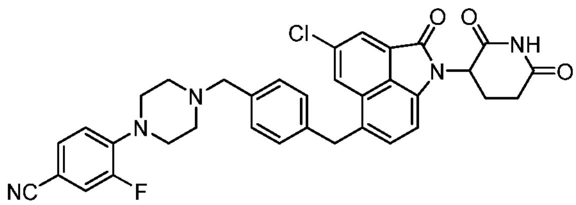

**17**

LCMS (ESI+): m/z 622.1 [M+H] +.

$^1$H NMR (400MHz, DMSO-$d_6$) $\delta$ 11.14 (br s, 1H), 8.39 (s, 1H), 8.12 (s, 1H), 7.71 - 7.63 (m, 1H), 7.58 - 7.52 (m, 1H), 7.47 - 7.41 (m, 1H), 7.24 (br s, 4H), 7.17 - 7.12 (m, 1H), 7.11 - 7.05 (m, 1H), 5.52 - 5.36 (m, 1H), 4.38 (br s, 2H), 3.46 (br s, 2H), 3.14 (br s, 4H), 3.02 - 2.87 (m, 1H), 2.82 - 2.59 (m, 2H), 2.48 - 2.44 (m, 4H), 2.10 (br s, 1H).

**Example 17.** Compound **18**

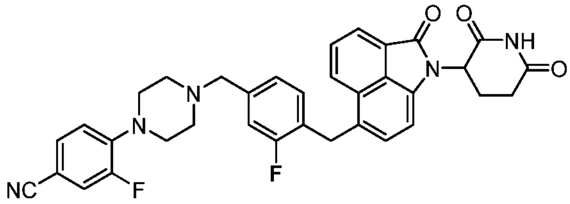

**18**

LCMS (ESI$^+$): m/z 606.2 $[M+H]^+$.

$^1$H NMR (400MHz, DMSO-$d_6$) $\delta$ 11.13 (s, 1H), 8.35 (d, $J$ = 8.0 Hz, 1H), 8.10 (d, $J$ = 7.2 Hz, 1H), 7.89 - 7.81 (m, 1H), 7.71 - 7.62 (m, 1H), 7.58 - 7.51 (m, 1H), 7.33 (d, $J$ = 7.2 Hz, 1H), 7.20 (t, $J$ = 8.0 Hz, 1H), 7.17 - 7.12 (m, 1H), 7.12 - 7.02 (m, 3H), 5.48 - 5.39 (m, 1H), 4.39 (s, 2H), 3.54 - 3.43 (m, 2H), 3.35 - 3.32 (m, 4H), 3.29 - 3.10 (m, 4H), 3.07 - 2.87 (m, 1H), 2.81 - 2.70 (m, 1H), 2.68 - 2.60 (m, 1H), 2.16 - 1.97 (m, 1H).

**Example 18.** Compound **19**

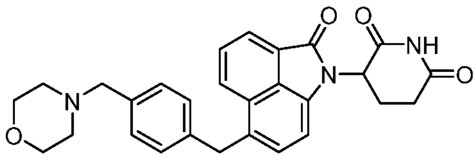

**19**

LCMS (ESI$^+$): m/z 470.4 $[M+H]^+$.

$^1$H NMR (400 MHz, DMSO-$d_6$) $\delta$ 11.13 (s, 1H), 8.34 - 8.30 (m, 1H), 8.10 - 8.05 (m, 1H), 7.85 - 7.77 (m, 1H), 7.43 - 7.36 (m, 1H), 7.27 - 7.15 (m, 4H), 7.13 - 7.07 (m, 1H), 5.49 - 5.41 (m, 1H), 4.40 - 4.34 (m, 2H), 3.54 - 3.50 (m, 4H), 3.37 (s, 2H), 3.01 - 2.89 (m, 1H), 2.81 - 2.69 (m, 1H), 2.67 - 2.61 (m, 1H), 2.32 - 2.21 (m, 4H), 2.13 - 2.04 (m, 1H).

**Example 19.** Compound **20**

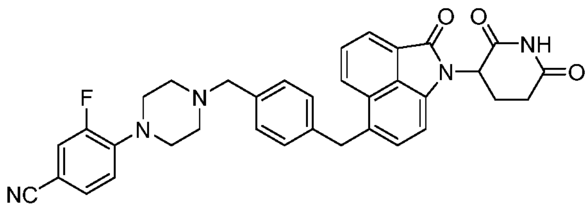

**20**

LCMS (ESI$^+$): m/z 588.1 $[M+H]^+$.

$^1$H NMR (400 MHz, DMSO-$d_6$) $\delta$ 11.09 - 11.24 (s, 1H), 8.26 - 8.48 (m, 1H), 8.03 - 8.13 (m, 1H), 7.78 - 7.88 (m, 1H), 7.65 - 7.73 (m, 1H), 7.52 - 7.59 (m, 1H), 7.38 - 7.45 (m, 1H), 7.17 - 7.31 (m, 4H), 7.02 - 7.15 (m, 2H), 5.35 - 5.62 (m, 1H), 4.39 (s, 2H), 3.45 (s, 2H), 3.08 - 3.18 (m, 4H), 2.90 - 2.99 (m, 1H), 2.74 - 2.81 (m, 1H), 2.64 - 2.68 (m, 1H), 2.43 - 2.49 (m, 4H), 2.04 - 2.15 (m, 1H).

**Example 20:** Compound 16

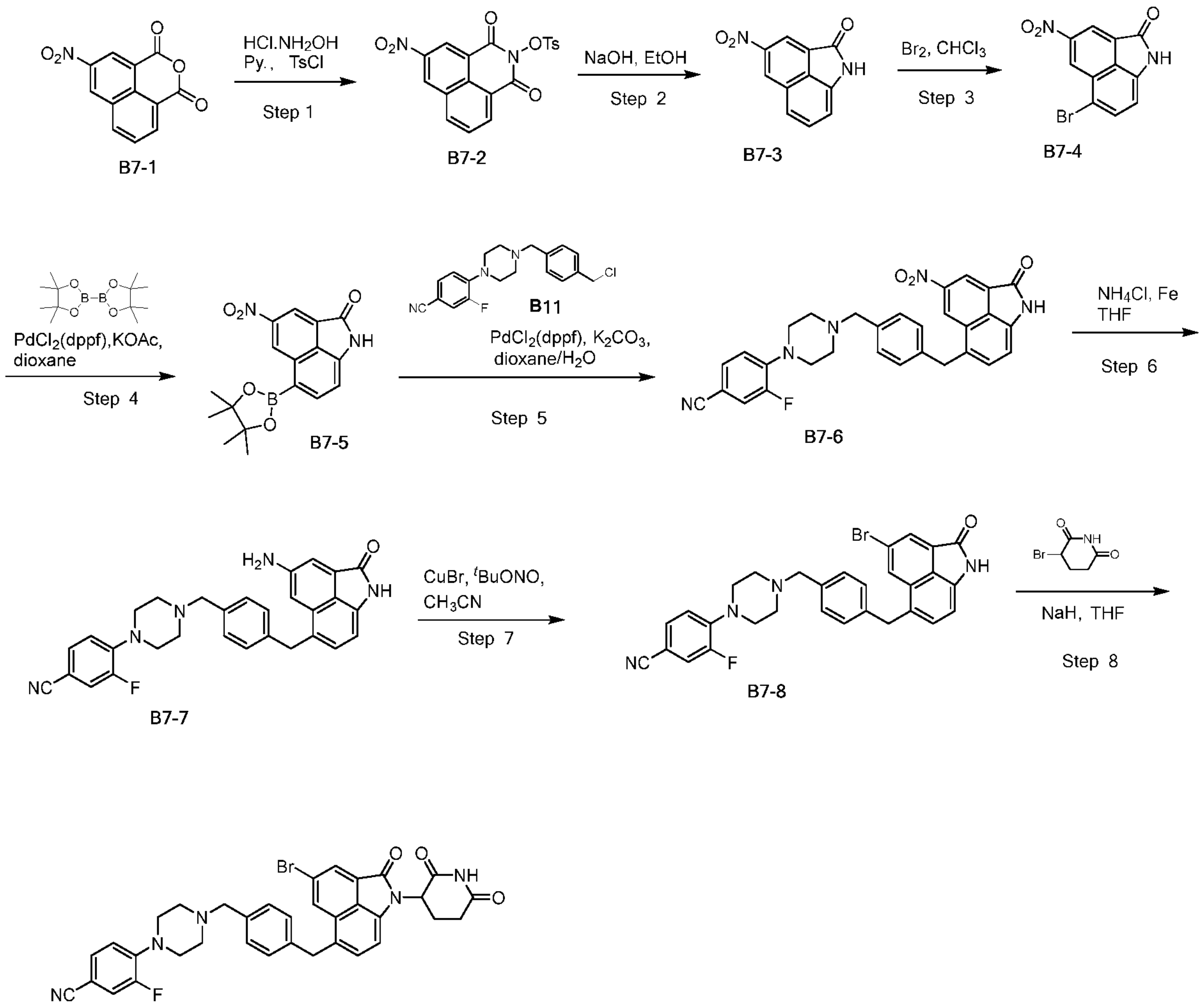


16

**Step1:** Synthesis of **B7-2**

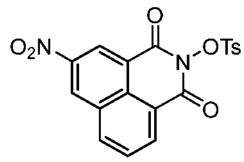

B7-2

A pale yellow suspension of intermediate **B7-1** (21.0 g, 86.4 mmol), hydroxylamine hydrochloride (6.00 g, 86.4 mmol) and pyridine (70 mL) was stirred at 100 °C for 1 hours. Then TosCl (32.9 g, 173 mmol) was added and the yellow suspension was stirred at 80 °C for 2 hours. The reaction mixture was concentrated to give a residue and then ice water (150 mL) was added and the yellow suspension was filtered. The filtered cake was concentrated to give a residue. Intermediate **B7-2** (35.5 g, 99.7% yield) was obtained as a pale yellow solid.

$^1$H NMR(400 MHz, DMSO-$d_6$) $\delta$ 9.56 (d, *J* = 2.4 Hz, 1H), 8.91 (d, *J* = 2.4 Hz, 1H), 8.88 - 8.82 (m, 1H), 8.67 (dd, *J* = 0.8, 7.2 Hz, 1H), 8.09 (t, *J* = 7.6 Hz, 1H), 7.95 (d, *J* = 8.4 Hz, 2H), 7.53 (d, *J* = 8.0 Hz, 2H), 2.48 (s, 3H).

**Step 2:** Synthesis of **B7-3**

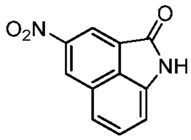

B7-3

To a brown suspension of intermediate **B7-2** (15.6 g, 37.83 mmol) in EtOH (64 mL) and $H_2O$ (50 mL) was added NaOH (2.7 M, 60.3 mL) and the reaction mixture was stirred at 100 °C for 1 hours. The reaction mixture was cooled to 80 °C. 12M HCl was added until pH=3 and the yellow suspension was filtered. The filtered cake was concentrated to afford intermediate **B7-3** (8.1 g, crude) as a yellow solid, without further purification for next step.
LCMS ($ESI^+$): m/z 215.1 $[M+H]^+$.

**Step 3:** Synthesis of **B7-4**

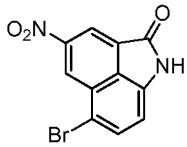

B7-4

To a yellow suspension of intermediate **B7-3** (8.1 g, crude) in $CHCl_3$ (80 mL) was added $Br_2$ (3.06 g, 19.12 mmol, 986 uL) at 0 °C. The yellow suspension was stirred at 20 °C for 16 hours. The reaction mixture was concentrated to give a residue. Intermediate **B7-4** (11.2 g, crude) was obtained as a yellow solid.
$^1$H NMR (400 MHz, DMSO-$d_6$) $\delta$ 11.22 (br s, 1H), 8.79 (d, *J*= 1.2 Hz, 1H), 8.59 (d, *J* = 1.2 Hz, 1H), 7.88 (d, *J* = 7.6 Hz, 1H), 7.04 (d, *J* = 7.6 Hz, 1H).

**Step 4:** Synthesis of **B7-5**

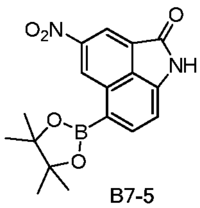

B7-5

A yellow suspension of intermediate **B7-4** (11.2 g, crude), Pd(dppf)$Cl_2$ (1.40 g, 1.92 mmol), KOAc (5.62 g, 57.3 mmol) and 4,4,4',4',5,5,5',5'-octamethyl-2,2'-bi(1,3,2-dioxaborolane) (7.62 g, 30.0 mmol) in dioxane (80 mL) was degassed for 3 times with $N_2$ and stirred at 90 °C for 16 hours. The reaction mixture was diluted with DCM (30 mL), filtered through a short silica gel column and the filtrate was concentrated to give intermediate **B7-5** (13.0 g, crude) as a yellow solid. The crude product was used for the next step without further purification.

**Step 5:** Synthesis of **B7-6**

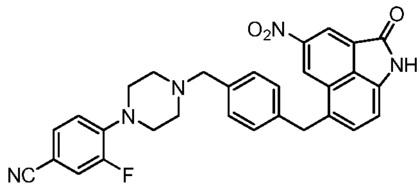

B7-6

A brown suspension of 4-[4-[[4-(chloromethyl)phenyl]methyl]piperazin-1-yl]-3-fluoro-benzonitrile (5.50 g, 16.00 mmol), 4-nitro-6-(4,4,5,5-tetramethyl-1,3,2-dioxaborolan-2-yl)benzo[cd]indol-2(1H)-one **B7-5** (13.0 g, crude), Pd(dppf)$Cl_2$ (1.76 g, 2.40 mmol) and $K_2CO_3$ (6.63 g, 48.0 mmol) in dioxane (50 mL) and $H_2O$ (2.5 mL) was exchanged with $N_2$ for 3 times and stirred at 80 °C for 16 hours under $N_2$. The reaction mixture was concentrated to give a residue. The residue was purified by column chromatography (PE: EA=3:1 to 1:1). Intermediate **B7-6** (1.60 g, 19.2% yield) was obtained as a yellow solid.

LCMS (ESI$^+$): m/z 522.3 [M+H] $^+$.
$^1$H NMR (400 MHz, DMSO-$d_6$) δ11.10 (s, 1H), 9.12 (d, *J* = 1.2 Hz, 1H), 8.56 (d, *J* = 1.2 Hz, 1H), 7.67 (dd, *J* = 1.6, 13.4 Hz, 1H), 7.55 (dd, *J* = 1.6, 8.4 Hz, 1H), 7.49 (d, *J* = 7.2 Hz, 1H), 7.28 - 7.21 (m, 4H), 7.14 (d, *J* = 7.2 Hz, 1H), 7.08 (t, *J* = 8.8 Hz, 1H), 4.46 (s, 2H), 3.46 (s, 2H), 3.18 - 3.09 (m, 4H), 2.50 - 2.44 (m, 4H).

**Step 6:** Synthesis of **B7-7**

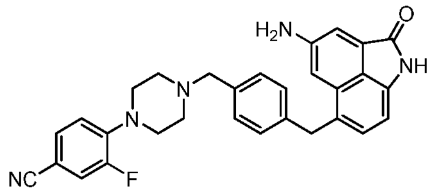

B7-7

A brown suspension of intermediate **B7-6** (660 mg, 1.27 mmol), Fe (707 mg, 12.7 mmol) and $NH_4Cl$ (677 mg, 12.7 mmol) in THF (10 mL) and $H_2O$ (3 mL) was stirred at 80 °C for 2 hours. The reaction mixture was diluted with DCM/MeOH (10:1, 20 mL). The organic layer was dried over $Na_2SO_4$, filtered and concentrated to give a residue. The residue was purified by column chromatography (PE: EA=1:1 to 0:1) to afford intermediate **B7-7** (350 mg, 56.3% yield) as a yellow solid.

**Step 7:** Synthesis of **B7-8**

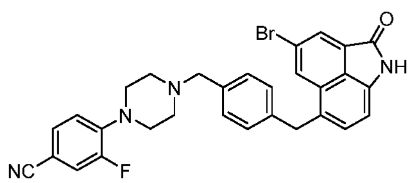

B7-8

A yellow suspension of intermediate **B7-7** (240 mg, 488 μmol) and tert-butyl nitrite (75.5 mg, 732 μmol, 87 μL) in MeCN (10 mL) was stirred at 25 °C for 30 min. Then the reaction mixture was cooled to 0 °C and CuBr (70.0 mg, 488 μmol) was added. The reaction mixture was allowed to warm to 25 °C and stirred at 25 °C for 16 hours. The reaction mixture was diluted with DCM/MeOH (10:1, 40 mL), washed with water (30 mL). The organic layer was dried over $Na_2SO_4$, filtered and concentrated to give a residue. The residue was purified by column chromatography (DCM: MeOH=1:0 to 20:1). Intermediate **B7-8** (80 mg, 113.78 umol, 23.3% yield) was obtained as a yellow solid.
LCMS (ESI$^+$): m/z 555.5 [M+H] $^+$.

**Step 8:** Synthesis of compound **16**

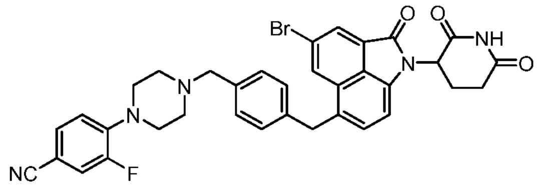

16

A yellow suspension of intermediate **B7-8** (80.0 mg, 144 μmol) in THF (2 mL) was stirred at 0 °C for 30 min. Then 3-bromopiperidine-2,6-dione (194 mg, 1.01 mmol) was added at 0 °C. The yellow suspension was allowed to warm to 60 °C and stirred at 60 °C for 2 hours. The reaction mixture was diluted with EA (50 mL) and quenched by ice water (50 mL). The organic layer was dried over $Na_2SO_4$, filtered and concentrated to give a residue. The residue was purified by prep-TLC (PE: EA=1:2). Compound **16** (18.0 mg, 18.3% yield) was obtained as a yellow solid.

LCMS (ESI$^+$): m/z 668.0[M+H]$^+$.
$^1$H NMR (400 MHz, DMSO-$d_6$) δ11.15 (s, 1H), 8.53 (s, 1H), 8.22 (s, 1H), 7.67 (d, *J* = 13.6 Hz, 1H), 7.55 (d, *J*= 8.4 Hz, 1H), 7.43 (d, *J*= 7.2 Hz, 1H), 7.23 (s, 4H), 7.15 (d, *J* = 7.2 Hz, 1H), 7.08 (t, *J* = 8.8 Hz, 1H), 5.44 (dd, *J* = 5.2, 12.4 Hz, 1H), 4.37 (s, 2H), 3.46 (s, 2H), 3.14 (br s, 4H), 3.02 - 2.86 (m, 1H), 2.79 - 2.70 (m, 1H), 2.68 - 2.62 (m, 1H), 2.49 - 2.41 (m, 4H), 2.15 - 2.07 (m, 1H).

**Degradation Assay for IKZF1 or SALL4 (HiBiT tag bioluminescence assay)**

The following is a general method for testing compounds-induced degradation of HiBiT-tagged IKZF1 or SALL4 overexpressed in a human cell line, for example, the HT-1080 cell line (American Type Culture Collection (ATCC), catalog number CCL-121). The HT-1080 IKZF1(or SALL4) HiBiT reporter cell line was engineered via lentiviral transduction to stably overexpress GSPT1(Δ1-138)/G575N and HiBiT-tagged IKZF1 or SALL4. The HT-1080 IKZF1(or SALL4) HiBiT reporter cell line was maintained in DMEM media supplemented with 10% fetal bovine serum, 1X sodium pyruvate, 1X non-essential amino acids, 1X glutamine, 100 U/ml penicillin, and 100 ug/mL streptomycin. The HT-1080 IKZF1(or SALL4) HiBiT reporter cells were seeded into 384-well plates (Cat# 3764, Corning) pre-spotted with DMSO or test compounds. Increasing concentrations of test compounds (ranging from 15.81 pM to 500 nM or from 0.3162 nM to 10 μM at a 3.162-fold increment) or DMSO were dispensed into an empty 384-well plate using an Echo 650 Acoustic Liquid Handler (Beckman Coulter). About 10,000 cells in complete DMEM cell culture media were seeded per well. Assay plates were incubated at 37°C with 5% $CO_2$ for 20 hours, followed by the assessment of IKZF1 or SALL4 degradation using the Nano-Glo HiBiT Lytic Detection Reagent (Cat# N3050, Promega) according to the manufacturer's instruction. Luminescence was then measured with an EnVision plate reader (PerkinElmer) or a PHERAstar plate reader (BMG Labtech). All IKZF1 or SALL4 degradation curves were processed using Collaborative Drug Discovery Vault softwares. IKZF1 or SALL4 levels in compound-treated wells were normalized to that of DMSO control and expressed as Percent of Control (PoC, y). A four Parameter Logistic Model was used to determine the compound's $EC_{50}$ and $DC_{50}$, using the following equation:

$$y = (A+ ((B-A)/ (1+ ((C/x)^A D))))$$

wherein,
A = $Y_{min}$ (lowest IKZF1 or SALL4 level normalized to DMSO control in response to compound treatment, as determined by curve fit)
B = $Y_{max}$ (IKZF1 or SALL4 level in DMSO control)
C = $EC_{50}$ half degradation concentration
D = Hill Slope
x = compound concentration
$EC_{50}$ = the concentration of compound when y = $(Y_{max}-Y_{min})/2$
$DC_{50}$ = test sample concentration when IKZF1 or SALL4 readings y = 50% DMSO-treated samples
y = IKZF1 or SALL4 readings after test sample treatment (normalized to IKZF1 or SALL4 readings in DMSO-treated samples)

$$D_{max} = (1- Y_{min}/Y_{max}) * 100\%$$

$D_{max}$ represents the maximum percentage of IKZF1 or SALL4 protein degradation induced by compound treatment.

Table 1. IKZF1 and SALL4 degradation $DC_{50}$ and $D_{max}$ values of selected compounds.

| **Compound No.** | **HiBiT IKZF1:** | **HiBiT IKZF1:** | **HiBiT SALL4:** | **HiBiT SALL4:** |
|---|---|---|---|---|
| | **$DC_{50}$ (nM)** | **$D_{max}$(%)** | **$DC_{50}$ (μM)** | **$D_{max}$(%)** |
| 1 | 0.025 | 93.2 | 0.117 | 57.4 |
| 2 | 0.022 | 91.8 | 0.4 | 53.2 |
| 3 | 0.038 | 93.3 | 0.0024 | 68 |
| 4 | 0.026 | 93.5 | 0.001 | 68.6 |
| 5 | 0.017 | 94.1 | 0.002 | 76.5 |
| 6 | 0.74 | 74.6 | >10 | 33.7 |
| 7 | 0.13 | 86.3 | >10 | 45.4 |
| 8 | 2.37 | 75.2 | >10 | 34.9 |
| 9 | 0.074 | 91.6 | 0.032 | 58.1 |
| 9-A | 0.23 | 89.9 | 0.328 | 55.2 |
| 9-B | 0.134 | 90.9 | 0.104 | 59.5 |
| 10 | 0.065 | 88.3 | >10 | 29.4 |
| 11 | 2.19 | 66.2 | >10 | 33.9 |
| 12 | 5.89 | 69.3 | >10 | 32.5 |
| 13 | 1.22 | 87.3 | >10 | 49.1 |
| Pomalidomide | 0.266 | 79.5 | 0.332 | 69.7 |

Table 2. IKZF1 and SALL4 degradation $DC_{50}$ and $D_{max}$ values of selected compounds

| **Compound No.** | **HiBiT IKZF1: $DC_{50}$ (nM)** | **HiBiT IKZF1: $D_{max}$ (%)** | **HiBiT SALL4: $DC_{50}$ (μM)** | **HiBiT SALL4: $D_{max}$ (%)** |
|---|---|---|---|---|
| 9-A | 0.185 | 93.1 | 0.292 | 57.0 |
| 9-B | 0.102 | 93.8 | 0.102 | 59.4 |
| 14 | 1.0 | 89.9 | 0.002 | 79.3 |
| 15 | 39.1 | 60.1 | >10 | 28.5 |
| 16 | 1.23 | 92.2 | 0.772 | 65.8 |
| 17 | >10 | 41.1 | >10 | 11.4 |
| 18 | 0.014 | 96.1 | <0.0003 | 80.5 |
| 19 | 0.157 | 93.5 | 0.0052 | 64.6 |
| 20 | 0.014 | 96.9 | <0.0003 | 79.3 |
| CC-92480 | 0.105 | 95.5 | 0.0034 | 75.8 |
| Note: 1) Data shown in Table 1 and Table 2 were performed once with technical duplicates. Some selected compounds in Table 1 were tested on different days, while all selected compounds in Table 2 were tested on the same day.<br>2) Pomalidomide (shown in Table 1) and CC-92480 (shown in Table 2) are known compounds with the following formula: | | | | |

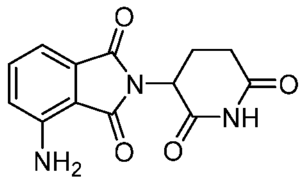

**Pomalidomide**

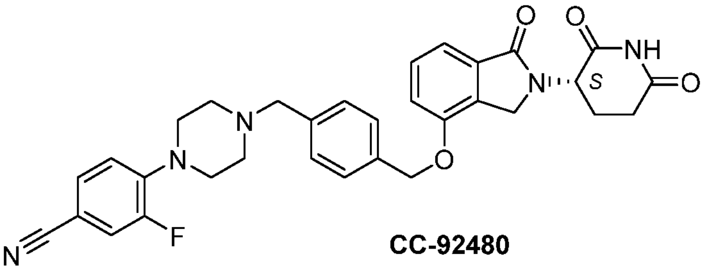

**CC-92480**

( *J. Med. Chem.* **2020**, 63, *13*, 6648–6676

**Comparison of IKZF1 protein degradation kinetics induced by compounds 9-B and 9-A**

The IKZF1 protein degradation kinetics of compounds 9-B and 9-A was evaluated using the IKZF1 HiBiT degradation assay as described above except that the incubation time was changed to 1, 2 or 4 hours.

The result shown that, compound 9-B is much more potent than compound 9-A in inducing IKZF1 degradation. After 1 hour of compound incubation, 9-B exhibited a $D_{max}$ of ~77% and a $DC_{50}$ of ~0.4 nM, while 9-A exhibited a $D_{max}$ of ~68% and a $DC_{50}$ of ~8 nM. After 4 hours of incubation, 9-A showed improved but still lower IKZF1 degradation potency and efficacy as compared to 9-B (9-A: $D_{max}$ = 88%, $DC_{50}$ = 0.6 nM; 9-B: $D_{max}$ = 92%, $DC_{50}$ < 0.2 nM). See Figure 1.

**MM.1S cell proliferation assay**

The following is a method for testing the antiproliferative activities of compounds in multiple myeloma cell lines, for example, the MM.1S (ATCC Cat# CRL-2974). This cell line was cultured in RPMI-1640 medium supplemented with 10% FBS, 1X sodium pyruvate, 1X non-essential amino acids, 1X glutamine, 100 U/ml penicillin, and 100 ug/mL streptomycin. MM. 1S cells were seeded into a 384-well plate (Cat# 3764, Corning), pre-spotted with DMSO or compounds. Increasing concentrations of test compounds ranging from 0.3162 pM to 10 nM at a 3.162-fold increment in a 10-point dose response assay or DMSO were dispensed into an empty 384-well plate using an a D300e digital dispenser (Tecan) or an Echo 650 Acoustic Liquid Handler (Beckman Coulter). About 3,000 cells in 50 μL of culture medium were seeded per well. Assay plates were incubated at 37 °C with 5% CO2 for 120 hours. After that, 20 μL of the CellTiter-Glo Luminescent Cell Viability Assay Reagent (Cat# G7573, Promega) was added to each well and incubated at room temperature for 30 minutes. Luminescence was then measured with an EnVision plate reader (PerkinElmer) or a PHERAstar plate reader (BMG LABTECH). All percentage of cell growth inhibition curves were processed using Collaborative Drug Discovery Vault softwares. Cell viability reads in compound-treated wells were normalized to that of DMSO control and expressed as Percent of Control (PoC, y). A four Parameter Logistic Model was used to determine the compound's EC50 and DC50, using the following equation:

$$y = (A+ ((B-A)/ (1+ ((C/x)^A D))))$$

wherein,

A = $Y_{min}$ (lowest cell viability read normalized to DMSO control in response to compound treatment, as determined by curve fit)
B = $Y_{max}$ (the maximum value, cell viability read in DMSO control)
C = $EC_{50}$ half inhibitory concentration
D = Hill Slope
x = compound concentration
$EC_{50}$ = the concentration of compound when y = $(Y_{max}-Y_{min})/2$
$IC_{50}$ = the concentration of the compound when y = 50% of DMSO control
y = cell viability read normalized to DMSO control

Table 3. Antiproliferative $IC_{50}$ and $Y_{min}$ values of selected compounds in MM.1S

| The compound | MM.1S: $IC_{50}$ (nM) | $Y_{min}$:(%) |
|---|---|---|
| **9** | 0.04 (n = 2) | 4.0 (n = 2) |
| **9-B** | 0.022 (n = 3) | 5.6 (n = 3) |
| **CC-92480** | 0.041 (n = 7) | 7.5 (n = 7) |
| **Pomalidomide** | >30 (n = 1) | ND (n = 1) |
| Note: 1) n, the biological replicates of each assay.<br>2) ND, not determined. The maximum concentration of Pomalidomide tested was not high enough for accurate determination of $Y_{min}$. | | |

**Pharmacokinetic study in mice**

Objective: The objective of this study was to determine the pharmacokinetics of test compound following a single intravenous (IV) bolus dose and a single oral (PO) administration in male CD1 mice by quantifying the concentrations of the test compound at different times in plasma using LC/MS/MS method.

Materials: Male CD1 mouse (20-30g, 6-8 weeks, Zhejiang Vital River Laboratory Animal Technology Co., Ltd.).

The clear solution of the test article was injected into CD1 mice via tail vein (free access to food and water), or orally administrated into CD1 mice (free access to food and water). After administration, blood was collected from the dorsal metatarsal vein at 5 min (0.0833h, IV only) , 15 min (0.25h) , 30 min (0.5 h) , 60 min (1h) , 2h, 4h, 6h, 8h and 24h. Blood samples were placed in an anticoagulant tube with EDTA-K2 at 4 °C, mixed adequately and centrifuged at 4000 g for 5 min to get the plasma. The plasma concentration was quantified by LC-MS/MS. The PK parameters were calculated using non-compartmental model by Phoenix WinNonlin 6.1 (Linear/log trapezoidal).

Table 4. In-vivo PK of Compound 9 in mice

| | | |
|---|---|---|
| IV (2 mg/kg) | Cl (mL/min/kg) | 1.57 |
| | $t_{1/2}$ (hr) | 10.2 |
| | $V_{ss}$ (L/kg) | 1.32 |
| | AUC $_{last}$(h*ng/mL) | 17203 |
| PO (10 mg/kg) | Cmax (ng/mL) | 1463 |
| | $t_{1/2}$ (hr) | NC |
| | AUC $_{last}$ (h*ng/mL) | 23298 |
| | F (%) | 27.1 |
| Note: NC: not calculated | | |

Table 5. In-vivo PK of Compound **9-B** in mice

| | | |
|---|---|---|
| PO (10 mg/kg) | $C_{max}$ (ng/mL) | 4390 |
| | $t_{1/2}$ (hr) | 8.30 |
| | $AUC_{last}$ (h*ng/mL) | 73578 |

The present application also includes the technical scheme of any one of the items numbered as follows.

1. A compound represented by structural formula (I),

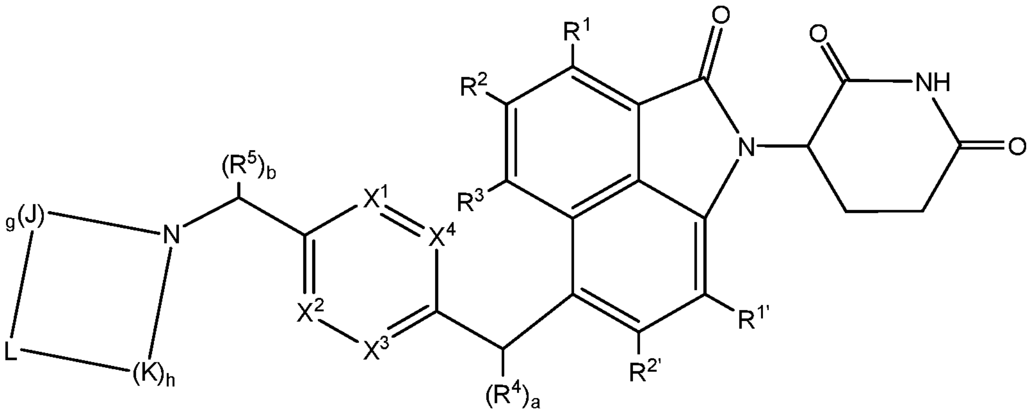

(I)

or a pharmaceutically acceptable salt thereof,
wherein:

$R^1$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^1$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^2$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{2'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^3$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino,

mercapto, -COOH, or $C_1$-$C_6$ alkyl;

$R^4$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^5$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

a is 0, 1 or 2;

b is 0, 1 or 2;

$X^1$, for each occurrence, is independently selected from $CR^{14}$ or N;

$X^2$, for each occurrence, is independently selected from $CR^{14'}$ or N;

$X^3$, for each occurrence, is independently selected from $CR^{14''}$ or N;

$X^4$, for each occurrence, is independently selected from $CR^{14'''}$ or N;

$R^{14}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14''}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14'''}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$

alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

L, for each occurrence, is independently selected from O, $NR^{21}$, $CR^{22}$, $CR^{23}R^{24}$, $NR^{25}$-$CR^{26}$, $CR^{27}$-$CR^{28}$,

$R^{21}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{22}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

when L is $CR^{23}R^{24}$, $R^{23}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{24}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or,

$R^{23}$ and $R^{24}$ are joined together with the connected carbon atom to form a monocyclic, bicyclic or tricyclic saturated or unsaturated ring system with 5-14 ring atoms, wherein 0, 1, 2, 3 or 4 of the ring atoms are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

when L is $NR^{25}$-$CR^{26}$, $R^{25}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{26}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or,

$R^{25}$ and $R^{26}$ are joined together with the connected nitrogen atom and carbon atom to form a monocyclic, bicyclic or tricyclic saturated or unsaturated ring system with 5-14 ring atoms, wherein 0, 1, 2, 3 or 4 of the remaining ring atoms except the nitrogen atom are heteroatoms selected from N, O and S, with remaining ring atoms being carbon, and the ring system is optionally substituted with 1, 2, 3 or 4 halogens, hydroxyl, nitro, cyano, amino, mercapto, - COOH, or $C_1$-$C_6$ alkyl;

when L is $CR^{27}$-$CR^{28}$, $R^{27}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{28}$, for each occurrence, is independently selected from absence, hydrogen, halogen, hydroxyl, nitro, cyano, amino, mercapto, -COOH, $C_1$-$C_6$ alkyl, $C_2$-$C_6$alkenyl, $C_2$-$C_6$alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or

$R^{27}$ and $R^{28}$ are joined together with the connected carbon atoms to form a monocyclic, bicyclic or tricyclic saturated or unsaturated ring system having 5-14 ring atoms, wherein 0, 1, 2, 3 or 4 of the ring atoms are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

J is $NR^{31}$, $CR^{32}R^{3}3$;

g is 0, 1 or 2;

K is $NR^{34}$, $CR^{35}R^{36}$;

h is 0, 1 or 2;

$R^{31}$, for each occurrence, is independently selected from absence, hydrogen, halogen, hydroxyl, nitro, cyano, amino, mercapto, -COOH, $C_1$-$C_6$ alkyl, $C_2$-$C_6$alkenyl, $C_2$-$C_6$alkynyl, $C_1$-$C_6$alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{32}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{33}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{34}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{35}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{36}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

2. The compound or a pharmaceutically acceptable salt thereof of item 1, wherein the compound of structural formula (I) is the compound of structural formula (II):

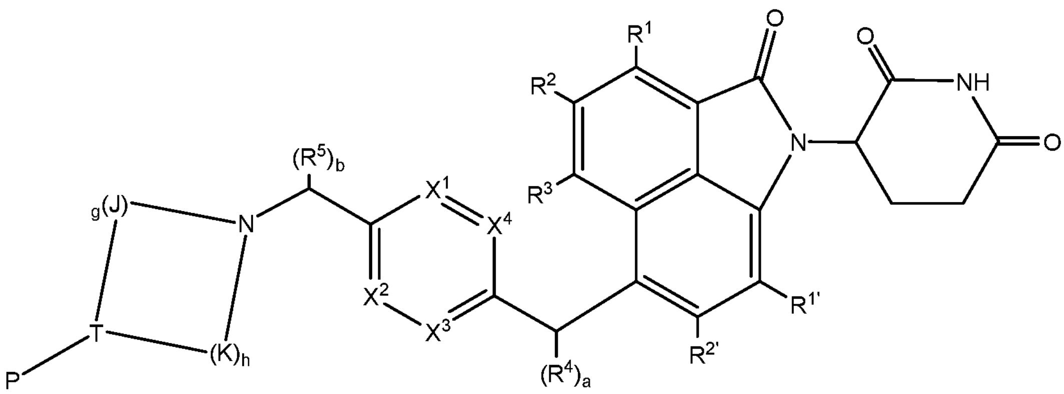

(II),

or a pharmaceutically acceptable salt thereof,
wherein:

T is N or CH;

P, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

3. The compound or a pharmaceutically acceptable salt thereof of item 1, wherein the compound of structural formula (I) is the compound of structural formula (II):

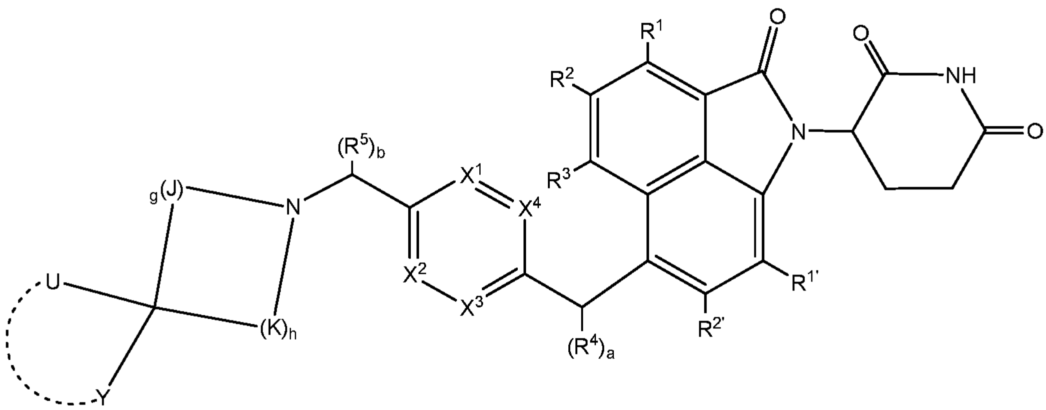

(III),

or a pharmaceutically acceptable salt thereof,
wherein the group

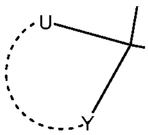

represents a monocyclic, bicyclic or tricyclic saturated or unsaturated ring system having 5-14 ring atoms, U is N, C or O, Y is N, C or O, the ring system further comprises additional 0, 1, 2, 3 or 4 heteroatoms selected from N, O, S, and the ring system is optionally substituted with 1, 2, 3 or 4 halogens, hydroxyl, nitro, cyano, amino, mercapto, -COOH, or $C_1$-$C_6$ alkyl.

4. The compound or a pharmaceutically acceptable salt thereof of item 1, wherein the compound of structural formula (I) is the compound of structural formula (II):

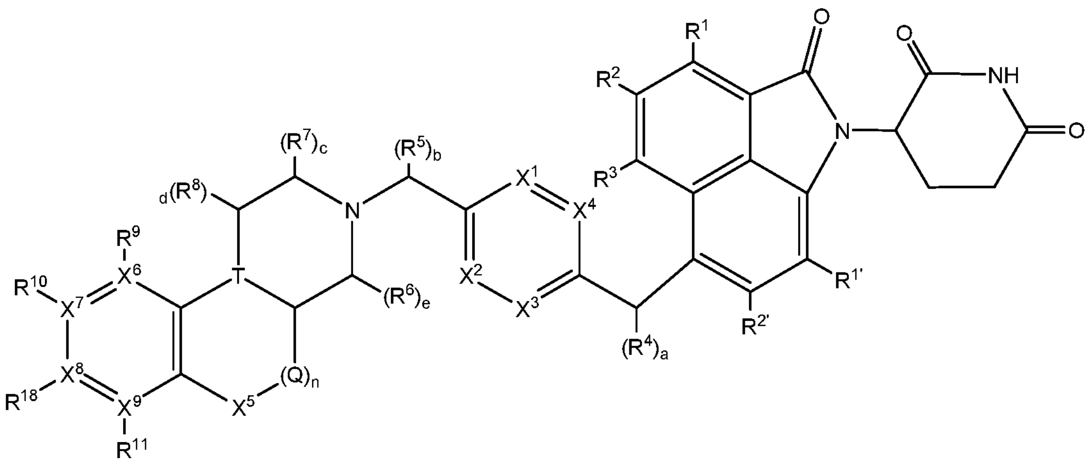

(IV),

or a pharmaceutically acceptable salt thereof,
wherein

$R^6$, for each occurrence, is independently selected from absence, hydrogen, halogen, hydroxyl, nitro, cyano, amino, mercapto, -COOH, $C_1$-$C_6$ alkyl, $C_2$-$C_6$alkenyl, $C_2$-$C_6$alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino,

mercapto, -COOH, or $C_1$-$C_6$ alkyl;

$R^7$, for each occurrence, is independently selected from absence, hydrogen, halogen, hydroxyl, nitro, cyano, amino, mercapto, -COOH, $C_1$-$C_6$ alkyl, $C_2$-$C_6$alkenyl, $C_2$-$C_6$alkynyl, $C_1$-$C_6$alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^8$, for each occurrence, is independently selected from absence, hydrogen, halogen, hydroxyl, nitro, cyano, amino, mercapto, -COOH, $C_1$-$C_6$ alkyl, $C_2$-$C_6$alkenyl, $C_2$-$C_6$alkynyl, $C_1$-$C_6$alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^9$, for each occurrence, is independently selected from absence, hydrogen, halogen, hydroxyl, nitro, cyano, amino, mercapto, -COOH, $C_1$-$C_6$ alkyl, $C_2$-$C_6$alkenyl, $C_2$-$C_6$alkynyl, $C_1$-$C_6$alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{10}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{11}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

c is 0, 1 or 2;

d is 0, 1 or 2;

e is 0, 1 or 2;

Q is $CR^{12}R^{13}$, n is 0, 1 or 2;

$X^5$ is selected from O, $CR^{15}R^{16}$, or $NR^{17}$.

$R^{12}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino,

mercapto, -COOH, or $C_1$-$C_6$ alkyl;

$R^{13}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{15}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{16}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{17}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{18}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

T is N or CH;

$X^6$ isN orC;

$X^7$ is N or C;

$X^8$ is N or C;

$X^9$ is N or C.

5. The compound or a pharmaceutically acceptable salt thereof of any one of the preceding items, wherein the compound is the compound of structural formula (II):

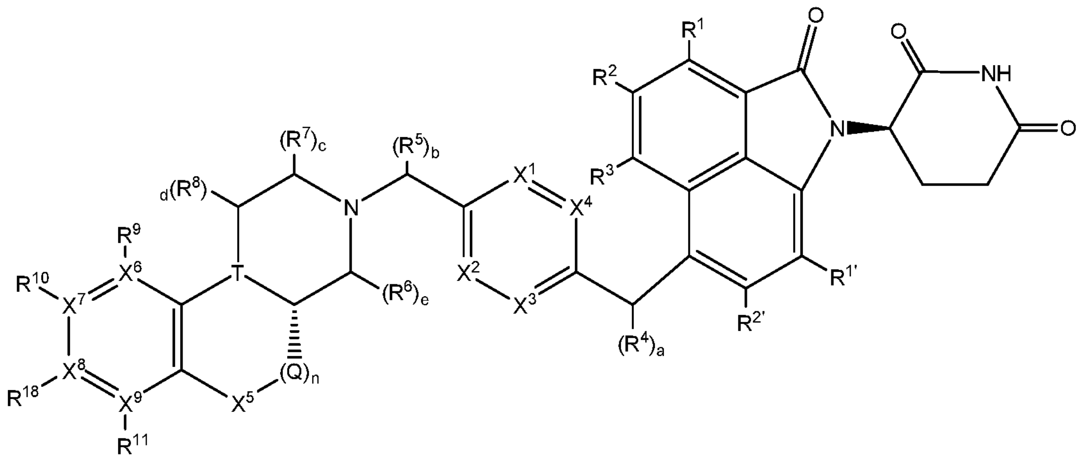

(V)

or a pharmaceutically acceptable salt thereof.

6. The compound or a pharmaceutically acceptable salt thereof of any one of the preceding items, wherein the compound is the compound of structural formula (VI):

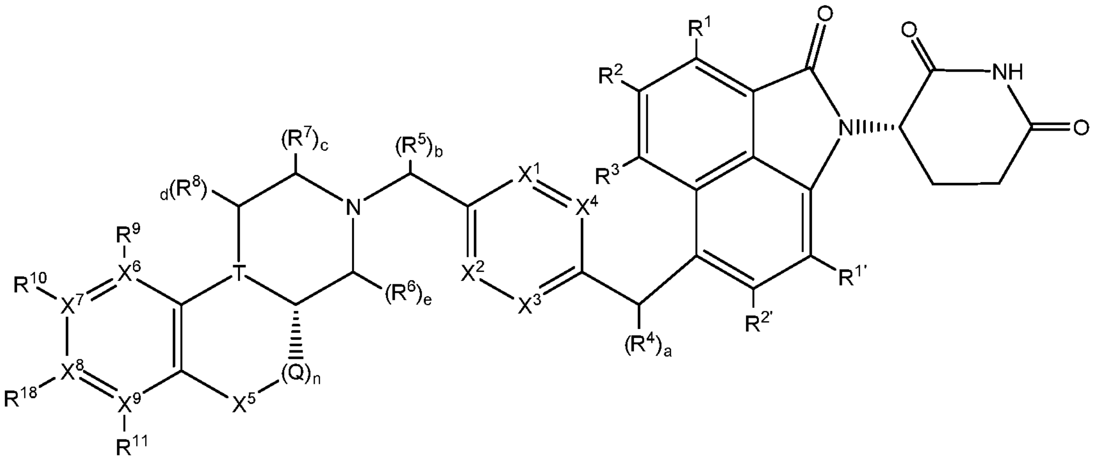

(VI)

or a pharmaceutically acceptable salt thereof.

7. The compound or a pharmaceutically acceptable salt thereof of any one of the preceding items, wherein the compound is the compound of structural formula (VII):

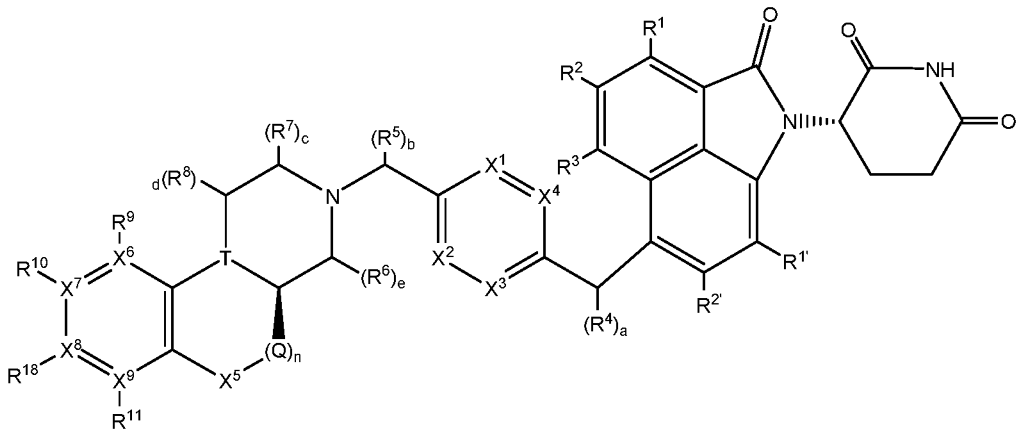

(VII)

or a pharmaceutically acceptable salt thereof.

8. The compound or a pharmaceutically acceptable salt thereof of any one of the preceding items, wherein the compound is the compound of structural formula (VIII):

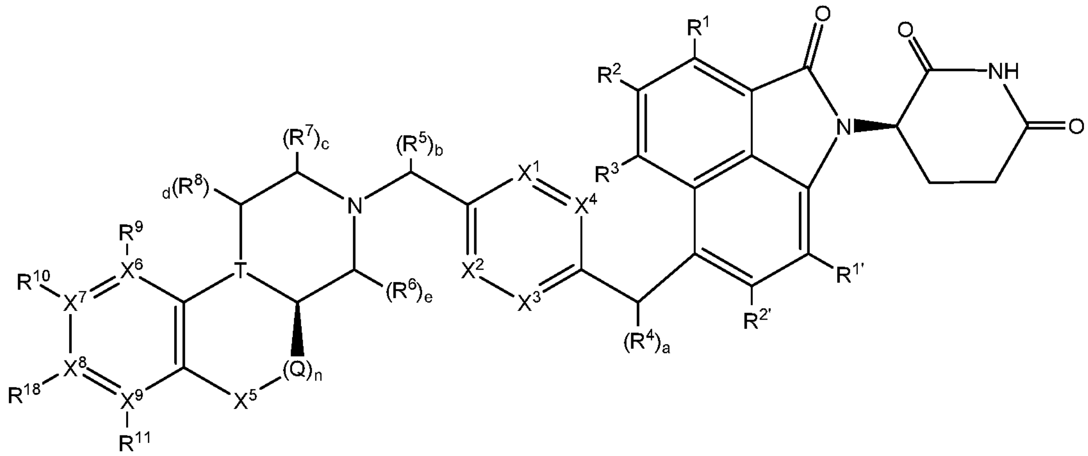

(VIII)

or a pharmaceutically acceptable salt thereof.

9. The compound or a pharmaceutically acceptable salt thereof of any one of the preceding items, wherein the compound is the compound of structural formula (IX):

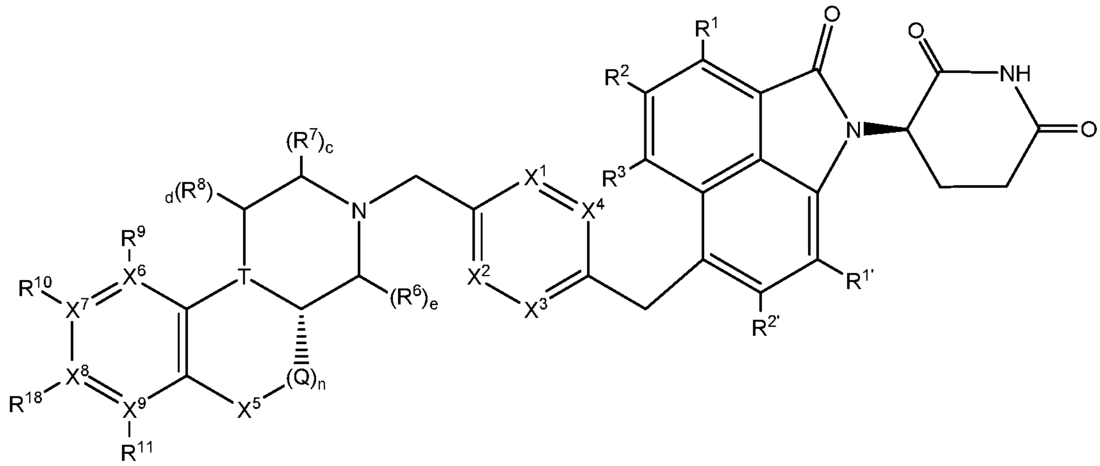

(IX)

or a pharmaceutically acceptable salt thereof.

10. The compound or a pharmaceutically acceptable salt thereof of any one of the preceding items, wherein the compound is the compound of structural formula (X):

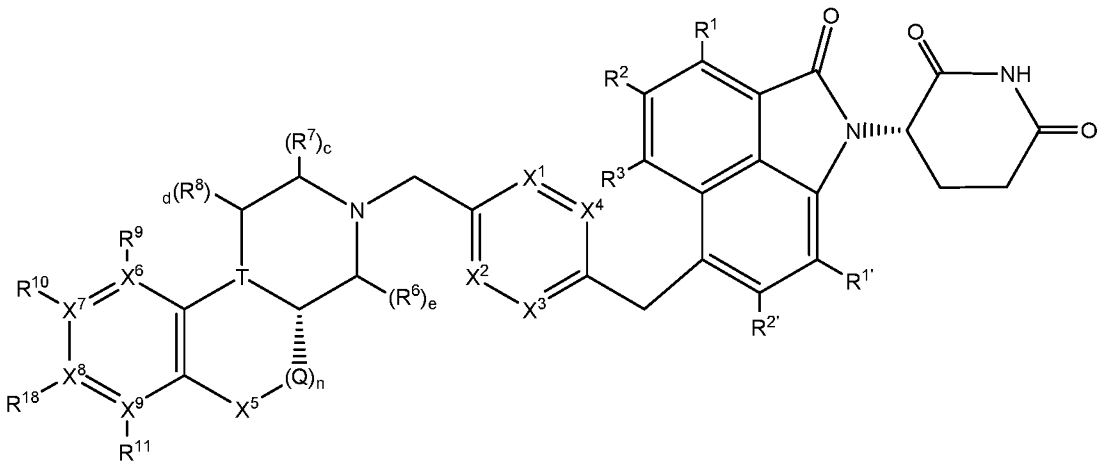

(X)

or a pharmaceutically acceptable salt thereof.

11. The compound or a pharmaceutically acceptable salt thereof of any one of the preceding items, wherein the compound is the compound of structural formula (XI):

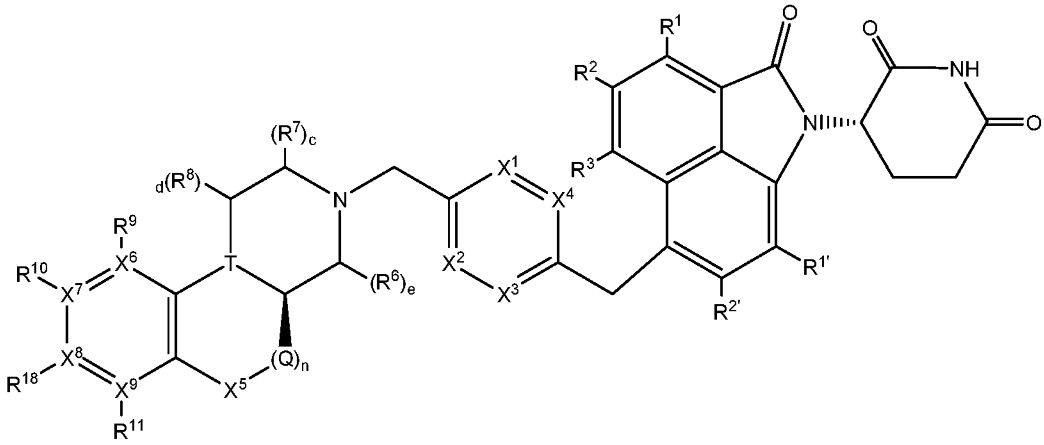

(XI)

or a pharmaceutically acceptable salt thereof.

12. The compound or a pharmaceutically acceptable salt thereof of any one of the preceding items, wherein the compound is the compound of structural formula (XII):

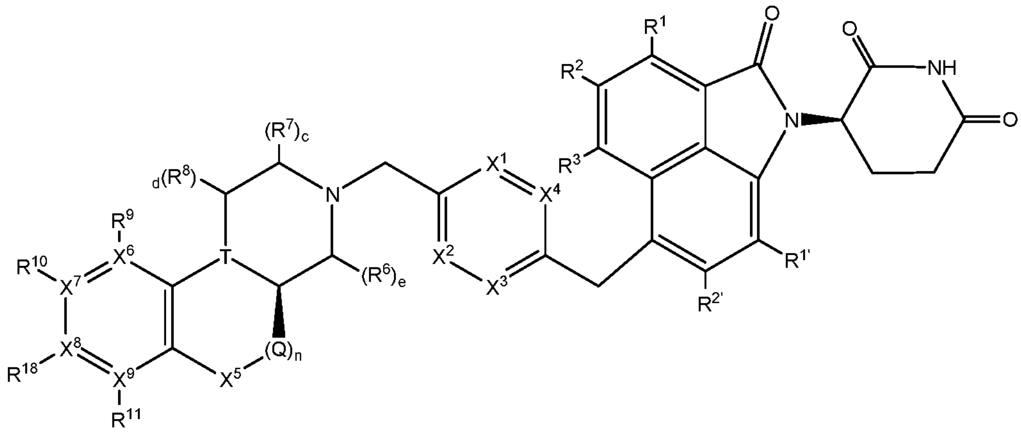

(XII)

or a pharmaceutically acceptable salt thereof.

13. The compound or a pharmaceutically acceptable salt thereof of any one of the preceding items, wherein the compound is the compound of structural formula (XIII):

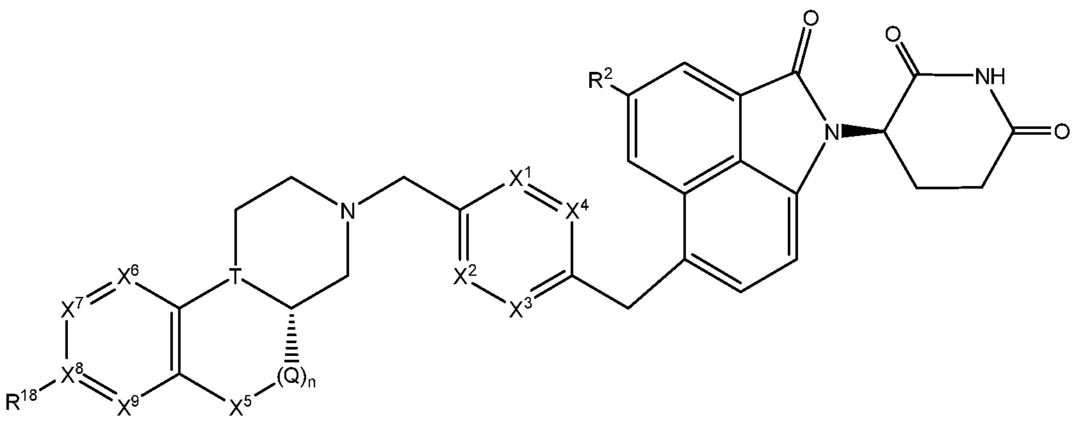

(XIII)

or a pharmaceutically acceptable salt thereof.

14. The compound or a pharmaceutically acceptable salt thereof of any one of the preceding items, wherein the compound is the compound of structural formula (XIV):

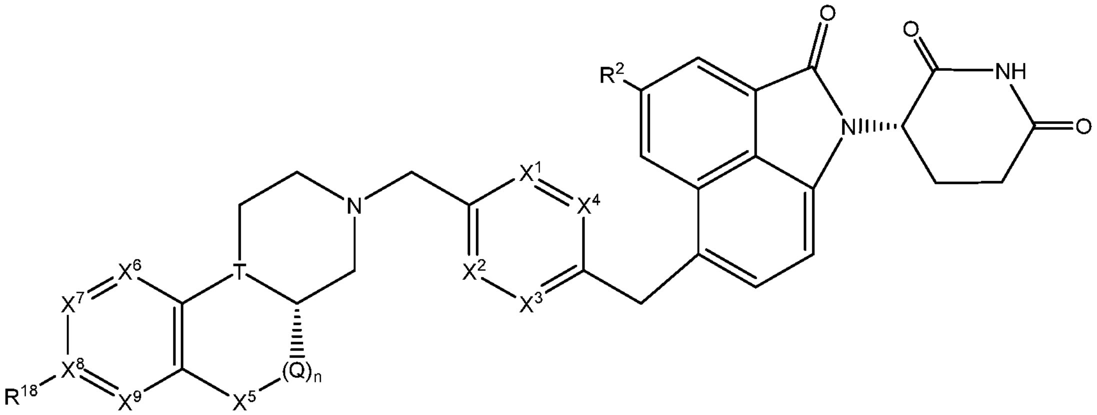

(XIV)

or a pharmaceutically acceptable salt thereof.

15. The compound or a pharmaceutically acceptable salt thereof of any one of the preceding items, wherein the compound is the compound of structural formula (XV):

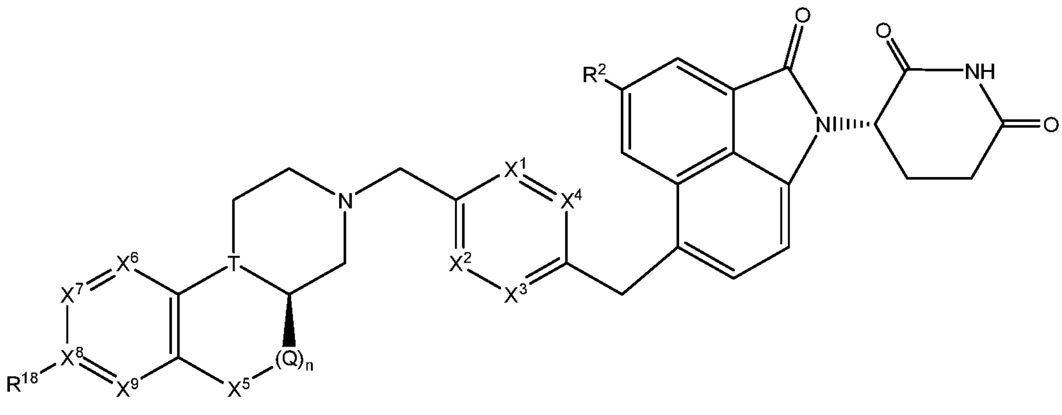

(XV)

or a pharmaceutically acceptable salt thereof.

16. The compound or a pharmaceutically acceptable salt thereof of any one of the preceding items, wherein the compound is the compound of structural formula (XVI):

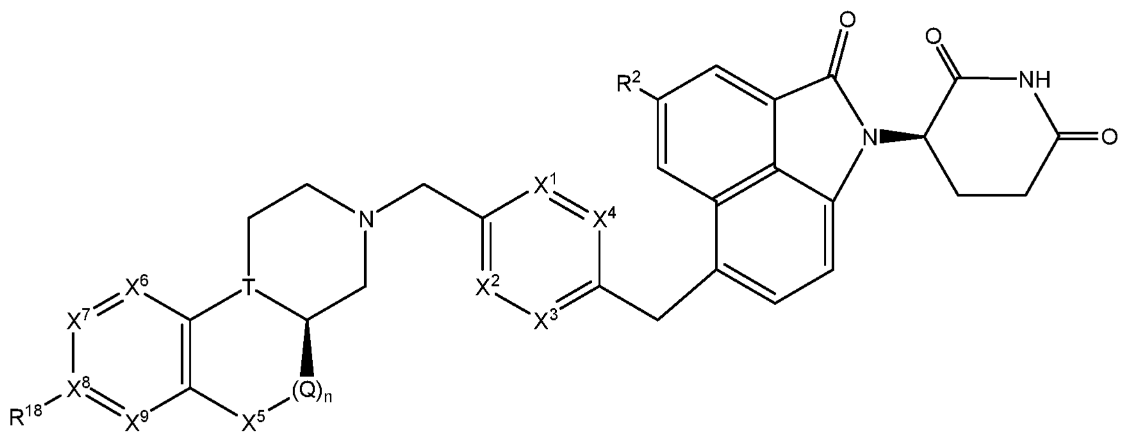

(XVI)

or a pharmaceutically acceptable salt thereof.

17. The compound or a pharmaceutically acceptable salt thereof of any one of the preceding items, wherein the compound is the compound of structural formula (XVII):

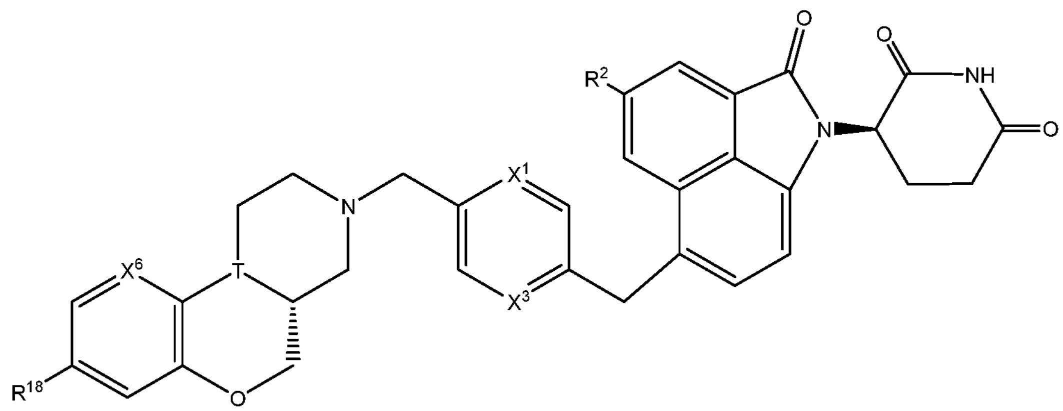

(XVII)

or a pharmaceutically acceptable salt thereof.

18. The compound or a pharmaceutically acceptable salt thereof of any one of the preceding items, wherein the compound is the compound of structural formula (XVIII):

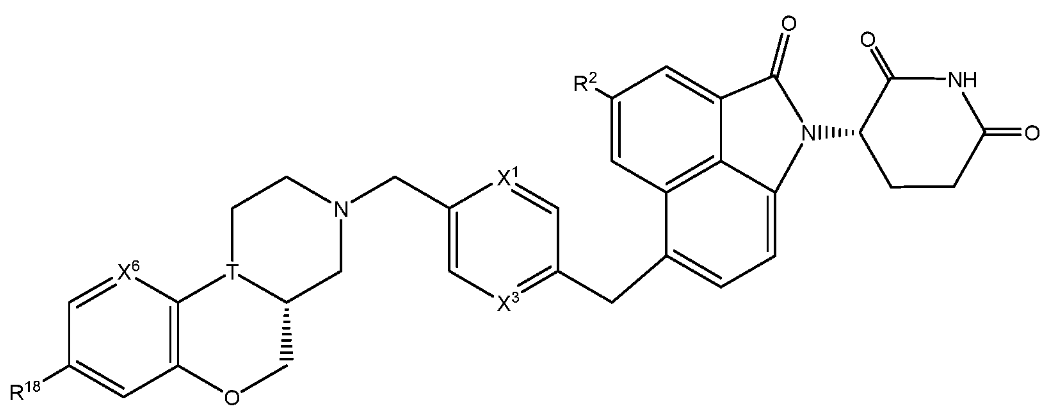

(XVIII)

or a pharmaceutically acceptable salt thereof.

19. The compound or a pharmaceutically acceptable salt thereof of any one of the preceding items, wherein the compound is the compound of structural formula (XIX):

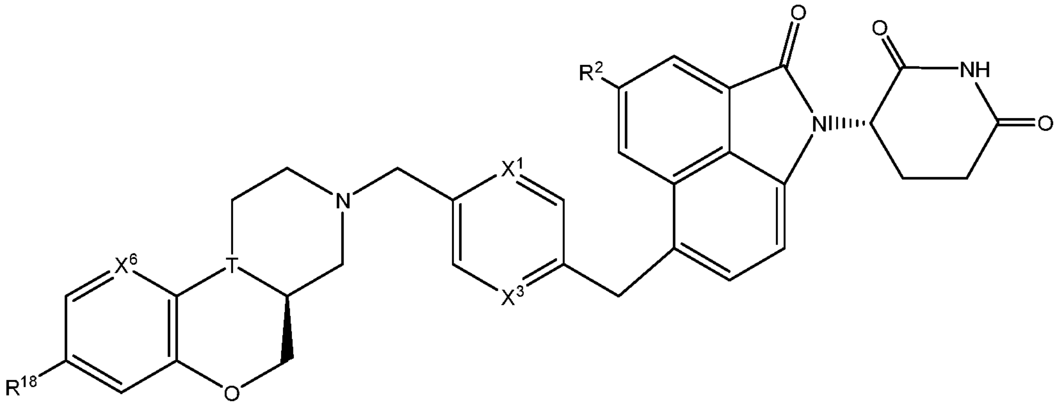

(XIX)

or a pharmaceutically acceptable salt thereof.

20. The compound or a pharmaceutically acceptable salt thereof of any one of the preceding items, wherein the compound is the compound of structural formula (XX):

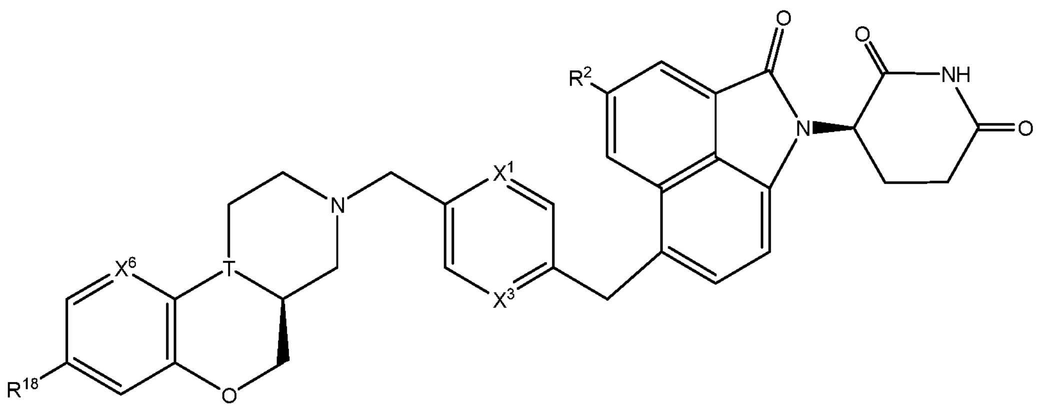

(XX)

or a pharmaceutically acceptable salt thereof.

21. The compound or a pharmaceutically acceptable salt thereof of any one of the preceding items, wherein the compound is the compound of structural formula (XXI):

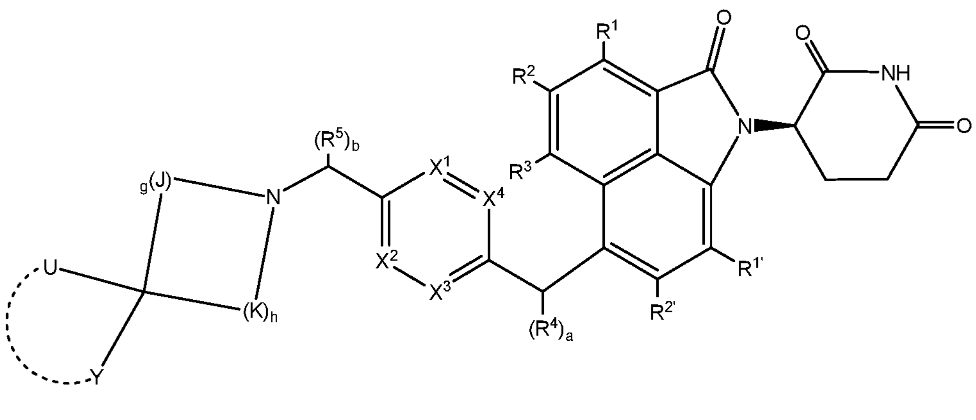

(XXI)

or a pharmaceutically acceptable salt thereof.

22. The compound or a pharmaceutically acceptable salt thereof of any one of the preceding items, wherein the compound is the compound of structural formula (XXII):

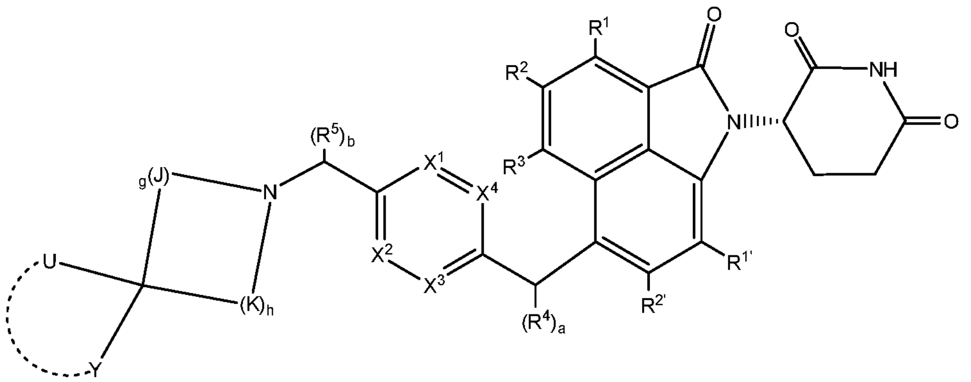

(XXII)

or a pharmaceutically acceptable salt thereof.

23. The compound or a pharmaceutically acceptable salt thereof of any one of the preceding items, wherein the compound is the compound of structural formula (XXIII):

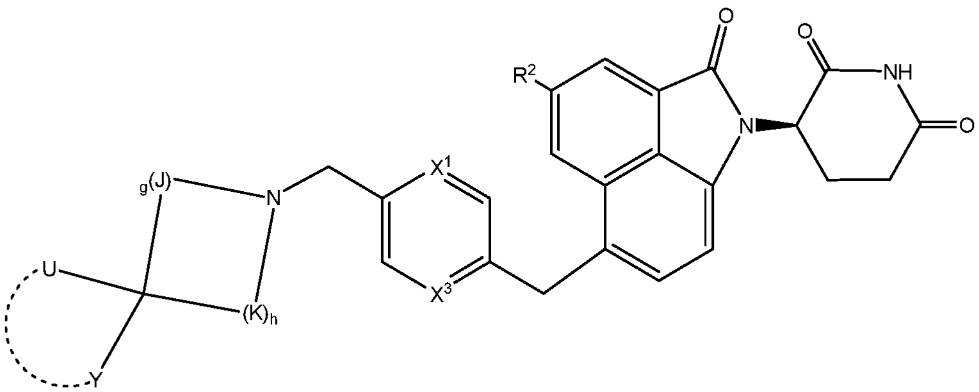

(XXIII)

or a pharmaceutically acceptable salt thereof.

24. The compound or a pharmaceutically acceptable salt thereof of any one of the preceding items, wherein the compound is the compound of structural formula (XXIV):

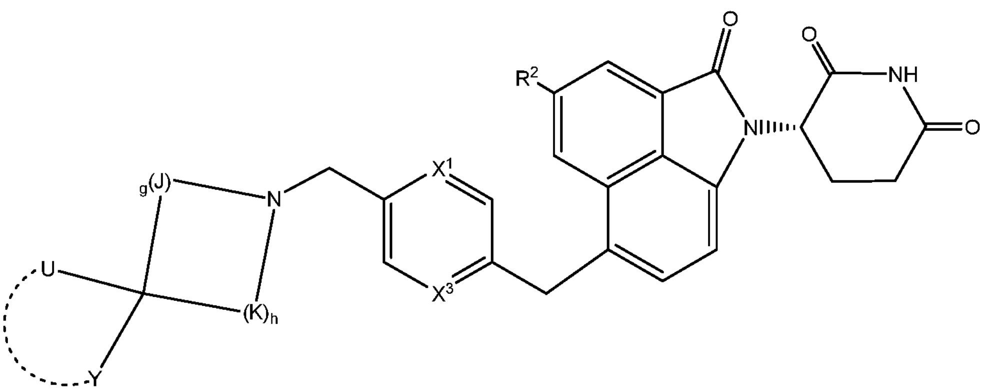

(XXIV)

or a pharmaceutically acceptable salt thereof.

25. The compound or a pharmaceutically acceptable salt thereof of any one of the preceding items, wherein the compound is the compound of structural formula (XXV):

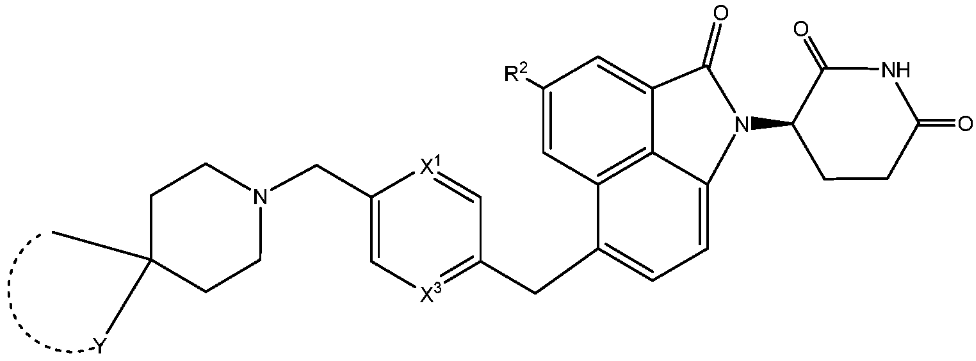

(XXV)

or a pharmaceutically acceptable salt thereof.

26. The compound or a pharmaceutically acceptable salt thereof of any one of the preceding items, wherein the compound is the compound of structural formula (XXVI):

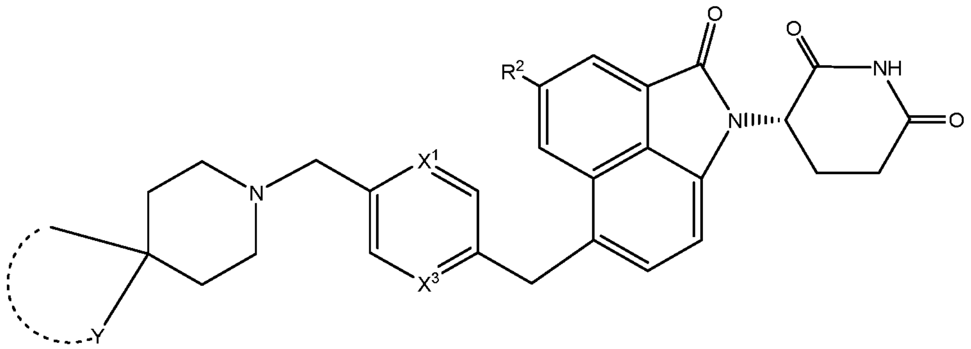

(XXVI)

or a pharmaceutically acceptable salt thereof.

27. The compound or a pharmaceutically acceptable salt thereof of any one of the preceding items, wherein the compound is the compound of structural formula (XXVII):

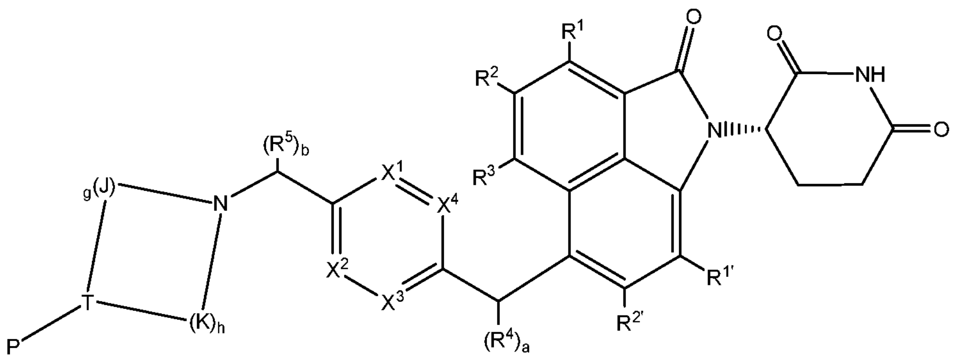

(XXVII)

or a pharmaceutically acceptable salt thereof.

28. The compound or a pharmaceutically acceptable salt thereof of any one of the preceding items, wherein the compound is the compound of structural formula (XXVIII):

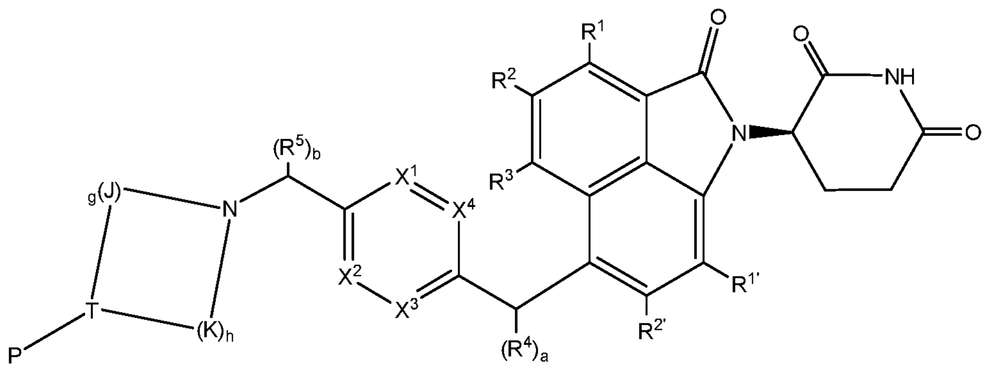

(XXVIII)

or a pharmaceutically acceptable salt thereof.

29. The compound or a pharmaceutically acceptable salt thereof of any one of the preceding items, wherein the compound is the compound of structural formula (XXIX):

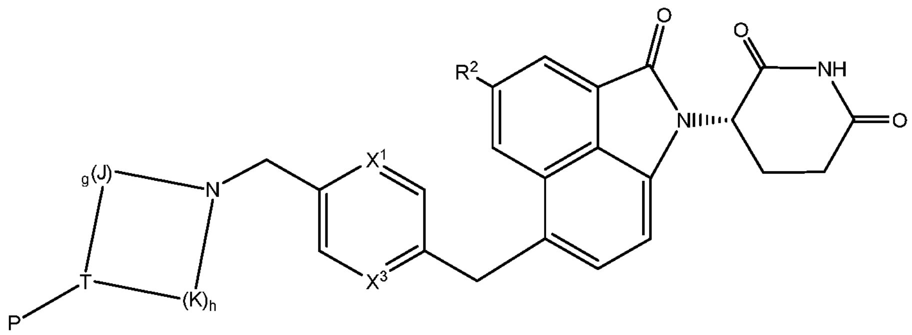

(XXIX)

or a pharmaceutically acceptable salt thereof.

30. The compound or a pharmaceutically acceptable salt thereof of any one of the preceding items, wherein the compound is the compound of structural formula (XXX):

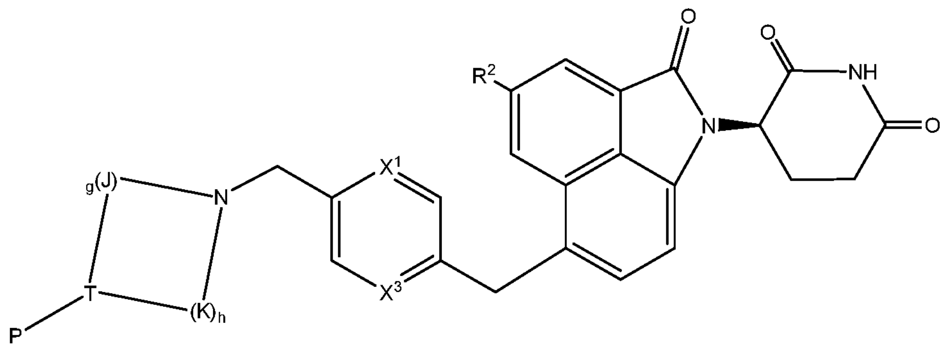

(XXX)

or a pharmaceutically acceptable salt thereof.

31. The compound or a pharmaceutically acceptable salt thereof of any one of the preceding items, wherein the compound is the compound of structural formula (XXXI):

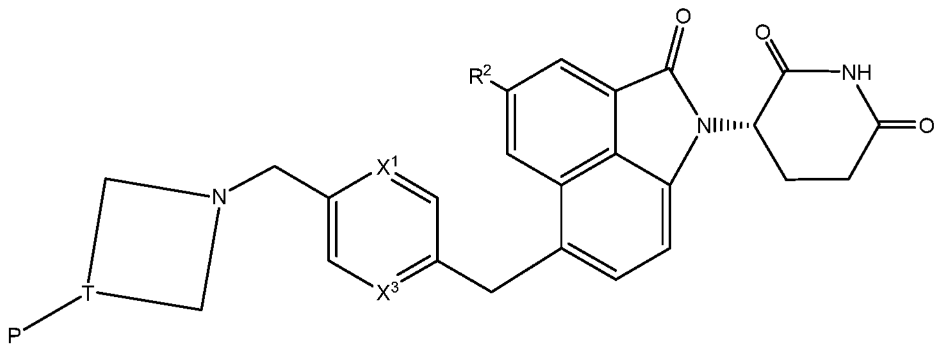

(XXXI)

or a pharmaceutically acceptable salt thereof.

32. The compound or a pharmaceutically acceptable salt thereof of any one of the preceding items, wherein the compound is the compound of structural formula (XXXII):

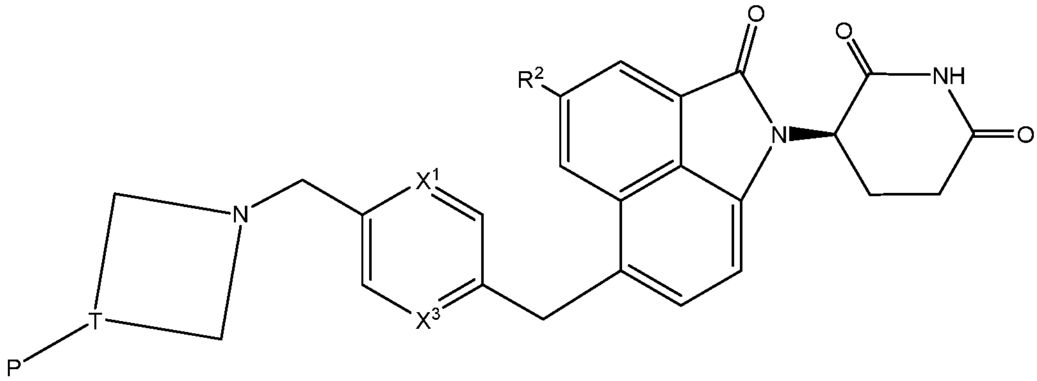

(XXXII)

or a pharmaceutically acceptable salt thereof.

33. The compound or a pharmaceutically acceptable salt thereof of any one of the preceding items, wherein the compound is the compound of structural formula (XXXIII):

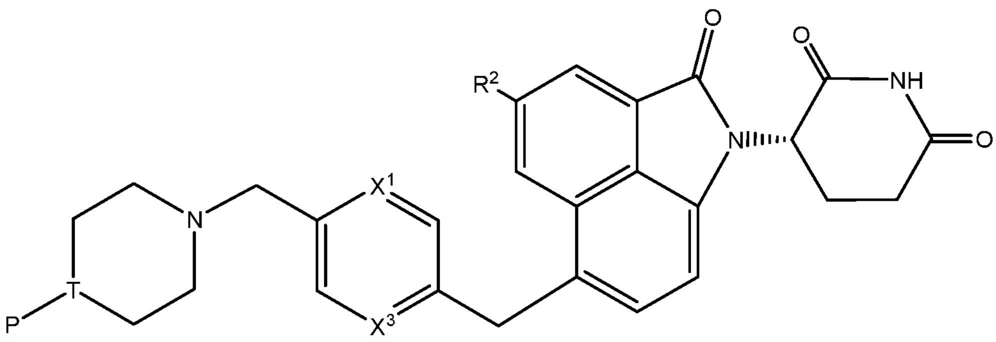

(XXXIII)

or a pharmaceutically acceptable salt thereof.

34. The compound or a pharmaceutically acceptable salt thereof of any one of the preceding items, wherein the compound is the compound of structural formula (XXXIV):

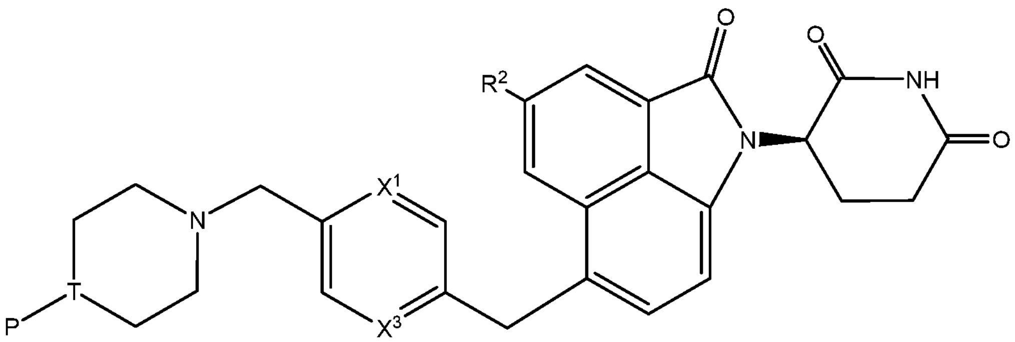

(XXXIV)

or a pharmaceutically acceptable salt thereof.

35. The compound or a pharmaceutically acceptable salt thereof of any one of the preceding items, wherein $R^1$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

36. The compound or a pharmaceutically acceptable salt thereof of any one of the preceding items, wherein $R^1$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

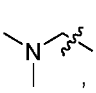, 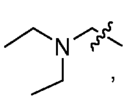, 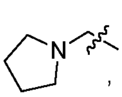, 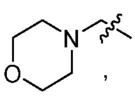, 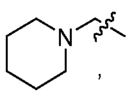, 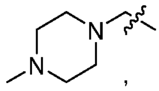, 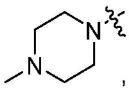, 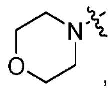,

trifluoromethyl, monofluoromethyl, difluoromethyl, 2-fluoroethyl, pentafluoroethyl.

37. The compound or a pharmaceutically acceptable salt thereof of any one of the preceding items, wherein $R^1$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

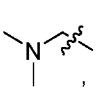, 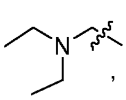, 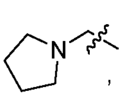, 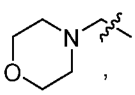, 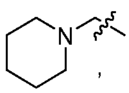, 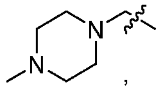, 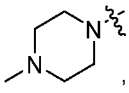, 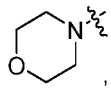,

trifluoromethyl, monofluoromethyl, difluoromethyl, 2-fluoroethyl, pentafluoroethyl.

38. The compound or a pharmaceutically acceptable salt thereof of any one of the preceding items, wherein $R^1$, for each occurrence, is independently selected from hydrogen, fluorine, cyano, methyl, isopropyl.

39. The compound or a pharmaceutically acceptable salt thereof of any one of the preceding items, wherein $R^{1'}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

40. The compound or a pharmaceutically acceptable salt thereof of any one of the preceding items, wherein $R^{1'}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

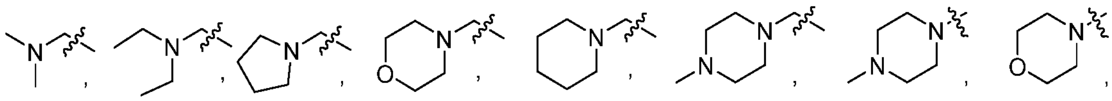

, , , , , , , ,

trifluoromethyl, monofluoromethyl, difluoromethyl, 2-fluoroethyl, pentafluoroethyl.

41. The compound or a pharmaceutically acceptable salt thereof of any one of the preceding items, wherein $R^{1'}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

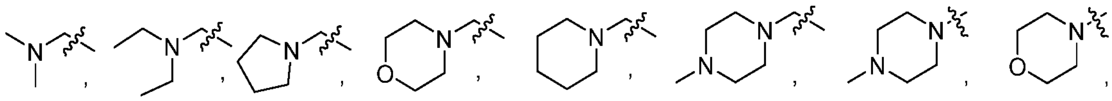

, , , , , , , ,

trifluoromethyl, monofluoromethyl, difluoromethyl, 2-fluoroethyl, pentafluoroethyl.

42. The compound or a pharmaceutically acceptable salt thereof of any one of the preceding items, wherein $R^1$, for each occurrence, is independently selected from hydrogen, fluorine, cyano, methyl, isopropyl.

43. The compound or a pharmaceutically acceptable salt thereof of any one of the preceding items, wherein $R^2$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

44. The compound or a pharmaceutically acceptable salt thereof of any one of the preceding items, wherein $R^2$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

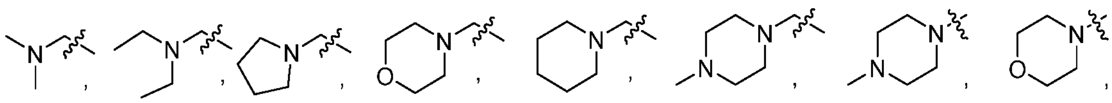

, , , , , , , ,

trifluoromethyl, monofluoromethyl, difluoromethyl, 2-fluoroethyl, pentafluoroethyl.

45. The compound or a pharmaceutically acceptable salt thereof of any one of the preceding items, wherein $R^2$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

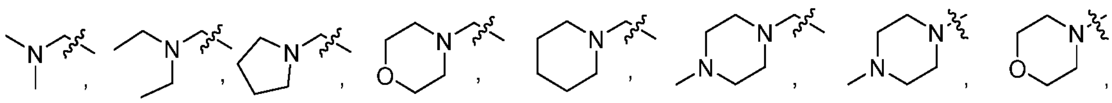

, , , , , , , ,

trifluoromethyl, monofluoromethyl, difluoromethyl, 2-fluoroethyl, pentafluoroethyl.

46. The compound or a pharmaceutically acceptable salt thereof of any one of the preceding items, wherein $R^2$, for each occurrence, is independently selected from hydrogen, fluorine, chlorine, bromine, cyano, trifluoromethyl, methoxy, ethoxy or methyl.

47. The compound or a pharmaceutically acceptable salt thereof of any one of the preceding items, wherein $R^{2'}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl,

$C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

48. The compound or a pharmaceutically acceptable salt thereof of any one of the preceding items, wherein $R^{2'}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

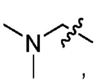, 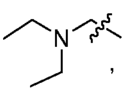, 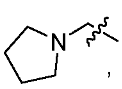, 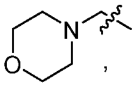, 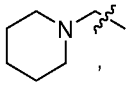, 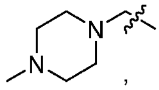, 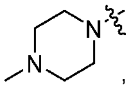, 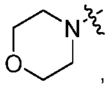,

trifluoromethyl, monofluoromethyl, difluoromethyl, 2-fluoroethyl, pentafluoroethyl.

49. The compound or a pharmaceutically acceptable salt thereof of any one of the preceding items, wherein $R^{2'}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

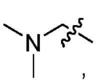, 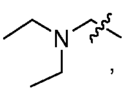, 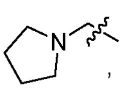, 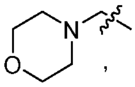, 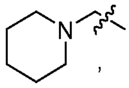, 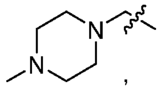, 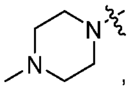, 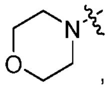,

trifluoromethyl, monofluoromethyl, difluoromethyl, 2-fluoroethyl, pentafluoroethyl.

50. The compound or a pharmaceutically acceptable salt thereof of any one of the preceding items, wherein $R^{2'}$, for each occurrence, is independently selected from hydrogen, fluorine, cyano, methyl, isopropyl.

51. The compound or a pharmaceutically acceptable salt thereof of any one of the preceding items, wherein $R^3$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

52. The compound or a pharmaceutically acceptable salt thereof of any one of the preceding items, wherein $R^3$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

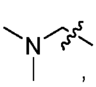, 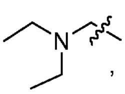, 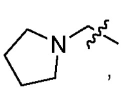, 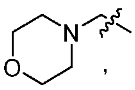, 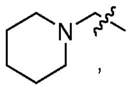, 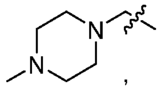, 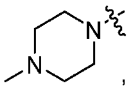, 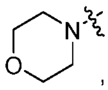,

trifluoromethyl, monofluoromethyl, difluoromethyl, 2-fluoroethyl, pentafluoroethyl.

53. The compound or a pharmaceutically acceptable salt thereof of any one of the preceding items, wherein $R^3$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

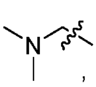, 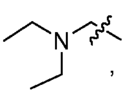, 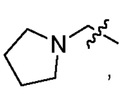, 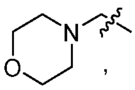, 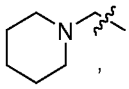, 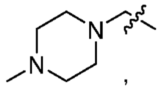, 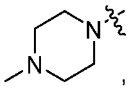, 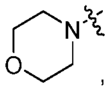,

trifluoromethyl, monofluoromethyl, difluoromethyl, 2-fluoroethyl, pentafluoroethyl.

54. The compound or a pharmaceutically acceptable salt thereof of any one of the preceding items, wherein $R^3$, for

each occurrence, is independently selected from hydrogen, fluorine, cyano, methyl, isopropyl.

55. The compound or a pharmaceutically acceptable salt thereof of any one of the preceding items, wherein $R^4$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

56. The compound or a pharmaceutically acceptable salt thereof of any one of the preceding items, wherein $R^3$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

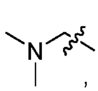, 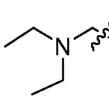, 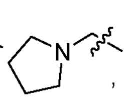, 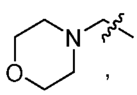, 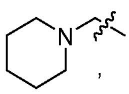, 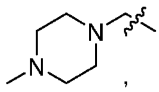, 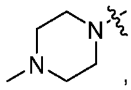, 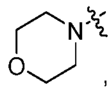,

trifluoromethyl, monofluoromethyl, difluoromethyl, 2-fluoroethyl, pentafluoroethyl.

57. The compound or a pharmaceutically acceptable salt thereof of any one of the preceding items, wherein $R^4$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

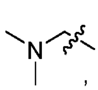, 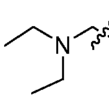, 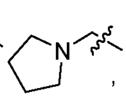, 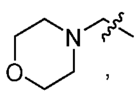, 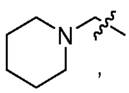, 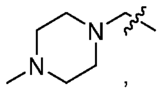, 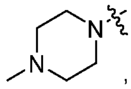, 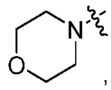,

trifluoromethyl, monofluoromethyl, difluoromethyl, 2-fluoroethyl, pentafluoroethyl.

58. The compound or a pharmaceutically acceptable salt thereof of any one of the preceding items, wherein $R^4$, for each occurrence, is independently selected from hydrogen, fluorine, cyano, methyl, isopropyl.

59. The compound or a pharmaceutically acceptable salt thereof of any one of the preceding items, wherein $R^5$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

60. The compound or a pharmaceutically acceptable salt thereof of any one of the preceding items, wherein $R^5$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

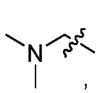, 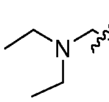, 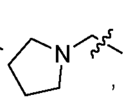, 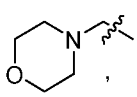, 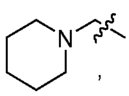, 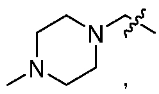, 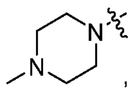, 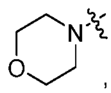,

trifluoromethyl, monofluoromethyl, difluoromethyl, 2-fluoroethyl, pentafluoroethyl.

61. The compound or a pharmaceutically acceptable salt thereof of any one of the preceding items, wherein $R^5$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

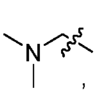, 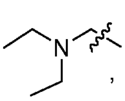, 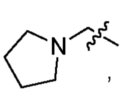, 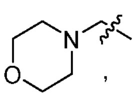, 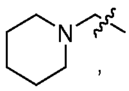, 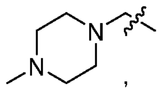, 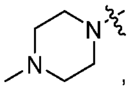, 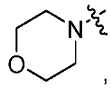,

trifluoromethyl, monofluoromethyl, difluoromethyl, 2-fluoroethyl, pentafluoroethyl.

62. The compound or a pharmaceutically acceptable salt thereof of any one of the preceding items, wherein $R^5$, for each occurrence, is independently selected from hydrogen, fluorine, cyano, methyl, isopropyl.

63. The compound or a pharmaceutically acceptable salt thereof of any one of the preceding items, wherein $R^6$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

64. The compound or a pharmaceutically acceptable salt thereof of any one of the preceding items, wherein $R^6$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

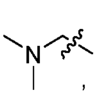, 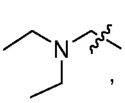, 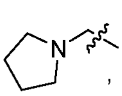, 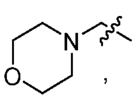, 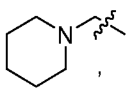, 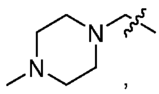, 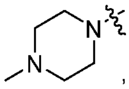, 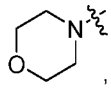,

trifluoromethyl, monofluoromethyl, difluoromethyl, 2-fluoroethyl, pentafluoroethyl.

65. The compound or a pharmaceutically acceptable salt thereof of any one of the preceding items, wherein $R^6$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

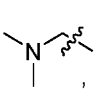, 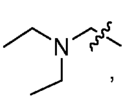, 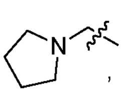, 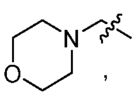, 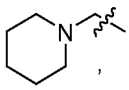, 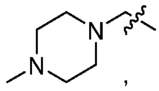, 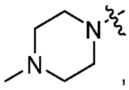, 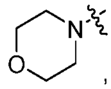,

trifluoromethyl, monofluoromethyl, difluoromethyl, 2-fluoroethyl, pentafluoroethyl.

66. The compound or a pharmaceutically acceptable salt thereof of any one of the preceding items, wherein $R^6$, for each occurrence, is independently selected from hydrogen, fluorine, cyano, methyl, isopropyl.

67. The compound or a pharmaceutically acceptable salt thereof of any one of the preceding items, wherein $R^7$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

68. The compound or a pharmaceutically acceptable salt thereof of any one of the preceding items, wherein $R^7$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

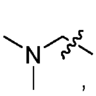, 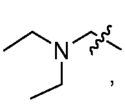, 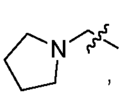, 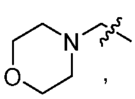, 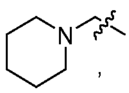, 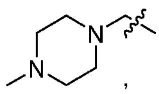, 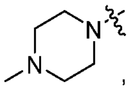, 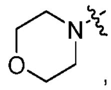,

trifluoromethyl, monofluoromethyl, difluoromethyl, 2-fluoroethyl, pentafluoroethyl.

69. The compound or a pharmaceutically acceptable salt thereof of any one of the preceding items, wherein $R^7$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

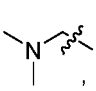, 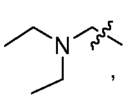, 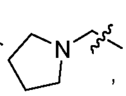, 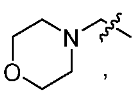, 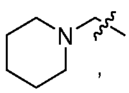, 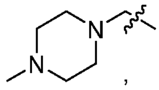, 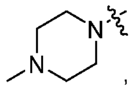, 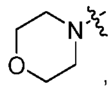,

trifluoromethyl, monofluoromethyl, difluoromethyl, 2-fluoroethyl, pentafluoroethyl.

70. The compound or a pharmaceutically acceptable salt thereof of any one of the preceding items, wherein $R^7$, for each occurrence, is independently selected from hydrogen, fluorine, cyano, methyl, isopropyl.

71. The compound or a pharmaceutically acceptable salt thereof of any one of the preceding items, wherein $R^8$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

72. The compound or a pharmaceutically acceptable salt thereof of any one of the preceding items, wherein $R^8$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

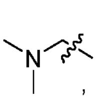, 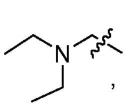, 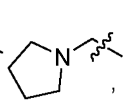, 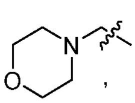, 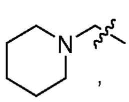, 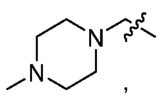, 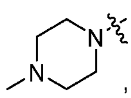, 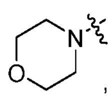,

trifluoromethyl, monofluoromethyl, difluoromethyl, 2-fluoroethyl, pentafluoroethyl.

73. The compound or a pharmaceutically acceptable salt thereof of any one of the preceding items, wherein $R^8$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

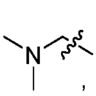, 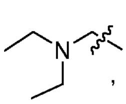, 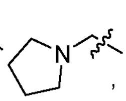, 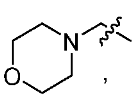, 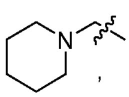, 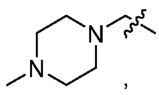, 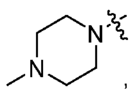, 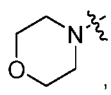,

trifluoromethyl, monofluoromethyl, difluoromethyl, 2-fluoroethyl, pentafluoroethyl.

74. The compound or a pharmaceutically acceptable salt thereof of any one of the preceding items, wherein $R^8$, for each occurrence, is independently selected from hydrogen, fluorine, cyano, methyl, isopropyl.

75. The compound or a pharmaceutically acceptable salt thereof of any one of the preceding items, wherein $R^9$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

76. The compound or a pharmaceutically acceptable salt thereof of any one of the preceding items, wherein $R^9$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

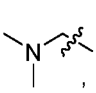, 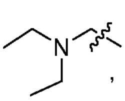, 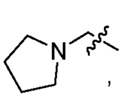, 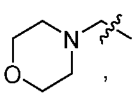, 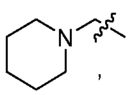, 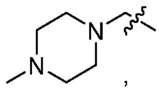, 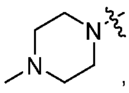, 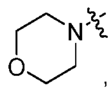,

trifluoromethyl, monofluoromethyl, difluoromethyl, 2-fluoroethyl, pentafluoroethyl.

77. The compound or a pharmaceutically acceptable salt thereof of any one of the preceding items, wherein $R^9$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

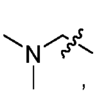, 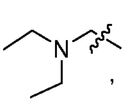, 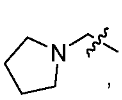, 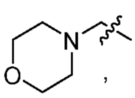, 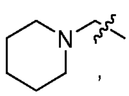, 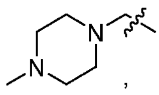, 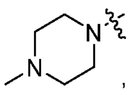, 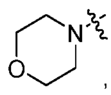,

trifluoromethyl, monofluoromethyl, difluoromethyl, 2-fluoroethyl, pentafluoroethyl.

78. The compound or a pharmaceutically acceptable salt thereof of any one of the preceding items, wherein $R^9$, for each occurrence, is independently selected from hydrogen, fluorine, cyano, methyl, isopropyl.

79. The compound or a pharmaceutically acceptable salt thereof of any one of the preceding items, wherein $R^{10}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

80. The compound or a pharmaceutically acceptable salt thereof of any one of the preceding items, wherein $R^{10}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

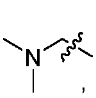, 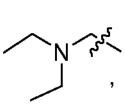, 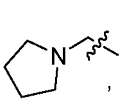, 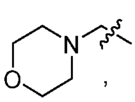, 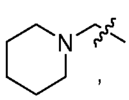, 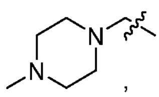, 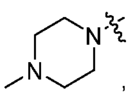, 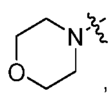,

trifluoromethyl, monofluoromethyl, difluoromethyl, 2-fluoroethyl, pentafluoroethyl.

81. The compound or a pharmaceutically acceptable salt thereof of any one of the preceding items, wherein $R^{10}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

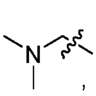, 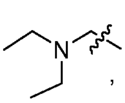, 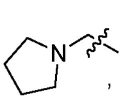, 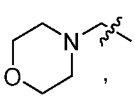, 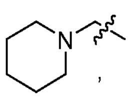, 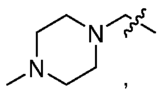, 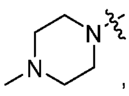, 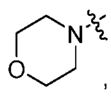,

trifluoromethyl, monofluoromethyl, difluoromethyl, 2-fluoroethyl, pentafluoroethyl.

82. The compound or a pharmaceutically acceptable salt thereof of any one of the preceding items, wherein $R^{10}$, for each occurrence, is independently selected from hydrogen, fluorine, cyano, methyl, isopropyl.

83. The compound or a pharmaceutically acceptable salt thereof of any one of the preceding items, wherein $R^{11}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted

with 1, 2, 3 or 4 halogens, hydroxyl, nitro, cyano, amino, mercapto, -COOH, or $C_1$-$C_6$ alkyl.

84. The compound or a pharmaceutically acceptable salt thereof of any one of the preceding items, wherein $R^{11}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

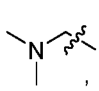, 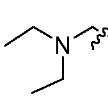, 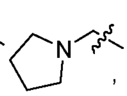, 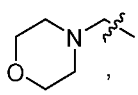, 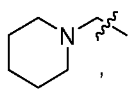, 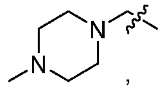, 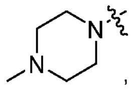, 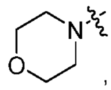,

trifluoromethyl, monofluoromethyl, difluoromethyl, 2-fluoroethyl, pentafluoroethyl.

85. The compound or a pharmaceutically acceptable salt thereof of any one of the preceding items, wherein $R^{11}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

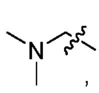, 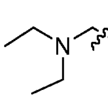, 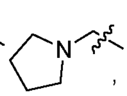, 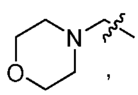, 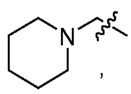, 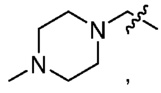, 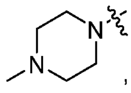, 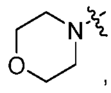,

trifluoromethyl, monofluoromethyl, difluoromethyl, 2-fluoroethyl, pentafluoroethyl.

86. The compound or a pharmaceutically acceptable salt thereof of any one of the preceding items, wherein $R^{11}$, for each occurrence, is independently selected from hydrogen, fluorine, cyano, methyl, isopropyl.

87. The compound or a pharmaceutically acceptable salt thereof of any one of the preceding items, wherein $R^{12}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

88. The compound or a pharmaceutically acceptable salt thereof of any one of the preceding items, wherein $R^{12}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

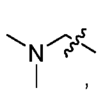, 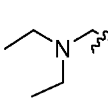, 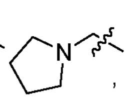, 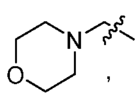, 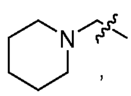, 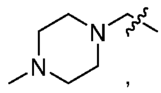, 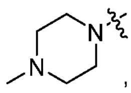, 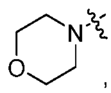,

trifluoromethyl, monofluoromethyl, difluoromethyl, 2-fluoroethyl, pentafluoroethyl.

89. The compound or a pharmaceutically acceptable salt thereof of any one of the preceding items, wherein $R^{12}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

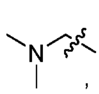, 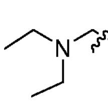, 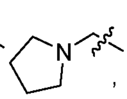, 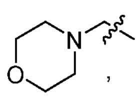, 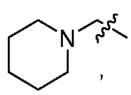, 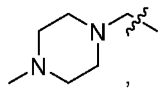, 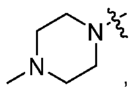, 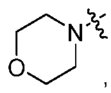,

trifluoromethyl, monofluoromethyl, difluoromethyl, 2-fluoroethyl, pentafluoroethyl.

90. The compound or a pharmaceutically acceptable salt thereof of any one of the preceding items, wherein $R^{12}$, for each occurrence, is independently selected from hydrogen, fluorine, cyano, methyl, isopropyl.

91. The compound or a pharmaceutically acceptable salt thereof of any one of the preceding items, wherein $R^{13}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

92. The compound or a pharmaceutically acceptable salt thereof of any one of the preceding items, wherein $R^{13}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

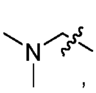, 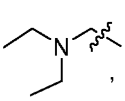, 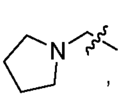, 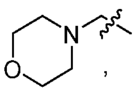, 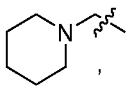, 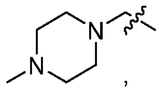, 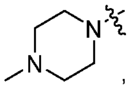, 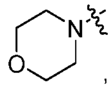,

trifluoromethyl, monofluoromethyl, difluoromethyl, 2-fluoroethyl, pentafluoroethyl.

93. The compound or a pharmaceutically acceptable salt thereof of any one of the preceding items, wherein $R^{13}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

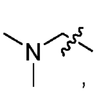, 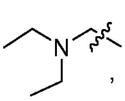, 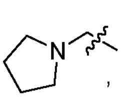, 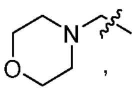, 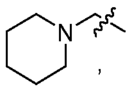, 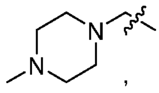, 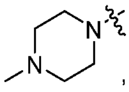, 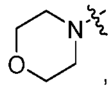,

trifluoromethyl, monofluoromethyl, difluoromethyl, 2-fluoroethyl, pentafluoroethyl.

94. The compound or a pharmaceutically acceptable salt thereof of any one of the preceding items, wherein $R^{13}$, for each occurrence, is independently selected from hydrogen, fluorine, cyano, methyl, isopropyl.

95. The compound or a pharmaceutically acceptable salt thereof of any one of the preceding items, wherein $R^{14}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

96. The compound or a pharmaceutically acceptable salt thereof of any one of the preceding items, wherein $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

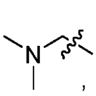, 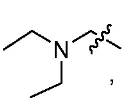, 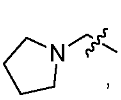, 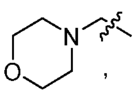, 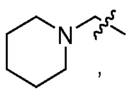, 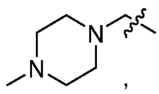, 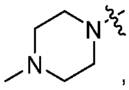, 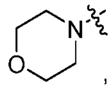,

trifluoromethyl, monofluoromethyl, difluoromethyl, 2-fluoroethyl, pentafluoroethyl.

97. The compound or a pharmaceutically acceptable salt thereof of any one of the preceding items, wherein $R^{14}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

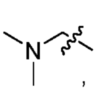, 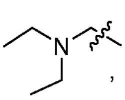, 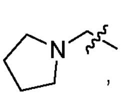, 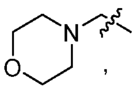, 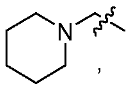, 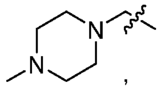, 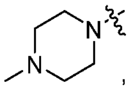, 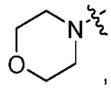,

trifluoromethyl, monofluoromethyl, difluoromethyl, 2-fluoroethyl, pentafluoroethyl.

98. The compound or a pharmaceutically acceptable salt thereof of any one of the preceding items, wherein $R^{14}$, for each occurrence, is independently selected from hydrogen, fluorine, cyano, methyl, isopropyl.

99. The compound or a pharmaceutically acceptable salt thereof of any one of the preceding items, wherein $R^{14}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

100. The compound or a pharmaceutically acceptable salt thereof of any one of the preceding items, wherein $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

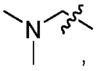, 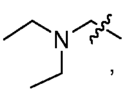, 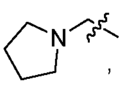, 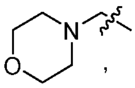, 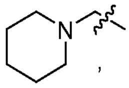, 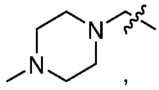, 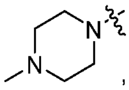, 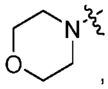,

trifluoromethyl, monofluoromethyl, difluoromethyl, 2-fluoroethyl, pentafluoroethyl.

101. The compound or a pharmaceutically acceptable salt thereof of any one of the preceding items, wherein $R^{14}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

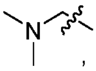, 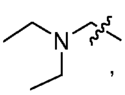, 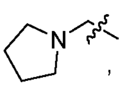, 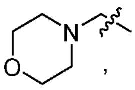, 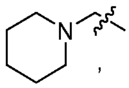, 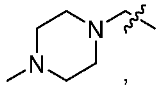, 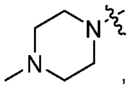, 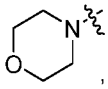,

trifluoromethyl, monofluoromethyl, difluoromethyl, 2-fluoroethyl, pentafluoroethyl.

102. The compound or a pharmaceutically acceptable salt thereof of any one of the preceding items, wherein $R^{14}$, for each occurrence, is independently selected from hydrogen, fluorine, cyano, methyl, isopropyl.

103. The compound or a pharmaceutically acceptable salt thereof of any one of the preceding items, wherein $R^{14''}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

104. The compound or a pharmaceutically acceptable salt thereof of any one of the preceding items, wherein $R^{14''}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

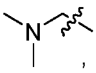, 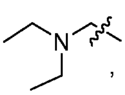, 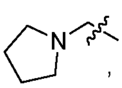, 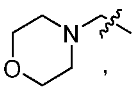, 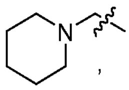, 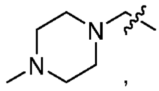, 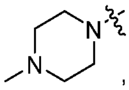, 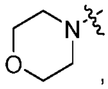,

trifluoromethyl, monofluoromethyl, difluoromethyl, 2-fluoroethyl, pentafluoroethyl.

106. The compound or a pharmaceutically acceptable salt thereof of any one of the preceding items, wherein $R^{14''}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

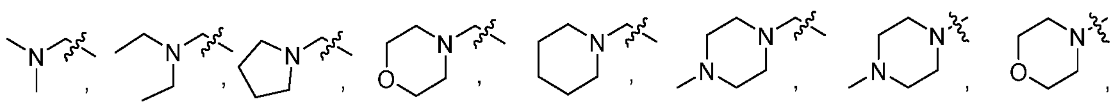

trifluoromethyl, monofluoromethyl, difluoromethyl, 2-fluoroethyl, pentafluoroethyl.

106. The compound or a pharmaceutically acceptable salt thereof of any one of the preceding items, wherein $R^{14''}$, for each occurrence, is independently selected from hydrogen, fluorine, cyano, methyl, isopropyl.

107. The compound or a pharmaceutically acceptable salt thereof of any one of the preceding items, wherein $R^{14'''}$ , for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

108. The compound or a pharmaceutically acceptable salt thereof of any one of the preceding items, wherein $R^{14'''}$ , for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

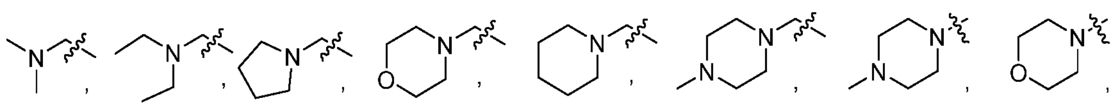

trifluoromethyl, monofluoromethyl, difluoromethyl, 2-fluoroethyl, pentafluoroethyl.

109. The compound or a pharmaceutically acceptable salt thereof of any one of the preceding items, wherein $R^{14'''}$ , for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

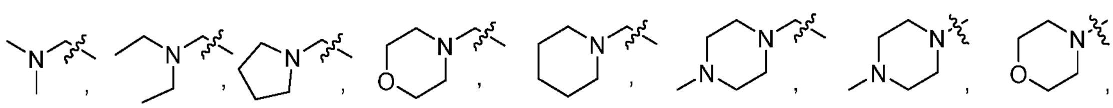

trifluoromethyl, monofluoromethyl, difluoromethyl, 2-fluoroethyl, pentafluoroethyl.

110. The compound or a pharmaceutically acceptable salt thereof of any one of the preceding items, wherein $R^{14'''}$ , for each occurrence, is independently selected from hydrogen, fluorine, cyano, methyl, isopropyl.

111. The compound or a pharmaceutically acceptable salt thereof of any one of the preceding items, wherein $R^{15}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

112. The compound or a pharmaceutically acceptable salt thereof of any one of the preceding items, wherein $R^{15}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

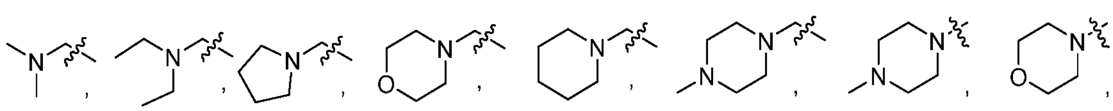

trifluoromethyl, monofluoromethyl, difluoromethyl, 2-fluoroethyl, pentafluoroethyl.

113. The compound or a pharmaceutically acceptable salt thereof of any one of the preceding items, wherein $R^{15}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

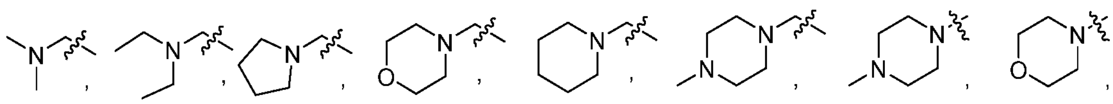
, , , , , , , ,

trifluoromethyl, monofluoromethyl, difluoromethyl, 2-fluoroethyl, pentafluoroethyl.

114. The compound or a pharmaceutically acceptable salt thereof of any one of the preceding items, wherein $R^{15}$, for each occurrence, is independently selected from hydrogen, fluorine, cyano, methyl, isopropyl.

115. The compound or a pharmaceutically acceptable salt thereof of any one of the preceding items, wherein $R^{16}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

116. The compound or a pharmaceutically acceptable salt thereof of any one of the preceding items, wherein $R^{16}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

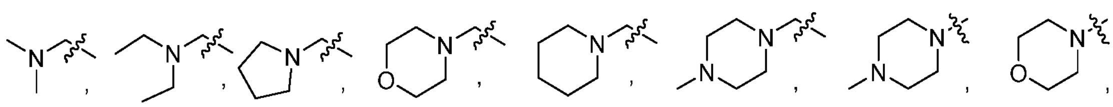
, , , , , , , ,

trifluoromethyl, monofluoromethyl, difluoromethyl, 2-fluoroethyl, pentafluoroethyl.

117. The compound or a pharmaceutically acceptable salt thereof of any one of the preceding items, wherein $R^{16}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

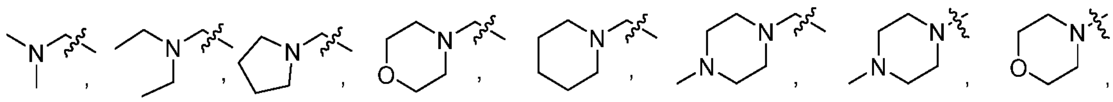
, , , , , , , ,

trifluoromethyl, monofluoromethyl, difluoromethyl, 2-fluoroethyl, pentafluoroethyl.

118. The compound or a pharmaceutically acceptable salt thereof of any one of the preceding items, wherein $R^{16}$, for each occurrence, is independently selected from hydrogen, fluorine, cyano, methyl, isopropyl.

119. The compound or a pharmaceutically acceptable salt thereof of any one of the preceding items, wherein $R^{17}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

120. The compound or a pharmaceutically acceptable salt thereof of any one of the preceding items, wherein $R^{17}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

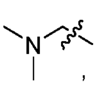, 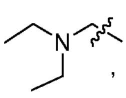, 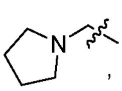, 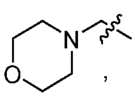, 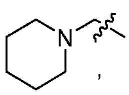, 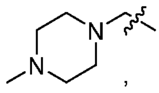, 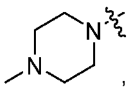, 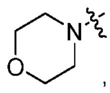,

trifluoromethyl, monofluoromethyl, difluoromethyl, 2-fluoroethyl, pentafluoroethyl.

121. The compound or a pharmaceutically acceptable salt thereof of any one of the preceding items, wherein $R^{17}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

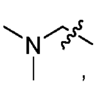, 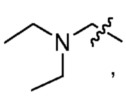, 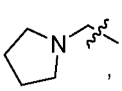, 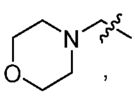, 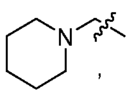, 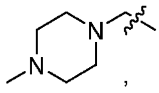, 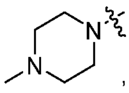, 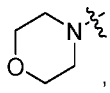,

trifluoromethyl, monofluoromethyl, difluoromethyl, 2-fluoroethyl, pentafluoroethyl.

122. The compound or a pharmaceutically acceptable salt thereof of any one of the preceding items, wherein $R^{17}$, for each occurrence, is independently selected from hydrogen, fluorine, cyano, methyl, isopropyl.

123. The compound or a pharmaceutically acceptable salt thereof of any one of the preceding items, wherein $R^{18}$, for each occurrence, is independently selected from absence, hydrogen, fluorine, chlorine, hydroxyl, nitro, cyano, amino, mercapto, -COOH, $C_1$-$C_6$ alkyl,, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

124. The compound or a pharmaceutically acceptable salt thereof of any one of the preceding items, wherein $R^{18}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

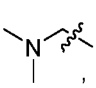, 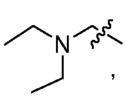, 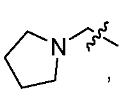, 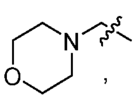, 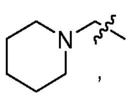, 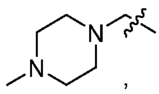, 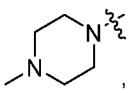, 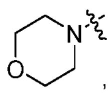,

trifluoromethyl, monofluoromethyl, difluoromethyl, 2-fluoroethyl, pentafluoroethyl.

125. The compound or a pharmaceutically acceptable salt thereof of any one of the preceding items, wherein $R^{18}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

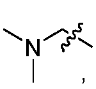, 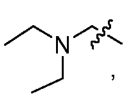, 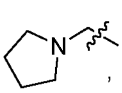, 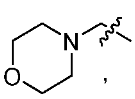, 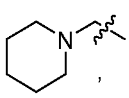, 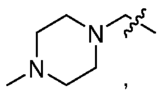, 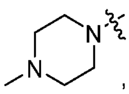, 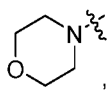,

trifluoromethyl, monofluoromethyl, difluoromethyl, 2-fluoroethyl, pentafluoroethyl.

126. The compound or a pharmaceutically acceptable salt thereof of any one of the preceding items, wherein $R^{18}$, for each occurrence, is independently selected from hydrogen, fluorine, chlorine, cyano, methyl, or isopropyl.

127. The compound or a pharmaceutically acceptable salt thereof of any one of the preceding items, wherein $R^{21}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted

with 1, 2, 3 or 4 halogens, hydroxyl, nitro, cyano, amino, mercapto, -COOH, or $C_1$-$C_6$ alkyl.

128. The compound or a pharmaceutically acceptable salt thereof of any one of the preceding items, wherein $R^{21}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

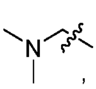, 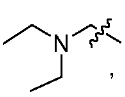, 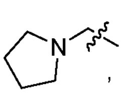, 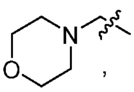, 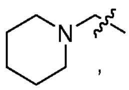, 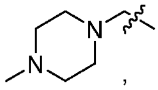, 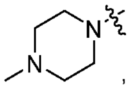, 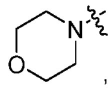,

trifluoromethyl, monofluoromethyl, difluoromethyl, 2-fluoroethyl, pentafluoroethyl.

129. The compound or a pharmaceutically acceptable salt thereof of any one of the preceding items, wherein $R^{21}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

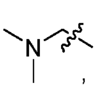, 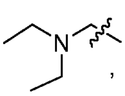, 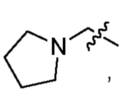, 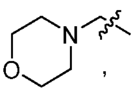, 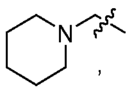, 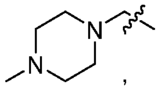, 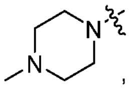, 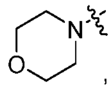,

trifluoromethyl, monofluoromethyl, difluoromethyl, 2-fluoroethyl, pentafluoroethyl.

130. The compound or a pharmaceutically acceptable salt thereof of any one of the preceding items, wherein $R^{21}$, for each occurrence, is independently selected from hydrogen, fluorine, cyano, methyl, isopropyl.

131. The compound or a pharmaceutically acceptable salt thereof of any one of the preceding items, wherein $R^{22}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

132. The compound or a pharmaceutically acceptable salt thereof of any one of the preceding items, wherein $R^{22}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

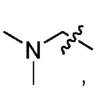, 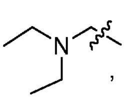, 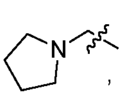, 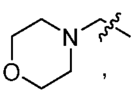, 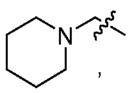, 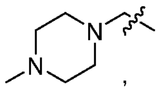, 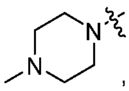, 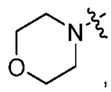,

trifluoromethyl, monofluoromethyl, difluoromethyl, 2-fluoroethyl, pentafluoroethyl.

133, $R^{22}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

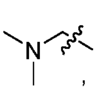, 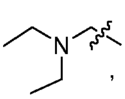, 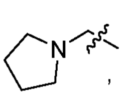, 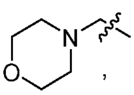, 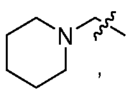, 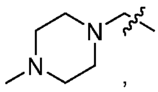, 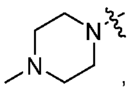, 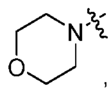,

trifluoromethyl, monofluoromethyl, difluoromethyl, 2-fluoroethyl, pentafluoroethyl.

134. The compound or a pharmaceutically acceptable salt thereof of any one of the preceding items, wherein $R^{22}$, for each occurrence, is independently selected from hydrogen, fluorine, cyano, methyl, isopropyl.

135. The compound or a pharmaceutically acceptable salt thereof of any one of the preceding items, wherein $R^{23}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

136. The compound or a pharmaceutically acceptable salt thereof of any one of the preceding items, wherein $R^{23}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

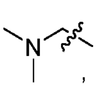, 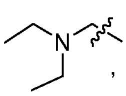, 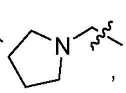, 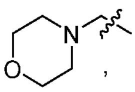, 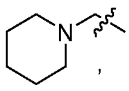, 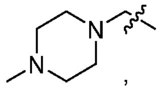, 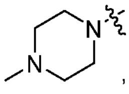, 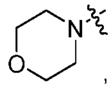,

trifluoromethyl, monofluoromethyl, difluoromethyl, 2-fluoroethyl, pentafluoroethyl.

137. The compound or a pharmaceutically acceptable salt thereof of any one of the preceding items, wherein $R^{23}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

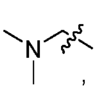, 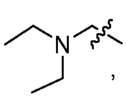, 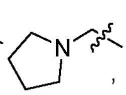, 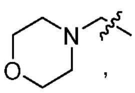, 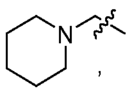, 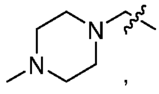, 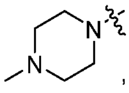, 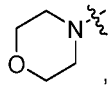,

trifluoromethyl, monofluoromethyl, difluoromethyl, 2-fluoroethyl, pentafluoroethyl.

138. The compound or a pharmaceutically acceptable salt thereof of any one of the preceding items, wherein $R^{23}$, for each occurrence, is independently selected from hydrogen, fluorine, cyano, methyl, isopropyl.

139. The compound or a pharmaceutically acceptable salt thereof of any one of the preceding items, wherein $R^{24}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

140. The compound or a pharmaceutically acceptable salt thereof of any one of the preceding items, wherein $R^{24}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

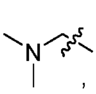, 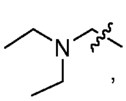, 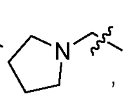, 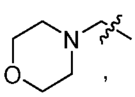, 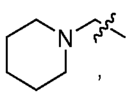, 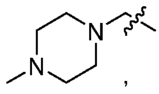, 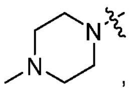, 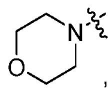,

trifluoromethyl, monofluoromethyl, difluoromethyl, 2-fluoroethyl, pentafluoroethyl.

141. The compound or a pharmaceutically acceptable salt thereof of any one of the preceding items, wherein $R^{24}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

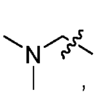, 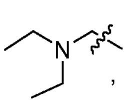, 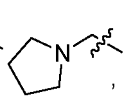, 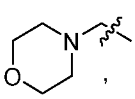, 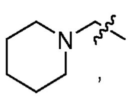, 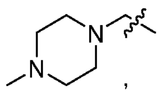, 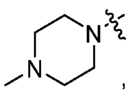, 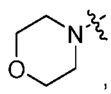,

trifluoromethyl, monofluoromethyl, difluoromethyl, 2-fluoroethyl, pentafluoroethyl.

142. The compound or a pharmaceutically acceptable salt thereof of any one of the preceding items, wherein $R^{24}$, for each occurrence, is independently selected from hydrogen, fluorine, cyano, methyl, isopropyl.

143. The compound or a pharmaceutically acceptable salt thereof of any one of the preceding items, wherein $R^{25}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

144. The compound or a pharmaceutically acceptable salt thereof of any one of the preceding items, wherein $R^{25}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

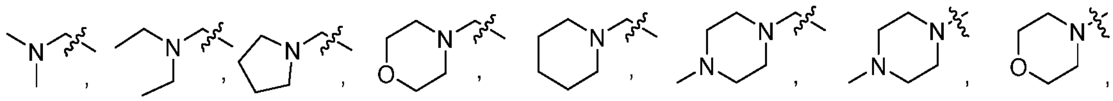

trifluoromethyl, monofluoromethyl, difluoromethyl, 2-fluoroethyl, pentafluoroethyl.

145. The compound or a pharmaceutically acceptable salt thereof of any one of the preceding items, wherein $R^{25}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

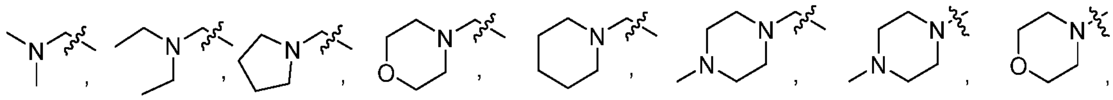

trifluoromethyl, monofluoromethyl, difluoromethyl, 2-fluoroethyl, pentafluoroethyl.

146. The compound or a pharmaceutically acceptable salt thereof of any one of the preceding items, wherein $R^{25}$, for each occurrence, is independently selected from hydrogen, fluorine, cyano, methyl, isopropyl.

147. The compound or a pharmaceutically acceptable salt thereof of any one of the preceding items, wherein $R^{26}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

148. The compound or a pharmaceutically acceptable salt thereof of any one of the preceding items, wherein $R^{26}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

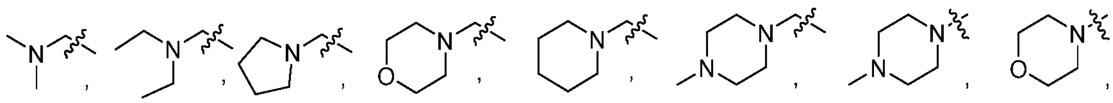

trifluoromethyl, monofluoromethyl, difluoromethyl, 2-fluoroethyl, pentafluoroethyl.

149. The compound or a pharmaceutically acceptable salt thereof of any one of the preceding items, wherein $R^{26}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

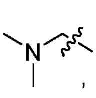, 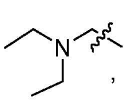, 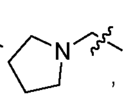, 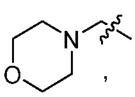, 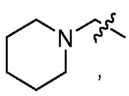, 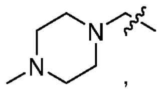, 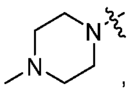, 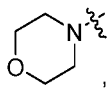,

trifluoromethyl, monofluoromethyl, difluoromethyl, 2-fluoroethyl, pentafluoroethyl.

150. The compound or a pharmaceutically acceptable salt thereof of any one of the preceding items, wherein $R^{26}$, for each occurrence, is independently selected from hydrogen, fluorine, cyano, methyl, isopropyl.

151. The compound or a pharmaceutically acceptable salt thereof of any one of the preceding items, wherein $R^{27}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

152. The compound or a pharmaceutically acceptable salt thereof of any one of the preceding items, wherein $R^{27}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

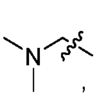, 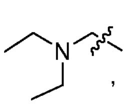, 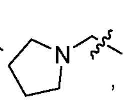, 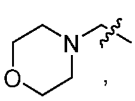, 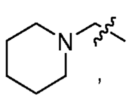, 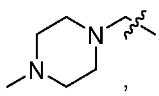, 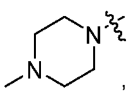, 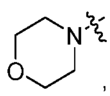,

trifluoromethyl, monofluoromethyl, difluoromethyl, 2-fluoroethyl, pentafluoroethyl.

153. The compound or a pharmaceutically acceptable salt thereof of any one of the preceding items, wherein $R^{27}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

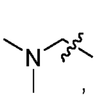, 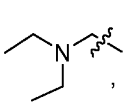, 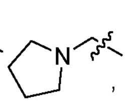, 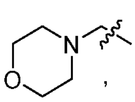, 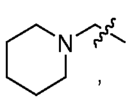, 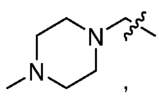, 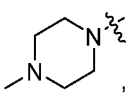, 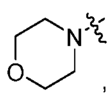,

trifluoromethyl, monofluoromethyl, difluoromethyl, 2-fluoroethyl, pentafluoroethyl.

154. The compound or a pharmaceutically acceptable salt thereof of any one of the preceding items, wherein $R^{27}$, for each occurrence, is independently selected from hydrogen, fluorine, cyano, methyl, isopropyl.

155. The compound or a pharmaceutically acceptable salt thereof of any one of the preceding items, wherein $R^{28}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

156. The compound or a pharmaceutically acceptable salt thereof of any one of the preceding items, wherein $R^{28}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

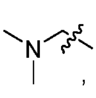, 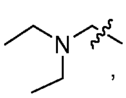, 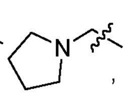, 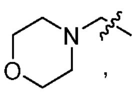, 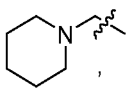, 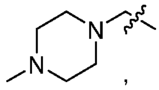, 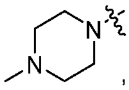, 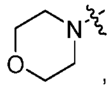,

trifluoromethyl, monofluoromethyl, difluoromethyl, 2-fluoroethyl, pentafluoroethyl.

157. The compound or a pharmaceutically acceptable salt thereof of any one of the preceding items, wherein $R^{28}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

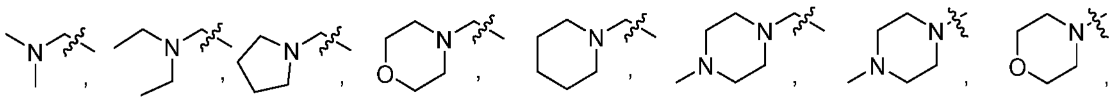

trifluoromethyl, monofluoromethyl, difluoromethyl, 2-fluoroethyl, pentafluoroethyl.

158. The compound or a pharmaceutically acceptable salt thereof of any one of the preceding items, wherein $R^{28}$, for each occurrence, is independently selected from hydrogen, fluorine, cyano, methyl, isopropyl.

159. The compound or a pharmaceutically acceptable salt thereof of any one of the preceding items, wherein $R^{31}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

160. The compound or a pharmaceutically acceptable salt thereof of any one of the preceding items, wherein $R^{31}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

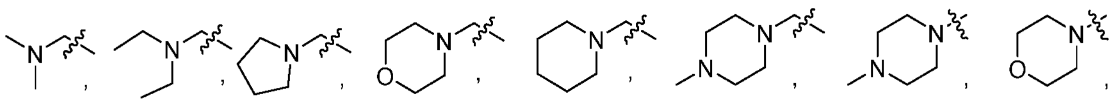

trifluoromethyl, monofluoromethyl, difluoromethyl, 2-fluoroethyl, pentafluoroethyl.

161. The compound or a pharmaceutically acceptable salt thereof of any one of the preceding items, wherein $R^{31}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

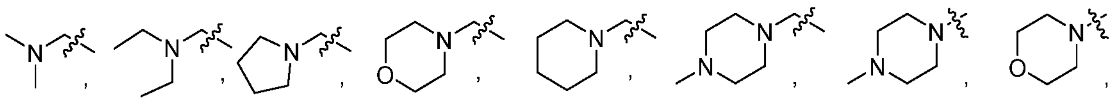

trifluoromethyl, monofluoromethyl, difluoromethyl, 2-fluoroethyl, pentafluoroethyl.

162. The compound or a pharmaceutically acceptable salt thereof of any one of the preceding items, wherein $R^{31}$, for each occurrence, is independently selected from hydrogen, fluorine, cyano, methyl, isopropyl.

163. The compound or a pharmaceutically acceptable salt thereof of any one of the preceding items, wherein $R^{32}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

164. The compound or a pharmaceutically acceptable salt thereof of any one of the preceding items, wherein $R^{32}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

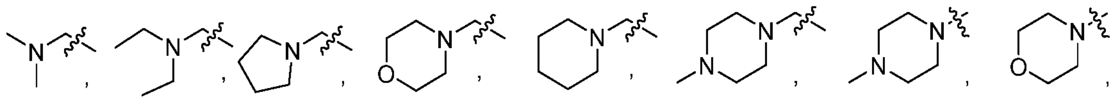 , , , , , , , ,

trifluoromethyl, monofluoromethyl, difluoromethyl, 2-fluoroethyl, pentafluoroethyl.

165. The compound or a pharmaceutically acceptable salt thereof of any one of the preceding items, wherein $R^{32}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

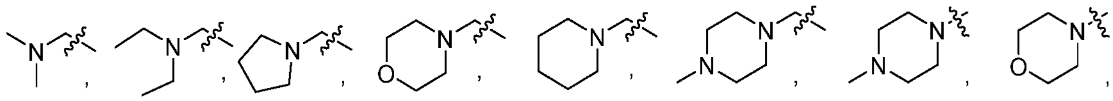 , , , , , , , ,

trifluoromethyl, monofluoromethyl, difluoromethyl, 2-fluoroethyl, pentafluoroethyl.

166. The compound or a pharmaceutically acceptable salt thereof of any one of the preceding items, wherein $R^{32}$, for each occurrence, is independently selected from hydrogen, fluorine, cyano, methyl, isopropyl.

167. The compound or a pharmaceutically acceptable salt thereof of any one of the preceding items, wherein $R^{33}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

168. The compound or a pharmaceutically acceptable salt thereof of any one of the preceding items, wherein $R^{33}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

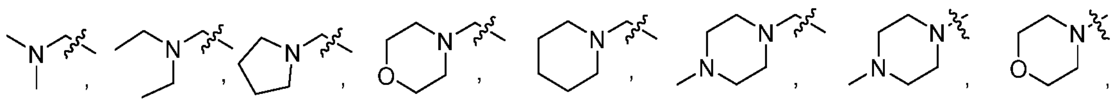 , , , , , , , ,

trifluoromethyl, monofluoromethyl, difluoromethyl, 2-fluoroethyl, pentafluoroethyl.

169. The compound or a pharmaceutically acceptable salt thereof of any one of the preceding items, wherein $R^{33}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

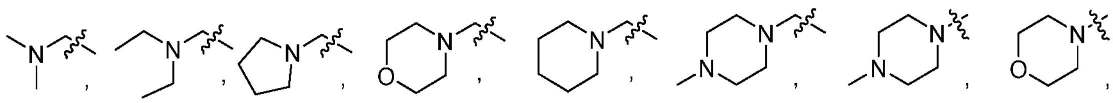 , , , , , , , ,

trifluoromethyl, monofluoromethyl, difluoromethyl, 2-fluoroethyl, pentafluoroethyl.

170. The compound or a pharmaceutically acceptable salt thereof of any one of the preceding items, wherein $R^{33}$, for each occurrence, is independently selected from hydrogen, fluorine, cyano, methyl, isopropyl.

171. The compound or a pharmaceutically acceptable salt thereof of any one of the preceding items, wherein $R^{34}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted

with 1, 2, 3 or 4 halogens, hydroxyl, nitro, cyano, amino, mercapto, -COOH, or $C_1$-$C_6$ alkyl.

172. The compound or a pharmaceutically acceptable salt thereof of any one of the preceding items, wherein $R^{34}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

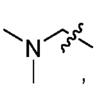, 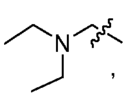, 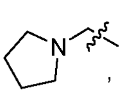, 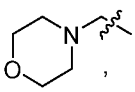, 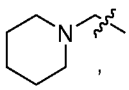, 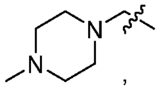, 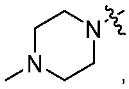, 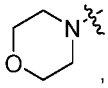,

trifluoromethyl, monofluoromethyl, difluoromethyl, 2-fluoroethyl, pentafluoroethyl.

173. The compound or a pharmaceutically acceptable salt thereof of any one of the preceding items, wherein $R^{34}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

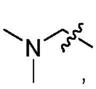, 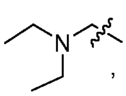, 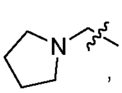, 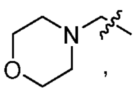, 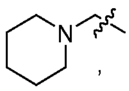, 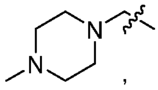, 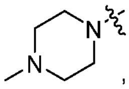, 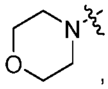,

trifluoromethyl, monofluoromethyl, difluoromethyl, 2-fluoroethyl, pentafluoroethyl.

174. The compound or a pharmaceutically acceptable salt thereof of any one of the preceding items, wherein $R^{34}$, for each occurrence, is independently selected from hydrogen, fluorine, cyano, methyl, isopropyl.

175. The compound or a pharmaceutically acceptable salt thereof of any one of the preceding items, wherein $R^{35}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

176. The compound or a pharmaceutically acceptable salt thereof of any one of the preceding items, wherein $R^{35}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

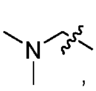, 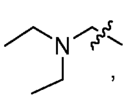, 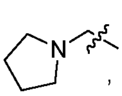, 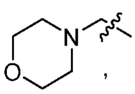, 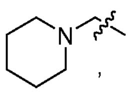, 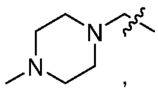, 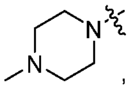, 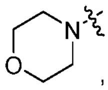,

trifluoromethyl, monofluoromethyl, difluoromethyl, 2-fluoroethyl, pentafluoroethyl.

177. The compound or a pharmaceutically acceptable salt thereof of any one of the preceding items, wherein $R^{35}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

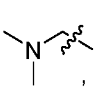, 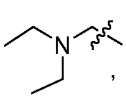, 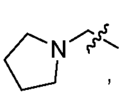, 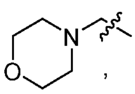, 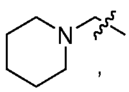, 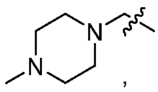, 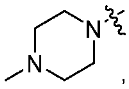, 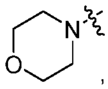,

trifluoromethyl, monofluoromethyl, difluoromethyl, 2-fluoroethyl, pentafluoroethyl.

178. The compound or a pharmaceutically acceptable salt thereof of any one of the preceding items, wherein $R^{35}$, for each occurrence, is independently selected from hydrogen, fluorine, cyano, methyl, isopropyl.

179. The compound or a pharmaceutically acceptable salt thereof of any one of the preceding items, wherein $R^{36}$, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

180. The compound or a pharmaceutically acceptable salt thereof of any one of the preceding items, wherein $R^{36}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

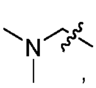, 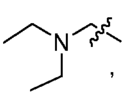, 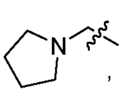, 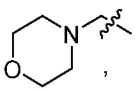, 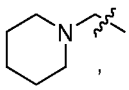, 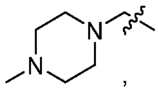, 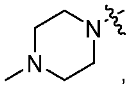, 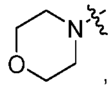,

trifluoromethyl, monofluoromethyl, difluoromethyl, 2-fluoroethyl, pentafluoroethyl.

181. The compound or a pharmaceutically acceptable salt thereof of any one of the preceding items, wherein $R^{36}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

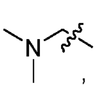, 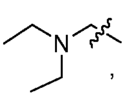, 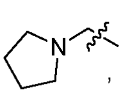, 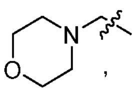, 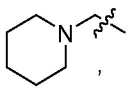, 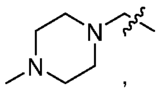, 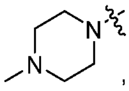, 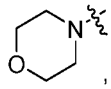,

trifluoromethyl, monofluoromethyl, difluoromethyl, 2-fluoroethyl, pentafluoroethyl.

182. The compound or a pharmaceutically acceptable salt thereof of any one of the preceding items, wherein $R^{36}$, for each occurrence, is independently selected from hydrogen, fluorine, cyano, methyl, isopropyl.

183. The compound or a pharmaceutically acceptable salt thereof of any one of the preceding items, wherein a is 0.

184. The compound or a pharmaceutically acceptable salt thereof of any one of the preceding items, wherein a is 1.

185. The compound or a pharmaceutically acceptable salt thereof of any one of the preceding items, wherein a is 2.

186. The compound or a pharmaceutically acceptable salt thereof of any one of the preceding items, wherein b is 0.

187. The compound or a pharmaceutically acceptable salt thereof of any one of the preceding items, wherein b is 1.

188. The compound or a pharmaceutically acceptable salt thereof of any one of the preceding items, wherein b is 2.

189. The compound or a pharmaceutically acceptable salt thereof of any one of the preceding items, wherein c is 0.

190. The compound or a pharmaceutically acceptable salt thereof of any one of the preceding items, wherein c is 1.

191. The compound or a pharmaceutically acceptable salt thereof of any one of the preceding items, wherein c is 2.

192. The compound or a pharmaceutically acceptable salt thereof of any one of the preceding items, wherein d is 0.

193. The compound or a pharmaceutically acceptable salt thereof of any one of the preceding items, wherein d is 1.

194. The compound or a pharmaceutically acceptable salt thereof of any one of the preceding items, wherein d is 2.

195. The compound or a pharmaceutically acceptable salt thereof of any one of the preceding items, wherein e is 0.

196. The compound or a pharmaceutically acceptable salt thereof of any one of the preceding items, wherein e is 1.

197. The compound or a pharmaceutically acceptable salt thereof of any one of the preceding items, wherein e is 2.

198. The compound or a pharmaceutically acceptable salt thereof of any one of the preceding items, wherein n is 0.

199. The compound or a pharmaceutically acceptable salt thereof of any one of the preceding items, wherein n is 1.

200. The compound or a pharmaceutically acceptable salt thereof of any one of the preceding items, wherein n is 2.

201. The compound or a pharmaceutically acceptable salt thereof of any one of the preceding items, wherein g is 0.

202. The compound or a pharmaceutically acceptable salt thereof of any one of the preceding items, wherein g is 1.

203. The compound or a pharmaceutically acceptable salt thereof of any one of the preceding items, wherein g is 2.

204. The compound or a pharmaceutically acceptable salt thereof of any one of the preceding items, wherein h is 0.

205. The compound or a pharmaceutically acceptable salt thereof of any one of the preceding items, wherein h is 1.

206. The compound or a pharmaceutically acceptable salt thereof of any one of the preceding items, wherein h is 2.

207. The compound or a pharmaceutically acceptable salt thereof of any one of the preceding items, wherein $X^1$ is N.

208. The compound or a pharmaceutically acceptable salt thereof of any one of the preceding items, wherein $X^1$ is $CR^{14}$.

209. The compound or a pharmaceutically acceptable salt thereof of any one of the preceding items, wherein $X^2$ is N.

210. The compound or a pharmaceutically acceptable salt thereof of any one of the preceding items, wherein $X^2$ is $CR^{14'}$.

211. The compound or a pharmaceutically acceptable salt thereof of any one of the preceding items, wherein $X^3$ is N.

212. The compound or a pharmaceutically acceptable salt thereof of any one of the preceding items, wherein $X^3$ is $CR^{14''}$.

213. The compound or a pharmaceutically acceptable salt thereof of any one of the preceding items, wherein $X^4$ is N.

214. A compound or a pharmaceutically acceptable salt thereof of any one of the preceding items, wherein $X^4$ is $CR^{14'''}$.

215. The compound or a pharmaceutically acceptable salt thereof of any one of the preceding items, wherein $X^5$ is O.

216. The compound or a pharmaceutically acceptable salt thereof of any one of the preceding items, wherein $X^5$ is $CR^{15}R^{16}$.

217. The compound or a pharmaceutically acceptable salt thereof of any one of the preceding items, wherein $X^5$ is $NR^{17}$.

218. The compound or a pharmaceutically acceptable salt thereof of any one of the preceding items, wherein $X^6$ is N.

219. The compound or a pharmaceutically acceptable salt thereof of any one of the preceding items, wherein $X^6$ is C.

220. The compound or a pharmaceutically acceptable salt thereof of any one of the preceding items, wherein $X^7$ is N.

221. The compound or a pharmaceutically acceptable salt thereof of any one of the preceding items, wherein $X^7$ is C.

222. The compound or a pharmaceutically acceptable salt thereof of any one of the preceding items, wherein $X^8$ is N.

223. The compound or a pharmaceutically acceptable salt thereof of any one of the preceding items, wherein $X^6$ is C.

224. The compound or a pharmaceutically acceptable salt thereof of any one of the preceding items, wherein $X^9$ is N.

225. The compound or a pharmaceutically acceptable salt thereof of any one of the preceding items, wherein $X^9$ is C.

226. The compound or a pharmaceutically acceptable salt thereof of any one of the preceding items, wherein T is N.

227. The compound or a pharmaceutically acceptable salt thereof of any one of the preceding items, wherein T is CH.

228. The compound or a pharmaceutically acceptable salt thereof of any one of the preceding items, wherein J is $NR^{31}$.

229. The compound or a pharmaceutically acceptable salt thereof of any one of the preceding items, wherein J is $CR^{32}R^{33}$.

230. The compound or a pharmaceutically acceptable salt thereof of any one of the preceding items, wherein K is $NR^{34}$.

231. The compound or a pharmaceutically acceptable salt thereof of any one of the preceding items, wherein K is $CR^{35}R^{36}$.

232. The compound or a pharmaceutically acceptable salt thereof of any one of the preceding items, wherein P, for each occurrence, is independently selected from absence, hydrogen, fluorine, chlorine, hydroxyl, nitro, cyano, amino, mercapto, -COOH, $C_1$-$C_6$ alkyl, , $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

233. The compound or a pharmaceutically acceptable salt thereof of any one of the preceding items, wherein P, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

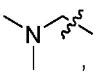, 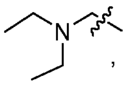, 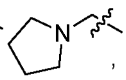, 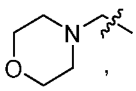, 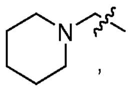, 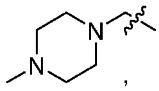, 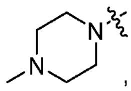, 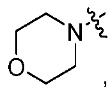,

trifluoromethyl, monofluoromethyl, difluoromethyl, 2-fluoroethyl, pentafluoroethyl, fluorophenylacetonitrile.

234. The compound or a pharmaceutically acceptable salt thereof of any one of the preceding items, wherein P, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

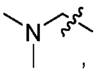, 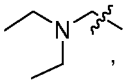, 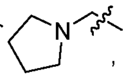, 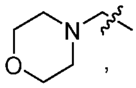, 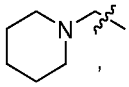, 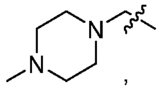, 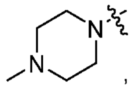, 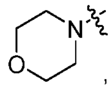,

trifluoromethyl, monofluoromethyl, difluoromethyl, 2-fluoroethyl, pentafluoroethyl, fluorophenylacetonitrile.

235. The compound or a pharmaceutically acceptable salt thereof of any one of the preceding items, wherein P, for each occurrence, is independently selected from hydrogen, fluorine, cyano, methyl, isopropyl, tert-butyl, trifluoromethyl, morpholinyl,

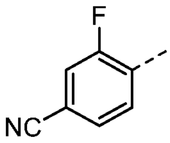 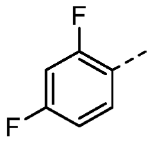 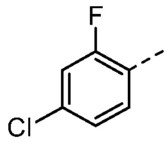 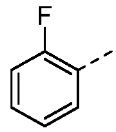

236. The compound or a pharmaceutically acceptable salt thereof of any one of the preceding items, wherein U is N.

237. The compound or a pharmaceutically acceptable salt thereof of any one of the preceding items, wherein U is C.

238. The compound or a pharmaceutically acceptable salt thereof of any one of the preceding items, wherein U is O.

236. The compound or a pharmaceutically acceptable salt thereof of any one of the preceding items, wherein Y is N.

237. The compound or a pharmaceutically acceptable salt thereof of any one of the preceding items, wherein Y is C.

238. The compound or a pharmaceutically acceptable salt thereof of any one of the preceding items, wherein Y is O.

239. The compound or a pharmaceutically acceptable salt thereof of any one of the preceding items, wherein L is O.

240. The compound or a pharmaceutically acceptable salt thereof of any one of the preceding items, wherein L is $NR^{21}$.

241. The compound or a pharmaceutically acceptable salt thereof of any one of the preceding items, wherein L is $CR^{22}$.

242. The compound or a pharmaceutically acceptable salt thereof of any one of the preceding items, wherein L is $CR^{23}R^{24}$.

243. The compound or a pharmaceutically acceptable salt thereof of any one of the preceding items, wherein L is $NR^{25}$-$CR^{26}$.

244. The compound or a pharmaceutically acceptable salt thereof of any one of the preceding items, wherein L is $CR^{27}$-$CR^{28}$.

245. The compound or a pharmaceutically acceptable salt thereof of any one of the preceding items, wherein

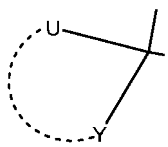

is a 5-membered monocyclic ring.

246. The compound or a pharmaceutically acceptable salt thereof of any one of the preceding items, wherein

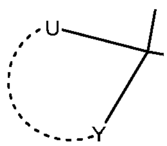

is a 6-membered monocyclic ring.

247. The compound or a pharmaceutically acceptable salt thereof of any one of the preceding items, wherein

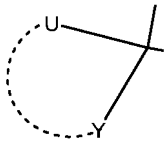

is a 7-membered monocyclic ring.

248. The compound or a pharmaceutically acceptable salt thereof of any one of the preceding items, wherein

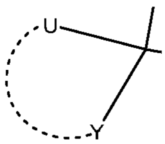

is substituted with 1 substituent.

249. The compound or a pharmaceutically acceptable salt thereof of any one of the preceding items, wherein

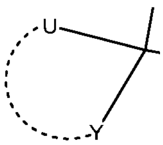

is substituted with 2 substituents.

250. The compound or a pharmaceutically acceptable salt thereof of any one of the preceding items, wherein

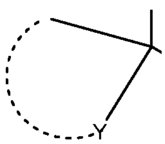

is a 5-membered monocyclic ring.

251. The compound or a pharmaceutically acceptable salt thereof of any one of the preceding items, wherein

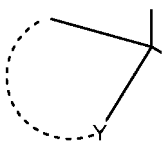

is a 6-membered monocyclic ring.

252. The compound or a pharmaceutically acceptable salt thereof of any one of the preceding items, wherein

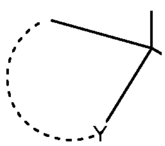

is a 7-membered monocyclic ring.

253. The compound or a pharmaceutically acceptable salt thereof of any one of the preceding items, wherein

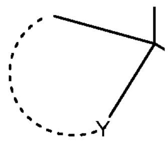

is substituted with 1 substituent.

254. The compound or a pharmaceutically acceptable salt thereof of any one of the preceding items, wherein

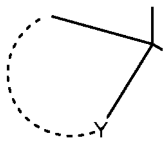

is substituted with 2 substituents.

255. The compound or a pharmaceutically acceptable salt thereof of any one of the preceding items, wherein

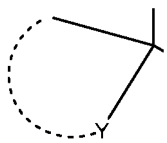

is

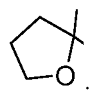

256. The compound or a pharmaceutically acceptable salt thereof of any one of the preceding items, wherein

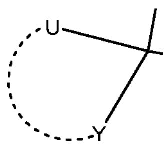

is

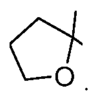

## Claims

**1.** A compound represented by structural formula (I):

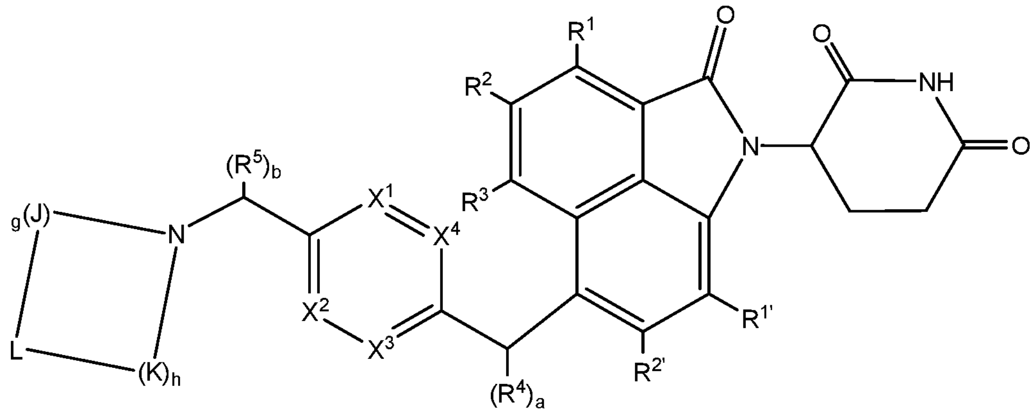

(I)

or a pharmaceutically acceptable salt thereof,
wherein:

$R^1$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino)$C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{1'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^2$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{2'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^3$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^4$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$

alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^5$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

a is 0, 1 or 2;

b is 0, 1 or 2;

$X^1$, for each occurrence, is independently selected from $CR^{14}$ or N;

$X^2$, for each occurrence, is independently selected from $CR^{14}$ or N;

$X^3$, for each occurrence, is independently selected from $CR^{14}$ or N;

$X^4$, for each occurrence, is independently selected from $CR^{14}$ or N;

$R^{14}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14'}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14''}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{14'''}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

L, for each occurrence, is independently selected from O, $NR^{21}$, $CR^{22}$, $CR^{23}R^{24}$, $NR^{25}$-$CR^{26}$, $CR^{27}$-$CR^{28}$,

$R^{21}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{22}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino,

mercapto, -COOH, or $C_1$-$C_6$ alkyl;

when L is $CR^{23}R^{24}$, $R^{23}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{24}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or,

$R^{23}$ and $R^{24}$ are joined together with the connected carbon atom to form a monocyclic, bicyclic or tricyclic saturated or unsaturated ring system with 5-14 ring atoms, wherein 0, 1, 2, 3 or 4 of the ring atoms are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

when L is $NR^{25}$-$CR^{26}$, $R^{25}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{26}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or,

$R^{25}$ and $R^{26}$ are joined together with the connected nitrogen atom and carbon atom to form a monocyclic, bicyclic or tricyclic saturated or unsaturated ring system with 5-14 ring atoms, wherein 0, 1, 2, 3 or 4 of the remaining ring atoms except the nitrogen atom are heteroatoms selected from N, O and S, with remaining ring atoms being carbon, and the ring system is optionally substituted with 1, 2, 3 or 4 halogens, hydroxyl, nitro, cyano, amino, mercapto, - COOH, or $C_1$-$C_6$ alkyl;

when L is $CR^{27}$-$CR^{28}$, $R^{27}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl,

$R^{28}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl, or

$R^{27}$ and $R^{28}$ are joined together with the connected carbon atom to form a monocyclic, bicyclic or tricyclic saturated or unsaturated ring system having 5-14 ring atoms, wherein 0, 1, 2, 3 or 4 of the ring atoms are heteroatoms selected from N, O and S, with remaining ring atoms being carbon, and the ring system is optionally substituted with 1, 2, 3 or 4 halogens, hydroxyl, nitro, cyano, amino, mercapto, -COOH, or $C_1$-$C_6$ alkyl;

J is $NR^{31}$, $CR^{32}R^{33}$;

g is 0, 1 or 2;

K is $NR^{14}$, $CR^{35}R^{36}$
h is 0, 1 or 2;
$R^{31}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{32}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{33}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{34}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{35}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{36}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

**2.** The compound or a pharmaceutically acceptable salt thereof of claim 1, wherein the compound of structural formula (I) is the compound of structural formula (II):

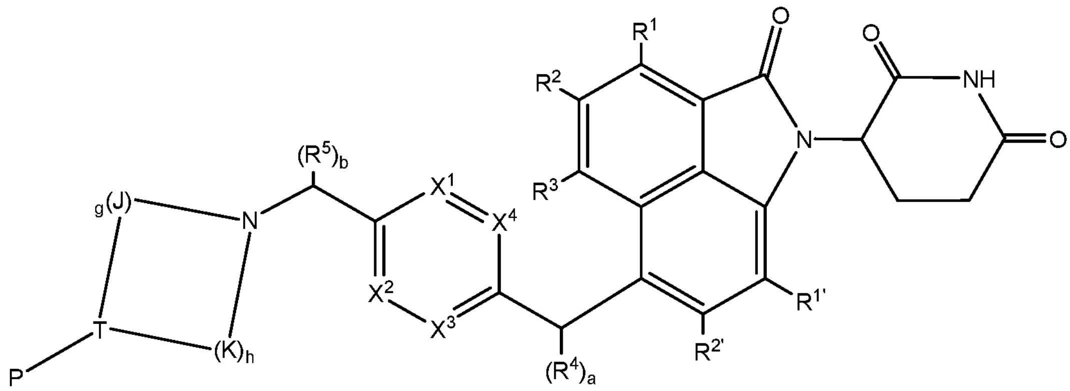

(II),

or a pharmaceutically acceptable salt thereof, wherein:

T is N or CH;

P, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.

**3.** The compound or a pharmaceutically acceptable salt thereof of claim 1, wherein the compound of structural formula (I) is the compound of structural formula (II):

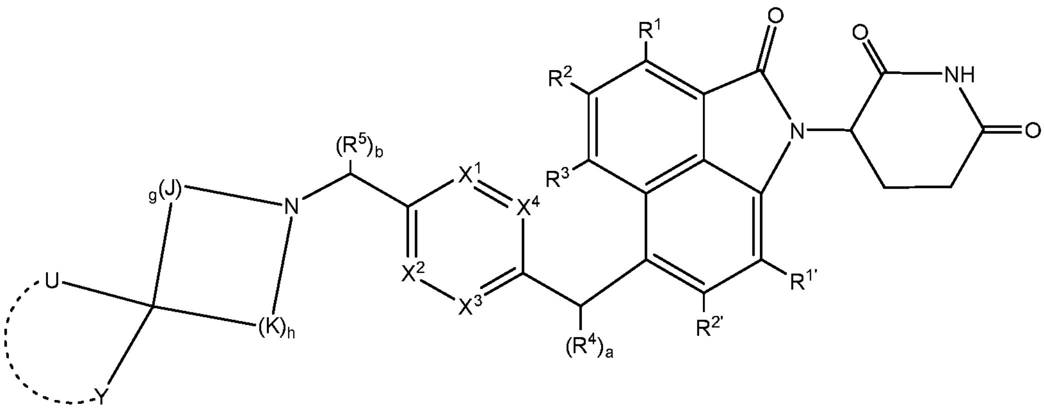

(III),

or a pharmaceutically acceptable salt thereof, wherein the group

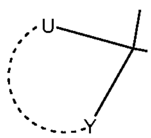

represents a saturated or unsaturated ring system having 5-14 ring atoms which is monocyclic, bicyclic or tricyclic, U is N, C or O, Y is N, C or O, the ring system further comprises additional 0, 1, 2, 3 or 4 heteroatoms selected from N, O, S, and the ring system is optionally substituted with 1, 2, 3 or 4 halogens, hydroxyl, nitro, cyano, amino, mercapto, -COOH, or $C_1$-$C_6$ alkyl.

**4.** The compound or a pharmaceutically acceptable salt thereof of claim 1, wherein the compound of structural formula (I) is the compound of structural formula (II):

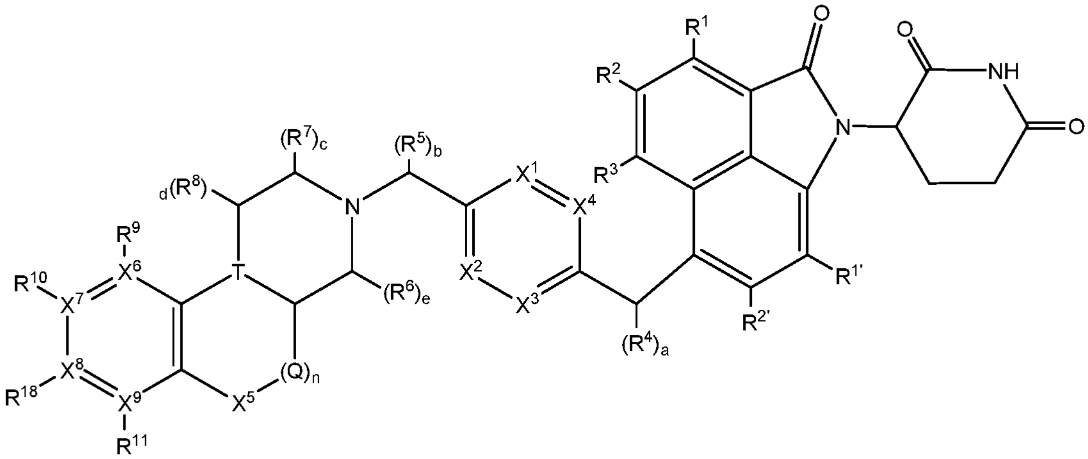

(IV),

or a pharmaceutically acceptable salt thereof,
wherein

$R^6$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^7$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^8$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^9$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{10}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;

$R^{11}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
c is 0, 1 or 2;
d is 0, 1 or 2;
e is 0, 1 or 2;
Q is $CR^{12}R^{13}$, n is 0, 1 or 2;
$X^5$ is selected from O, $CR^{15}R^{16}$, or $NR^{17}$,
$R^{12}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{13}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{15}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{16}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{17}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
$R^{18}$, for each occurrence, is independently selected from absence, hydrogen, halogen, hydroxyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl;
T is N or CH;
$X^6$ isN orC;
$X^7$ is N or C;
$X^8$ is N or C;

$X^9$ is N or C.

5. The compound or a pharmaceutically acceptable salt thereof of any one of the preceding claims, wherein the compound is the compound of structural formula (II):

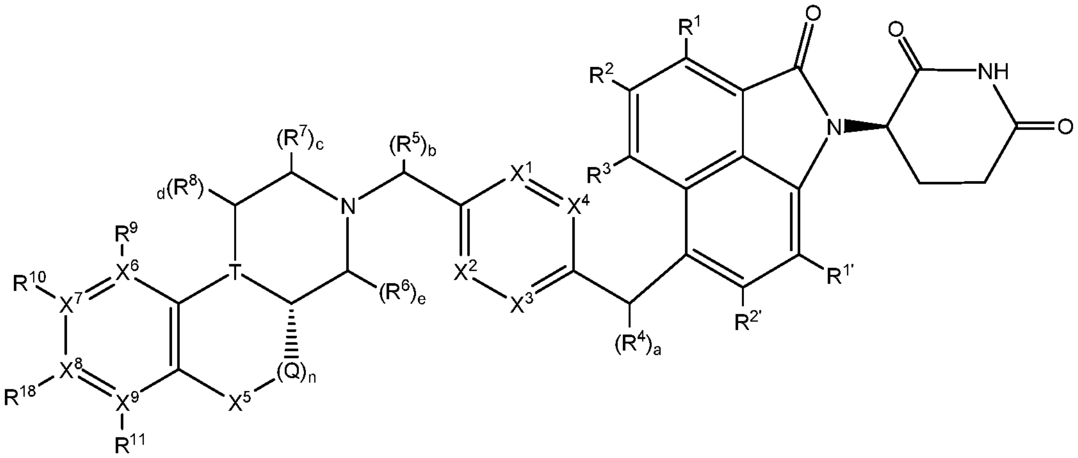

(V),

or a pharmaceutically acceptable salt thereof.

6. The compound or a pharmaceutically acceptable salt thereof of any one of the preceding claims, wherein the compound is the compound of structural formula (VI):

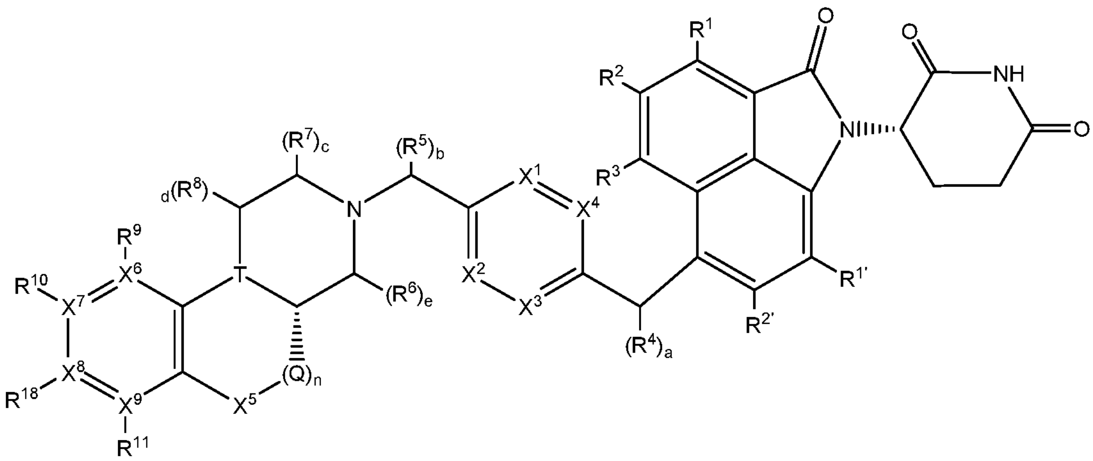

(VI),

or a pharmaceutically acceptable salt thereof.

7. The compound or a pharmaceutically acceptable salt thereof of any one of the preceding claims, wherein the compound is the compound of structural formula (VII):

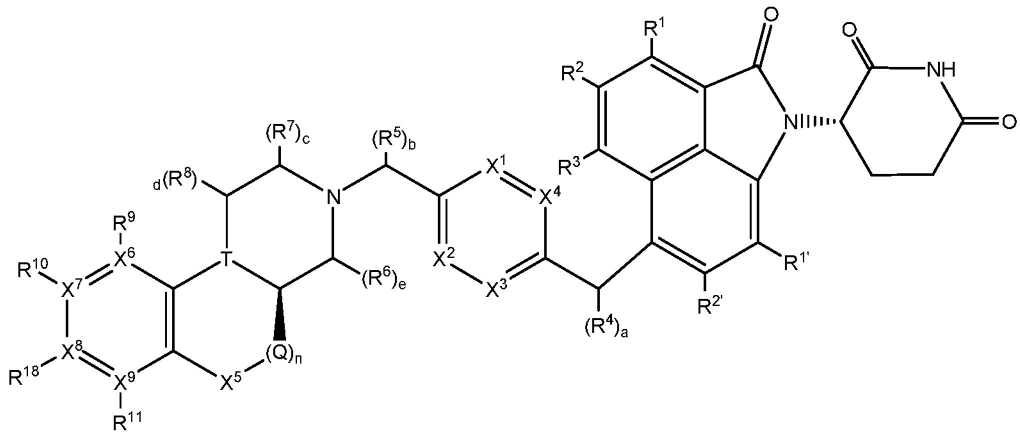

(VII),

or a pharmaceutically acceptable salt thereof.

**8.** The compound or a pharmaceutically acceptable salt thereof of any one of the preceding claims, wherein the compound is the compound of structural formula (VIII):

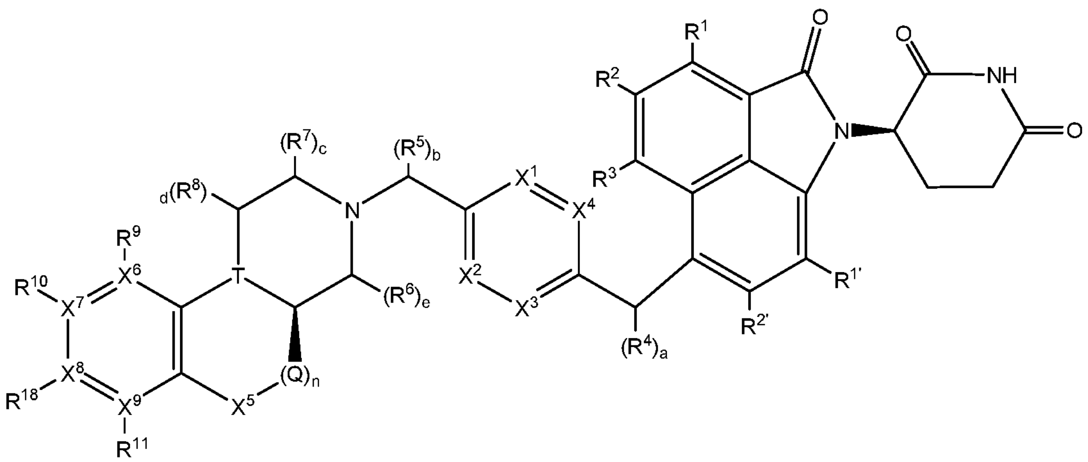

(VIII),

or a pharmaceutically acceptable salt thereof.

**9.** The compound or a pharmaceutically acceptable salt thereof of any one of the preceding claims, wherein the compound is the compound of structural formula (IX):

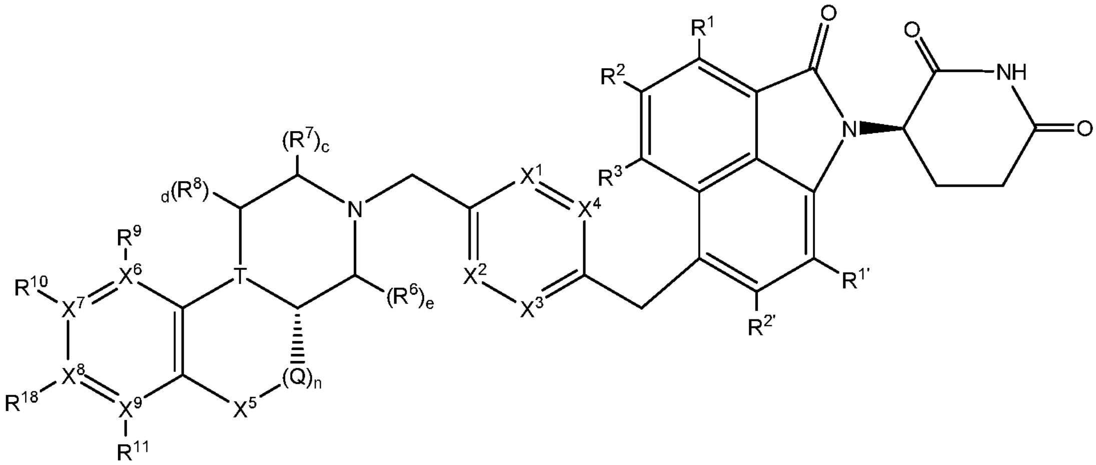

(IX),

or a pharmaceutically acceptable salt thereof.

**10.** The compound or a pharmaceutically acceptable salt thereof of any one of the preceding claims, wherein the compound is the compound of structural formula (X):

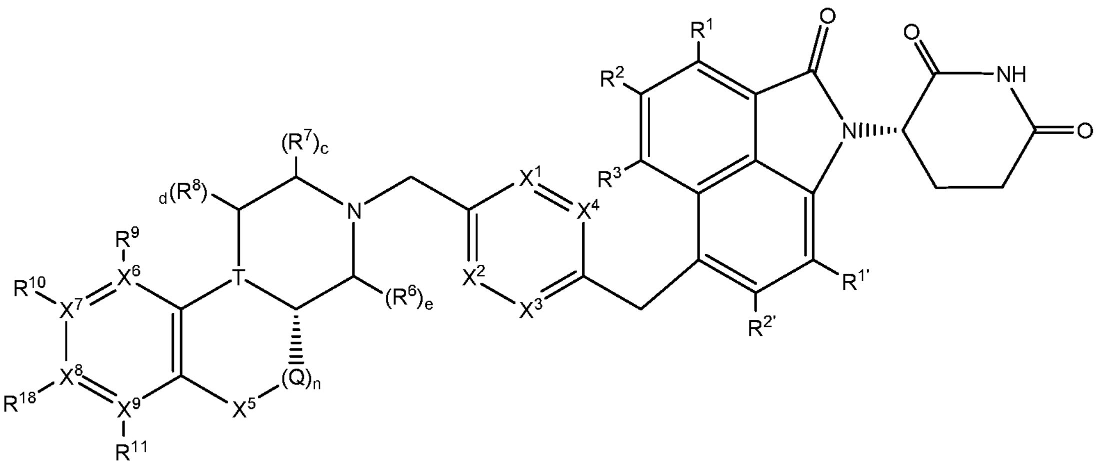

(X),

or a pharmaceutically acceptable salt thereof.

**11.** The compound or a pharmaceutically acceptable salt thereof of any one of the preceding claims, wherein the compound is the compound of structural formula (XI):

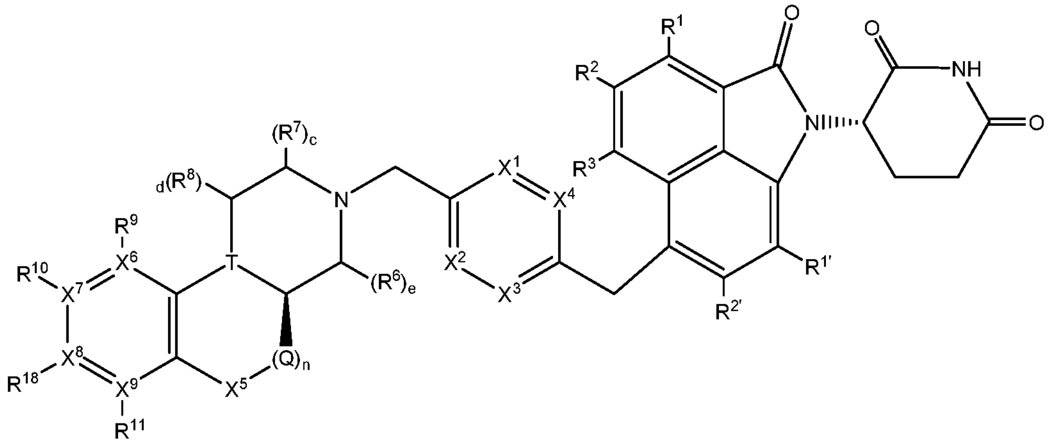

(XI),

or a pharmaceutically acceptable salt thereof.

**12.** The compound or a pharmaceutically acceptable salt thereof of any one of the preceding claims, wherein the compound is the compound of structural formula (XII):

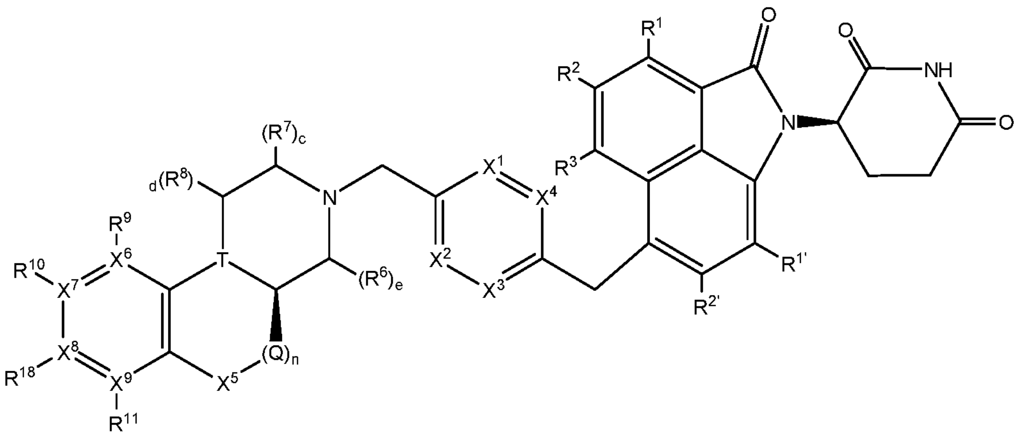

(XII),

or a pharmaceutically acceptable salt thereof.

**13.** The compound or a pharmaceutically acceptable salt thereof of any one of the preceding claims, wherein the compound is the compound of structural formula (XIII):

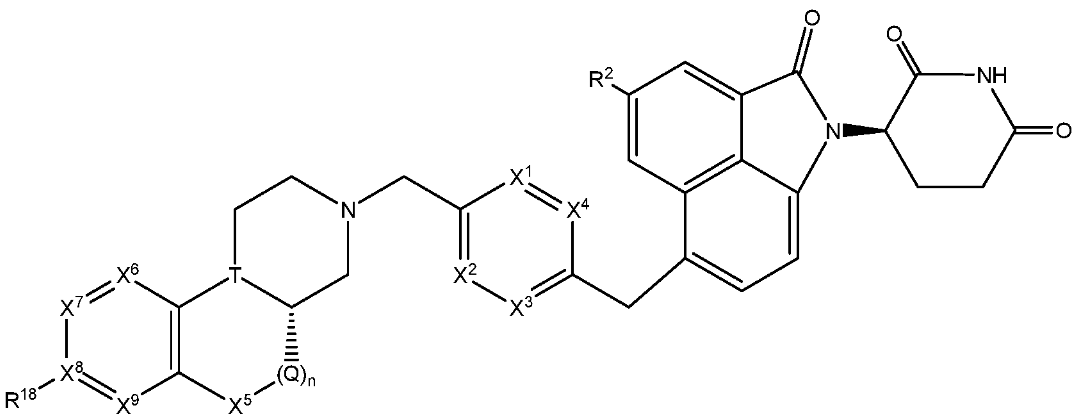

(XIII),

or a pharmaceutically acceptable salt thereof.

**14.** The compound or a pharmaceutically acceptable salt thereof of any one of the preceding claims, wherein the compound is the compound of structural formula (XIV):

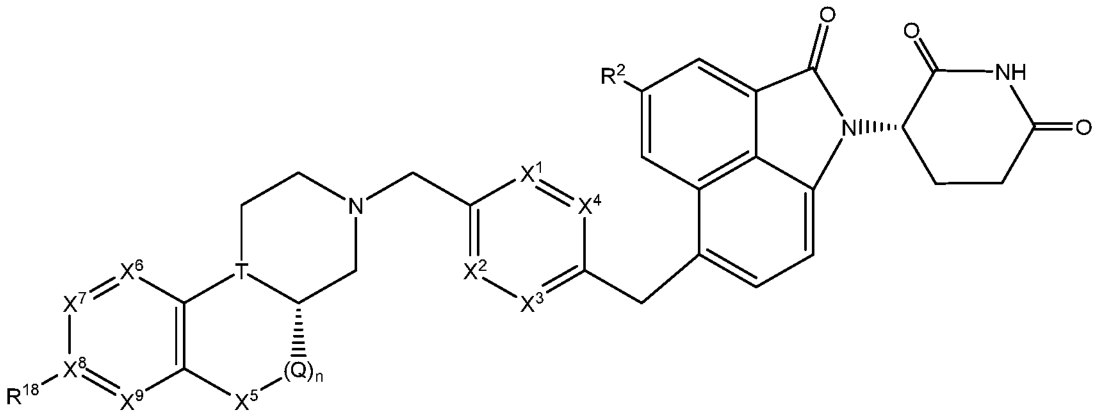

(XIV),

or a pharmaceutically acceptable salt thereof.

**15.** The compound or a pharmaceutically acceptable salt thereof of any one of the preceding claims, wherein the compound is the compound of structural formula (XV):

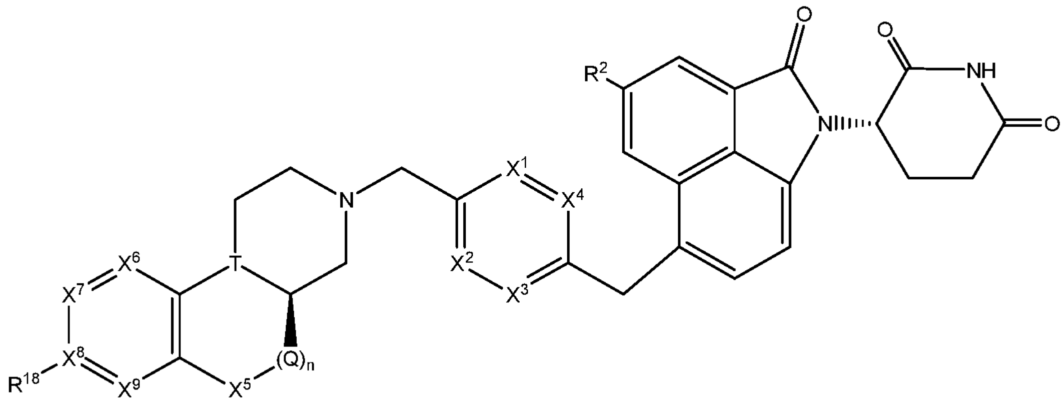

(XV),

or a pharmaceutically acceptable salt thereof.

**16.** The compound or a pharmaceutically acceptable salt thereof of any one of the preceding claims, wherein the compound is the compound of structural formula (XVI):

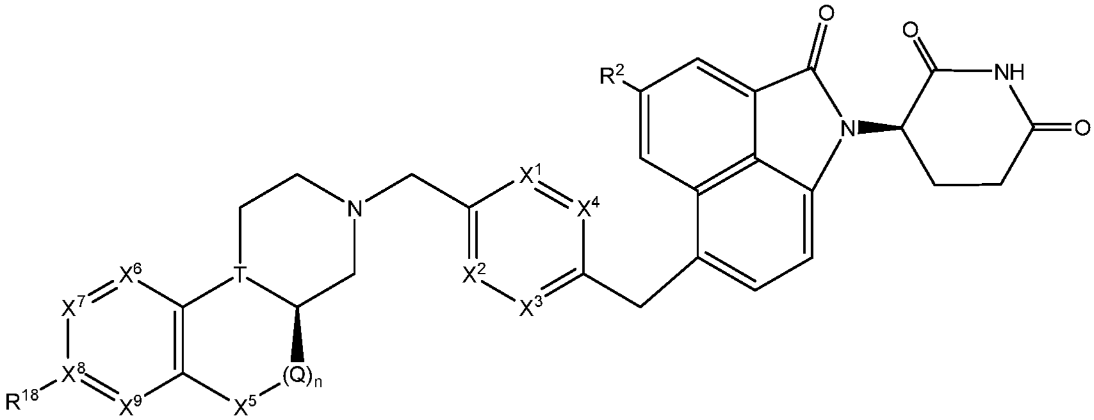

(XVI),

or a pharmaceutically acceptable salt thereof.

**17.** The compound or a pharmaceutically acceptable salt thereof of any one of the preceding claims, wherein the compound is the compound of structural formula (XVII):

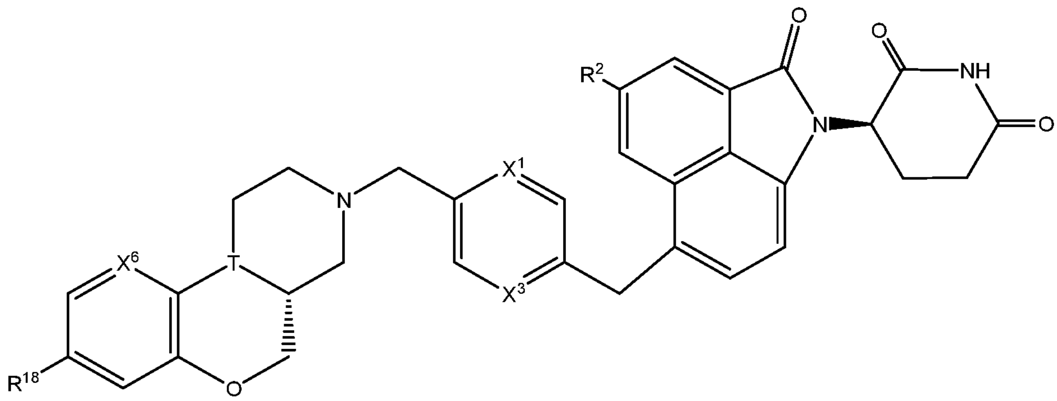

(XVII),

or a pharmaceutically acceptable salt thereof.

**18.** The compound or a pharmaceutically acceptable salt thereof of any one of the preceding claims, wherein the compound is the compound of structural formula (XVIII):

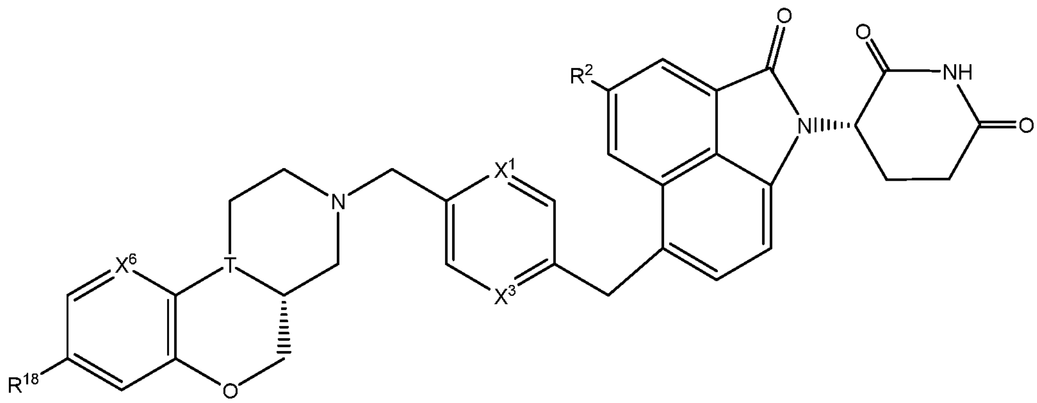

(XVIII),

or a pharmaceutically acceptable salt thereof.

**19.** The compound or a pharmaceutically acceptable salt thereof of any one of the preceding claims, wherein the compound is the compound of structural formula (XIX):

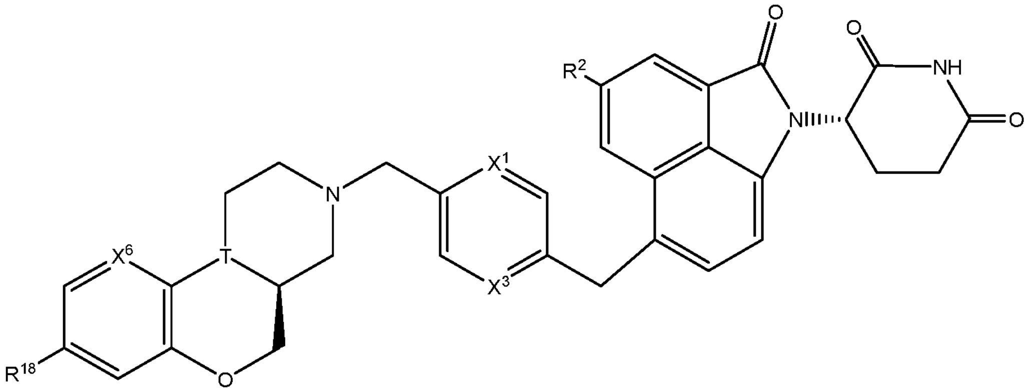

(XIX),

or a pharmaceutically acceptable salt thereof.

**20.** The compound or a pharmaceutically acceptable salt thereof of any one of the preceding claims, wherein the compound is the compound of structural formula (XX):

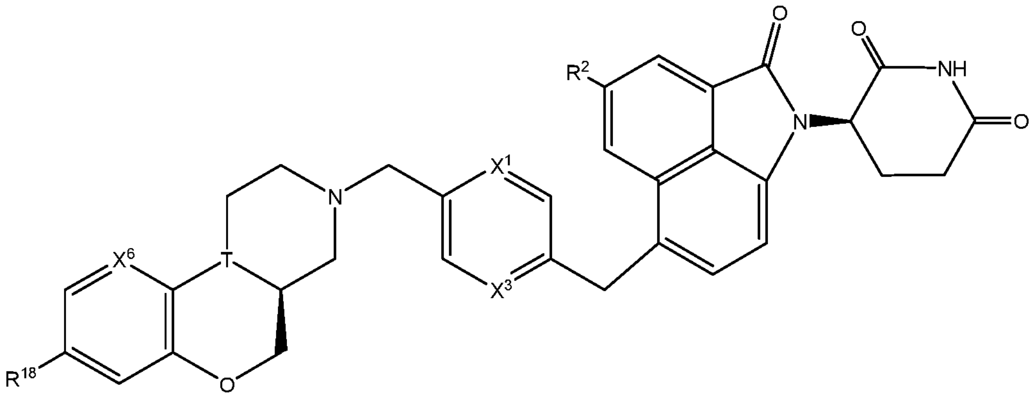

(XX),

or a pharmaceutically acceptable salt thereof.

**21.** The compound or a pharmaceutically acceptable salt thereof of any one of the preceding claims, wherein the compound is the compound of structural formula (XXI):

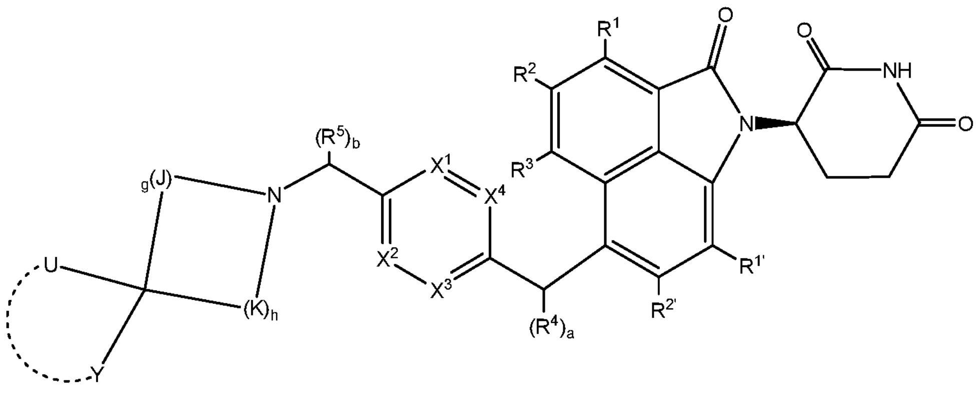

(XXI),

or a pharmaceutically acceptable salt thereof.

**22.** The compound or a pharmaceutically acceptable salt thereof of any one of the preceding claims, wherein the compound is the compound of structural formula (XXII):

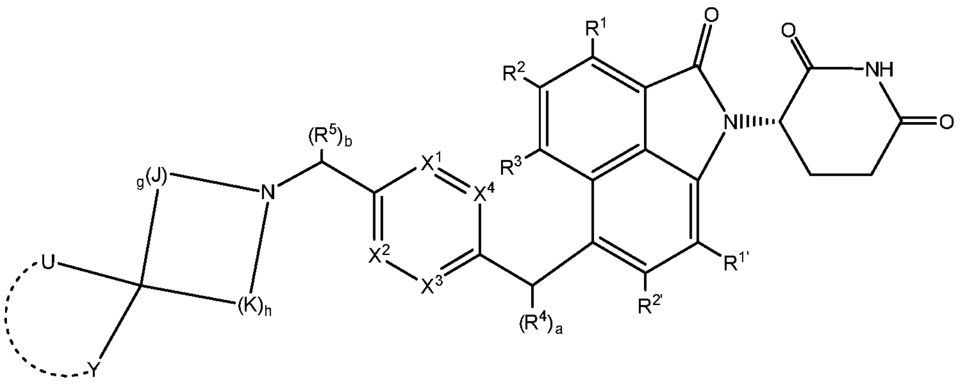

(XXII),

or a pharmaceutically acceptable salt thereof.

**23.** The compound or a pharmaceutically acceptable salt thereof of any one of the preceding claims, wherein the compound is the compound of structural formula (XXIII):

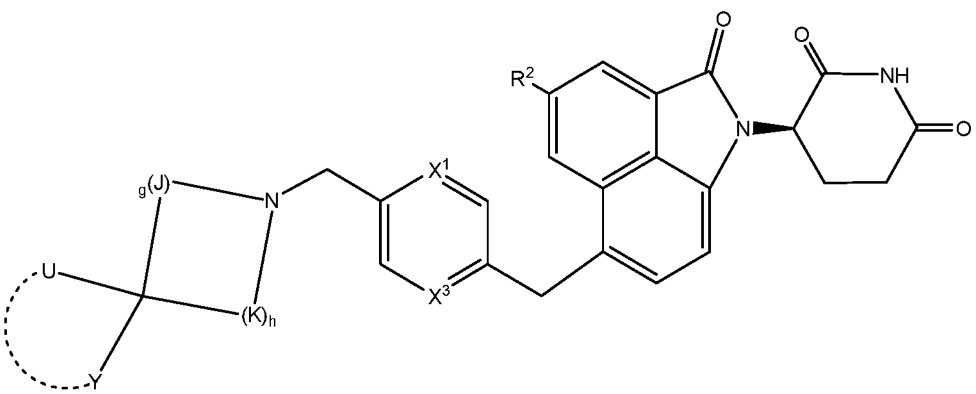

(XXIII),

or a pharmaceutically acceptable salt thereof.

**24.** The compound or a pharmaceutically acceptable salt thereof of any one of the preceding claims, wherein the compound is the compound of structural formula (XXIV):

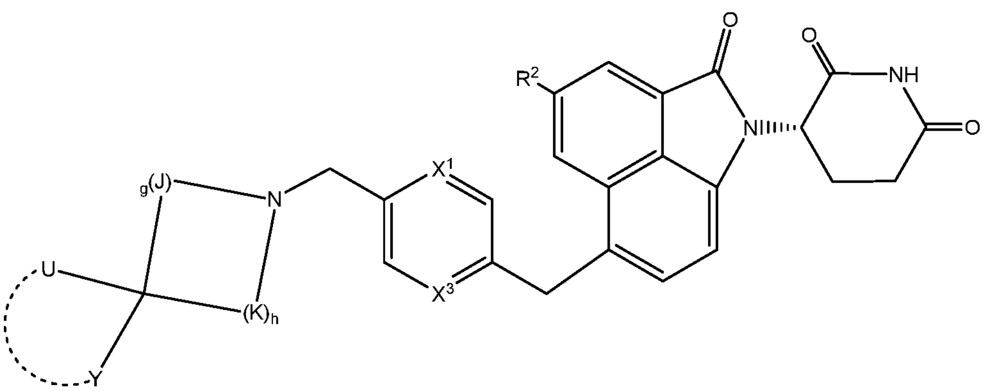

(XXIV),

or a pharmaceutically acceptable salt thereof.

**25.** The compound or a pharmaceutically acceptable salt thereof of any one of the preceding claims, wherein the compound is the compound of structural formula (XXV):

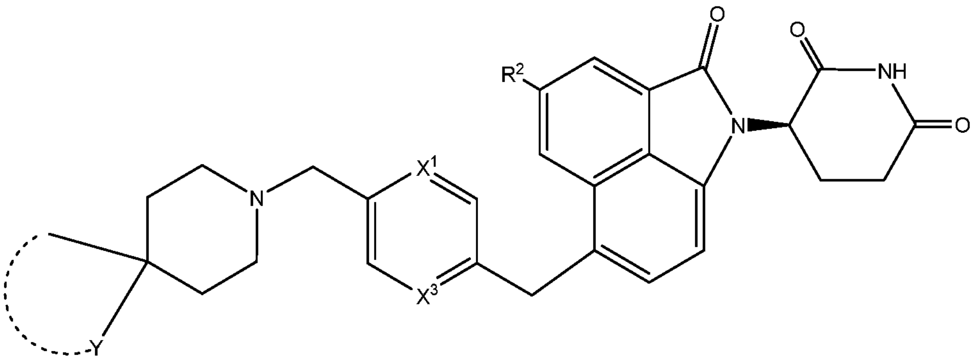

(XXV),

or a pharmaceutically acceptable salt thereof.

**26.** The compound or a pharmaceutically acceptable salt thereof of any one of the preceding claims, wherein the compound is the compound of structural formula (XXVI):

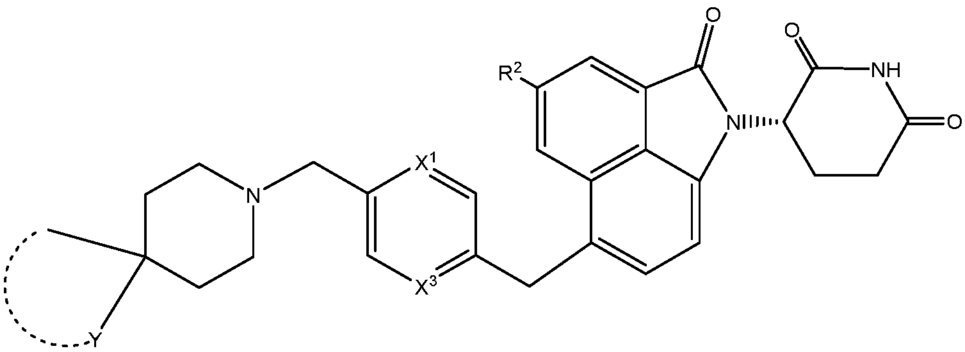

(XXVI),

or a pharmaceutically acceptable salt thereof.

**27.** The compound or a pharmaceutically acceptable salt thereof of any one of the preceding claims, wherein the compound is the compound of structural formula (XXVII):

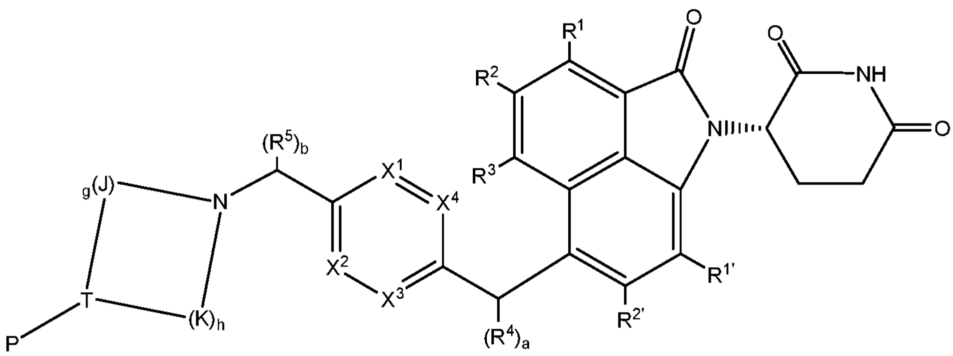

(XXVII),

or a pharmaceutically acceptable salt thereof.

**28.** The compound or a pharmaceutically acceptable salt thereof of any one of the preceding claims, wherein the

compound is the compound of structural formula (XXVIII):

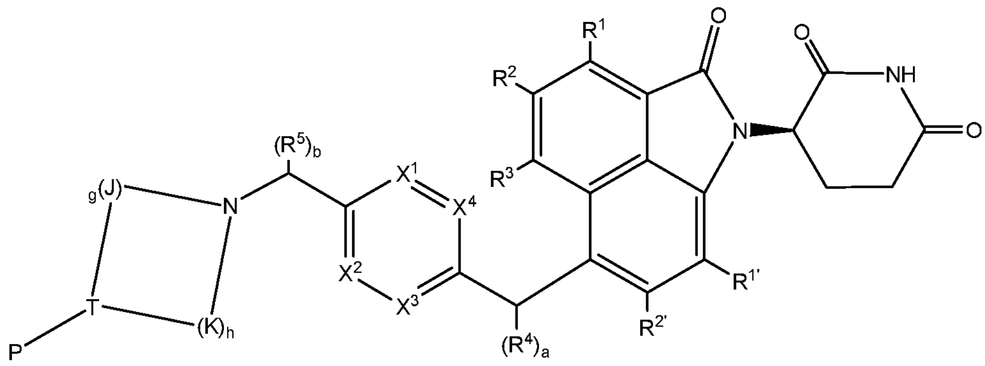

(XXVIII),

or a pharmaceutically acceptable salt thereof.

**29.** The compound or a pharmaceutically acceptable salt thereof of any one of the preceding claims, wherein the compound is the compound of structural formula (XXIX):

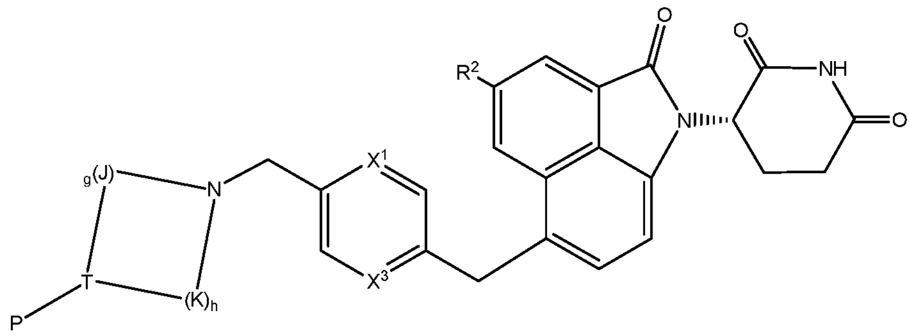

(XXIX),

or a pharmaceutically acceptable salt thereof.

**30.** The compound or a pharmaceutically acceptable salt thereof of any one of the preceding claims, wherein the compound is the compound of structural formula (XXX):

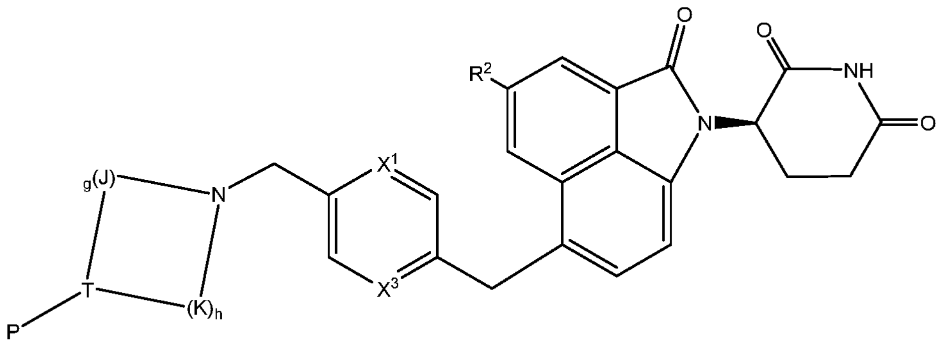

(XXX),

or a pharmaceutically acceptable salt thereof.

**31.** The compound or a pharmaceutically acceptable salt thereof of any one of the preceding claims, wherein the compound is the compound of structural formula (XXXI):

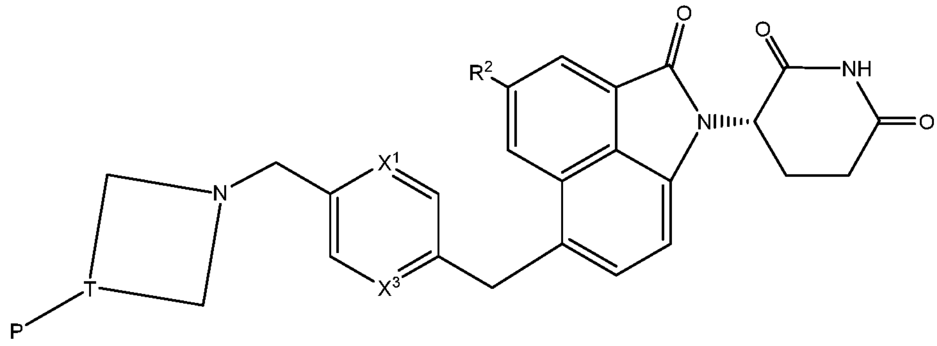

(XXXI),

or a pharmaceutically acceptable salt thereof.

**32.** The compound or a pharmaceutically acceptable salt thereof of any one of the preceding claims, wherein the compound is the compound of structural formula (XXXII):

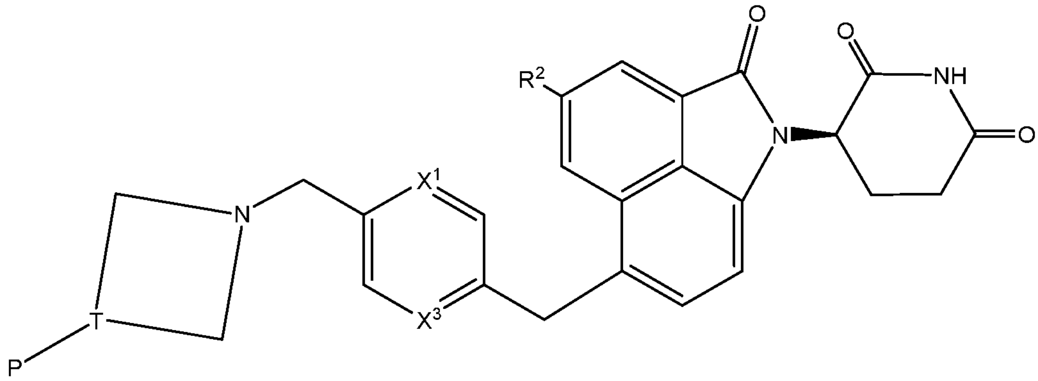

(XXXII),

or a pharmaceutically acceptable salt thereof.

**33.** The compound or a pharmaceutically acceptable salt thereof of any one of the preceding claims, wherein the compound is the compound of structural formula (XXXIII):

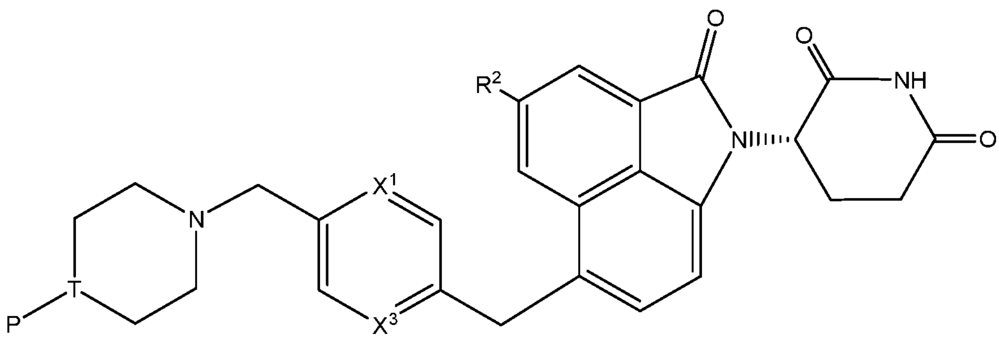

(XXXIII),

or a pharmaceutically acceptable salt thereof.

**34.** The compound or a pharmaceutically acceptable salt thereof of any one of the preceding claims, wherein the compound is the compound of structural formula (XXXIV):

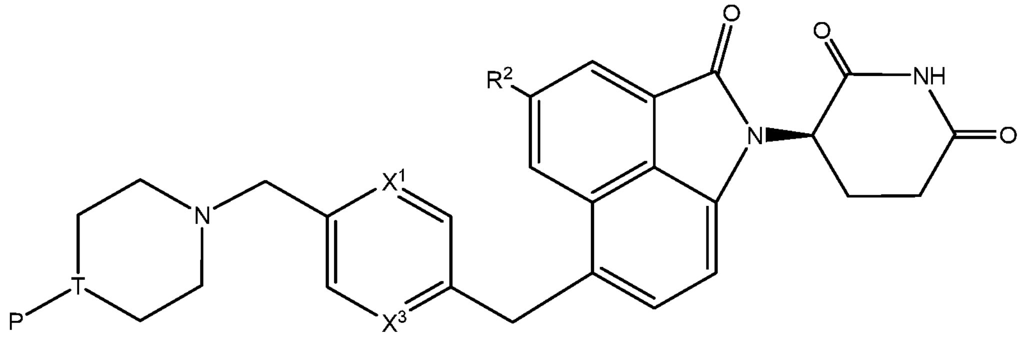

(XXXIV),

or a pharmaceutically acceptable salt thereof.

**35.** The compound or a pharmaceutically acceptable salt thereof of any one of the preceding claims, wherein $R^1$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^1$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

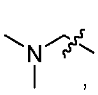, 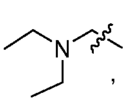, 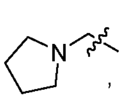, 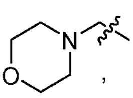, 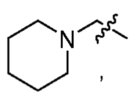, 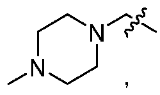, 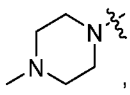, 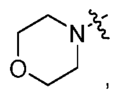,

trifluoromethyl, monofluoromethyl, difluoromethyl, 2-fluoroethyl, pentafluoroethyl; more preferably, $R^1$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

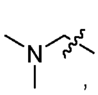, 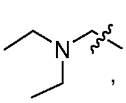, 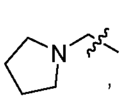, 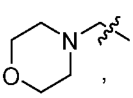, 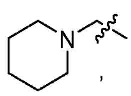, 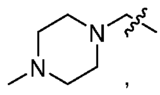, 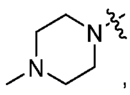, 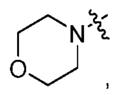,

trifluoromethyl, monofluoromethyl, difluoromethyl, 2-fluoroethyl, pentafluoroethyl; most preferably, $R^1$, for each occurrence, is independently selected from hydrogen, fluorine, cyano, methyl, isopropyl.

**36.** The compound or a pharmaceutically acceptable salt thereof of any one of the preceding claims, wherein $R^{1'}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{1'}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

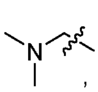, 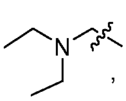, 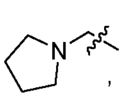, 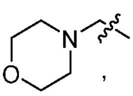, 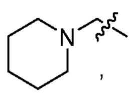, 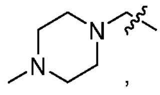, 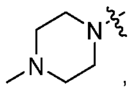, 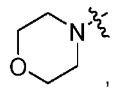,

trifluoromethyl, monofluoromethyl, difluoromethyl, 2-fluoroethyl, pentafluoroethyl; more preferably, $R^{1'}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

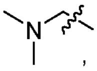, 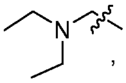, 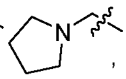, 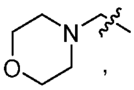, 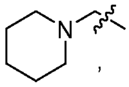, 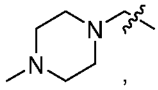, 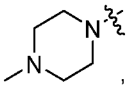, 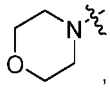,

trifluoromethyl, monofluoromethyl, difluoromethyl, 2-fluoroethyl, pentafluoroethyl; most preferably, $R^1$, for each occurrence, is independently selected from hydrogen, fluorine, cyano, methyl, isopropyl.

**37.** The compound or a pharmaceutically acceptable salt thereof of any one of the preceding claims, $R^2$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^2$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

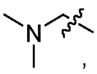, 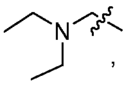, 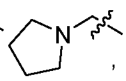, 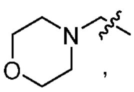, 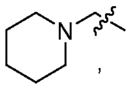, 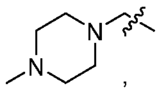, 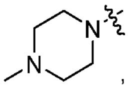, 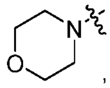,

trifluoromethyl, monofluoromethyl, difluoromethyl, 2-fluoroethyl, pentafluoroethyl; more preferably, $R^2$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

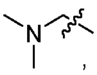, 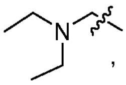, 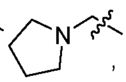, 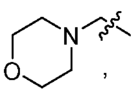, 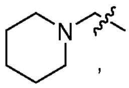, 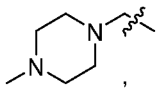, 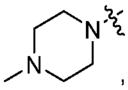, 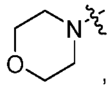,

trifluoromethyl, monofluoromethyl, difluoromethyl, 2-fluoroethyl, pentafluoroethyl; most preferably, $R^2$, for each occurrence, is independently selected from hydrogen, fluorine, cyano, methyl, isopropyl.

**38.** The compound or a pharmaceutically acceptable salt thereof of any one of the preceding claims, wherein $R^{2'}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl.; preferably, $R^{2'}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

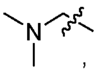, 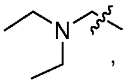, 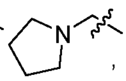, 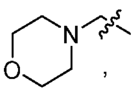, 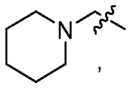, 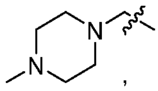, 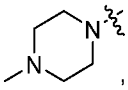, 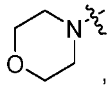,

trifluoromethyl, monofluoromethyl, difluoromethyl, 2-fluoroethyl, pentafluoroethyl; more preferably, $R^{2'}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

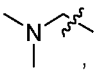, 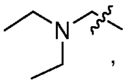, 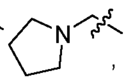, 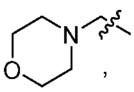, 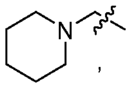, 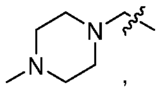, 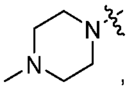, 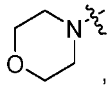,

trifluoromethyl, monofluoromethyl, difluoromethyl, 2-fluoroethyl, pentafluoroethyl; most preferably, $R^{2'}$, for each occurrence, is independently selected from hydrogen, fluorine, cyano, methyl, isopropyl.

**39.** The compound or a pharmaceutically acceptable salt thereof of any one of the preceding claims, wherein $R^3$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^3$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

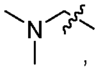, 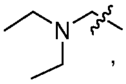, 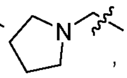, 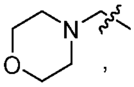, 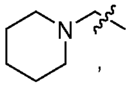, 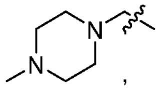, 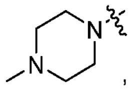, 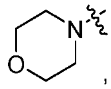,

trifluoromethyl, monofluoromethyl, difluoromethyl, 2-fluoroethyl, pentafluoroethyl; more preferably, $R^3$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

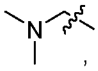, 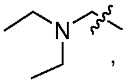, 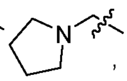, 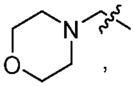, 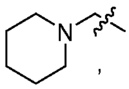, 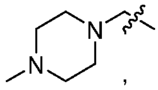, 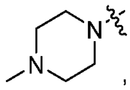, 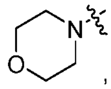,

trifluoromethyl, monofluoromethyl, difluoromethyl, 2-fluoroethyl, pentafluoroethyl; most preferably, $R^3$, for each occurrence, is independently selected from hydrogen, fluorine, cyano, methyl, isopropyl.

**40.** The compound or a pharmaceutically acceptable salt thereof of any one of the preceding claims, wherein $R^4$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^4$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

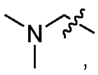, 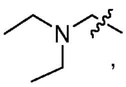, 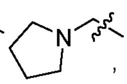, 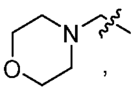, 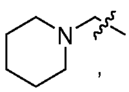, 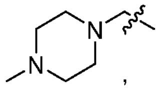, 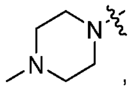, 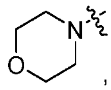,

trifluoromethyl, monofluoromethyl, difluoromethyl, 2-fluoroethyl, pentafluoroethyl; more preferably, $R^4$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

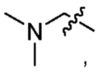, 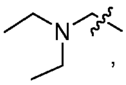, 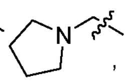, 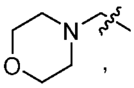, 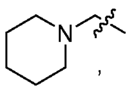, 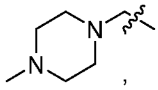, 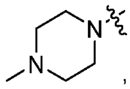, 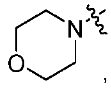,

trifluoromethyl, monofluoromethyl, difluoromethyl, 2-fluoroethyl, pentafluoroethyl; most preferably, $R^4$, for each occurrence, is independently selected from hydrogen, fluorine, cyano, methyl, isopropyl.

**41.** The compound or a pharmaceutically acceptable salt thereof of any one of the preceding claims, wherein $R^5$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl,

and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^5$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

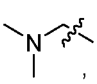, 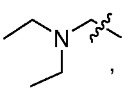, 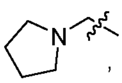, 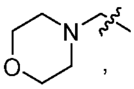, 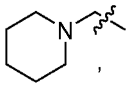, 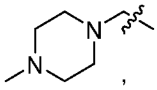, 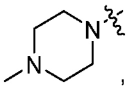, 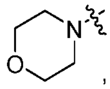,

trifluoromethyl, monofluoromethyl, difluoromethyl, 2-fluoroethyl, pentafluoroethyl; more preferably, $R^5$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

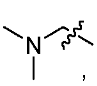, 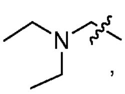, 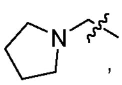, 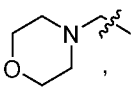, 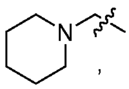, 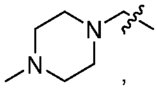, 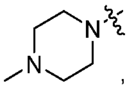, 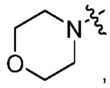,

trifluoromethyl, monofluoromethyl, difluoromethyl, 2-fluoroethyl, pentafluoroethyl; most preferably, $R^5$, for each occurrence, is independently selected from hydrogen, fluorine, cyano, methyl, isopropyl.

**42.** The compound or a pharmaceutically acceptable salt thereof of any one of the preceding claims, wherein $R^6$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^6$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

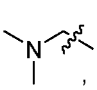, 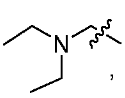, 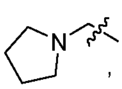, 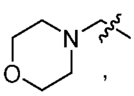, 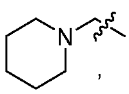, 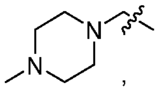, 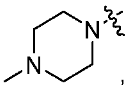, 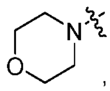,

trifluoromethyl, monofluoromethyl, difluoromethyl, 2-fluoroethyl, pentafluoroethyl; more preferably, $R^6$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

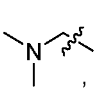, 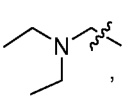, 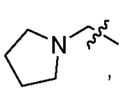, 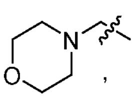, 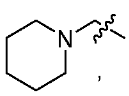, 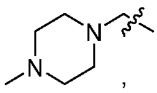, 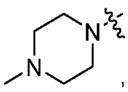, 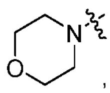,

trifluoromethyl, monofluoromethyl, difluoromethyl, 2-fluoroethyl, pentafluoroethyl; most preferably, $R^6$, for each occurrence, is independently selected from hydrogen, fluorine, cyano, methyl, isopropyl.

**43.** The compound or a pharmaceutically acceptable salt thereof of any one of the preceding claims, wherein $R^7$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^7$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

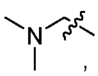, 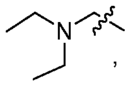, 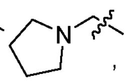, 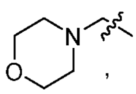, 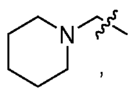, 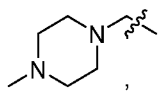, 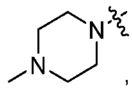, 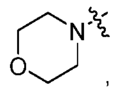,

trifluoromethyl, monofluoromethyl, difluoromethyl, 2-fluoroethyl, pentafluoroethyl; more preferably, $R^7$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

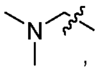, 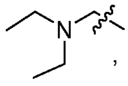, 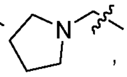, 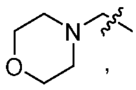, 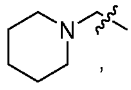, 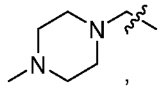, 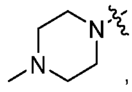, 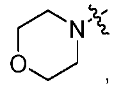,

trifluoromethyl, monofluoromethyl, difluoromethyl, 2-fluoroethyl, pentafluoroethyl; most preferably, $R^7$, for each occurrence, is independently selected from hydrogen, fluorine, cyano, methyl, isopropyl.

**44.** The compound or a pharmaceutically acceptable salt thereof of any one of the preceding claims, wherein $R^8$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^8$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

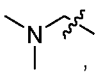, 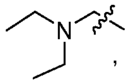, 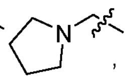, 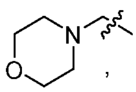, 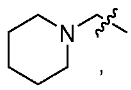, 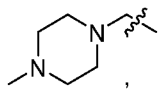, 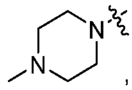, 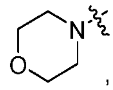,

trifluoromethyl, monofluoromethyl, difluoromethyl, 2-fluoroethyl, pentafluoroethyl; more preferably, $R^8$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

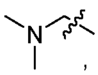, 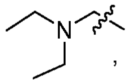, 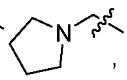, 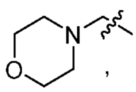, 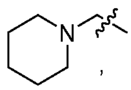, 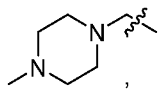, 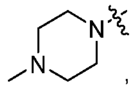, 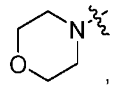,

trifluoromethyl, monofluoromethyl, difluoromethyl, 2-fluoroethyl, pentafluoroethyl; most preferably, $R^8$, for each occurrence, is independently selected from hydrogen, fluorine, cyano, methyl, isopropyl.

**45.** The compound or a pharmaceutically acceptable salt thereof of any one of the preceding claims, wherein $R^9$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^9$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

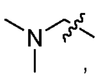, 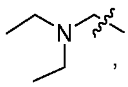, 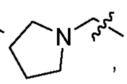, 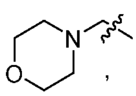, 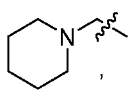, 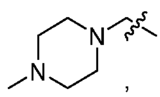, 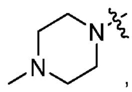, 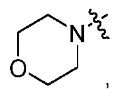,

trifluoromethyl, monofluoromethyl, difluoromethyl, 2-fluoroethyl, pentafluoroethyl; more preferably, $R^9$, for each occurrence,

currence, is independently selected from hydrogen, fluorine, chlorine, cyano, amino, mercapto, -COOH, methyl, ethyl, n-propyl, isopropyl, methoxy, ethoxy, n-propoxy, isopropoxy,

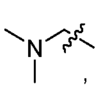 , 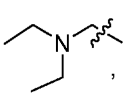 , 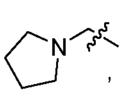 , 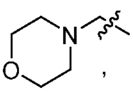 , 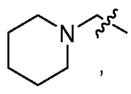 , 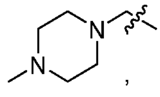 , 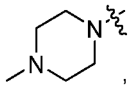 , 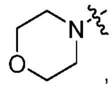 ,

trifluoromethyl, monofluoromethyl, difluoromethyl, 2-fluoroethyl, pentafluoroethyl; most preferably, $R^9$, for each occurrence, is independently selected from hydrogen, fluorine, cyano, methyl, isopropyl.

**46.** The compound or a pharmaceutically acceptable salt thereof of any one of the preceding claims, wherein $R^{10}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{10}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

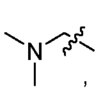 , 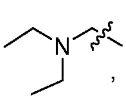 , 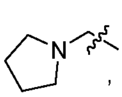 , 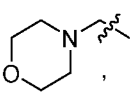 , 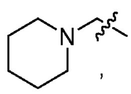 , 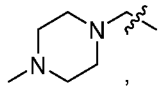 , 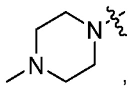 , 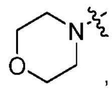 ,

trifluoromethyl, monofluoromethyl, difluoromethyl, 2-fluoroethyl, pentafluoroethyl; more preferably, $R^{10}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

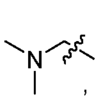 , 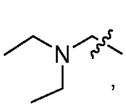 , 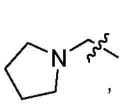 , 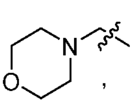 , 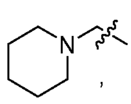 , 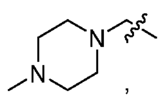 , 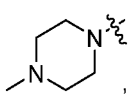 , 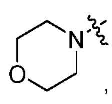 ,

trifluoromethyl, monofluoromethyl, difluoromethyl, 2-fluoroethyl, pentafluoroethyl; most preferably, $R^{10}$, for each occurrence, is independently selected from hydrogen, fluorine, cyano, methyl, isopropyl.

**47.** The compound or a pharmaceutically acceptable salt thereof of any one of the preceding claims, wherein $R^{11}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{11}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

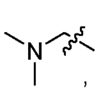 , 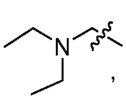 , 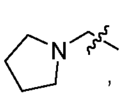 , 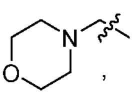 , 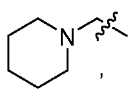 , 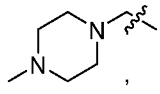 , 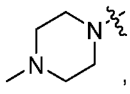 , 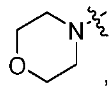 ,

trifluoromethyl, monofluoromethyl, difluoromethyl, 2-fluoroethyl, pentafluoroethyl; more preferably, $R^{11}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

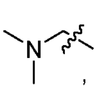, 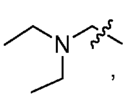, 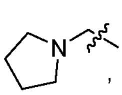, 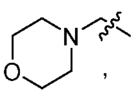, 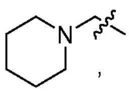, 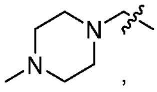, 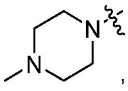, 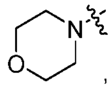,

trifluoromethyl, monofluoromethyl, difluoromethyl, 2-fluoroethyl, pentafluoroethyl; most preferably, $R^{11}$, for each occurrence, is independently selected from hydrogen, fluorine, cyano, methyl, isopropyl.

**48.** The compound or a pharmaceutically acceptable salt thereof of any one of the preceding claims, wherein $R^{12}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{12}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

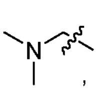, 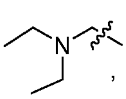, 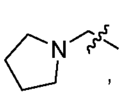, 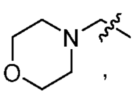, 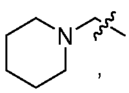, 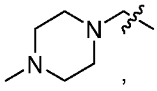, 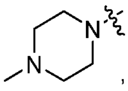, 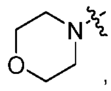,

trifluoromethyl, monofluoromethyl, difluoromethyl, 2-fluoroethyl, pentafluoroethyl; more preferably, $R^{12}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

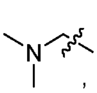, 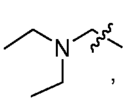, 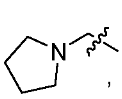, 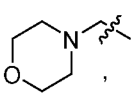, 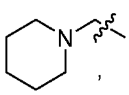, 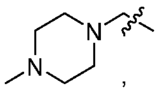, 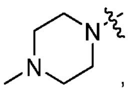, 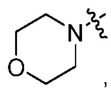,

trifluoromethyl, monofluoromethyl, difluoromethyl, 2-fluoroethyl, pentafluoroethyl; most preferably, $R^{12}$, for each occurrence, is independently selected from hydrogen, fluorine, cyano, methyl, isopropyl.

**49.** The compound or a pharmaceutically acceptable salt thereof of any one of the preceding claims, wherein $R^{13}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{13}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

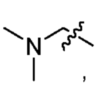, 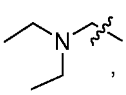, 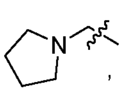, 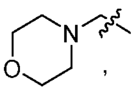, 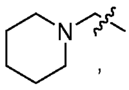, 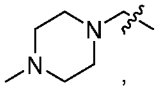, 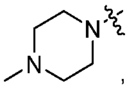, 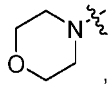,

trifluoromethyl, monofluoromethyl, difluoromethyl, 2-fluoroethyl, pentafluoroethyl; more preferably, $R^{13}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

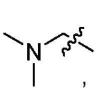, 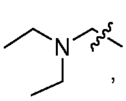, 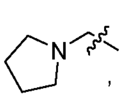, 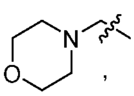, 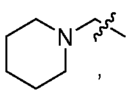, 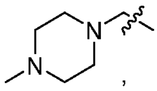, 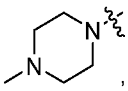, 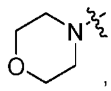,

trifluoromethyl, monofluoromethyl, difluoromethyl, 2-fluoroethyl, pentafluoroethyl; most preferably, $R^{13}$, for each

occurrence, is independently selected from hydrogen, fluorine, cyano, methyl, isopropyl.

**50.** The compound or a pharmaceutically acceptable salt thereof of any one of the preceding claims, wherein $R^{14}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

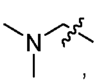, 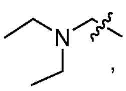, 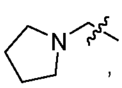, 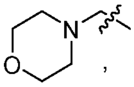, 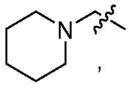, 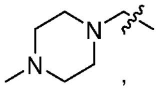, 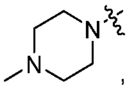, 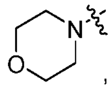,

trifluoromethyl, monofluoromethyl, difluoromethyl, 2-fluoroethyl, pentafluoroethyl; more preferably, $R^{14}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

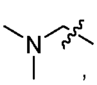, 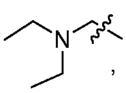, 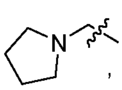, 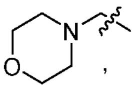, 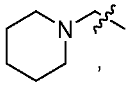, 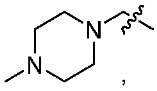, 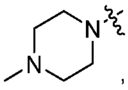, 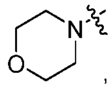,

trifluoromethyl, monofluoromethyl, difluoromethyl, 2-fluoroethyl, pentafluoroethyl; most preferably, $R^{14}$, for each occurrence, is independently selected from hydrogen, fluorine, cyano, methyl, isopropyl.

**51.** The compound or a pharmaceutically acceptable salt thereof of any one of the preceding claims, wherein $R^{14}$ , for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{14}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

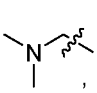, 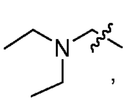, 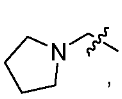, 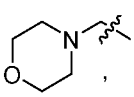, 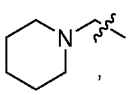, 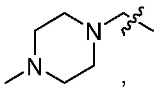, 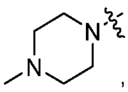, 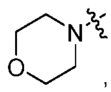,

trifluoromethyl, monofluoromethyl, difluoromethyl, 2-fluoroethyl, pentafluoroethyl; more preferably, $R^{14}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

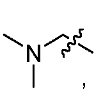, 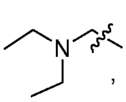, 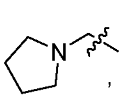, 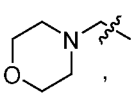, 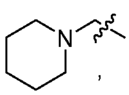, 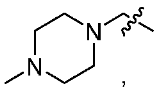, 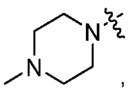, 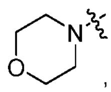,

trifluoromethyl, monofluoromethyl, difluoromethyl, 2-fluoroethyl, pentafluoroethyl; most preferably, $R^{14'}$, for each occurrence, is independently selected from hydrogen, fluorine, cyano, methyl, isopropyl.

**52.** The compound or a pharmaceutically acceptable salt thereof of any one of the preceding claims, wherein $R^{14''}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl,

and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{14}$ , for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

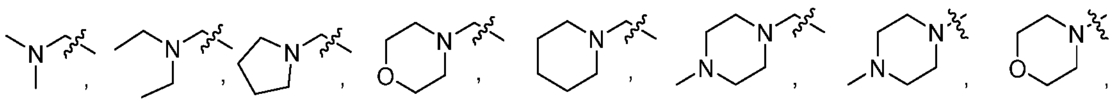
, , , , , , , ,

trifluoromethyl, monofluoromethyl, difluoromethyl, 2-fluoroethyl, pentafluoroethyl; more preferably, $R^{14''}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

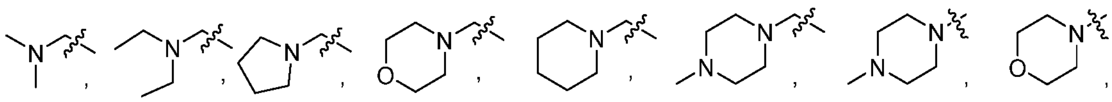
, , , , , , , ,

trifluoromethyl, monofluoromethyl, difluoromethyl, 2-fluoroethyl, pentafluoroethyl; most preferably, $R^{14''}$, for each occurrence, is independently selected from hydrogen, fluorine, cyano, methyl, isopropyl.

**53.** The compound or a pharmaceutically acceptable salt thereof of any one of the preceding claims, wherein $R^{14'''}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{14'''}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

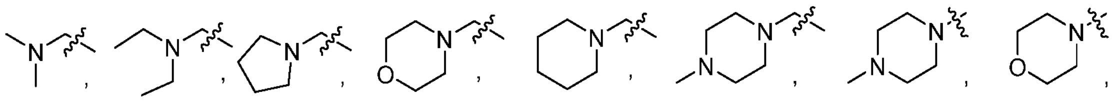
, , , , , , , ,

trifluoromethyl, monofluoromethyl, difluoromethyl, 2-fluoroethyl, pentafluoroethyl; more preferably, $R^{14'''}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

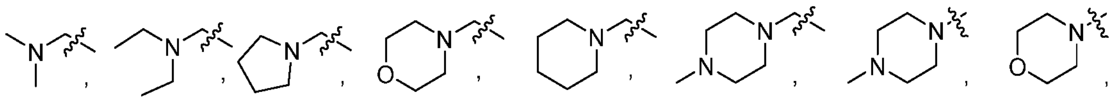
, , , , , , , ,

trifluoromethyl, monofluoromethyl, difluoromethyl, 2-fluoroethyl, pentafluoroethyl; most preferably, $R^{14'''}$, for each occurrence, is independently selected from hydrogen, fluorine, cyano, methyl, isopropyl.

**54.** The compound or a pharmaceutically acceptable salt thereof of any one of the preceding claims, wherein $R^{15}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{15}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

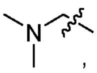, 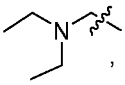, 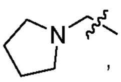, 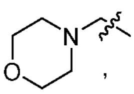, 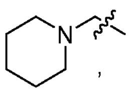, 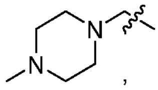, 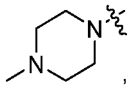, 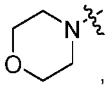,

trifluoromethyl, monofluoromethyl, difluoromethyl, 2-fluoroethyl, pentafluoroethyl; more preferably, $R^{15}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

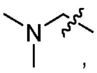, 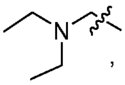, 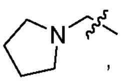, 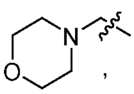, 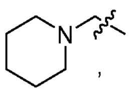, 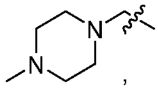, 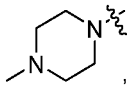, 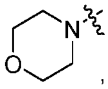,

trifluoromethyl, monofluoromethyl, difluoromethyl, 2-fluoroethyl, pentafluoroethyl; most preferably, $R^{15}$, for each occurrence, is independently selected from hydrogen, fluorine, cyano, methyl, isopropyl.

**55.** The compound or a pharmaceutically acceptable salt thereof of any one of the preceding claims, wherein $R^{16}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{16}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

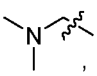, 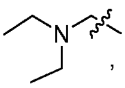, 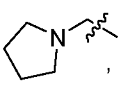, 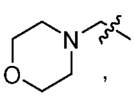, 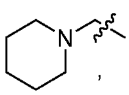, 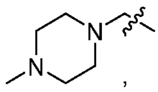, 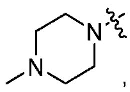, 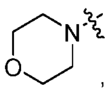,

trifluoromethyl, monofluoromethyl, difluoromethyl, 2-fluoroethyl, pentafluoroethyl; more preferably, $R^{16}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

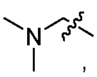, 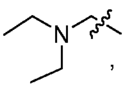, 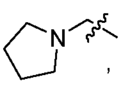, 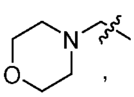, 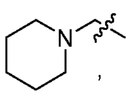, 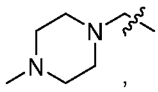, 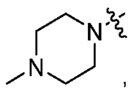, 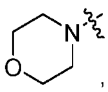,

trifluoromethyl, monofluoromethyl, difluoromethyl, 2-fluoroethyl, pentafluoroethyl; most preferably, $R^{16}$, for each occurrence, is independently selected from hydrogen, fluorine, cyano, methyl, isopropyl.

**56.** The compound or a pharmaceutically acceptable salt thereof of any one of the preceding claims, wherein $R^{17}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{17}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

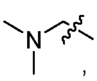, 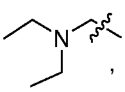, 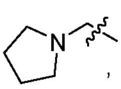, 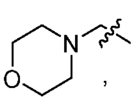, 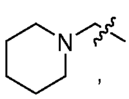, 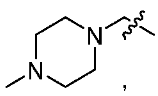, 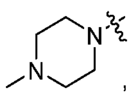, 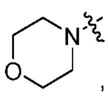,

trifluoromethyl, monofluoromethyl, difluoromethyl, 2-fluoroethyl, pentafluoroethyl; more preferably, $R^{17}$, for each

occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

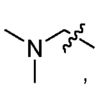, 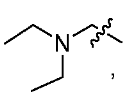, 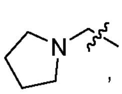, 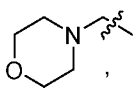, 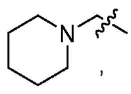, 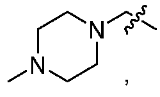, 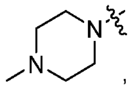, 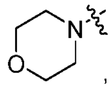,

trifluoromethyl, monofluoromethyl, difluoromethyl, 2-fluoroethyl, pentafluoroethyl; most preferably, $R^{17}$, for each occurrence, is independently selected from hydrogen, fluorine, cyano, methyl, isopropyl.

**57.** The compound or a pharmaceutically acceptable salt thereof of any one of the preceding claims, wherein $R^{18}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{18}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

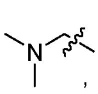, 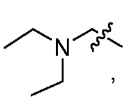, 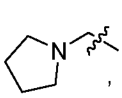, 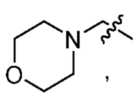, 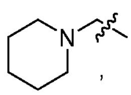, 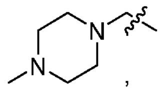, 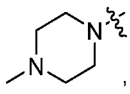, 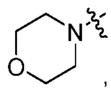,

trifluoromethyl, monofluoromethyl, difluoromethyl, 2-fluoroethyl, pentafluoroethyl; more preferably, $R^{18}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

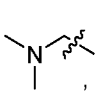, 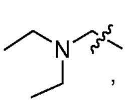, 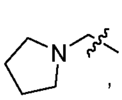, 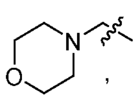, 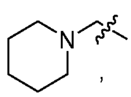, 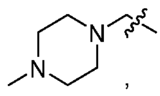, 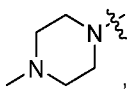, 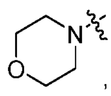,

trifluoromethyl, monofluoromethyl, difluoromethyl, 2-fluoroethyl, pentafluoroethyl; most preferably, $R^{18}$, for each occurrence, is independently selected from hydrogen, fluorine, cyano, methyl, isopropyl.

**58.** The compound or a pharmaceutically acceptable salt thereof of any one of the preceding claims, wherein $R^{21}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{21}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

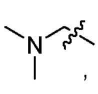, 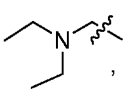, 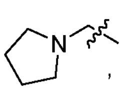, 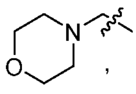, 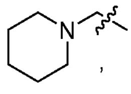, 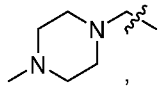, 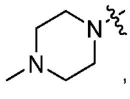, 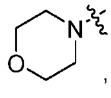,

trifluoromethyl, monofluoromethyl, difluoromethyl, 2-fluoroethyl, pentafluoroethyl; more preferably, $R^{21}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

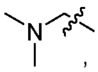, 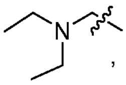, 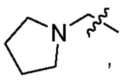, 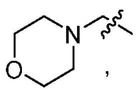, 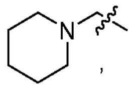, 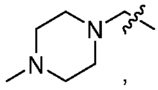, 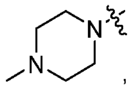, 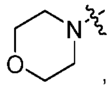,

trifluoromethyl, monofluoromethyl, difluoromethyl, 2-fluoroethyl, pentafluoroethyl; most preferably, $R^{21}$, for each occurrence, is independently selected from hydrogen, fluorine, cyano, methyl, isopropyl.

**59.** The compound or a pharmaceutically acceptable salt thereof of any one of the preceding claims, wherein $R^{22}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{22}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

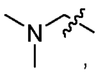, 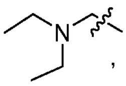, 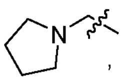, 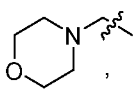, 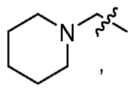, 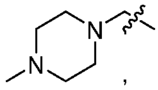, 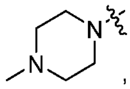, 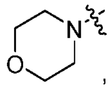,

trifluoromethyl, monofluoromethyl, difluoromethyl, 2-fluoroethyl, pentafluoroethyl; more preferably, $R^{22}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

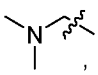, 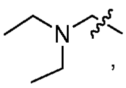, 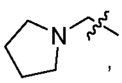, 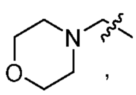, 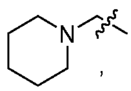, 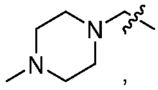, 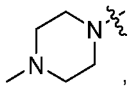, 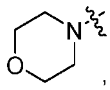,

trifluoromethyl, monofluoromethyl, difluoromethyl, 2-fluoroethyl, pentafluoroethyl; most preferably, $R^{22}$, for each occurrence, is independently selected from hydrogen, fluorine, cyano, methyl, isopropyl.

**60.** The compound or a pharmaceutically acceptable salt thereof of any one of the preceding claims, wherein $R^{23}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{23}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

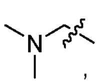, 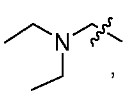, 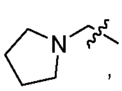, 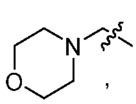, 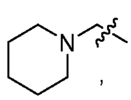, 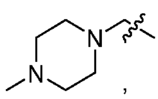, 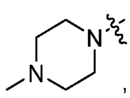, 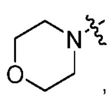,

trifluoromethyl, monofluoromethyl, difluoromethyl, 2-fluoroethyl, pentafluoroethyl; more preferably, $R^{23}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

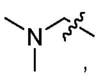, 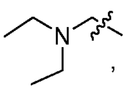, 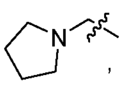, 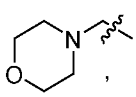, 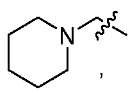, 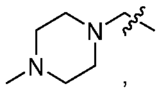, 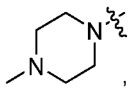, 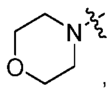,

trifluoromethyl, monofluoromethyl, difluoromethyl, 2-fluoroethyl, pentafluoroethyl; most preferably, $R^{23}$, for each

occurrence, is independently selected from hydrogen, fluorine, cyano, methyl, isopropyl.

**61.** The compound or a pharmaceutically acceptable salt thereof of any one of the preceding claims, wherein $R^{24}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{24}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

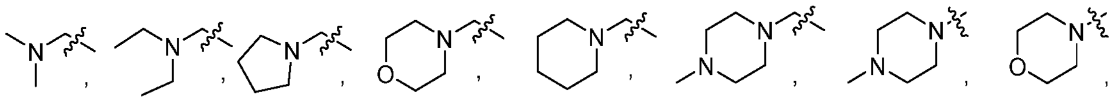

trifluoromethyl, monofluoromethyl, difluoromethyl, 2-fluoroethyl, pentafluoroethyl; more preferably, $R^{24}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

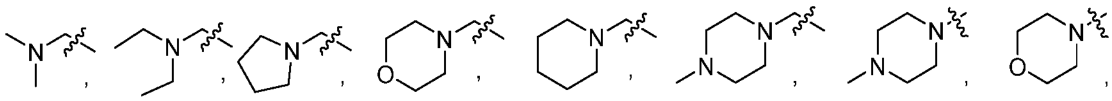

trifluoromethyl, monofluoromethyl, difluoromethyl, 2-fluoroethyl, pentafluoroethyl; most preferably, $R^{24}$, for each occurrence, is independently selected from hydrogen, fluorine, cyano, methyl, isopropyl.

**62.** The compound or a pharmaceutically acceptable salt thereof of any one of the preceding claims, wherein $R^{25}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{25}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

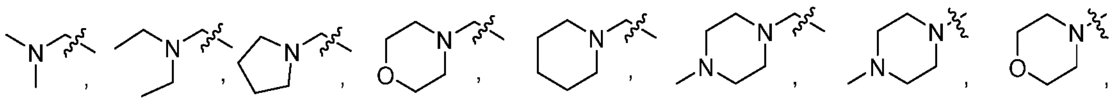

trifluoromethyl, monofluoromethyl, difluoromethyl, 2-fluoroethyl, pentafluoroethyl; more preferably, $R^{25}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

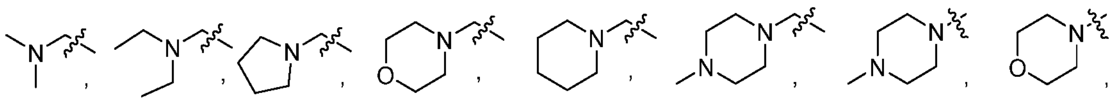

trifluoromethyl, monofluoromethyl, difluoromethyl, 2-fluoroethyl, pentafluoroethyl; most preferably, $R^{25}$, for each occurrence, is independently selected from hydrogen, fluorine, cyano, methyl, isopropyl.

**63.** The compound or a pharmaceutically acceptable salt thereof of any one of the preceding claims, wherein $R^{26}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl,

and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{26}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

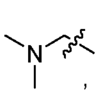 , 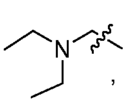 , 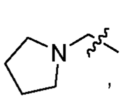 , 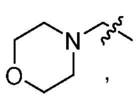 , 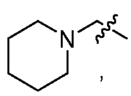 , 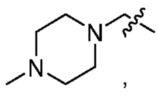 , 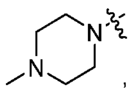 , 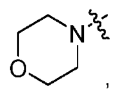 ,

trifluoromethyl, monofluoromethyl, difluoromethyl, 2-fluoroethyl, pentafluoroethyl; more preferably, $R^{26}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

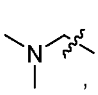 , 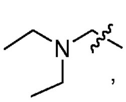 , 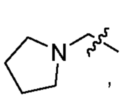 , 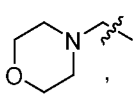 , 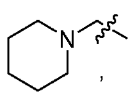 , 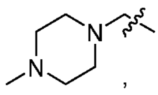 , 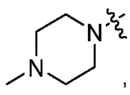 , 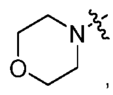 ,

trifluoromethyl, monofluoromethyl, difluoromethyl, 2-fluoroethyl, pentafluoroethyl; most preferably, $R^{26}$, for each occurrence, is independently selected from hydrogen, fluorine, cyano, methyl, isopropyl.

**64.** The compound or a pharmaceutically acceptable salt thereof of any one of the preceding claims, wherein $R^{27}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{27}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

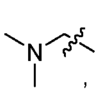 , 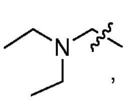 , 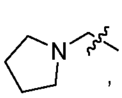 , 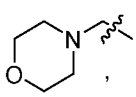 , 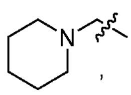 , 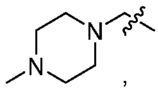 , 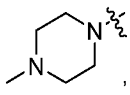 , 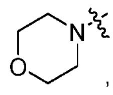 ,

trifluoromethyl, monofluoromethyl, difluoromethyl, 2-fluoroethyl, pentafluoroethyl; more preferably, $R^{27}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

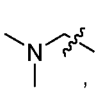 , 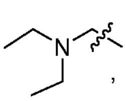 , 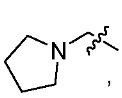 , 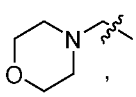 , 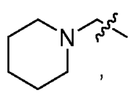 , 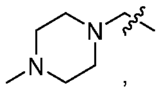 , 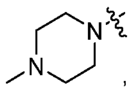 , 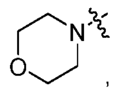 ,

trifluoromethyl, monofluoromethyl, difluoromethyl, 2-fluoroethyl, pentafluoroethyl; most preferably, $R^{27}$, for each occurrence, is independently selected from hydrogen, fluorine, cyano, methyl, isopropyl.

**65.** The compound or a pharmaceutically acceptable salt thereof of any one of the preceding claims, wherein $R^{28}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{28}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

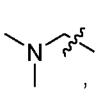, 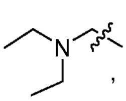, 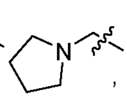, 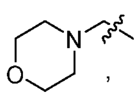, 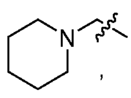, 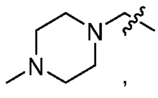, 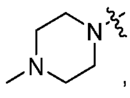, 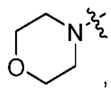,

trifluoromethyl, monofluoromethyl, difluoromethyl, 2-fluoroethyl, pentafluoroethyl; more preferably, $R^{28}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

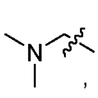, 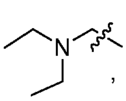, 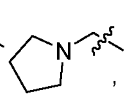, 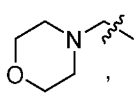, 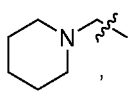, 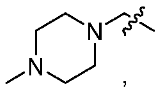, 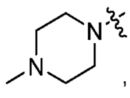, 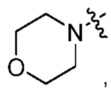,

trifluoromethyl, monofluoromethyl, difluoromethyl, 2-fluoroethyl, pentafluoroethyl; most preferably, $R^{28}$, for each occurrence, is independently selected from hydrogen, fluorine, cyano, methyl, isopropyl.

**66.** The compound or a pharmaceutically acceptable salt thereof of any one of the preceding claims, wherein $R^{31}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{31}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

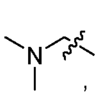, 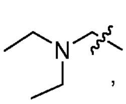, 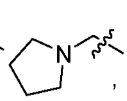, 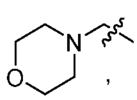, 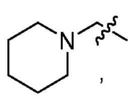, 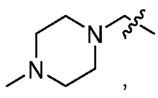, 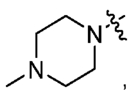, 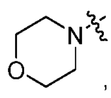,

trifluoromethyl, monofluoromethyl, difluoromethyl, 2-fluoroethyl, pentafluoroethyl; more preferably, $R^{31}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

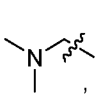, 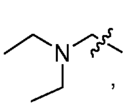, 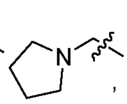, 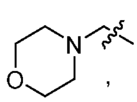, 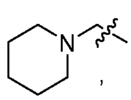, 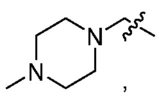, 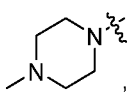, 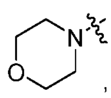,

trifluoromethyl, monofluoromethyl, difluoromethyl, 2-fluoroethyl, pentafluoroethyl; most preferably, $R^{31}$, for each occurrence, is independently selected from hydrogen, fluorine, cyano, methyl, isopropyl.

**67.** The compound or a pharmaceutically acceptable salt thereof of any one of the preceding claims, wherein $R^{32}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{32}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

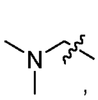, 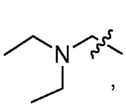, 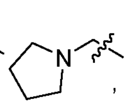, 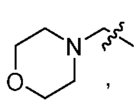, 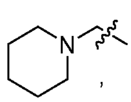, 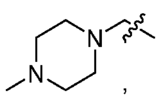, 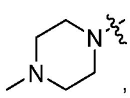, 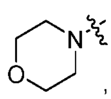,

trifluoromethyl, monofluoromethyl, difluoromethyl, 2-fluoroethyl, pentafluoroethyl; more preferably, $R^{32}$, for each

occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

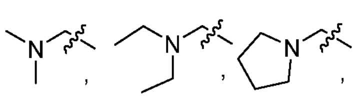 , , , 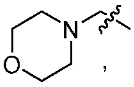 , 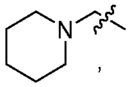 , 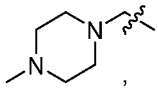 , 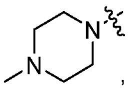 , 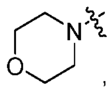 ,

trifluoromethyl, monofluoromethyl, difluoromethyl, 2-fluoroethyl, pentafluoroethyl; most preferably, $R^{32}$, for each occurrence, is independently selected from hydrogen, fluorine, cyano, methyl, isopropyl.

**68.** The compound or a pharmaceutically acceptable salt thereof of any one of the preceding claims, wherein $R^{33}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{33}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

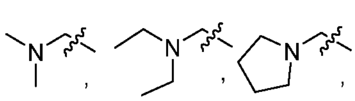 , , , 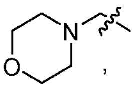 , 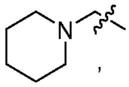 , 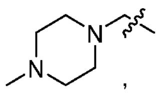 , 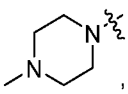 , 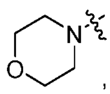 ,

trifluoromethyl, monofluoromethyl, difluoromethyl, 2-fluoroethyl, pentafluoroethyl; more preferably, $R^{33}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

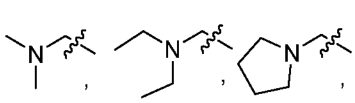 , , , 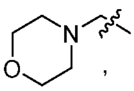 , 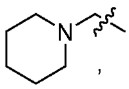 , 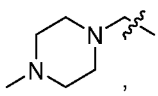 , 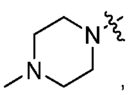 , 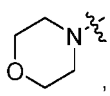 ,

trifluoromethyl, monofluoromethyl, difluoromethyl, 2-fluoroethyl, pentafluoroethyl; most preferably, $R^{33}$, for each occurrence, is independently selected from hydrogen, fluorine, cyano, methyl, isopropyl.

**69.** The compound or a pharmaceutically acceptable salt thereof of any one of the preceding claims, wherein $R^{34}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{34}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

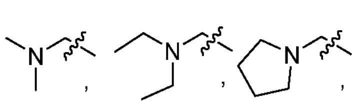 , , , 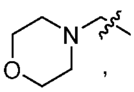 , 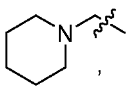 , 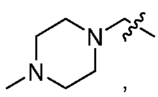 , 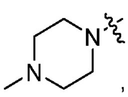 , 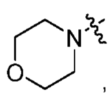 ,

trifluoromethyl, monofluoromethyl, difluoromethyl, 2-fluoroethyl, pentafluoroethyl; more preferably, $R^{34}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

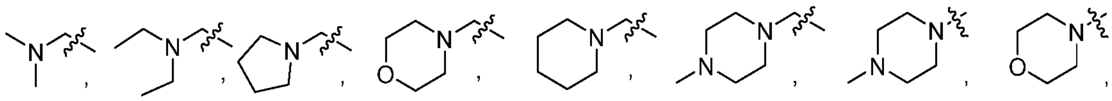
, , , , , , , ,

trifluoromethyl, monofluoromethyl, difluoromethyl, 2-fluoroethyl, pentafluoroethyl; most preferably, $R^{34}$, for each occurrence, is independently selected from hydrogen, fluorine, cyano, methyl, isopropyl.

**70.** The compound or a pharmaceutically acceptable salt thereof of any one of the preceding claims, wherein $R^{35}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{35}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

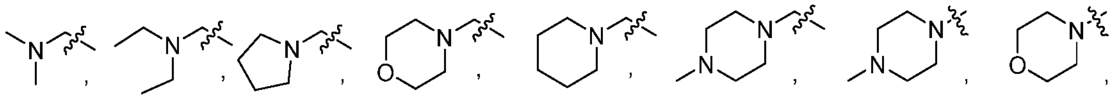
, , , , , , , ,

trifluoromethyl, monofluoromethyl, difluoromethyl, 2-fluoroethyl, pentafluoroethyl; more preferably, $R^{35}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

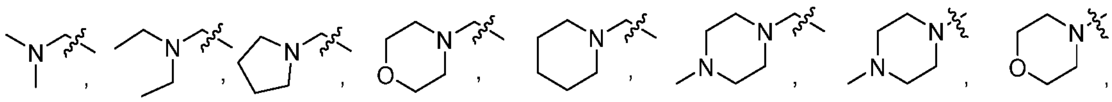
, , , , , , , ,

trifluoromethyl, monofluoromethyl, difluoromethyl, 2-fluoroethyl, pentafluoroethyl; most preferably, $R^{35}$, for each occurrence, is independently selected from hydrogen, fluorine, cyano, methyl, isopropyl.

**71.** The compound or a pharmaceutically acceptable salt thereof of any one of the preceding claims, wherein $R^{36}$, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, $R^{36}$, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

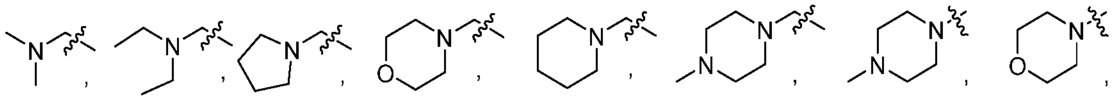
, , , , , , , ,

trifluoromethyl, monofluoromethyl, difluoromethyl, 2-fluoroethyl, pentafluoroethyl; more preferably, $R^{36}$, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

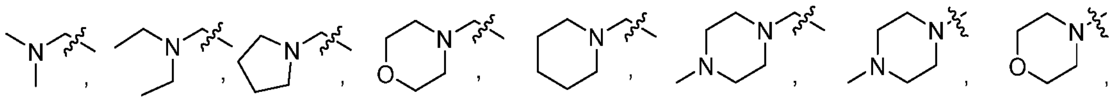
, , , , , , , ,

trifluoromethyl, monofluoromethyl, difluoromethyl, 2-fluoroethyl, pentafluoroethyl; most preferably, $R^{36}$, for each

occurrence, is independently selected from hydrogen, fluorine, cyano, methyl, isopropyl.

**72.** The compound or a pharmaceutically acceptable salt thereof of any one of the preceding claims, wherein: P, for each occurrence, is independently selected from absence, hydrogen, fluorine, chlorine, hydroxyl, nitro, cyano, amino, mercapto, -COOH, $C_1$-$C_6$ alkyl, $C_1$-$C_6$ alkoxy, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3 or 4 halogens, hydroxyl, nitro, cyano, amino, mercapto, -COOH, or $C_1$-$C_6$ alkyl; preferably, P, for each occurrence, is independently selected from absence, hydrogen, fluorine, chlorine, cyano, amino, mercapto, -COOH, methyl, ethyl, n-propyl, isopropyl, methoxy, ethoxy, n-propoxy, isopropoxy,

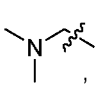, 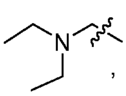, 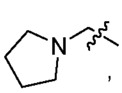, 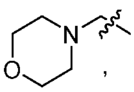, 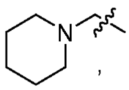, 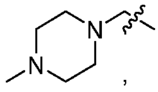, 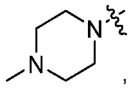, 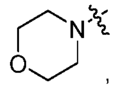,

trifluoromethyl, monofluoromethyl, difluoromethyl, 2-fluoroethyl, pentafluoroethyl, fluorophenylacetonitrile; more preferably, P, for each occurrence, is independently selected from hydrogen, fluorine, chlorine, cyano, amino, mercapto, -COOH, methyl, ethyl, n-propyl, isopropyl, methoxy, ethoxy, n-propoxy, isopropoxy,

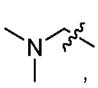, 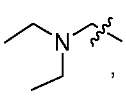, 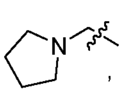, 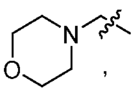, 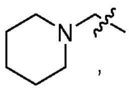, 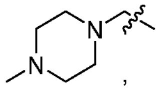, 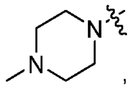, 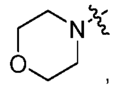,

trifluoromethyl, monofluoromethyl, difluoromethyl, 2-fluoroethyl, pentafluoroethyl, fluorophenylacetonitrile; most preferably, P, for each occurrence, is independently selected from hydrogen, fluorine, cyano, methyl, isopropyl, tert-butyl, trifluoromethyl, morpholinyl,

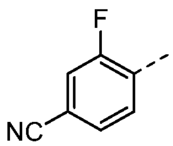 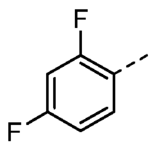 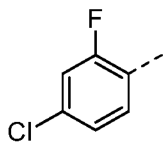 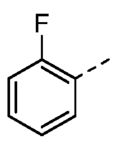.

**73.** The compound or a pharmaceutically acceptable salt thereof of any one of the preceding claims, wherein: T is N.

**74.** The compound or a pharmaceutically acceptable salt thereof of any one of the preceding claims, wherein: $R^2$ is hydrogen, fluorine, chlorine, bromine, cyano, trifluoromethyl, methoxy, ethoxy or methyl.

**75.** The compound or a pharmaceutically acceptable salt thereof of any one of the preceding claims, wherein: $X^8$ is C and $R^{18}$ is hydrogen, fluorine, chlorine, cyano, or methyl.

**76.** The compound or a pharmaceutically acceptable salt thereof of any one of the preceding claims, wherein: $-(Q)_n-$ is $-(CH_2)-$ or $-(CH_2)_2-$.

**77.** The compound or a pharmaceutically acceptable salt thereof of any one of the preceding claims, wherein: $X^5$ is O.

**78.** The compound or a pharmaceutically acceptable salt thereof of any one of the preceding claims, wherein: $X^1$ is N, CH, CF, $C$-$CH_3$.

**79.** The compound or a pharmaceutically acceptable salt thereof of any one of the preceding claims, wherein: $X^2$ is N, CH, CF, $C$-$CH_3$.

**80.** The compound or a pharmaceutically acceptable salt thereof of any one of the preceding claims, wherein: $X^3$ is N, CH, CF, $C$-$CH_3$.

**81.** The compound or a pharmaceutically acceptable salt thereof of any one of the preceding claims, wherein: $X^4$ is N,

CH, CF, C-$CH_3$.

**82.** The compound or a pharmaceutically acceptable salt thereof of any one of the preceding claims, wherein: $X^6$ is N, CH, CF, C-$CH_3$.

**83.** The compound or a pharmaceutically acceptable salt thereof of any one of the preceding claims, wherein: $X^7$ is N, CH, CF, C-$CH_3$.

**84.** The compound or a pharmaceutically acceptable salt thereof of any one of the preceding claims, wherein: $X^9$ is N, CH, CF, C-$CH_3$.

**85.** The compound or a pharmaceutically acceptable salt thereof of any one of the preceding claims, wherein: g is 1 or 2.

**86.** The compound or a pharmaceutically acceptable salt thereof of any one of the preceding claims, wherein: h is 1 or 2.

**87.** The compound or a pharmaceutically acceptable salt thereof of any one of the preceding claims, wherein: J is $CH_2$.

**88.** The compound or a pharmaceutically acceptable salt thereof of any one of the preceding claims, wherein: K is $CH_2$.

**89.** The compound or a pharmaceutically acceptable salt thereof of any one of the preceding claims, wherein: P is hydrogen, fluorine, cyano, methyl, isopropyl, tert-butyl, trifluoromethyl, morpholinyl,

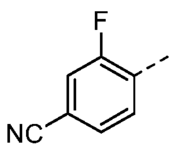 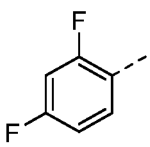 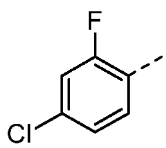 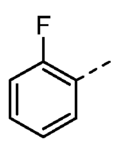 .

**90.** The compound or a pharmaceutically acceptable salt thereof of any one of the preceding claims, wherein: U is C, N or O.

**91.** The compound or a pharmaceutically acceptable salt thereof of any one of the preceding claims, wherein: Y is C, N or O.

**92.** The compound or a pharmaceutically acceptable salt thereof of any one of the preceding claims, wherein:

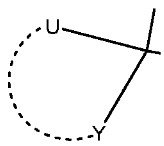

is a 5, 6 or 7-membered monocyclic ring.

**93.** The compound or a pharmaceutically acceptable salt thereof of any one of the preceding claims, wherein:

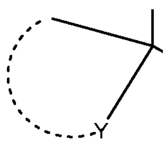

is a 5, 6 or 7-membered monocyclic ring.

**94.** The compound or a pharmaceutically acceptable salt thereof of any one of the preceding claims, wherein the compound is the following compound or a pharmaceutically acceptable salt thereof:

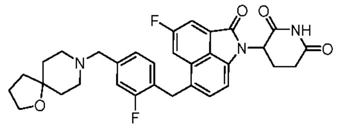
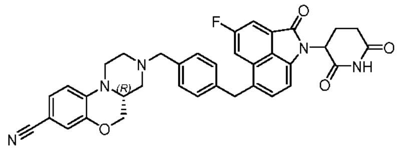
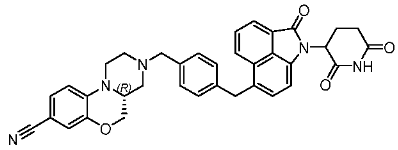
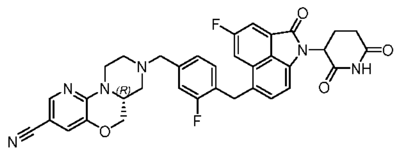
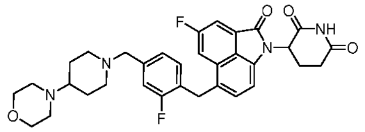
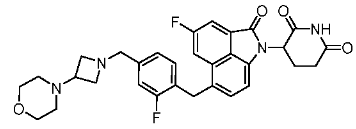
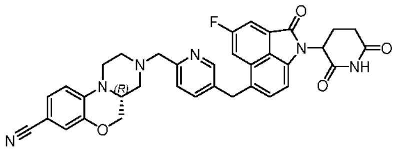
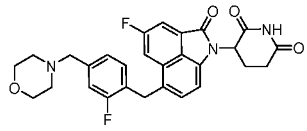
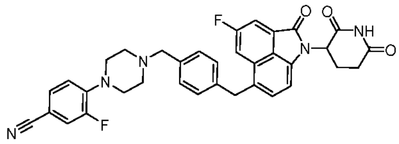
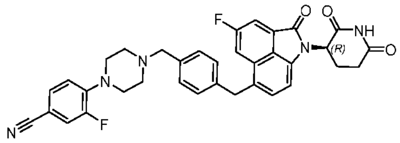
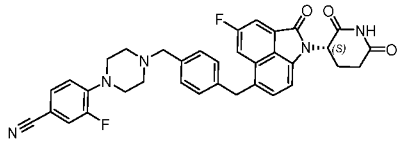
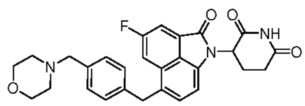
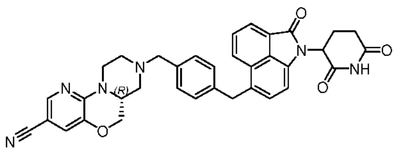
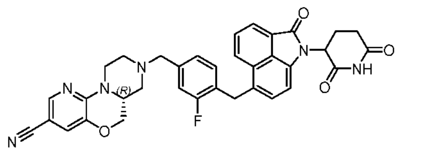
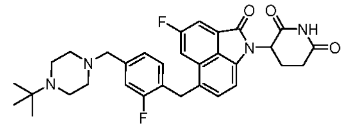
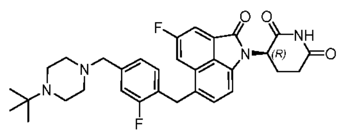
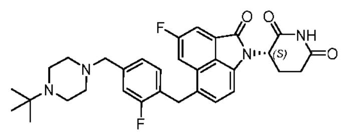
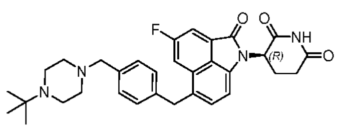
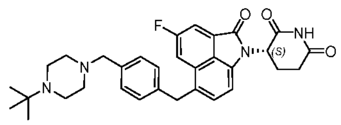
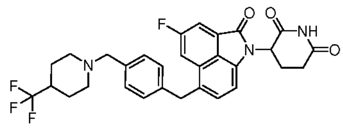
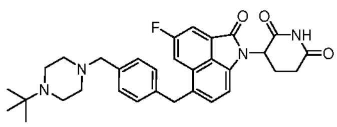
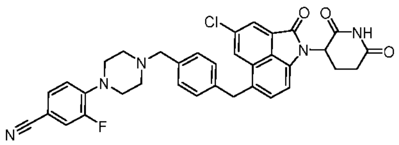
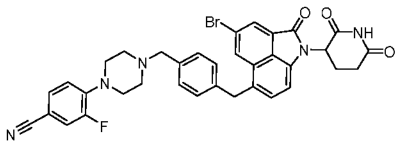
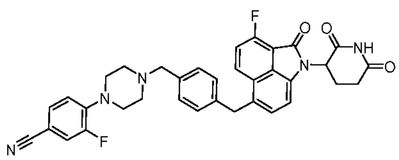
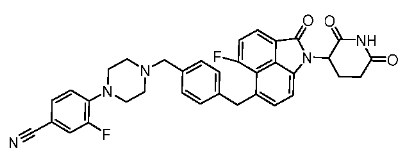

**95.** The compound or a pharmaceutically acceptable salt thereof of any one of the preceding claims, wherein the compound is the following compound or a pharmaceutically acceptable salt thereof:

**96.** A pharmaceutical composition comprising a compound or a pharmaceutically acceptable salt thereof of any one of

claims 1 to 94, and a pharmaceutically acceptable carrier.

**97.** The pharmaceutical composition of claim 96, wherein the pharmaceutical composition is a tablet, a capsule, a granule, a syrup, a suspension, a solution, a dispersion, a slow release preparation for oral or non-oral administration, an intravenous injection preparation, a subcutaneous injection preparation, an inhalation preparation, a transdermal preparation, a rectal or vaginal suppository.

**98.** Use of the compound or a pharmaceutically acceptable salt thereof of any one of claims 1 to 94 in the manufacture of a medicament for the treatment of proliferative disorders.

**99.** A compound or a pharmaceutically acceptable salt thereof of any one of claims 1 to 94, for use in the treatment of proliferative disorders.

**100.** A method of treating proliferative disorders in a subject in need thereof, comprising administering to a subject in need thereof a therapeutically effective amount of the compound or a pharmaceutically acceptable salt thereof of any one of claims 1 to 94.

**101.** The use, the compound or a pharmaceutically acceptable salt thereof or the method of claims 98 to 100, wherein the proliferative disorders comprises breast cancer, colon cancer, brain cancer, prostate cancer, kidney cancer, pancreatic cancer, ovarian cancer, head and neck cancer, melanoma, colorectal cancer, gastric cancer, squamous cancer, small-cell lung cancer, non small-cell lung cancer, testicular cancer, Merkel cell carcinoma, glioblastoma, neuroblastoma, cancers of lymphatic organs and hematological malignancy including Leukemia (Acute lymphoblastic leukemia (ALL), Acute myelogenous leukemia (AML), Chronic lymphocytic leukemia (CLL), Chronic myelogenous leukemia (CML), Acute monocytic leukemia (AMOL), Hairy cell leukemia (HCL), T-cell prolymphocytic leukemia (T-PLL), Large granular lymphocytic leukemia, Adult T-cell leukemia), Lymphoma (small lymphocytic lymphoma (SLL), Hodgkin’s lymphomas (Nodular sclerosis, Mixed cellularity, Lymphocyte-rich, Lymphocyte depleted or not depleted, and Nodular lymphocyte-predominant Hodgkin lymphoma), Non-Hodgkin’s lymphomas (all subtypes), Chronic lymphocytic leukemia/Small lymphocytic lymphoma, B-cell prolymphocytic leukemia, Lymphoplasmacytic lymphoma (such as Waldenström macroglobulinemia), Splenic marginal zone lymphoma, Plasma cell neoplasms (Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma, Burkitt lymphoma/leukemia, T cell prolymphocytic leukemia, T cell large granular lymphocytic leukemia, Aggressive NK cell leukemia, Adult T cell leukemia/lymphoma, Extranodal NK/T cell lymphoma (nasal type), Enteropathy-type T cell lymphoma, Hepatosplenic T cell lymphoma, Blastic NK cell lymphoma, Mycosis fungoides / Sezary syndrome, Primary cutaneous CD30-positive T cell lymphoproliferative disorders, Primary cutaneous anaplastic large cell lymphoma, Lymphomatoid papulosis, Angioimmunoblastic T cell lymphoma, Peripheral T cell lymphoma (unspecified), Anaplastic large cell lymphoma), multiple myeloma (plasma cell myeloma or Kahler’s disease).

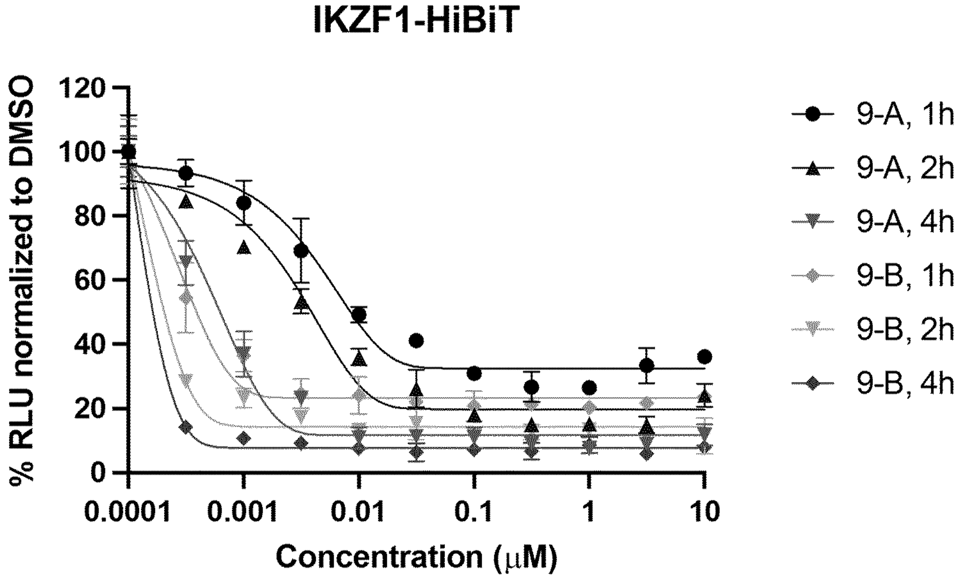


**Figure 1. IKZF1 protein degradation kinetics of compounds 9-A and 9-B**

**INTERNATIONAL SEARCH REPORT**

International application No.
**PCT/CN2022/114058**

**A. CLASSIFICATION OF SUBJECT MATTER**

C07D 401/14(2006.01)i; C07D 419/14(2006.01)i; C07D 209/92(2006.01)i; A61K 31/4035(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, STN(CAPLUS, REGISTRY, MARPAT): 异吲哚啉酮, 癌症, 肿瘤, IKZF, SALL4, cancer, tumour, tumor, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2020210630 A1 (C4 THERAPEUTICS INC.) 15 October 2020 (2020-10-15) description, pages 4-6, page 17, line 6 to page 18, line 12, page 31, line 7 to page 32, line 6, page 63, line 1 to page 65, line 5, pages 120-129, page 182, line 4 to page 187, line 6, page 221, paragraph 2, and claims 175-176 | 1-3, 21-41, 50-53, 58-74, 78-81, 85-101 |
| A | WO 2020210630 A1 (C4 THERAPEUTICS INC.) 15 October 2020 (2020-10-15) description, pages 4-6, page 17, line 6 to page 18, line 12, page 31, line 7 to page 32, line 6, page 63, line 1 to page 65, line 5, pages 120-129, page 182, line 4 to page 187, line 6, page 221, paragraph 2, and claims 175-176 | 4-20, 42-49, 54-57, 75-77, 82-84 |
| PX | WO 2022081927 A1 (C4 THERAPEUTICS INC.) 21 April 2022 (2022-04-21) description, pages 347-550, and claim 1 | 1-3, 21-41, 50-53, 58-74, 78-81, 85-101 |
| PX | WO 2022032132 A1 (C4 THERAPEUTICS INC.) 10 February 2022 (2022-02-10) claims 1-85 | 1-3, 21-41, 50-53, 58-74, 78-81, 85-101 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2022** | **10 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/114058**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **100-101**
because they relate to subject matter not required to be searched by this Authority, namely:

[1] The subject matter of claims 100-101 relates to a disease treatment method, belonging to an object that is not granted a patent; and in the search report, a search was conducted on the basis of a corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/114058**

| Patent document cited in search report | | | Publication date (day/month/year) | Patent family member(s) | | | Publication date (day/month/year) |
|---|---|---|---|---|---|---|---|
| WO | 2020210630 | A1 | 15 October 2020 | KR | 20210152515 | A | 15 December 2021 |
| | | | | IL | 287116 | A | 01 December 2021 |
| | | | | CA | 3130469 | A1 | 15 October 2020 |
| | | | | JP | 2022527216 | A | 31 May 2022 |
| | | | | US | 11407732 | B1 | 09 August 2022 |
| | | | | EP | 3953332 | A1 | 16 February 2022 |
| | | | | MA | 55628 | A | 16 February 2022 |
| | | | | SG | 11202109024 Y | A | 29 September 2021 |
| | | | | EA | 202192738 | A1 | 17 March 2022 |
| | | | | AU | 2020272978 | A1 | 16 September 2021 |
| | | | | CN | 113677664 | A | 19 November 2021 |
| WO | 2022081927 | A1 | 21 April 2022 | | None | | |
| WO | 2022032132 | A1 | 10 February 2022 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Science,* 2014, vol. 343, 301-305
- *Nature Chemical Biology,* 2018, vol. 14, 981-987
- **PAQUETTE, LEO A.** Principles of Modern Heterocyclic Chemistry. W. A. Benjamin, 1968
- The Chemistry of Heterocyclic Compounds, A series of Monographs. John Wiley & Sons, 1950
- *J. Am. Chem. Soc.,* 1960, vol. 82, 5566
- REMINGTON'S PHARMACEUTICAL SCIENCES. Mack Publishing Company, 1995